(19) Europäisches Patentamt
European Patent Office
Office européen des brevets

(11) EP 2 809 354 B1

(12) **EUROPEAN PATENT SPECIFICATION**

(45) Date of publication and mention of the grant of the patent:
**14.04.2021 Bulletin 2021/15**

(21) Application number: **13703328.8**

(22) Date of filing: **31.01.2013**

(51) Int Cl.:
*A61K 47/64* (2017.01)
*A61K 47/59* (2017.01)
*A61K 47/54* (2017.01)
*A61K 39/39* (2006.01)
*A61K 39/00* (2006.01)
*A61K 39/12* (2006.01)
*A61P 37/00* (2006.01)
*A61P 31/00* (2006.01)
*A61P 35/00* (2006.01)

(86) International application number:
**PCT/EP2013/000292**

(87) International publication number:
**WO 2013/113502 (08.08.2013 Gazette 2013/32)**

(54) **NEGATIVELY CHARGED NUCLEIC ACID COMPRISING COMPLEXES FOR IMMUNOSTIMULATION**

NEGATIV GELADENE NUKLEINSÄURE MIT KOMPLEXEN ZUR IMMUNSTIMULATION

COMPLEXES COMPRENANT DES ACIDES NUCLÉIQUES CHARGÉS NÉGATIVEMENT DESTINÉS À L'IMMUNO-STIMULATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.01.2012 PCT/EP2012/000418**

(43) Date of publication of application:
**10.12.2014 Bulletin 2014/50**

(60) Divisional application:
**20216421.6**

(73) Proprietor: **CureVac AG**
**72076 Tübingen (DE)**

(72) Inventor: **BAUMHOF, Patrick**
**72706 Tübingen (DE)**

(74) Representative: **Graf von Stosch, Andreas et al**
**Graf von Stosch**
**Patentanwaltsgesellschaft mbH**
**Prinzregentenstraße 22**
**80538 München (DE)**

(56) References cited:
**WO-A1-01/54720**
**WO-A1-2010/037539**
**WO-A1-2012/013326**
**WO-A2-03/000227**

- **ROZENFELD J H K ET AL: "Stable assemblies of cationic bilayer fragments and CpG oligonucleotide with enhanced immunoadjuvant activity in vivo", JOURNAL OF CONTROLLED RELEASE 20120610 ELSEVIER NLD, vol. 160, no. 2, 21 October 2011 (2011-10-21), pages 367-373, XP055063715, ISSN: 0168-3659**
- **S. Hamm ET AL: "Immunostimulatory RNA is a potent inducer of antigen-specific cytotoxic and humoral immune response in vivo", INTERNATIONAL IMMUNOLOGY., vol. 19, no. 3, 7 February 2007 (2007-02-07), pages 297-304, XP055296417, GB ISSN: 0953-8178, DOI: 10.1093/intimm/dxl146**
- **WEIDE BENJAMIN ET AL: "Direct injection of protamine-protected mRNA: results of a phase 1/2 vaccination trial in metastatic melanoma patients", JOURNAL OF IMMUNOTHERAPY, RAVEN PRESS, NEW YORK, NY, US, vol. 32, no. 5, 1 June 2009 (2009-06-01), pages 498-507, XP009154222, ISSN: 1053-8550, DOI: 10.1097/CJI.0B013E3181A00068**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 809 354 B1

SEQUENCE LISTING

<110> CureVac GmbH

<120> Negatively charged nucleic acid comprising complexes for immunostimulation

<130> Cu01P125WO1

<150> PCT/EP2012/000418
<151> 2012-01-31

<160> 108

<170> PatentIn version 3.5

<210> 1
<211> 20
<212> RNA
<213> artificial

<220>
<223> nucleic acid according to formula (II) (GlxmGn)

<400> 1
gguuuuuuuu uuuuuuuggg 20

<210> 2
<211> 20
<212> RNA
<213> artificial

<220>
<223> nucleic acid according to formula (II) (GlXmGn)

<400> 2
gggggguuuuu uuuuuggggg 20

<210> 3
<211> 40
<212> RNA
<213> artificial

<220>
<223> nucleic acid according to formula (II) (GlxmGn)

<400> 3
gggggguuuu uuuuuuuuuu uuuuuuuuuu uuuuuggggg 40

<210> 4
<211> 39
<212> RNA
<213> artificial

<220>
<223> nucleic acid according to formula (II) (GlxmGn)

<400> 4
gugugugugu guuuuuuuuu uuuuuuugug ugugugugu 39

<210> 5
<211> 39
<212> RNA
<213> artificial

<220>
<223> nucleic acid according to formula (II) (GlxmGn)

<400> 5
gguugguugg uuuuuuuuuu uuuuuuuggu ugguugguu 39

<210> 6
<211> 20
<212> RNA
<213> artificial

<220>
<223> nucleic acid according to formula (II) (GlXmGn)

<400> 6
gggggggggu uugggggggg 20

<210> 7
<211> 20
<212> RNA
<213> artificial

<220>
<223> nucleic acid according to formula (II) (GlxmGn)

<400> 7
ggggggggguu uugggggggg 20

<210> 8
<211> 20
<212> RNA
<213> artificial

<220>
<223> nucleic acid according to formula (II) (GlXmGn)

<400> 8
ggggggguuu uuuggggggg 20

<210> 9
<211> 20
<212> RNA
<213> artificial

<220>
<223> nucleic acid according to formula (II) (GlxmGn)

<400> 9
gggggggguuu uuuugggggg 20

<210> 10
<211> 20
<212> RNA
<213> artificial

<220>
<223> nucleic acid according to formula (II) (GlXmGn)

<400> 10
ggggggguuuu uuuuggggg 20

<210> 11
<211> 20
<212> RNA
<213> artificial

<220>
<223> nucleic acid according to formula (II) (GlXmGn)

<400> 11
ggggguuuu uuuuugggg 20

<210> 12
<211> 20
<212> RNA
<213> artificial

<220>
<223> nucleic acid according to formula (II) (GlxmGn)

<400> 12
gggggguuuu uuuuuugggg 20

<210> 13
<211> 20
<212> RNA
<213> artificial

<220>
<223> nucleic acid according to formula (II) (GlxmGn)

<400> 13
ggggguuuuu uuuuuugggg 20

<210> 14
<211> 20
<212> RNA

<213> artificial

<220>
<223> nucleic acid according to formula (II) (GlxmGn)

<400> 14
gggggguuuuu uuuuuuuggg 20

<210> 15
<211> 20
<212> RNA
<213> artificial

<220>
<223> nucleic acid according to formula (II) (GlxmGn)

<400> 15
gggguuuuuu uuuuuuuggg 20

<210> 16
<211> 20
<212> RNA
<213> artificial

<220>
<223> nucleic acid according to formula (II) (GlxmGn)

<400> 16
ggggguuuuuu uuuuuuuugg 20

<210> 17
<211> 20
<212> RNA
<213> artificial

<220>
<223> nucleic acid according to formula (II) (GlxmGn)

<400> 17
gguuuuuuuu uuuuuuuugg 20

<210> 18
<211> 20
<212> RNA
<213> artificial

<220>
<223> nucleic acid according to formula (II) (GlxmGn)

<400> 18
guuuuuuuuu uuuuuuuuug 20

<210> 19
<211> 22
<212> RNA
<213> artificial

<220>

<223> nucleic acid according to formula (II) (GlxmGn)

<400> 19
gggggggggg uuuggggggg gg 22

<210> 20
<211> 22
<212> RNA
<213> artificial

<220>
<223> nucleic acid according to formula (II) (GlxmGn)

<400> 20
gggggggggu uuuggggggg gg 22

<210> 21
<211> 22
<212> RNA
<213> artificial

<220>
<223> nucleic acid according to formula (II) (GlxmGn)

<400> 21
gggggggguu uuuugggggg gg 22

<210> 22
<211> 22
<212> RNA
<213> artificial

<220>
<223> nucleic acid according to formula (II) (GlxmGn)

<400> 22
gggggggguu uuuuuggggg gg 22

<210> 23
<211> 22
<212> RNA
<213> artificial

<220>
<223> nucleic acid according to formula (II) (GlXmGn)

<400> 23
ggggggguuu uuuuuggggg gg 22

<210> 24
<211> 22
<212> RNA
<213> artificial

<220>
<223> nucleic acid according to formula (II) (GlXmGn)

<400> 24

ggggggguuu uuuuuugggg gg 22

<210> 25
<211> 22
<212> RNA
<213> artificial

<220>
<223> nucleic acid according to formula (II) (GlXmGn)

<400> 25
ggggggguuu uuuuuuuggg gg 22

<210> 26
<211> 22
<212> RNA
<213> artificial

<220>
<223> nucleic acid according to formula (II) (GlXmGn)

<400> 26
gggggguuuu uuuuuuuggg gg 22

<210> 27
<211> 22
<212> RNA
<213> artificial

<220>
<223> nucleic acid according to formula (II) (GlxmGn)

<400> 27
gggggguuuu uuuuuuuugg gg 22

<210> 28
<211> 22
<212> RNA
<213> artificial

<220>
<223> nucleic acid according to formula (II) (GlxmGn)

<400> 28
ggggguuuuu uuuuuuuugg gg 22

<210> 29
<211> 22
<212> RNA
<213> artificial

<220>
<223> nucleic acid according to formula (II) (GlxmGn)

<400> 29
ggggguuuuu uuuuuuuuug gg 22

<210> 30

<211> 22
<212> RNA
<213> artificial

<220>
<223> nucleic acid according to formula (II) (GlxmGn)

<400> 30
ggguuuuuuu uuuuuuuuug gg 22

<210> 31
<211> 22
<212> RNA
<213> artificial

<220>
<223> nucleic acid according to formula (II) (GlxmGn)

<400> 31
gguuuuuuuu uuuuuuuuuu gg 22

<210> 32
<211> 24
<212> RNA
<213> artificial

<220>
<223> nucleic acid according to formula (II) (GlxmGn)

<400> 32
gggggggggg guuugggggg gggg 24

<210> 33
<211> 24
<212> RNA
<213> artificial

<220>
<223> nucleic acid according to formula (II) (GlxmGn)

<400> 33
gggggggggg uuuugggggg gggg 24

<210> 34
<211> 24
<212> RNA
<213> artificial

<220>
<223> nucleic acid according to formula (II) (GlxmGn)

<400> 34
gggggggggu uuuuuggggg gggg 24

<210> 35
<211> 24
<212> RNA
<213> artificial

<220>
<223> nucleic acid according to formula (II) (GlxmGn)

<400> 35
gggggggggu uuuuuugggg gggg 24

<210> 36
<211> 24
<212> RNA
<213> artificial

<220>
<223> artificial

<400> 36
ggggggggguu uuuuuugggg gggg 24

<210> 37
<211> 24
<212> RNA
<213> artificial

<220>
<223> nucleic acid according to formula (II) (GlXmGn)

<400> 37
gggggggguu uuuuuuuggg gggg 24

<210> 38
<211> 24
<212> RNA
<213> artificial

<220>
<223> nucleic acid according to formula (II) (GlxmGn)

<400> 38
ggggggggguu uuuuuuuugg gggg 24

<210> 39
<211> 24
<212> RNA
<213> artificial

<220>
<223> nucleic acid according to formula (II) (GlxmGn)

<400> 39
gggggggguuu uuuuuuuugg gggg 24

<210> 40
<211> 24
<212> RNA
<213> artificial

<220>
<223> nucleic acid according to formula (II) (GlxmGn)

<400> 40
ggggggguuu uuuuuuuuug gggg 24

<210> 41
<211> 24
<212> RNA
<213> artificial

<220>
<223> nucleic acid according to formula (II) (GlxmGn)

<400> 41
gggggguuuu uuuuuuuuug gggg 24

<210> 42
<211> 24
<212> RNA
<213> artificial

<220>
<223> nucleic acid according to formula (II) (GlxmGn)

<400> 42
gggggguuuu uuuuuuuuuu gggg 24

<210> 43
<211> 24
<212> RNA
<213> artificial

<220>
<223> nucleic acid according to formula (II) (GlxmGn)

<400> 43
gggguuuuuu uuuuuuuuuu gggg 24

<210> 44
<211> 24
<212> RNA
<213> artificial

<220>
<223> nucleic acid according to formula (II) (GlxmGn)

<400> 44
ggguuuuuuu uuuuuuuuuu uggg 24

<210> 45
<211> 32
<212> RNA
<213> artificial

<220>
<223> nucleic acid according to formula (II) (GlxmGn)

<400> 45
guuuuuuuuu uuuuuuuuuu uuuuuuuuuu ug 32

<210> 46
<211> 34
<212> RNA
<213> artificial

<220>
<223> nucleic acid according to formula (II) (GlXmGn)

<400> 46
gguuuuuuuu uuuuuuuuuu uuuuuuuuuu uugg 34

<210> 47
<211> 36
<212> RNA
<213> artificial

<220>
<223> nucleic acid according to formula (II) (GlXmGn)

<400> 47
ggguuuuuuu uuuuuuuuuu uuuuuuuuuu uuuggg 36

<210> 48
<211> 37
<212> RNA
<213> artificial

<220>
<223> nucleic acid according to formula (II) (GlXmGn)

<400> 48
gggguuuuuu uuuuuuuuuu uuuuuuuuuu uuuuggg 37

<210> 49
<211> 39
<212> RNA
<213> artificial

<220>
<223> nucleic acid according to formula (II) (GlxmGn)

<400> 49
ggggguuuuu uuuuuuuuuu uuuuuuuuuu uuuuugggg 39

<210> 50
<211> 41
<212> RNA
<213> artificial

<220>
<223> nucleic acid according to formula (II) (GlXmGn)

<400> 50
gggggguuuu uuuuuuuuuu uuuuuuuuuu uuuuuugggg g 41

<210> 51
<211> 43
<212> RNA

<213> artificial

<220>
<223> nucleic acid according to formula (II) (GlxmGn)

<400> 51
gggggggguuu uuuuuuuuuu uuuuuuuuuu uuuuuuuggg ggg 43

<210> 52
<211> 45
<212> RNA
<213> artificial

<220>
<223> nucleic acid according to formula (II) (GlxmGn)

<400> 52
ggggggggguu uuuuuuuuuu uuuuuuuuuu uuuuuuuugg ggggg 45

<210> 53
<211> 47
<212> RNA
<213> artificial

<220>
<223> nucleic acid according to formula (II) (GlxmGn)

<400> 53
gggggggggu uuuuuuuuuu uuuuuuuuuu uuuuuuuuug ggggggg 47

<210> 54
<211> 7
<212> RNA
<213> artificial

<220>
<223> nucleic acid according to formula (II) (GlXmGn)

<400> 54
gguuugg 7

<210> 55
<211> 8
<212> RNA
<213> artificial

<220>
<223> nucleic acid according to formula (II) (GlXmGn)

<400> 55
gguuuugg 8

<210> 56
<211> 9
<212> RNA
<213> artificial

<220>

<223> nucleic acid according to formula (II) (GlXmGn)

<400> 56
gguuuuugg 9

<210> 57
<211> 10
<212> RNA
<213> artificial

<220>
<223> nucleic acid according to formula (II) (GlXmGn)

<400> 57
gguuuuuugg 10

<210> 58
<211> 11
<212> RNA
<213> artificial

<220>
<223> nucleic acid according to formula (II) (GlXmGn)

<400> 58
gguuuuuuug g 11

<210> 59
<211> 12
<212> RNA
<213> artificial

<220>
<223> nucleic acid according to formula (II) (GlXmGn)

<400> 59
gguuuuuuuu gg 12

<210> 60
<211> 13
<212> RNA
<213> artificial

<220>
<223> nucleic acid according to formula (II) (GlxmGn)

<400> 60
gguuuuuuuu ugg 13

<210> 61
<211> 14
<212> RNA
<213> artificial

<220>
<223> nucleic acid according to formula (II) (GlxmGn)

<400> 61

gguuuuuuuu uugg 14

<210> 62
<211> 15
<212> RNA
<213> artificial

<220>
<223> nucleic acid according to formula (II) (GlxmGn)

<400> 62
gguuuuuuuu uuugg 15

<210> 63
<211> 16
<212> RNA
<213> artificial

<220>
<223> nucleic acid according to formula (II) (GlXmGn)

<400> 63
gguuuuuuuu uuuugg 16

<210> 64
<211> 17
<212> RNA
<213> artificial

<220>
<223> nucleic acid according to formula (II) (GlXmGn)

<400> 64
gguuuuuuuu uuuuugg 17

<210> 65
<211> 18
<212> RNA
<213> artificial

<220>
<223> nucleic acid according to formula (II) (GlXmGn)

<400> 65
gguuuuuuuu uuuuuugg 18

<210> 66
<211> 19
<212> RNA
<213> artificial

<220>
<223> nucleic acid according to formula (II) (GlxmGn)

<400> 66
gguuuuuuuu uuuuuuugg 19

<210> 67

<211> 9
<212> RNA
<213> artificial

<220>
<223> nucleic acid according to formula (II) (GlxmGn)

<400> 67
ggguuuggg 9

<210> 68
<211> 10
<212> RNA
<213> artificial

<220>
<223> nucleic acid according to formula (II) (GlXmGn)

<400> 68
ggguuuuggg 10

<210> 69
<211> 11
<212> RNA
<213> artificial

<220>
<223> nucleic acid according to formula (II) (GlxmGn)

<400> 69
ggguuuuugg g 11

<210> 70
<211> 12
<212> RNA
<213> artificial

<220>
<223> nucleic acid according to formula (II) (GlxmGn)

<400> 70
ggguuuuuug gg 12

<210> 71
<211> 13
<212> RNA
<213> artificial

<220>
<223> nucleic acid according to formula (II) (GlxmGn)

<400> 71
ggguuuuuuu ggg 13

<210> 72
<211> 14
<212> RNA
<213> artificial

<220>
<223> nucleic acid according to formula (II) (GlxmGn)

<400> 72
ggguuuuuuu uggg 14

<210> 73
<211> 15
<212> RNA
<213> artificial

<220>
<223> nucleic acid according to formula (II) (GlxmGn)

<400> 73
ggguuuuuuu uuggg 15

<210> 74
<211> 16
<212> RNA
<213> artificial

<220>
<223> nucleic acid according to formula (II) (GlxmGn)

<400> 74
ggguuuuuuu uuuggg 16

<210> 75
<211> 17
<212> RNA
<213> artificial

<220>
<223> nucleic acid according to formula (II) (GlxmGn)

<400> 75
ggguuuuuuu uuuuggg 17

<210> 76
<211> 18
<212> RNA
<213> artificial

<220>
<223> nucleic acid according to formula (II) (GlxmGn)

<400> 76
ggguuuuuuu uuuuuggg 18

<210> 77
<211> 19
<212> RNA
<213> artificial

<220>
<223> nucleic acid according to formula (II) (GlxmGn)

<400> 77
ggguuuuuuu uuuuuuggg 19

<210> 78
<211> 57
<212> RNA
<213> artificial

<220>
<223> nucleic acid according to formula (II) (GlxmGn)

<400> 78
ggguuuuuuu uuuuuuuugg guuuuuuuuu uuuuuugggu uuuuuuuuuu uuuuggg 57

<210> 79
<211> 42
<212> RNA
<213> artificial

<220>
<223> nucleic acid according to formula (II) (GlxmGn)

<400> 79
ggguuuuuuu uuuuuuuugg ggggguuuuuu uuuuuuuuug gg 42

<210> 80
<211> 51
<212> RNA
<213> artificial

<220>
<223> nucleic acid according to formula (II) (GlxmGn)

<400> 80
ggguuugggu uuggguuugg guuuggguuu ggguuugggu uuggguuugg g 51

<210> 81
<211> 20
<212> RNA
<213> artificial

<220>
<223> nucleic acid according to formula (II) (GlxmGn)

<400> 81
gguuuuuuuu uuuuuuuggg 20

<210> 82
<211> 57
<212> RNA
<213> artificial

<220>
<223> nucleic acid according to formula (III) (Clxmcn)

<400> 82
cccuuuuuuu uuuuuuuucc cuuuuuuuuu uuuuuucccu uuuuuuuuuu uuuuccc 57

<210> 83
<211> 51
<212> RNA
<213> artificial

<220>
<223> nucleic acid according to formula (III) (Clxmcn)

<400> 83
cccuuucccu uucccuuucc cuuucccuuu cccuuucccu uucccuuucc c 51

<210> 84
<211> 42
<212> RNA
<213> artificial

<220>
<223> nucleic acid according to formula (III) (ClxmCn)

<400> 84
cccuuuuuuu uuuuuuuucc ccccuuuuuu uuuuuuuuuc cc 42

<210> 85
<211> 60
<212> RNA
<213> artificial

<220>
<223> nucleic acid molecule according to formula (IV) (NuGlxmGnNv)a

<400> 85
uagcgaagcu cuuggaccua gguuuuuuuu uuuuuuuggg ugcguuccua gaaguacacg 60

<210> 86
<211> 120
<212> RNA
<213> artificial

<220>
<223> nucleic acid molecule according to formula (IV) (NuGlxmGnNv)a

<400> 86

uagcgaagcu cuuggaccua gguuuuuuuu uuuuuuuggg ugcguuccua gaaguacacg 60
aucgcuucga gaaccuggau ccaaaaaaaa aaaaaaaccc acgcaaggau cuucaugugc 120

<210> 87
<211> 229
<212> RNA
<213> artificial

<220>
<223> nucleic acid molecule according to formula (IV) (NuGlxmGnNv)a

<400> 87

```
gggagaaagc ucaagcuugg agcaaugccc gcacauugag gaaaccgagu ugcauaucuc    60
agaguauugg cccccgugua gguuauucuu gacagacagu ggagcuuauu cacucccagg   120
auccgagucg cauacuacgg uacuggugac agaccuaggu cgucaguuga ccaguccgcc   180
acuagacgug aguccgucaa agcaguuaga uguuacacuc uauuagauc               229
```

<210> 88
<211> 547
<212> RNA
<213> artificial

<220>
<223> nucleic acid molecule according to formula (IV) (NuGlxmGnNv)a

<400> 88

```
gggagaaagc ucaagcuugg agcaaugccc gcacauugag gaaaccgagu ugcauaucuc    60
agaguauugg cccccgugua gguuauucuu gacagacagu ggagcuuauu cacucccagg   120
auccgagucg cauacuacgg uacuggugac agaccuaggu cgucaguuga ccaguccgcc   180
acuagacgug aguccgucaa agcaguuaga uguuacacuc uauuagaucu cggauuacag   240
cuggaaggag caggaguagu guucuugcuc uaaguaccga gugugcccaa uacccgauca   300
gcuuauuaac gaacggcucc uccucuuaga cugcagcgua agugcggaau cuggggauca   360
aauuacugac ugccuggauu acccucggac auauaaccuu guagcacgcu guugcuguau   420
aggugaccaa cgcccacucg aguagaccag cucucuuagu ccggacaaug auaggaggcg   480
cggucaaucu acuucuggcu aguuaagaau aggcugcacc gaccucuaua aguagcgugu   540
ccucuag                                                             547
```

<210> 89
<211> 1083
<212> RNA
<213> artificial

<220>
<223> nucleic acid molecule according to formula (IV) (NuGlxmGnNv)a

<400> 89

```
gggagaaagc ucaagcuugg agcaaugccc gcacauugag gaaaccgagu ugcauaucuc      60
agaguauugg cccccgugua gguuauucuu gacagacagu ggagcuuauu cacucccagg     120
auccgagucg cauacuacgg uacuggugac agaccuaggu cgucaguuga ccaguccgcc     180
acuagacgug aguccgucaa agcaguuaga uguuacacuc uauuagaucu cggauuacag     240
cuggaaggag caggaguagu guucuugcuc uaaguaccga gugugcccaa uacccgauca     300
gcuuauuaac gaacggcucc uccucuuaga cugcagcgua agugcggaau cuggggauca     360
aauuacugac ugccuggauu acccucggac auauaaccuu guagcacgcu guugcuguau     420
aggugaccaa cgcccacucg aguagaccag cucucuuagu ccggacaaug auaggaggcg     480
cggucaaucu acuucuggcu aguuaagaau aggcugcacc gaccucuaua aguagcgugu     540
ccucuagagc uacgcagguu cgcaauaaaa gcguugauua gugugcauag aacagaccuc     600
uuauucggug aaacgccaga augcuaaauu ccaauaacuc uucccaaaac gcguacggcc     660
gaagacgcgc gcuuaucuug uguacguucu cgcacaugga agaaucagcg ggcauggugg     720
uagggcaaua ggggagcugg guagcagcga aaaagggccc cugcgcacgu agcuucgcug     780
uucgucugaa acaacccggc auccguugua gcgaucccgu uaucaguguu auucuugugc     840
gcacuaagau ucauggugua gucgacaaua acagcgucuu ggcagauucu ggucacgugc     900
ccuaugcccg ggcuugugcc ucucaggugc acagcgauac uuaaagccuu caagguacuc     960
gacgugggua ccgauucgug acacuuccua agauuauucc acuguguuag ccccgcaccg    1020
ccgaccuaaa cugguccaau guauacgcau ucgcugagcg gaucgauaau aaaagcuuga    1080
auu                                                                  1083
```

<210> 90
<211> 229
<212> RNA
<213> artificial

<220>
<223> nucleic acid molecule according to formula (IV) (NuGlxmGnNv)a

<400> 90

```
gggagaaagc ucaagcuuau ccaaguaggc uggucaccug uacaacguag ccgguauuuu      60
uuuuuuuuuu uuuuuuuuga ccgucucaag guccaaguua gucugccuau aaaggugcgg     120
auccacagcu gaugaaagac uugugcggua cgguuaaucu ccccuuuuuu uuuuuuuuuu     180
uuuuuaguaa augcgucuac ugaauccagc gaugaugcug gcccagauc                 229
```

<210> 91
<211> 546
<212> RNA
<213> artificial

<220>
<223> nucleic acid molecule according to formula (IV) (NuGlxmGnNv)a

<400> 91

gggagaaagc ucaagcuuau ccaaguaggc uggucaccug uacaacguag ccgguauuuu 60
uuuuuuuuuu uuuuuuuuga ccgucucaag guccaaguua gucugccuau aaaggugcgg 120
auccacagcu gaugaaagac uugugcggua cgguuaaucu ccccuuuuuu uuuuuuuuuu 180
uuuuuaguaa augcgucuac ugaauccagc gaugaugcug gcccagaucu ucgaccacaa 240
gugcauauag uagucaucga gggucgccuu uuuuuuuuuu uuuuuuuuuu uggcccaguu 300
cugagacuuc gcuagagacu acaguuacag cugcaguagu aaccacugcg gcuauugcag 360
gaaaucccgu ucagguuuuu uuuuuuuuuu uuuuuuccgc ucacuaugau uaagaaccag 420
guggaguguc acugcucucg aggucucacg agagcgcucg auacaguccu uggaagaauc 480
uuuuuuuuuu uuuuuuuuuu uugugcgacg aucacagaga acuucuauuc augcaggucu 540
gcucua 546

<210> 92
<211> 1083
<212> RNA
<213> artificial

<220>
<223> nucleic acid molecule according to formula (IV) (NuGlxmGnNv)a

<400> 92

gggagaaagc ucaagcuuau ccaaguaggc uggucaccug uacaacguag ccgguauuuu 60
uuuuuuuuuu uuuuuuuuga ccgucucaag guccaaguua gucugccuau aaaggugcgg 120
auccacagcu gaugaaagac uugugcggua cgguuaaucu ccccuuuuuu uuuuuuuuuu 180
uuuuuaguaa augcgucuac ugaauccagc gaugaugcug gcccagaucu ucgaccacaa 240
gugcauauag uagucaucga gggucgccuu uuuuuuuuuu uuuuuuuuuu uggcccaguu 300
cugagacuuc gcuagagacu acaguuacag cugcaguagu aaccacugcg gcuauugcag 360
gaaaucccgu ucagguuuuu uuuuuuuuuu uuuuuuccgc ucacuaugau uaagaaccag 420
guggaguguc acugcucucg aggucucacg agagcgcucg auacaguccu uggaagaauc 480
uuuuuuuuuu uuuuuuuuuu uugugcgacg aucacagaga acuucuauuc augcaggucu 540
gcucuagaac gaacugaccu gacgccugaa cuuaugagcg ugcguauuuu uuuuuuuuuu 600
uuuuuuuuuc cucccaacaa augucgauca auagcugggc uguuggagac gcgucagcaa 660
augccguggc uccauaggac guguagacuu cuauuuuuuu uuuuuuuuuu uuuucccggg 720
accacaaaua auauucuugc uugguugggc gcaagggccc cguaucaggu cauaaacggg 780
uacauguugc acaggcuccu uuuuuuuuuu uuuuuuuuuu uucgcugagu uauuccgguc 840
ucaaaagacg gcagacguca gucgacaaca cggucuaaag cagugcuaca aucugccgug 900
uucguguuuu uuuuuuuuuu uuuuuuguga accuacacgg cgugcacugu aguucgcaau 960
ucauagggua ccggcucaga guuaugccuu gguugaaaac ugcccagcau acuuuuuuuu 1020
uuuuuuuuuu uucauauucc caugcuaagc aagggaugcc gcgagucaug uuaagcuuga 1080
auu 1083

<210> 93
<211> 59
<212> RNA
<213> artificial

<220>
<223> nucleic acid molecule according to formula (v) (NuClxmCnNv)a

<400> 93
uagcgaagcu cuuggaccua ccuuuuuuuu uuuuuucccu gcguuccuag aaguacacg 59

<210> 94
<211> 120
<212> RNA
<213> artificial

<220>
<223> nucleic acid molecule according to formula (v) (NuClxmCnNv)a

<400> 94

uagcgaagcu cuuggaccua ccuuuuuuuu uuuuuuuccc ugcguuccua gaaguacacg 60
aucgcuucga gaaccuggau ggaaaaaaaa aaaaaaaggg acgcaaggau cuucaugugc 120

<210> 95
<211> 7
<212> PRT
<213> artificial

<220>
<223> cationic peptide according to sum formula (I) {(Arg)l; (Lys)m; (His)n; (Orn)o; (Xaa)x}

<400> 95

Arg Arg Arg Arg Arg Arg Arg
1 5

<210> 96
<211> 9
<212> PRT
<213> artificial

<220>
<223> cationic peptide according to sum formula (I) {(Arg)l; (Lys)n); (His)n; (Orn)o; (Xaa)x}

<400> 96

Arg Arg Arg Arg Arg Arg Arg Arg Arg
1 5

<210> 97
<211> 12
<212> PRT
<213> artificial

<220>
<223> cationic peptide according to sum formula (I) {(Arg)l; (Lys)m; (His)n; (Orn)o; (Xaa)x}

<400> 97

Arg Arg Arg Arg Arg Arg Arg Arg Arg Arg Arg Arg

1 5 10

<210> 98
<211> 14
<212> PRT
<213> artificial

<220>
<223> cationic peptid (cys-Arg12-cys)

<400> 98

Cys Arg Arg Arg Arg Arg Arg Arg Arg Arg Arg Arg Arg Cys
1 5 10

<210> 99
<211> 20
<212> DNA

<213> Artificial Sequence

<220>
<223> CpG 2216

<400> 99
gggggacgat cgtcgggggg 20

<210> 100
<211> 20
<212> DNA
<213> artificial

<220>
<223> CpG-ODN

<400> 100
tccatgacgt tcctgatgct 20

<210> 101
<211> 547
<212> RNA
<213> artificial

<220>
<223> nucleic acid molecule according to formula (IV) (NuGlxmGnNv)a

<400> 101

gggagaaagc ucaagcuuau ccaaguaggc uggucaccug uacaacguag ccgguauuuu 60
uuuuuuuuuu uuuuuuuuga ccgucucaag guccaaguua gucugccuau aaaggugcgg 120
auccacagcu gaugaaagac uugugcggua cgguuaaucu ccccuuuuuu uuuuuuuuuu 180
uuuuuaguaa augcgucuac ugaauccagc gaugaugcug gcccagaucu ucgaccacaa 240
gugcauauag uagucaucga gggucgccuu uuuuuuuuuu uuuuuuuuuu uggcccaguu 300
cugagacuuc gcuagagacu acaguuacag cugcaguagu aaccacugcg gcuauugcag 360
gaaaucccgu ucagguuuuu uuuuuuuuuu uuuuuuccgc ucacuaugau uaagaaccag 420
guggaguguc acugcucucg aggucucacg agagcgcucg auacaguccu uggaagaauc 480
uuuuuuuuuu uuuuuuuuuu uugugcgacg aucacagaga acuucuauuc augcaggucu 540

gcucuag 547

<210> 102
<211> 20
<212> DNA
<213> artificial

<220>
<223> CpG-DNA

<400> 102
tccatgacgt tcctgacgtt 20

<210> 103
<211> 8
<212> PRT
<213> Artificial sequence

<220>
<223> chicken OVA 257-264 peptide

<400> 103

Ser Ile Ile Asn Phe Glu Lys Leu
1               5

<210> 104
<211> 14
<212> PRT
<213> Artificial sequence

<220>
<223> chicken OVA 323-336 peptide

<400> 104

Ile Ser Gln Ala Val His Ala Ala His Ala Glu Ile Asn Glu
1               5                   10

<210> 105
<211> 9
<212> PRT
<213> Artificial sequence

<220>
<223> mouse mastozytoma P815-derived peptide P1A

<400> 105

Leu Pro Tyr Leu Gly Trp Leu Val Phe
1               5

<210> 106
<211> 9
<212> PRT
<213> Artificial sequence

<220>
<223> CSP-peptide

<400> 106

Ser Tyr Val Pro Ser Ala Glu Gln Ile

1               5

<210> 107
<211> 9
<212> PRT
<213> Artificial Sequence

<220>
<223> LLO-peptide

<400> 107

```
Gly Tyr Lys Asp Gly Asn Glu Tyr Ile
1               5
```

<210> 108
<211> 8
<212> PRT
<213> Artificial Sequence

<220>
<223> MC1R-peptide

<400> 108

```
Trp Gly Pro Phe Phe Leu His Leu
1               5
```

## Description

The present invention is directed to a pharmaceutical composition including (e.g., for use as an adjuvant) a (negatively charged) nucleic acid comprising complex comprising as a carrier cationic or polycationic compounds ( peptides, proteins or polyimines) and as a cargo at least one nucleic acid (molecule) and at least one antigen or a fragment, variant and/or derivative thereof according to the claims. The inventive pharmaceutical composition (e.g. an adjuvanted vaccine) allows for efficient induction of an adaptive immune response directed against the at least one antigen comprised therein, particularly of a Th1-shifted immune response. The present invention furthermore provides kits or kits of parts comprising the components of the inventive nucleic acid comprising complex or of the inventive pharmaceutical composition, as well as the use of the inventive pharmaceutical composition or the inventive kit or kit of parts as a vaccine, particularly in the treatment of infectious diseases, allergies, autoimmune diseases and tumour or cancer diseases. Furthermore the invention provides: (a) the nucleic acid comprising complex for use in therapy in combination with at least one protein or peptide antigen or a functional fragment, variant and/or derivative thereof; and (b) at least one peptide or protein antigen or a functional fragment, variant and/or derivative thereof for use in therapy in combination with the nucleic acid comprising complex, in each case (a) and (b), particularly for use in therapy of infectious diseases, allergies, autoimmune diseases and tumour or cancer diseases.

Many diseases today require administration of adjuvants to provide an innate immune response to support an adaptive immune response, particularly in the context of vaccinations.

Vaccination is generally believed to be one of the most effective and cost-efficient ways to prevent or treat diseases. Nevertheless several problems in vaccine development have proved difficult to solve: Vaccines are often inefficient for the very young and the very old; many vaccines need to be given several times, and the protection they confer wanes over time, requiring booster administrations, and, for some diseases such as HIV, development of efficient vaccines is urgently needed. As generally accepted, many of these vaccines would be enabled or improved if they could elicit a stronger and more durable immune response.

Accordingly, the development of new efficient and safe adjuvants for vaccination purposes which support induction and maintenance of an adaptive immune response by initiating or boosting a parallel innate immune response represents a main challenging problem.

Adjuvants are usually defined as compounds that can increase and/or modulate the intrinsic immunogenicity of an antigen. To reduce negative side effects, new vaccines have a more defined composition that often leads to lower immunogenicity compared with previous whole-cell or virus-based vaccines. Adjuvants are therefore required to assist new vaccines to induce potent and persistent immune responses, with the additional benefit that less antigen and fewer injections are needed. Now it is clear that the adaptive immune response mainly depends on the level and specificity of the initial danger signals perceived by innate immune cells following infection or vaccination (Guy, B. (2007), Nat Rev Microbiol 5(7): 505-17.). In particular for new generation vaccine candidates, which will increasingly comprise highly purified recombinant proteins and, although very safe, are poorly immunogenic, efficient adjuvants will become increasingly necessary.

Unfortunately, only a few licensed adjuvants are available so far. Most prominent is Alum, which is known to be safe, but also represents a very weak adjuvant. Many further adjuvants have been developed, e.g. including the administration of pathogens, CpG-nucleotides, etc. Most of these new or "established" adjuvants, however, still do not satisfy the above requirements, since many new and emerging problems have to be considered and solved. These problems *inter alia* include new and re-emerging infectious diseases, repeated administrations, threat of pandemic flu, etc.

Furthermore, the new vaccine targets are usually more difficult to develop and - due to their specifically tailored immune responses - require more potent adjuvants to enable success. Moreover, there are still a significant number of important pathogens for which we do not even have effective vaccines at present. This represents a very challenging future target. To enable vaccine development against such targets, more potent pharmaceutical compositions that include adjuvants and such targets will be necessary. Therefore, the new adjuvants in such compositions will need to offer advantages, including more heterologous antibody responses, covering pathogen diversity, induction of potent functional antibody responses, ensuring pathogen killing or neutralization and induction of more effective T cell responses, for direct and indirect pathogen killing, particularly the induction of cytotoxic T cells which are part of a Th1 immune response. In addition, adjuvants may be necessary to achieve more pragmatic effects, including antigen dose reduction and overcoming antigen competition in combination vaccines. Moreover, against the background of an aging population, which is increasingly susceptible to infectious diseases, new adjuvants will be necessary to overcome the natural deterioration of the immune response with age (O'Hagan, D. T. and E. De Gregorio (2009), Drug Discov Today 14(11-12): 541-51.).

The review of O'Hagan (2009; supra) summarizes some reasons for the urgent need of new effective adjuvants e.g. the requirement of a lower antigen dose in vaccines, the necessity to increase the breadth of an immune response and the heterologous activity, to enable complex combination vaccines, and to overcome antigenic competition, to overcome limited immune response in some groups of the population, such as the elderly, the young children, and infants, patients with chronic diseases and the immunocompromised, to increase effector T cell response and antibody titers, to induce protective responses more rapidly and also to extend the duration of response by enhancing memory B and T cell responses.

Furthermore, it is known from the prior art that peptide or protein antigens or inactivated or attenuated virus or cell based vaccine presenting protein antigens preferably induce a Th2-shifted immune response by themselves. For example, Huber *et al.* showed that BALB/c mice typically respond to inactivated influenza vaccines and subunit vaccines with a Th2-type immune response which is associated with the stimulation of IgG1 antibodies. But the major antibody isotype necessary in the sera of mice to survive viral infections is IgG2a, which is stimulated during Th1-type immune responses. Therefore, stimulation of IgG2a antibodies has been associated with increased efficacy of influenza vaccination. Additionally, monoclonal antibodies of the IgG2a isotype are more efficient at clearing influenza, Ebola, and yellow fever virus infections than monoclonal antibodies of the IgG1 isotyp displaying similar antigenic specificities. (Huber et al., (2006) Clincal and Vaccine Immunology 13(9): 981-990).

Summarizing the above, new efficient and safe immunostimulating agents or adjuvants are required, which are preferably efficient in inducing an innate immune response, particularly in inducing the anti-viral cytokine IFN-alpha; and therefore switching a Th2-shifted immune response into a Th1-shifted immune response which is especially important for peptide or protein vaccines (or virus or cell preparations displaying peptide or protein antigens) which mainly induces a Th2-shifted immune response.

As already explained above adjuvants or immunostimulating agents usually act via their capability to induce an innate immune response. The innate immune system forms the dominant system of host defense in most organisms and comprises barriers such as humoral and chemical barriers including, e.g., inflammation, the complement system and cellular barriers. The innate immune system is typically based on a small number of receptors, called pattern recognition receptors. They recognize conserved molecular patterns that distinguish foreign organisms, like viruses, bacteria, fungi and parasites, from cells of the host. Such pathogen-associated molecular patterns (PAMP) include viral nucleic acids, components of bacterial and fungal walls, flagellar proteins, and more. The first family of pattern recognition receptors (PAMP receptors) studied in detail was the Toll-like receptor (TLR) family. TLRs are transmembrane proteins which recognize ligands of the extracellular milieu or of the lumen of endosomes. Following ligand-binding they transduce the signal via cytoplasmic adaptor proteins which leads to triggering of a host-defence response and entailing production of antimicrobial peptides, proinflammatory chemokines and cytokines, antiviral cytokines, etc. (see e.g. Meylan, E., J. Tschopp, et al. (2006), Nature 442(7098): 39-44). Further relevant components of the immune system include e.g. the endosomal TLRs, cytoplasmic receptors, Type I interferons and cytoplasmic receptors. Therefore, the immunostimulating agents or adjuvants are defined herein preferably as inducers of an innate immune response, which activate pattern recognition receptors (PAMP receptors). Hereby, a cascade of signals is elicited, which e.g. may result in the release of cytokines (e.g. IFN-alpha) supporting the innate immune response. Accordingly, it is preferably a feature of an immunostimulating agent or adjuvant to bind to such receptors and activate such PAMP receptors. Ideally, such as an agent or adjuvant additionally supports the adaptive immune response by e.g. shifting the immune response such that the preferred class of Th cells is activated. Depending on the disease or disorder to be treated a shift to a Th1-based immune response may be preferred or, in other cases, a shift to a Th2 immune response may be preferred.

In the prior art, there are some promising adjuvant candidates which fulfil at least some, but not all, of the above defined required characteristics.

As an example, among the above developed new adjuvants, some nucleic acids like CpG DNA oligonucleotides or isRNA (immunostimulating RNA) turned out to be promising candidates for new immunostimulating agents or adjuvants as they allow the therapeutic or prophylactic induction of an innate immune response. Comprehensibly, such nucleic acid based adjuvants usually have to be delivered effectively to the site of action to allow induction of an effective innate immune response without unnecessary loss of adjuvant activity and, in some cases, without the necessity to increase the administered volume above systemically tolerated levels.

One approach to solve this issue may be the transfection of cells which are part of the innate immune system (e.g. dendritic cells, plasmacytoid dendritic cells (pDCs)) with immunostimulatory nucleic acids, which are ligands of PAMP receptors, (e.g. Toll-like receptors (TLRs)), and thus may lead to immunostimulation by the nucleic acid ligand. Further approaches may be the direct transfection of nucleic acid based adjuvants. All of these approaches, however, are typically impaired by inefficient delivery of the nucleic acid and consequently diminished adjuvant activity, in particular when administered locally.

However, one main disadvantage of such nucleic acid based adjuvant approaches until today is their limited ability to cross the plasma membrane of mammalian cells, resulting in poor cellular access and inadequate therapeutic efficacy. Until today this hurdle represents a major challenge for nucleic acid transfection based applications, e.g. biomedical developments and accordingly the commercial success of many biopharmaceuticals (see e.g. Foerg, C. & Merkle, H.P., J Pharm Sci97, 144-62 (2008).

Transfection of nucleic acids or genes into cells or tissues has been investigated up to date in the context of *in vitro* transfection purposes and in the context of gene therapeutic approaches. However, no adjuvants are available so far which are based on such gene delivery techniques which are efficient and safe, in particular no licensed adjuvants. This is presumably due to the complex requirements of adjuvants in general in combination with stability issues to be solved in the case of nucleic acid based adjuvants.

Nevertheless, transfection of nucleic acids or genes into cells or tissues for eliciting an (innate and/or adaptive) immune response appears to provide a promising approach to provide new adjuvants.

Even if a lot of transfection methods are known in the art, transfer or insertion of nucleic acids or genes into an individual's cells still represents a major challenge today and is not yet solved satisfactorily. To address this complex issue a variety of methods were developed in the last decade. These include transfection by calcium phosphate, cationic lipids, cationic polymers, and liposomes. Further methods for transfection are electroporation and viral transduction.

Many of these approaches utilize transfection of nucleic acids or genes into cells or tissues without the purpose to induce an innate immune response. There are even some gene therapeutic therapies, which have to strictly avoid induction of an innate immune response. Even in the rare cases, where vaccination is carried out to induce an adaptive antigen-specific immune response using administration of nucleic acids, e.g. in tumour vaccinations using DNA or mRNA encoded antigens, induction of an adaptive immune response is typically carried out as an active immunization against the encoded antigen but not as an accompanying adjuvant therapy and thus may require additional administration of a separate adjuvant to induce an innate immune response.

Thus, many of the above described approaches have been assessed for transfection of nucleic acids in terms of translation (e.g. DNA, mRNA) or inhibition of translation (e.g. siRNA, shRNA) and only a few studies have been done assessing the effect of such nucleic acid comprising complexes on the stimulation of immunocompetent cells.

In the above mentioned transfection methods of coding nucleic acids the ultimate goal is the final destination of the cargo in the cytosol or nucleus. To reach this goal the route of transfer across the cell membranes is of less importance and the efficiency of the transfer is measured by level of expression.

In contrast to this, the goal of nucleic acids for stimulation of immunocompetent cells is the presentation of the nucleic acid to different pattern-recognition receptors. Such receptors are localized in different compartments and to get an optimal immune response the formulation must enter such compartments to ensure presentation. Therefore, the efficiency of the immunostimulatory formulation is mainly dependent on the route of cellular uptake.

As an example, TLR-7, TLR-8, and TLR9 receptors which are the main PAMP receptors of immunostimulatory nucleic acids are located in the endosome. Thus, transfection of cells with immunostimulatory nucleic acids, may advantageously lead to the uptake of the immunostimulatory nucleic acid into endosomes and depending on the specific carrier molecule to immunostimulation by the immunostimulatory nucleic acid. Also it is desirable to transfer immunostimulatory nucleic acids, particularly immunostimulatory RNA into the cytosol of the cell to present it to cytosolic PAMP receptors as for example the RIG-I or the PKR receptor.

In the recent years immunostimulatory nucleic acids complexed to different carriers were only examined for their capacity to induce an innate immune response and not for their capacity to support an adaptive immune response. For example, Scheel *et al.* has shown that protamine complexed mRNA molecules are danger signals for cells and therefore are able to induce an innate immune response (Scheel, B et al. (2004). Eur J Immunol 34, 537-47 and Scheel, B et al. (2005). Eur J Immunol 35, 1557-1566).

Other reports have shown that also cationic lipids or cationic polymers such as PEI may be utilized to present immunostimulating RNA *in vivo* (Heil, F et al., (2004) Science 303: 1526-9).

Furthermore, Diebold *et al.* reported the effect of PEI complexed ssRNAs on TLR7 induced production of inflammatory cytokines. They emphasized the recognition of endosomal ssRNA by cells of the innate immune system for detection of RNA virus infection (Diebold, S.S. et al., (2004) Science 303: 1529-31). Interestingly the authors reduced the role of PEI complexation to simple protection against RNAse degradation.

Another report by Hornung *et al.* discloses the effect of certain sequences of RNA oligonucleotides delivered by cationic liposomes and develops an algorithm that can predict the immunostimulatory capacity of RNA sequences. Also in this study the role of the carrier molecule on the immunostimulatory capacity was not assessed (Hornung, V. et al., (2005) Nat Med 11: 263-70).

Only in a few studies the role of the carrier molecule on the immunostimulatory capacity of nucleic acids was examined.

Recently Fotin-Mleczek *et al.* has examined the effect of different cargo/carrier ratios on the immunostimulatory capacity of RNA (WO 2009/030481). Those formulations efficiently induced the cytokine production of IL-6 and TNFalpha in immunocompetent cells, but neither the induction of the preferable anti-viral cytokine IFN-alpha nor the support of an adaptive immune response caused by a peptide or protein antigen was examined.

In a further study, Fotin-Mleczek *et al.* reported on an immunostimulatory composition comprising an adjuvant component, comprising an (m)RNA, complexed with a cationic or polycationic compound, and at least one free mRNA for use as an antigen (WO 2010/037539 and Fotin-Mleczek et al. (2011) J Immunother 34: 1-15). They could show that the adjuvant component can support an immune response directed against an mRNA vaccine which itself induces already a Th1-shifted immune response without support of an adjuvant. But neither the induction of the anti-viral cytokine IFNalpha nor the support of an adaptive immune response directed against a peptide or protein antigen was examined. WO 01/54720 discloses a pharmaceutical composition for use as a vaccine, wherein the composition comprises a polycationic peptide, e.g poly-L-arginine and CpG-ODN to be combined with a protein antigen.

Postpublished PCT application WO 2012/013326 describes compositions containing RNA/peptide complexes with the peptide component being either ovalbumine or the ovalbumine-derived model peptide SIINFEKL.

Summarizing the above, the prior art does not provide feasible means or methods, which allow to establish efficient adjuvants for vaccination purposes, particularly in case peptide or protein antigens are used for vaccination and therefore a switch to a Th1-shifted immune response is desired and/or necessary.

Accordingly, it is the object of the present invention to provide approaches, which address one or more of these problems.

The object underlying the present invention is solved by the subject matter of the present invention as claimed Any subject-matter falling outside the scope of the claims is provided for information purposes only.

For the sake of clarity and readability the following definitions are provided. Any technical features disclosed thereby can be part of each and every embodiment of the invention. Additional definitions and explanations can be provided in the context of this disclosure.

Nucleic acid: The term nucleic acid means any DNA- or RNA-molecule and is used synonymous with polynucleotide. Furthermore, modifications or derivatives of the nucleic acid as defined herein are explicitly included in the general term "nucleic acid". For example, PNA is also included in the term "nucleic acid".

Monocistronic RNA: A monocistronic RNA may typically be a RNA, preferably a mRNA, that encodes only one open reading frame. An open reading frame in this context is a sequence of several nucleotide triplets (codons) that can be translated into a peptide or protein.

Bi-/multicistronic RNA: RNA, preferably a mRNA, that typically may have two (bicistronic) or more (multicistronic) open reading frames (ORF). An open reading frame in this context is a sequence of several nucleotide triplets (codons) that can be translated into a peptide or protein.

5'-Cap structure: A 5' Cap is typically a modified nucleotide, particularly a guanine nucleotide, added to the 5' end of a RNA-molecule. Preferably, the 5'-Cap is added using a 5'-5'-triphosphate linkage.

Poly(C) sequence: A poly(C) sequence is typically a long sequence of cytosine nucleotides, typically about 10 to about 200 cytidine nucleotides, preferably about 10 to about 100 cytidine nucleotides, more preferably about 10 to about 70 cytidine nucleotides or even more preferably about 20 to about 50 or even about 20 to about 30 cytidine nucleotides. A poly(C) sequence may preferably be located 3' of the coding region comprised by a nucleic acid.

Poly(A) tail: A poly(A) tail also called "3'-poly(A) tail" is typically a long sequence of adenine nucleotides of up to about 400 adenosine nucleotides, e.g. from about 25 to about 400, preferably from about 50 to about 400, more preferably from about 50 to about 300, even more preferably from about 50 to about 250, most preferably from about 60 to about 250 adenosine nucleotides, added to the 3' end of a RNA.

Stabilized nucleic acid: A stabilized nucleic acid, typically, may be essentially resistant to *in vivo* degradation (e.g. degradation by an exo- or endo-nuclease) and/or ex vivo degradation (e.g. by the manufacturing process prior to vaccine administration, e.g. in the course of the preparation of the vaccine solution to be administered). Stabilization of mRNA can, e.g., be achieved by providing a 5'-Cap structure, a poly(A) tail, a poly(C) tail, or any other UTR modification. It can also be achieved by backbone modification or modification of the G/C-content of the nucleic acid. Various other methods are conceivable in the context of the invention.

Modification of a nucleic acid (modified nucleic acid): Modification of a nucleic acid molecule may contain backbone modifications, sugar modifications or base modifications. A backbone modification in connection with the present invention is a modification in which phosphates of the backbone of the nucleotides contained in the nucleic acid molecule are chemically modified. A sugar modification in connection with the present invention is a chemical modification of the sugar of the nucleotides of the nucleic acid. Furthermore, a base modification in connection with the present invention is a chemical modification of the base moiety of the nucleotides of the nucleic acid molecule. Therefore a modified nucleic acid is also defined herein as a nucleic acid molecule which may include nucleotide analogues. Furthermore a modification of a nucleic acid molecule can contain a lipid modification. Such a lipid-modified nucleic acid typically comprises a nucleic acid as defined herein. Such a lipid-modified nucleic acid molecule typically further comprises at least one linker covalently linked with that nucleic acid molecule, and at least one lipid covalently linked with the respective linker. Alternatively, the lipid-modified nucleic acid molecule comprises at least one nucleic acid molecule as defined herein and at least one (bifunctional) lipid covalently linked (without a linker) with that nucleic acid molecule. According to a third alternative, the lipid-modified nucleic acid molecule comprises a nucleic acid molecule as defined herein, at least one linker covalently linked with that nucleic acid molecule, and at least one lipid covalently linked with the respective linker, and also at least one (bifunctional) lipid covalently linked (without a linker) with that nucleic acid molecule.

A modification of a nucleic acid may also comprise the modification of the G/C content of the coding region of a nucleic acid molecule, especially if the nucleic acid molecule is in the form of an mRNA. In this context it is particularly preferred that the G/C content of the coding region of the nucleic acid molecule is increased, compared to the G/C content of the coding region of its particular wild type coding sequence, i.e. the unmodified mRNA. The encoded amino acid sequence of the nucleic acid sequence is preferably not modified compared to the coded amino acid sequence of the particular wild type mRNA.The modification of the G/C-content of the nucleic acid molecule, especially if the nucleic acid molecule is in the form of an mRNA or codes for an mRNA, is based on the fact that the sequence of any mRNA region to be translated is important for efficient translation of that mRNA. Thus, the composition and the sequence of various nucleotides are important. In particular, sequences having an increased G (guanosine)/C (cytosine) content are more stable than sequences having an increased A (adenosine)/U (uracil) content. Therefore, the codons of the coding sequence or mRNA are therefore varied compared to its wild type coding sequence or mRNA, while retaining the translated amino acid sequence, such that they include an increased amount of G/C nucleotides. In respect to the fact that several codons code for one and the same amino acid (so-called degeneration of the genetic code), the most favourable codons for the stability can be determined (so-called alternative codon usage). Preferably, the G/C content of the coding region of the nucleic acid molecule, especially if the nucleic acid is in the form of an mRNA or codes for an mRNA, is increased by at least 7%, more preferably by at least 15%, particularly preferably by at least 20%, compared to the G/C content of the coded region of the wild type mRNA. According to a specific embodiment at least 5%, 10%, 20%, 30%, 40%, 50%, 60%, more preferably at least 70%, even more preferably at least 80% and most preferably at least 90%, 95% or even 100% of the substitutable codons in the region coding for a protein or peptide as defined herein or its fragment, variant and/or derivative thereof or the whole sequence of the wild type mRNA sequence or coding sequence are substituted, thereby increasing the G/C content of said sequence. In this context, it is particularly preferable to increase the G/C content of the nucleic acid molecule, especially if the nucleic acid is in the form of an mRNA or codes for an mRNA, to the maximum (i.e. 100% of the substitutable codons), in particular in the region coding for a protein, compared to the wild type sequence. Furthermore, a modification of the nucleic acid, especially if the nucleic acid is in the form of an mRNA or codes for an mRNA, is based on the finding that the translation efficiency is also determined by a different frequency in the occurrence of tRNAs in cells. The frequency in the occurrence of tRNAs in a cell, and thus the codon usage in said cell, is dependent on the species the cell is derived from. Accordingly, a yeast cell generally exhibits a different codon usage than a mammalian cell, such as a human cell. Thus, if so-called "rare codons" are present in the nucleic acid molecule (with respect to the respective expression system), especially if the nucleic acid is in the form of an mRNA or codes for an mRNA, to an increased extent, the corresponding modified nucleic acid molecule is translated to a significantly poorer degree than in the case where codons coding for relatively "frequent" tRNAs are present. Therefore, especially if the modified nucleic acid molecule is in the form of an mRNA or codes for an mRNA, the coding region of the modified nucleic acid is preferably modified compared to the corresponding region of the wild type mRNA or coding sequence such that at least one codon of the wild type sequence which codes for a tRNA which is relatively rare in the cell is exchanged for a codon which codes for a tRNA which is relatively frequent in the cell and carries the same amino acid as the relatively rare tRNA. By this modification, the sequences of the nucleic acid molecule, especially if the nucleic acid is in the form of an mRNA or codes for an mRNA, is modified such that codons for which frequently occurring tRNAs are available are inserted. In other words, by this modification all codons of the wild type sequence which code for a tRNA which is relatively rare in the cell can in each case be exchanged for a codon which codes for a tRNA which is relatively frequent in the cell and which, in each case, carries the same amino acid as the relatively rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. It is particularly preferred that a nucleic acid sequence coding for a protein used in the present invention is codon optimized for the human codon usage. The codons which use for the particular amino acid the tRNA which occurs the most frequently, e.g. the Gly codon, which uses the tRNA which occurs the most frequently in the (human) cell, are particularly preferred. In this context, it is particularly preferable to link the sequential G/C content which is increased, in particular maximized, in the modified nucleic acid molecule, especially if the nucleic acid is in the form of an mRNA or codes for an mRNA, with the "frequent" codons without modifying the amino acid sequence of the protein encoded by the coding region of the nucleic acid molecule. This preferred embodiment allows provision of a particularly efficiently translated and stabilized (modified) nucleic acid, especially if the nucleic acid is in the form of an mRNA or codes for an mRNA.

Derivative of a nucleic acid molecule: A derivative of a nucleic acid molecule is defined herein in the same manner as a modified nucleic acid, as defined above.

Nucleotide analogues: Nucleotides structurally similar (analogue) to naturally occurring nucleotides which include phosphate backbone modifications, sugar modifications, or modifications of the nucleobase.

UTR modification: A nucleic acid molecule, especially if the nucleic acid is in the form of a coding nucleic acid molecule, preferably has at least one 5' and/or 3' stabilizing sequence (UTR modification). These stabilizing sequences in the 5' and/or 3' untranslated regions have the effect of increasing the half-life of the nucleic acid in the cytosol. These stabilizing sequences can have 100% sequence identity to naturally occurring sequences which occur in viruses, bacteria and eukaryotes, but can also be partly or completely synthetic. The untranslated sequences (UTR) of the (alpha-)globin gene, e.g. from *Homo sapiens* or *Xenopus laevis* may be mentioned as an example of stabilizing sequences which can be used for a stabilized nucleic acid. Another example of a stabilizing sequence has the general formula (C/U)CCAN$_x$-CCC(U/A)Py$_x$UC(C/U)CC which is contained in the 3'UTR of the very stable RNA which codes for (alpha-)globin, type(I)-collagen, 15-lipoxygenase or for tyrosine hydroxylase (cf. Holcik et al., Proc. Natl. Acad. Sci. USA 1997, 94: 2410 to 2414). Such stabilizing sequences can of course be used individually or in combination with one another and also in combination with other stabilizing sequences known to a person skilled in the art. In the context of the present invention, a UTR modification preferably means a modification of a coding nucleic acid, such as a gene or mRNA, by adding or exchanging a 5'- and/or 3'-UTR, preferably by adding or exchanging for a stabilizing 5'- and/or 3'-UTR, e.g., as specified above.

Nucleic acid synthesis: Nucleic acid molecules used according to the invention as defined herein may be prepared using any method known in the art, including synthetic methods such as e.g. solid phase synthesis, as well as *in vitro* methods, such as *in vitro* transcription reactions.

For preparation of a nucleic acid molecule, especially if the nucleic acid is in the form of an mRNA, a corresponding DNA molecule may be, e.g., transcribed *in vitro*. This DNA matrix preferably comprises a suitable promoter, e.g. a T7 or SP6 promoter, for *in vitro* transcription, which is followed by the desired nucleotide sequence coding for the nucleic acid molecule, e.g. mRNA, to be prepared and a termination signal for *in vitro* transcription. The DNA molecule, which forms the matrix of the at least one RNA of interest, may be prepared by fermentative proliferation and subsequent isolation as part of a plasmid which can be replicated in bacteria. Plasmids which may be mentioned as suitable for the present invention are e.g. the plasmids pT7Ts (GenBank accession number U26404; Lai et al., Development 1995, 121: 2349 to 2360), pGEM® series, e.g. pGEM®-1 (GenBank accession number X65300; from Promega) and pSP64 (GenBank accession number X65327); cf. also Mezei and Storts, Purification of PCR Products, in: Griffin and Griffin (ed.), PCR Technology: Current Innovation, CRC Press, Boca Raton, FL, 2001.

Protein: A protein typically consists of one or more polypeptides folded into 3-dimensional form, facilitating a biological function.

Peptide: A peptide is typically a short polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units, more specifically between 50 and 300 monomeric units. Furthermore a "peptide" is defined herein also to include any peptidyl molecule, including peptide analogues.

Peptide analogues: A peptide analogue may comprise naturally or non-naturally occurring amino acids which may be used for the purpose of the invention. For example they can comprise amino acids selected from an isostere or a chiral analog (D-amino acid or L-amino acid) of an amino acid. Additionally, the analog may comprise one or more amino acids, preferably selected from hydroxyproline, β-alanine, 2,3-diaminopropionic acid, α-aminoisobutyric acid, N-methylglycine (sarcosine), ornithine, citrulline, t-butylalanine, t-butylglycine, N-methylisoleucine, phenylglycine, cyclohexylalanine, norleucine, naphthylalanine, pyridylalanine, 3-benzothienyl alanine, 4-chlorophenylalanine, 2-fluorophenylalanine, 3-fluorophenylalanine, 4-fluorophenylalanine, penicillamine, 1,2,3,4-tetrahydro-isoquinoline-3-carboxylic acid, [beta]-2-thienylalanine, methionine sulfoxide, homoarginine, N-acetyl lysine, 2,4-diamino butyric acid, p-aminophenylalanine, N-methylvaline, homocysteine, homoserine, ε-amino hexanoic acid, δ-amino valeric acid, 2,3-diaminobutyric acid. A peptide analogue as defined herein may further contain modified peptides. The term specifically includes peptide back-bone modifications (i.e., amide bond mimetics) known to those skilled in the art. Such modifications include modifications of the amide nitrogen, the α-carbon, amide carbonyl, complete replacement of the amide bond, extensions, deletions or backbone crosslinks. Several peptide backbone modifications are known, including Ψ[OPH2S], ΨCH2NH], Ψ[CSNH2], Ψ[NHCO], Ψ[COCH2], and Ψ[(E) or (Z) CH=CH]. In the nomenclature used above, Ψ indicates the absence of an amide bond. The structure that replaces the amide group is specified within the brackets. Other modifications include, for example, an N-alkyl (or aryl) substitution (Ψ[CONR]), or backbone crosslinking to construct lactams and other cyclic structures, C-terminal hydroxymethyl modifications, O-modified modifications (e.g., C-terminal hydroxymethyl benzyl ether), N-terminal modifications including substituted amides such as alkylaniides and hydrazides.

Peptide synthesis: A peptide, a peptide analogue, or a derivative thereof is preferably synthesized using a chemical method known to the skilled artisan. For example, synthetic peptides are prepared using known techniques of solid phase, liquid phase, or peptide condensation, or any combination thereof, and can include natural and/or unnatural amino acids. Generally, chemical synthesis methods comprise the sequential addition of one or more amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complementary (amino or carboxyl) group suitably protected, under conditions that allow for the formation of an amide linkage. The protecting group is then removed from the newly added amino acid residue and the next amino acid (suitably protected) is then added, and so forth. After the desired amino acids have been linked in the proper sequence, any remaining protecting groups (and any solid support, if solid phase synthesis techniques are used) are removed sequentially or concurrently, to render the final polypeptide. These methods are suitable for synthesis of a peptide used for the purpose of the present invention (such as a peptide analogue) or derivative thereof. Typical protecting groups include t-butyloxycarbonyl (Boc), 9-fluorenylmethoxycarbonyl (Fmoc) benzyloxycarbonyl (Cbz); p-toluenesulfonyl (Tx); 2,4-dinitrophenyl; benzyl (Bzl); biphenylisopropyloxycarboxy-carbonyl, t-amyloxycarbonyl, isobornyloxycarbonyl, o-bromobenzyloxycarbonyl, cyclohexyl, isopropyl, acetyl, o-nitrophenylsulfonyl and the like. Typical solid supports are cross-linked polymeric supports. These can include divinylbenzene cross-linked-styrene-based polymers, for example, divinylbenzene-hydroxymethylstyrene copolymers, divinylbenzene-chloromethylstyrene copolymers and divinylbenzene-benzhydrylaminopolystyrene copolymers.

Recombinant peptide or protein production: A peptide or protein or derivative thereof may be produced using recombinant protein or peptide production. To facilitate the production of a recombinant peptide or protein, at least one nucleic acid encoding the same is preferably isolated or synthesized. Typically, the nucleic acid encoding the recombinant protein or peptide is isolated using a known method, such as, for example, amplification (e.g., using PCR) or isolated from nucleic acid from an organism using one or more restriction enzymes or isolated from a library of nucleic acids. For expressing a protein or peptide by recombinant means, a protein/peptide-encoding nucleic acid is placed in operable connection with a promoter or other regulatory sequence capable of regulating expression in a cell-free system or cellular system. For example, nucleic acid comprising a sequence that encodes a peptide or protein is placed in operable connection with a suitable promoter and maintained in a suitable cell for a time and under conditions sufficient for expression to occur. Typical expression vectors for in vitro expression, cell-free expression or cell-based expression have been described and are well known for the skilled person. In this context cell-free expression systems may include *E. coli* S30 fraction, rabbit reticulocyte lysate and wheat germ extract and a cellular system may be selected from bacterial (e.g. *E. coli*), insect, plant, or mammalian cells (e.g., 293, COS, CHO, 1OT cells, 293T cells).

Secretory signal peptide: Such signal peptides are sequences, which typically exhibit a length of about 15 to 30 amino acids and are preferably located at the N-terminus of the encoded peptide, without being limited thereto. Signal peptides as defined herein preferably allow the transport of the protein or peptide into a defined cellular compartment, preferably the cell surface, the endoplasmic reticulum (ER) or the endosomal-lysosomal compartment.

Carrier: A carrier in the context of the invention may typically be a compound that facilitates transport and/or complexation of another compound. A carrier, in the context of the present invention, is preferably suitable as carrier for nucleic acid molecules, e.g. for mediating dissolution in physiological acceptable liquids, transport and cellular uptake of the nucleic acid molecules or a vector. Accordingly, a carrier, in the context of the present invention, may be a component which may be suitable for depot and delivery of a nucleic acid molecule or vector. Such carriers may be, for example, cationic or polycationic carriers or compounds which may serve as transfection or complexation agent. Particularly preferred carriers in this context are cationic or polycationic compounds, including protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*), pAntp, plsl, FGF, Lactoferrin, Transportan, Buforin-2, Bac715-24, SynB, SynB(1), pVEC, hCT-derived peptides, SAP, or histones. Furthermore, such cationic or polycationic carriers may be cationic or polycationic peptides or proteins, thus, a carrier in the context of the present invention may, for example, be a peptidic cationic component. The cationic or polycationic carrier may also be a lipidic cationic component, such as lipids or liposomes.

Cationic component: The term "cationic component" typically refers to a charged molecule, which is positively charged (cation) at a pH value of about typically 1 to 9, preferably of a pH value of or below 9 (e.g. 5 to 9), of or below 8 (e.g. 5 to 8), of or below 7 (e.g. 5 to 7), most preferably at physiological pH values, e.g. about 7.3 to 7.4. Accordingly, a cationic peptide, protein or polymer according to the present invention is positively charged under physiological conditions, particularly under physiological salt conditions of the cell *in vivo.* A cationic peptide or protein contains a larger number of cationic amino acids, e.g. a larger number of Arg, His, Lys or Orn, than negatively charged or neutral amino acids. In a preferred embodiment, a cationic peptide or protein in the context of the present invention contains a larger number of cationic amino acids, e.g. a larger number of Arg, His, Lys or Orn, than other residues. The definition "cationic" may also refer to "polycationic" components.

The charge of a compound, complex or component, such as the cationic component or complex (A) as defined herein is preferably determined or assessed under physiological conditions, e.g. at a pH of between about 5.5 and 7.5, preferably at a pH of between about 6.0 and 7.4, such as about 7.0, at a temperature of between about 25°C and 40°C, preferably at a temperature of about 35 and 38°C, such as about 37°C, at a physiological salt concentration of, e.g. between about 130 and 160 mM, preferably between about 137 mM and 150 mM, such as at about 137 mM. Particularly preferred conditions for determining or assessing the charge of a compound, complex or component as defined herein are the conditions found in a 100% Ringer lactate solution at 25°C.

Zetapotential: The "zetapotential" is a widely used parameter for the electrical surface charge of a particle. It is typically determined by moving the charged particle through an electrical field. In the context of the present invention, the zetapotential is the preferred parameter for characterizing the charge of a particle, e.g. of complex (A) of the pharmaceutical compositions according to the present invention. Thus, in the context of the present invention, the charge of a particle is preferably determined by determining the zetapotential by the laser Doppler electrophoresis method using a Zetasizer Nano instrument (Malvern Instruments, Malvern, UK) at 25°C and a scattering angle of 173°. The surface charge of a given particle also depends on the ionic strength of the utilized matrix (e.g. salt containing buffer) and the pH of the solution. Therefore, the actual zetapotential of a given complex (A) at a charge ratio (N/P) may differ slightly between different buffers used for injection. For the measurement, the particles, such as complex (A) of the pharmaceutical compositions according to the present invention are preferably suspended in Ringer Lactate solution. The present invention claims therefore the use of a negativley charged complex (A) under the conditions of a given injection buffer, preferably under the conditions of a Ringer lactate solution, assessed by its Zetapotential. A Ringer lactate solution according to the present invention preferably contains 130 mmol/L sodium ions, 109 mmol/L chloride ions, 28 mmol/L lactate, 4 mmol/L potassium ions and 1.5 mmol/L cacium ion. The sodium, chloride, potassium and lactate typically come from NaCl (sodium chloride), $NaC_3H_5O_3$ (sodium lactate), CaCl2 (calcium chloride), and KCl (potassium chloride). The osmolarity of the Ringer lactate solution is 273 mOsm/L and the pH is adjusted to 6.5.

Pharmaceutically effective amount: A pharmaceutically effective amount in the context of the invention is typically understood to be an amount that is sufficient to induce an immune response.

Immune system: The immune system may protect organisms from infection. If a pathogen breaks through a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts contains so called humoral and cellular components.

Immune response: An immune response may typically either be a specific reaction of the adaptive immune system to a particular antigen (so called specific or adaptive immune response) or an unspecific reaction of the innate immune system (so called unspecific or innate immune response). In essence, the invention is associated with specific reactions (adaptive immune responses) of the adaptive immune system. However, this specific response can be supported by an additional unspecific reaction (innate immune response). Therefore, the invention also relates to a compound or composition for simultaneous stimulation of the innate and the adaptive immune system to evoke an efficient adaptive immune response.

Adaptive immune response: The adaptive immune response is typically understood to be antigen-specific. Antigen specificity allows for the generation of responses that are tailored to specific antigens, antigen-expressing cells, pathogens or pathogen-infected cells. The ability to mount these tailored responses is maintained in the body by "memory cells". Should a pathogen infect the body more than once, these specific memory cells are used to quickly eliminate it. In this context, the first step of an adaptive immune response is the activation of naive antigen-specific T cells or different immune cells able to induce an antigen-specific immune response by antigen-presenting cells. This occurs in the lymphoid tissues and organs through which naive T cells are constantly passing. Cell types that can serve as antigen-presenting cells are inter alia dendritic cells, macrophages, and B cells. Each of these cells has a distinct function in eliciting immune responses. Dendritic cells take up antigens by phagocytosis and macropinocytosis and are stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. Presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind antigen directly, but instead recognize short peptide fragments e.g. of pathogen-derived protein antigens, which are bound to MHC molecules on the surfaces of other cells.

Adaptive immune system: The adaptive immune system is, typically, composed of highly specialized, systemic cells and processes that eliminate or prevent pathogenic growth. The adaptive immune response provides the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of that cell will then inherit genes encoding the same receptor specificity, including the Memory B cells and Memory T cells that are the keys to long-lived specific immunity. Immune network theory is a theory of how the adaptive immune system works, that is based on interactions between the variable regions of the receptors of T cells, B cells and of molecules made by T cells and B cells that have variable regions.

Innate immune system: The innate immune system, also known as non-specific immune system, comprises the cells and mechanisms that defend the host from infection by other organisms in a non-specific manner. This means that the cells of the innate system recognize and respond to pathogens in a generic way, but unlike the adaptive immune system, it does not confer long-lasting or protective immunity to the host. The innate immune system may be e.g. activated by ligands of pathogen-associated molecular patterns

(PAMP) receptors, e.g. Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. Typically a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system through a process known as antigen presentation; and/or acting as a physical and chemical barrier to infectious agents.

Cellular immunity/cellular immune response: Cellular immunity relates typically to the activation of macrophages, natural killer cells (NK), antigen-specific cytotoxic T-lymphocytes, and the release of various cytokines in response to an antigen. In a more general way, cellular immunity is not related to antibodies but to the activation of cells of the immune system. A cellular immune response is characterized e.g. by activating antigen-specific cytotoxic T-lymphocytes that are able to induce apoptosis in body cells displaying epitopes of an antigen on their surface, such as virus-infected cells, cells with intracellular bacteria, and cancer cells displaying tumor antigens; activating macrophages and natural killer cells, enabling them to destroy pathogens; and stimulating cells to secrete a variety of cytokines that influence the function of other cells involved in adaptive immune responses and innate immune responses.

Humoral immunity/humoral immune response: Humoral immunity refers typically to antibody production and the accessory processes that may accompany it. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Antigen: According to the present invention, the term "antigen" refers to a substance which is recognized by the immune system and is capable of triggering an antigen-specific immune response, e.g. by formation of antibodies or antigen-specific T-cells as part of an adaptive immune response. Typically, an antigen is a protein or peptide, but may also be a sugar, lipid, nucleic acid etc. structure. In this context, the first step of an adaptive immune response is the activation of naive antigen-specific T cells by antigen-presenting cells. This occurs in the lymphoid tissues and organs through which naive T cells are constantly passing. The three cell types that can serve as antigen-presenting cells are dendritic cells, macrophages, and B cells. Each of these cells has a distinct function in eliciting immune responses. Tissue dendritic cells take up antigens by phagocytosis and macropinocytosis and are stimulated by infection to migrate to the local lymphoid tissue, where they differentiate into mature dendritic cells. Macrophages ingest particulate antigens such as bacteria and are induced by infectious agents to express MHC class II molecules. The unique ability of B cells to bind and internalize soluble protein antigens via their receptors may be important to induce T cells. By presenting the antigen on MHC molecules leads to activation of T cells which induces their proliferation and differentiation into armed effector T cells. The most important function of effector T cells is the killing of infected cells by $CD8^+$ cytotoxic T cells and the activation of macrophages by TH1 cells which together make up cell-mediated immunity, and the activation of B cells by both TH2 and TH1 cells to produce different classes of antibody, thus driving the humoral immune response. T cells recognize an antigen by their T cell receptors which does not recognize and bind antigen directly, but instead recognize short peptide fragments e.g. of pathogens' protein antigens, which are bound to MHC molecules on the surfaces of other cells.

T cells fall into two major classes that have different effector functions. The two classes are distinguished by the expression of the cell-surface proteins CD4 and CD8. These two types of T cells differ in the class of MHC molecule that they recognize. There are two classes of MHC molecules - MHC class I and MHC class II molecules - which differ in their structure and expression pattern on tissues of the body. $CD4^+$ T cells bind to a MHC class II molecule and $CD8^+$ T cells to a MHC class I molecule. MHC class I and MHC class II molecules have distinct distributions among cells that reflect the different effector functions of the T cells that recognize them. MHC class I molecules present peptides from pathogens, commonly viruses to $CD8^+$ T cells, which differentiate into cytotoxic T cells that are specialized to kill any cell that they specifically recognize. Almost all cells express MHC class I molecules, although the level of constitutive expression varies from one cell type to the next. But not only pathogenic peptides from viruses are presented by MHC class I molecules, also self-antigens like tumour antigens are presented by them. MHC class I molecules bind peptides from proteins degraded in the cytosol and transported in the endoplasmic reticulum. Thereby MHC class I molecules on the surface of cells infected with viruses or other cytosolic pathogens display peptides from these pathogen. The $CD8^+$ T cells that recognize MHC class I:peptide complexes are specialized to kill any cells displaying foreign peptides and so rid the body of cells infected with viruses and other cytosolic pathogens. The main function of $CD4^+$ T cells ($CD4^+$ helper T cells) that recognize MHC class II molecules is to activate other effector cells of the immune system. Thus MHC class II molecules are normally found on B lymphocytes, dendritic cells, and macrophages, cells that participate in immune responses, but not on other tissue cells. Macrophages, for example, are activated to kill the intravesicular pathogens they harbour, and B cells to secrete immunoglobulins against foreign molecules. MHC class II molecules are prevented from binding to peptides in the endoplasmic reticulum and thus MHC class II molecules bind peptides from proteins which are degraded in endosomes. They can capture peptides from pathogens that have entered the vesicular system of macrophages, or from antigens internalized by immature dendritic cells or the immunoglobulin receptors of B cells. Pathogens that accumulate in large numbers inside macrophage and dendritic cell vesicles tend to stimulate the differentiation of TH1 cells, whereas extracellular antigens tend to stimulate the production of TH2 cells. TH1 cells activate the microbicidal properties of macrophages and induce B cells to make IgG antibodies that are very effective of opsonising extracellular pathogens for ingestion by phagocytic cells, whereas TH2 cells initiate the humoral response by activating naive B cells to secrete IgM, and induce the production of weakly opsonising antibodes such as IgG1 and IgG3 (mouse) and IgG2 and IgG4 (human) as well as IgA and IgE (mouse and human).

Vaccine: A vaccine is typically understood to be a prophylactic or therapeutic material providing at least one antigen or antigenic function. The antigen or antigenic function stimulates the body's adaptive immune system to provide an adaptive immune response.

Immunostimulating agent: The term "immunostimulating agent" is typically understood not to include agents as e.g. antigens (of whatever chemical structure), which elicit an adaptive/cytotoxic immune response, e.g. a "humoral" or "cellular" immune response, in other words elicit immune reponses (and confer immunity by themselves) which are characterized by a specific response to structural properties of an antigen recognized to be foreign by immune competent cells. Rather, by "immunostimulating agent", it is typically understood to mean agents/compounds/complexes which do not trigger any adaptive/cytotoxic immune response by themselves, but which may exlusively enhance such an adaptive/cytotoxic immune reponse in an unspecific way, by e.g. activating "PAMP" receptors and thereby triggering the release of cytokines which support the actual adaptive/cytotoxic immune response. Accordingly, any immunostimulation by agents (e.g. antigens) which evoke an adaptive and/or cytotoxic immune response by themselves (conferring immunity by themselves directly or indirectly) is typically disclaimed by the phrase "immunostimulating agent".

Adjuvant: The term "adjuvant" is also understood not to comprise agents which confer immunity by themselves. Accordingly, adjuvants do not by themselves typically confer immunity, but assist the immune system in various ways to enhance the antigen-specific immune response by e.g. promoting presentation of an antigen to the immune system. Hereby, an adjuvant may preferably e.g. modulate the antigen-specific immune response by e.g. shifting the dominating Th1-based antigen specific response to a more Th2-based antigen specific response or vice versa. Accordingly, the terms "immunostimulating agent" and "adjuvant" in the context of the present invention are typically understood to mean agents, compounds or complexes which do not confer immunity by themselves, but exclusively support the immune reponse in an unspecific way (in contrast to an antigen-specific immune response) by effects, which modulate the antigen-specific (adaptive cellular and/or humoral immune response) by unspecific measures, e.g. cytokine expression/secretion, improved antigen presentation, shifting the nature of the arms of the immune response etc.. Accordingly, any agents evoking by themselves immunity are typically disclaimed by the terms "adjuvant" or "immunostimulating agent".

Immunostimulatory RNA: An immunostimulatory RNA (isRNA) in the context of the invention may typically be a RNA that is able to induce an innate immune response itself. It usually does not have an open reading frame and thus does not provide a peptide-antigen but elicits an innate immune response e.g. by binding to a specific kind of pathogen-associated molecular patterns (PAMP) receptors (e.g. Toll-like-receptor (TLR) or other suitable receptors). However, of course also mRNAs having an open reading frame and coding for a peptide/protein (e.g. an antigenic function) may induce an innate immune response.

Fragment of a sequence: a fragment of a sequence is typically a shorter portion of a full-length sequence of e.g. a nucleic acid sequence or an amino acid sequence. Accordingly, a fragment of a sequence, typically, consists of a sequence that is identical to the corresponding stretch or corresponding stretches within the full-length sequence. A preferred fragment of a sequence in the context of the present invention, consists of a continuous stretch of entities, such as nucleotides or amino acids, corresponding to a continuous stretch of entities in the molecule the fragment is derived from, which represents at least 5%, preferably at least 20%, preferably at least 30%, more preferably at least 40%, more preferably at least 50%, even more preferably at least 60%, even more preferably at least 70%, and most preferably at least 80% of the total (i.e. full-length) molecule from which the fragment is derived. Thus, for example, a fragment of a protein or peptide antigen preferably corresponds to a continuous stretch of entities in the protein or peptide antigen the fragment is derived from, which represents at least 5%, preferably at least 20%, preferably at least 30%, more preferably at least 40%, more preferably at least 50%, even more preferably at least 60%, even more preferably at least 70%, and most preferably at least 80% of the total (i.e. full-length) protein or peptide antigen. It is particularly preferred that the fragment of a sequence is a functional fragment, i.e. that the fragment fulfills one or more of the functions fulfilled by the sequence the fragment is derived from. For example, a fragment of a protein or peptide antigen preferably exhibits at least one antigenic function (e.g. is capable of eliciting a specific immune reaction against at least one antigen determinant in said protein or peptide antigen) of the protein or peptide antigen the fragment is derived from.

Fragments of proteins: "Fragments" of proteins or peptides, i.e., fragments of amino acid sequences, in the context of the present invention may comprise a sequence of a protein or peptide as defined herein, which is, with regard to its amino acid sequence (or its encoding nucleic acid molecule), N-terminally, C-terminally and/or intrasequentially truncated compared to the amino acid sequence of the original (native) protein (or its encoded nucleic acid molecule). Such truncation may thus occur either on the amino acid level or correspondingly on the nucleic acid level. A sequence identity with respect to such a fragment as defined herein may therefore preferably refer to the entire protein or peptide as defined herein or to the entire (coding) nucleic acid molecule of such a protein or peptide.

Likewise, "fragments" of nucleic acid sequences in the context of the present invention may comprise a sequence of a nucleic acid as defined herein, which is, with regard to its nucleic acid molecule 5'-, 3'- and/or intrasequentially truncated compared to the nucleic acid molecule of the original (native) nucleic acid molecule. A sequence identity with respect to such a fragment as defined herein may therefore preferably refer to the entire nucleic acid as defined herein.

Preferred fragments of proteins or peptides in the context of the present invention may furthermore comprise a sequence of a protein or peptide as defined herein, which has a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These preferred fragments are typically recognized by T-cells in form of a complex consisting of the peptide fragment and an MHC molecule, i.e. the fragments are typically not recognized in their native form. Fragments of proteins or peptides may comprise at least one epitope of those proteins or peptides. Furthermore, also domains of a protein, like the extracellular domain, the intracellular domain or the transmembrane domain and shortened or truncated versions of a protein may be understood to comprise a fragment of a protein.

Epitope (also called "antigen determinant"): T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form.

B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Variant: A variant of an entity, such as a variant of a sequence, e.g. of a nucleotide or amino acid sequence, refers to a modified entity, such as a modified sequence, e.g. a modified nucleotide or amino acid sequence. For example, a variant of a sequence may exhibit one or more nucleotide or amino acid deletions, insertions, additions and/or substitutions compared to the sequence the variant is derived from. Preferably, a variant of a sequence in the context of the present invention is at least 40%, preferably at least 50%, more preferably at least 60%, more preferably at least 70%, even more preferably at least 80%, even more preferably at least 90%, most preferably at least 95% identical to the sequence the variant is derived from. Accordingly, a variant of a peptide or protein antigen in the context of the present invention is preferably at least 40%, preferably at least 50%, more preferably at least 60%, more preferably at least 70%, even more preferably at least 80%, even more preferably at least 90%, most preferably at least 95% identical to the sequence of the protein or peptide antigen the variant is derived from. Preferably, the variant is a functional variant, i.e.

that the variant fulfills one or more of the functions fulfilled by the sequence the variant is derived from. For example, a variant of a protein or peptide antigen preferably exhibits at least one antigenic function (e.g. is capable of eliciting a specific immune reaction against at least one antigen determinant in said protein or peptide antigen) of the protein or peptide antigen the variant is derived from.

"Variants" of proteins or peptides as defined in the context of the present invention may be generated, having an amino acid sequence which differs from the original sequence in one or more mutation(s), such as one or more substituted, inserted and/or deleted amino acid(s). Preferably, these fragments and/or variants have the same biological function or specific activity compared to the full-length native protein, e.g. its specific antigenic property. "Variants" of proteins or peptides as defined in the context of the present invention may comprise, e.g., conservative amino acid substitution(s) compared to their native, i.e. non-mutated physiological, sequence. Those amino acid sequences as well as their encoding nucleotide sequences in particular fall under the term variants as defined herein. Substitutions in which amino acids, which originate from the same class, are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).

Additionally, variants of proteins or peptides may comprise peptide analogues as defined herein. Furthermore, variants of proteins or peptides as defined herein, which may be encoded by a nucleic acid molecule, may also comprise those sequences, wherein nucleotides of the nucleic acid are exchanged according to the degeneration of the genetic code, without leading to an alteration of the respective amino acid sequence of the protein or peptide, i.e. the amino acid sequence or at least part thereof may not differ from the original sequence in one or more mutation(s) within the above meaning.

Sequence identity: In order to determine the percentage to which two sequences are identical, e.g. nucleic acid sequences or amino acid sequences as defined herein, the sequences can be aligned in order to be subsequently compared to one another. Therefore, e.g. a position of a first sequence may be compared with the corresponding position of the second sequence. If a position in the first sequence is occupied by the same component as is the case at a position in the second sequence, the two sequences are identical at this position. If this is not the case, the sequences differ at this position. If insertions occur in the second sequence in comparison to the first sequence, gaps can be inserted into the first sequence to allow a further alignment. If deletions occur in the second sequence in comparison to the first sequence, gaps can be inserted into the second sequence to allow a further alignment. The percentage to which two sequences are identical is then a function of the number of identical positions divided by the total number of positions including those positions which are only occupied in one sequence. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic Acids Res., 25:3389-3402. Such an algorithm is integrated in the BLAST program. Sequences which are identical to the sequences of the present invention to a certain extent can be identified by this program. A "variant" of a protein or peptide may have,e.g., at least 70%, 75%, 80%, 85%, 90%, 95%, 98% or 99% amino acid identity over a stretch of 10, 20, 30, 50, 75 or 100 amino acids, preferably over the full length sequence, of such protein or peptide. Analogously, a "variant" of a nucleic acid sequence may have, e.g., at least 70%, 75%, 80%, 85%, 90%, 95%, 98% or 99% nucleotide identity over a stretch of 10, 20, 30, 50, 75 or 100 nucleotides, preferably over the full length sequence, of such nucleic acid sequence.

Derivative of a protein or peptide: A derivative of a peptide or protein is a molecule that is derived from another molecule, such as said peptide or protein. A "derivative" of a peptide or protein also encompasses fusions comprising a peptide or protein used in the present invention. For example, the fusion comprises a label, such as, for example, an epitope, e.g., a FLAG epitope or a V5 epitope or an HA epitope. For example, the epitope is a FLAG epitope. Such a tag is useful for, for example, purifying the fusion protein. The term "derivative" of a peptide or protein also encompasses a derivatised peptide or protein, such as, for example, a peptide or protein modified to contain one or more-chemical moieties other than an amino acid. The chemical moiety may be linked covalently to the peptide or protein e.g., via an amino terminal amino acid residue, a carboxyl terminal amino acid residue, or at an internal amino acid residue. Such modifications include the addition of a protective or capping group on a reactive moiety in the peptide or protein, addition of a detectable label, and other changes that do not adversely destroy the activity of the peptide or protein compound. For example, a derivative may comprise a PEG moiety, radionuclide, coloured latex, etc. A derivative generally possesses or exhibits an improved characteristic relative to a e.g., enhanced protease resistance and/or longer half-life and/or enhanced transportability between cells or tissues of the human or animal body and/or reduced adverse effect(s) and/or enhanced affinity or immunogenicity. WO 2010/003193 describes various methodologies to provide peptide or protein derivatives which may be employed separately or in combination using standard procedures known to the person of ordinary skill, including derivatisation of a protein or peptide by e.g. PEGylation, HESylation, or glycosylation.

According to a first aspect, one or more objects underlying the present invention are solved by a pharmaceutical composition comprising:

(A) a complex, comprising:

a) at least one cationic and/or polycationic peptide or protein or a polyimine as a carrier,
and
b) at least one nucleic acid molecule as a cargo, wherein the at least one nucleic acid molecule is RNA,
wherein the cationic and/or polycationic component of the carrier and the nucleic acid molecule comprised as cargo in said complex are provided in an N/P ratio in the range of 0.4-0.9,
and

(B) at least one protein or peptide antigen, that is selected from the group consisting of:

(i) an antigen from a pathogen associated with infectious disease;
(ii) an antigen associated with allergy or allergic disease;
(iii) an antigen associated with autoimmune disease; and
(iv) an antigen associated with a cancer or tumour disease,
or

a functional fragment, variant and/or derivative of said protein or peptide antigen,
wherein the at least one protein or peptide antigen is provided as a separate component of the pharmaceutical composition and wherein component (B) is not ovalbumine or the ovalbumine-derived peptide SIINFEKL.

The cationic and/or polycationic components and the nucleic acid molecule comprised in said complex (A) are provided in an N/P ratio of below 1, i.e. in the in the range of 0.4-0.9, or in the range of 0.5-0.9. In some embodiments, the cationic and/or polycationic components and the nucleic acid molecule comprised in said complex (A) are provided in an N/P ratio of below 0.7, such as below 0.6, i.e. in the range of 0.4-0.6.

Generally, if the N/P ratio of the cationic and/or polycationic components and the nucleic acid molecule is below 1, the complex formed by the cationic and/or polycationic components and the nucleic acid molecule is negatively charged, thus, its empirically determined zetapotential is usually negative (within the scope of typical measurement errors).

Preferably, the charge of complex (A) is negative, preferably the zetapotential of complex (A) (measured as defined herein) is negative, i.e. below 0 mV, preferably below -1 mV, more preferably below -2 mV, even more preferably below -3 mV, and most preferably below -4 mV, such as between about -1 mV and -50 mV, between about -2 mV and -40 mV, or between about -5 mV and -30 mV.

The at least one nucleic acid molecule of complex (A) of the pharmaceutical compositions according to the present invention is not a CpG-DNA.The at least one nucleic acid molecule of complex (A) of the pharmaceutical compositions according to the present invention is not an oligodeoxynucleotide (ODN) containing one or more cytosine-guanine dinucleotides (CpG). Thus, the at least one nucleic acid molecule is not a CpG-ODN.The at least one nucleic acid molecule does not comprise or consist of the sequence 5'-TCC ATG ACG TTC CTG ATG CT-3' (SEQ ID NO: 100). Preferably, the at least one nucleic acid molecule is at least 21, preferably at least 25, preferably at least 30, more preferably at least 50 nucleotides in length. The at least one nucleic acid molecule is RNA, preferably an isRNA, for example, comprising or consisting of a sequence according to any one of Formulas II-V as defined herein, such as a sequence selected from the group consisting of SEQ ID NOs: 1-94 and 101 or a sequence which is at least 60%, preferably at least 70%, more preferably at least 80%, even more preferably at least 90%, and most preferably at least 95% identical to a sequence according to any one of SEQ ID NOs: 1-94 and 101, e.g. a sequence according to SEQ ID NO: 91 or 101 or a sequence which is at least 60%, preferably at least 70%, more preferably at least 80%, even more preferably at least 90%, and most preferably at least 95% identical to a sequence according to SEQ ID NO: 91 or 101.

In certain embodiments, the complex is for use as an adjuvant. For example, it is used as an adjuvant, and/or has adjuvant properties, as may be readily determined by the person of ordinary skill using routine methodologies, and including methodologies as described herein.

As a first ingredient the inventive pharmaceutical composition includes (e.g. as an adjuvant) at least one complex (A), comprising

a) as a carrier at least one cationic and/or polycationic peptide or protein or a polyimine and
b) as a cargo at least one nucleic acid molecule, wherein the at least one nucleic acid molecule is RNA;

wherein the cationic and/or polycationic component of the carrier and the nucleic acid molecule comprised as cargo in said complex are provided in an N/P ratio in the range of 0.4-0.9.

The cationic and/or polycationic components of the carrier and the nucleic acid molecule cargo comprised in said complex are provided in an N/P ratio in the range of 0.4-0.9, more specifically in the range of 0.5-0.9, or in the range of 0.4-0.6.

The charge of complex (A) is negative, preferably wherein the zetapotential of complex (A) (measured as defined herein) is negative, i.e. below 0 mV, preferably below -1 mV, more preferably below -2 mV, even more preferably below -3 mV, and most preferably below -4 mV, such as between about -1 mV and -50 mV, between about -2 mV and -40 mV, or between about -5 mV and -30 mV.

The complex comprised in the inventive pharmaceutical composition allows provision of a more efficient adjuvant for vaccination purposes. Advantageously, the complex is suited for *in vivo* delivery of nucleic acids, particularly to antigen-presenting cells (e.g. $CD19^+$ cells like B cells and follicular dendritic cells). The inventors surprisingly found that complexes comprising as a carrier cationic and/or polycationic components and as a cargo at least one nucleic acid molecule, wherein the cationic and/or polycationic components of the carrier and the nucleic acid molecule cargo comprised in said complex are provided in an N/P ratio in the range of 0.4-0.9, or more specifically in the range of 0.5-0.9; are preferably taken up by antigen-presenting cells (e.g. $CD19^+$ cells). This N/P ratio below 1 leads to a negative charge of the complexes which leads to a preferred uptake into $CD19^+$ cells, whereas positively charged complexes (which is the result of a N/P ratio higher than 1) are preferably taken up by $CD3^+$ cells (e.g. T cells). Therefore, these negatively charged complexes are preferably suited for adjuvant purposes because they can target particularly antigen-presenting cells, which are the most important cells for initiating an adaptive immune response. Furthermore, these negatively charged complexes preferably induce the anti-viral cytokine IFNalpha and consequently a Th1-shifted immune response. Therefore, these negatively charged complexes are particularly appropriate for the prophylactic or therapeutic treatment of diseases which is dependent on the induction of a Th1-shifted immune response (e.g. tumour or cancer diseases or infectious diseases like RSV infections) and for the use as adjuvant for protein or peptide antigens which mainly induce a Th2-shifted immune response.

In this context, the cationic and/or polycationic components, which form basis for the carrier of the complex, are selected from any suitable cationic or polycationic peptide, protein or polyimine suitable for this purpose, i.e. any cationic or polycationic peptide, protein or polyimine capable to complex a nucleic acid as defined according to the present invention, and thereby preferably condensing the nucleic acid. The cationic or polycationic peptide, protein or polyimine and, optionally, other additional polymers, is preferably a linear molecule, however, branched cationic or polycationic peptides, proteins or polyimines and, optionally, other additional polymers may also be used.

According to one first alternative, at least one cationic (or polycationic) component of the carrier may be selected from cationic or polycationic peptides or proteins. Such cationic or polycationic peptides or proteins preferably exhibit a length of about 3 to 100 amino acids, preferably a length of about 3 to 50 amino acids, more preferably a length of about 3 to 25 amino acids, e.g. a length of about 3 to 10; 5 to 20; 5 to 15; 8 to 15, 16 or 17; 10 to 15, 16, 17, 18, 19, or 20; or 15 to 25 amino acids. Alternatively or additionally, such cationic or polycationic peptides or proteins may exhibit a molecular weight of about 0.01 kDa to about 100 kDa, including a molecular weight of about 0.5 kDa to about 100 kDa, preferably of about 10 kDa to about 50 kDa, even more preferably of about 10 kDa to about 30 kDa. In this context also analogues and derivatives of proteins or peptides as defined herein are explicitly encompassed.

In the specific case that the cationic component of the carrier comprises or consists of a cationic or polycationic peptide or protein, the cationic properties of the cationic or polycationic peptide or protein or of the entire carrier, if the carrier is composed of cationic or polycationic peptides or proteins, may be determined based on its content of cationic amino acids, in particular based on its content of cationic amino acids in excess over anionic and neutral amino acids at a given pH (determined by the respectively pKs values of the acidic or basic residues), and thus, based on its net positive charge. Preferably, the content of cationic amino acids in the cationic or polycationic peptide or protein and/or the carrier is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even more preferably in the range of about 20% to 50%, e.g. 20, 30, 40 or 50%, or in a range formed by any two of the afore mentioned values, provided, that the content of all amino acids, e.g. cationic, lipophilic, hydrophilic, aromatic and further amino acids, in the cationic or polycationic peptide or protein, or in the entire carrier, if the carrier is entirely composed of cationic or polycationic peptides or proteins, is 100%.

In this context, cationic amino acids are preferably the naturally occurring amino acids Arg (Arginine), Lys (Lysine), His (Histidine), and Orn (Ornithin). However, in a broader sense any (non-natural) amino acid carrying a cationic charge on its side chain may also be envisaged to carry out the invention. However, those cationic amino acids are preferred which comprise side chains which are positively charged under physiological pH conditions. In a more preferred embodiment, these amino acids are Arg, Lys, and Orn.

Preferably, such cationic or polycationic peptides or proteins of the carrier, are selected from, without being restricted thereto, cationic peptides or proteins such as protamine, nucleoline, spermine or spermidine, oligo- or poly-L-lysine (PLL), basic polypeptides, oligo or poly-arginine, cell penetrating peptides (CPPs), chimeric CPPs, such as Transportan, or MPG peptides, HIV-binding peptides, Tat, HIV-1 Tat (HIV), Tat-derived peptides, members of the penetratin family, e.g. Penetratin, Antennapedia-derived peptides (particularly from *Drosophila antennapedia*), pAntp, pIsl, etc., antimicrobial-derived CPPs e.g. Buforin-2, Bac715-24, SynB, SynB(1), pVEC, hCT-derived peptides, SAP, MAP, KALA, PpTG20, Loligomere, FGF, Lactoferrin, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc.

In an alternative embodiment, cationic or polycationic peptides or proteins of the carrier do not consist of, preferably do not comprise any of the following: polylysine, polyarginine, defensins, cathelicidin, HIV-REV, HIV-TAT, antennapedia peptides, cathelin, synthetic peptides containing at least two KLK-motifs separated by a linker of 3 to 7 hydrophobic amino acids, and cationic peptides derived from said proteins.

In some embodiments, the cationic or polycationic peptides or proteins of the carrier do not consist of, preferably do not comprise, polylysine or polyarginine. In particular, it is preferred that the cationic or polycationic peptides or proteins of the carrier do not consist of, preferably do not comprise, poly-L-arginine with an average degree of polymerization of 60 arginine residues.

Furthermore, it is preferred that in embodiments, wherein the cationic or polycationic peptides or proteins of the carrier comprise polylysine or polyarginine peptides or proteins, said peptides or proteins comprise amino acids other than lysines and/or arginines. For example, it is preferred that if the cationic or polycationic peptides or proteins of the carrier comprise polyarginine and/or polylysine peptides, said peptides further comprise at least one cysteine residue.

Alternatively or additionally, such cationic or polycationic peptides or proteins of the carrier, are selected from, without being restricted thereto, following cationic peptides having the following sum formula (I):

$$\{(Arg)_l;(Lys)_m;(His)_n;(Orn)_o;(Xaa)_x\};$$

wherein [ + m + n + o + x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred. Even more preferred peptides of this formula are oligoarginines such as e.g. $Arg_7$, $Arg_8$, $Arg_9$, $Arg_{12}$, His3Arg9, Arg9His3, His3Arg9His3, $His_6Arg_9His_6$, His3Arg4His3, His6Arg4His6, $TyrSer_2Arg_9Ser_2Tyr$, (ArgLysHis)4, Tyr(ArgLysHis)2Arg, etc.

According to a particular preferred embodiment, such cationic or polycationic peptides or proteins of the carrier having the empirical sum formula (I) as shown above, may, without being restricted thereto, comprise at least one of the following subgroup of formulae:

Arg7, $Arg_8$, $Arg_9$, $Arg_{10}$, $Arg_{11}$, $Arg_{12}$, $Arg_{13}$, $Arg_{14}$, $Arg_{15-30}$;
$Lys_7$, $Lys_8$, $Lys_9$, $Lys_{10}$, $Lys_{11}$, $Lys_{12}$, $Lys_{13}$, $Lys_{14}$, $Lys_{15-30}$;
$His_7$, $His_8$, $His_9$, $His_{10}$, $His_{11}$, $His_{12}$, $His_{13}$, $His_{14}$, $His_{15-30}$;
$Orn_7$, $Orn_8$, $Orn_9$, $Orn_{10}$, $Orn_{11}$, $Orn_{12}$, $Orn_{13}$, $Orn_{14}$, $Orn_{15-30}$.

According to a further particularly preferred embodiment, cationic or polycationic peptides or proteins of the carrier, having the empirical sum formula (I) as shown, may be preferably selected from, without being restricted thereto, at least one of the following subgroup of formulae. The following formulae do not specify any amino acid order, but are intended to reflect empirical formulae by exclusively specifying the (number of) amino acids as components of the respective peptide. Accordingly, as an example, empirical formula $Arg_{(7-29)}Lys_1$ is intended to mean that peptides falling under this formula contain 7 to 19 Arg residues and 1 Lys residue of whatsoever order. If the peptides contain 7 Arg residues and 1 Lys residue, all variants having 7 Arg residues and 1 Lys residue are encompassed. The Lys residue may therefore be positioned anywhere in the e.g. 8 amino acid long sequence composed of 7 Arg and 1 Lys residues. The subgroup preferably comprises:

Arg(4-29)Lysi, $Arg_{(4-29)}His_1$, $Arg_{(4-29}Orn_1$, $Lys_{(4-29)}His_1$, $Lys_{(4-29)}Orn_1$, $His_{(4-29)}Orn_1$, Arg(3-28)Lys2, $Arg_{(3-28)}His_2$, $Arg_{(3-28)}Orn_2$, $Lys_{(3-28)}His_2$, $Lys_{(3-28)}Orn_2$, $His_{(3-28)}Orn_2$, $Arg_{(2-27)}Lys_3$, $Arg_{(2-27)}His_3$, $Arg_{(2-27)}Orn_3$, $Lys_{(2-27)}His_3$, Lys(2-27)Orn3, $His_{(2-27)}Orn_3$, $Arg_{(1-26)}Lys_4$, $Arg_{(1-26)}HiS_4$, $Arg_{(1-26)}Orn_4$, $Lys_{(1-26)}His_4$, $LyS_{(1-26)}Orn_4$, $His_{(1-26)}Orn_4$,

$Arg_{(3-28)}Lys_1His_1$, $Arg_{(3-28)}Lys_1Orn_1$, $Arg_{(3-28)}His_1Orn_1$, $Arg_1Lys_{(3-28)}His_1$, $Arg_1Lys_{(3-28)}Orn_1$, $Lys_{(3-28)}His_1Orn_1$, $Arg_1$ $Lys_1$ $His_{(3-28)}$, $Arg_1$ $His_{(3-28)}Orn_1$, $Lys_1His_{(3-28)}Orn_1$; $Arg_{(2-27)}Lys_2His_1$, Arg($_{2-27)}Lys_1His_2$, $Arg_{(2-27)}Lys_2Orn_1$, $Arg_{(2-27)}Lys_1Orn_2$, $Arg_{(2-27)}His_2Orn_1$, $Arg_{(2-27)}His_1Orn_2$, Arg2Lys(2-27)Hisi, $Arg_1Lys_{(2-27)}His_2$, $Arg_2Lys_{(2-27)}Orn_1$, $Arg_1Lys_{(2-27)}Orn_2$, $Lys_{(2-27)}His_2Orn_1$, $Lys_{(2-27)}His_1Orn_2$, $Arg_2Lys_1His_{(2-27)}$, $Arg_1Lys_2His_{(2-27)}$, $Arg_2His_{(2-27)}Orn_1$, $Arg_1His_{(2-27)}Orn_2$, $Lys_2His_{(2-27)}Orn_1$, $Lys_1His_{(2-27)}Orn_2$;

$Arg_{(1-26)}Lys_3His_1$, $Arg_{(1-26)}Lys_2His_2$, $Arg_{(1-26)}Lys_1His_3$, $Arg_{(1-26)}Lys_3Orn_1$, $Arg_{(1-26)}Lys_2Orn_2$, $Arg_{(1-26)}Lys_1Orn_3$, $Arg_{(1-26)}His_3Orn_1$, $Arg_{(1-26)}His_2Orn_2$, $Arg_{(1-26)}His_1Orn_3$, $Arg_3Lys_{(1-26)}His_1$, $Arg_2Lys_{(1-26)}His_2$, $Arg_1Lys_{(1-26)}His_3$, $Arg_3LyS_{(1-26)}Orn_1$, $Arg_2Lys_{(1-26)}Orn_2$, $Arg_1Lys_{(1-26)}Orn_3$, $Lys_{(1-26)}HiS_3Orn_1$, $Lys_{(1-26)}His_2Orn_2$, $Lys_{(1-26)}His_1Orn_{3,}$ $Arg_3Lys_1His_{(1-26)}$, $Arg_2Lys_2His_{(1-26),}$ $Arg_1Lys_3His_{(1-26),}$ $Arg_3His_{(1-26)}Orn_1$, $Arg_2His_{(1-26)}Orn_2$, $Arg_1His_{(1-26)}Orn_3$, $Lys_3His_{(1-26)}Orn_1$, $Lys_2His_{(1-26)}Orn_2$, $Lys_1His_{(1-26)}Orn_3$;

$Arg_{(2-27)}Lys_1His_1Orn_1$, $Arg_1Lys_{(2-27)}His_1Orn_1$, $Arg_1Lys_1His_{(2-27)}Orn_1$, $Arg_1Lys_1His_1Orn_{(2-27)}$;

$Arg_{(1-26)}$ $Lys_2His_1Orn_1$, $Arg_{(1-26)}Lys_1His_2Orn_1$, $Arg_{(1-26)}Lys_1His_1Orn_2$, $Arg_2Lys_{(1-26)}His_1Orn_1$, $Arg_1Lys_{(1-26)}His_2Orn_1$, $Arg_1Lys_{(1-26)}His_1$Orn2, $Arg_2Lys_1His_{(1-26)}Orn_1$, $Arg_1Lys_2HiS_{(1-26)}Orn_1$, $Arg_1Lys_1His_{(1-26)}Orn_2$, $Arg_2Lys_1His_1Orn_{(1-26)}$, $Arg_1Lys_2His_1Orn_{(1-26)}$, $Arg_1Lys_1His_2Orn_{(1-26)}$;

According to a further particular preferred embodiment, cationic or polycationic peptides or proteins of the carrier, having the empirical sum formula (I) as shown above may be, without being restricted thereto, selected from the subgroup consisting of generic formulae Arg7 (also termed as $R_{7;}$ SEQ ID NO. 95), $Arg_9$ (also termed $R_9$, SEQ ID NO. 96), $Arg_{12}$ (also termed as $R_{12}$, SEQ ID NO. 97).

In certain embodiments of all aspects of the invention the cationic and/or polycationic components of the carrier are not selected from cationic and/or polycationic components containing at least one -SH moiety. Therefore, the complex may not consist of or may not comprise a carrier formed by disulfide-crosslinked cationic and/or polycationic components.

Said cationic or polycationic peptides or proteins may be prepared by all methods known to a person of ordinary skill or by recombinant peptide or protein production or by peptide synthesis as described herein.

Alternatively, the at least one cationic or polycationic compound may be a (non-peptidic) cationic or polycationic polymer, which is selected from a polyimine, e.g. poly(ethylene imine) PEI or poly(propylene imine).

It is also disclosed that the at least one cationic (or polycationic) component of the carrier may be selected from e.g. any (non-peptidic) cationic or polycationic polymer suitable in this context. Thus, likewise as defined herein, the carrier may comprise the same or different cationic or polycationic polymers.

In the specific case that the cationic component of the carrier comprises a (non-peptidic) cationic or polycationic polymer the cationic properties of the (non-peptidic) cationic or polycationic polymer may be determined upon its content of cationic charges when compared to the overall charges of the components of the cationic polymer. Preferably, the content of cationic charges, preferably the net cationic charges (i.e. upon subtraction of anionic charges), in the cationic polymer at a (physiological) pH as defined herein is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 30% to 100%, even preferably in the range of about 50% to 100%, e.g. 50, 60, 70, 80%, 90% or 100%, or in a range formed by any two of the afore mentioned values, provided, that the content of all charges, e.g. positive and negative charges at a (physiological) pH as defined herein, in the entire cationic polymer is 100%.

Preferably, the (non-peptidic) cationic component of the carrier represents a cationic or polycationic polymer, typically exhibiting a molecular weight of about 0.1 or 0.5 kDa to about 100 kDa, preferably of about 1 kDa to about 75 kDa, more preferably of about 5 kDa to about 50 kDa, even more preferably of about 5 kDa to about 30 kDa, or a molecular weight of about 10 kDa to about 50 kDa, even more preferably of about 10 kDa to about 30 kDa.

According to the present invention as defined by the attached claims, polyimine (such as poly(ethylene imine) PEI or poly(propylene imine)) is used as a carrier. In certain non-claimed embodiments further disclosed herein, the (non-peptidic) cationic component of the carrier may be selected from cationic polysaccharides, for example chitosan, polybrene, cationic polymers, cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, Lipofectamine®, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyallylamine, sugar backbone based polymers, such as cyclodextrin based polymers, dextran based polymers, Chitosan, etc., silan backbone based polymers, such as PMOXA-PDMS copolymers, etc., blockpolymers consisting of a combination of one or more cationic blocks (e.g. selected of a cationic polymer as mentioned above) and of one or more hydrophilic or hydrophobic blocks (e.g polyethyleneglycole); etc.

In some embodiments disclosed herein, the non-peptidic cationic component does not consist of, preferably does not comprise any of the following: chitosan, derivatives of chitin, or fragments thereof.

If the cationic component of the carrier disclosed herein is a non-peptidic cationic component or comprises a non-peptidic cationic component, it is particularly preferred that the non-peptidic cationic component is based on lipids, preferably on liposomes, i.e. that the non-peptidic cationic component comprises or consists of a lipidic cationic component. Thus, in a preferred aspect of the disclosure, the cationic component of the carrier comprises or consists of lipids, preferably liposomes or micelles. Said lipidic cationic component or liposomes or micelles may be composed, e.g. of a mixture of lipids, for example, of a mixture of cationic and neutral lipids. Any lipid based compositions available for transfection of mammalian cells may, for example, be used as non-peptidic cationic component of the carrier in the context of the present disclosure. Examples for such lipidic cationic components are cationic components comprising or consisting of DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, Lipofectamine®, or any lipid consisting of 1-4 alkylchains carrying 12 to 20 carbon units and a cationic head group which may be a basic amino acid residue and/or a basic sugar moiety (e.g. Glucosamine) and/or another residue which confers a protonable (e.g. amines) or permamently cationic charged group (e.g quarternized amines). Preferred lipidic cationic components are components comprising or consisting of Lipofectamine® reagents, such as Lipofectamine® or Lipofectamine 2000® (obtainable from Life Technologies) or Oligofectamine™ (obtainable from Life Technologies), or comprising of consisting of DOTAP or DOTMA.

If the cationic component of the carrier is a non-peptidic cationic component or comprises a non-peptidic cationic component, such as a lipidic cationic component, the non-peptidic cationic component, such as the lipidic cationic component as defined above, e.g. the Lipofectamine reagent, and the nucleic acid molecule comprised in complex (A) are preferably provided in a "cationic component" : "nucleic acid molecule" mass ratio in the range of 1:1.2 to 1:15, preferably in the range of 1:1.5 to 1:10, more preferably in the range of 1:1.5 and 1:5, such as 1:2, 1:3 or 1:4. Preferably, the zetapotential of complex (A) (measured as defined herein) is negative, i.e. below 0 mV, preferably below -1 mV, more preferably below -2 mV, even more preferably below -3 mV, and most preferably below -4 mV, such as between about -1 mV and -50 mV, between about -2 mV and -40 mV, or between about -5 mV and -30 mV. Thus, in a preferred aspect of the disclosure, complex (A) comprises or consists of a lipidic cationic component as defined above, such as Lipofectamine® etc., and a nucleic acid molecule, such as an immunostimulating RNA, in a "cationic component" : "nucleic acid molecule" mass ratio range of 1:1.2 to 1:15, preferably in the range of 1:1.5 to 1:10, more preferably in the range of 1:1.5 and 1:5, such as 1:2, 1:3 or 1:4, wherein the zetapotential of complex (A) (measured as defined herein) is below 0 mV, preferably below -1 mV, more preferably below -2 mV, even more preferably below -3 mV, and most preferably below -4 mV, such as between about -1 mV and -50 mV, between about -2 mV and -40 mV, or between about -5 mV and -30 mV.

In the context of the carrier, the cationic components, which form basis for the carrier may be the same or different from each other. It is also particularly preferred that the carrier of the present invention comprises mixtures of cationic peptides, proteins or polymers and optionally further components as defined herein. The complex (A) according to the present invention comprises cationic and/or polycationic peptides or proteins or polyimines.

In this context, the complex due to its variable carrier advantageously allows to combine desired properties of different (short) cationic or polycationic peptides, proteins or polyimines and, optionally, further polymers or other components. The carrier, e.g., allows to efficiently compact nucleic acids for the purpose of efficient transfection of nucleic acids, and particularly for adjuvant therapy. Preferably, the complex may induce the anti-viral cytokine IFN-alpha and therefore support a Th1-shifted immune response, particularly in antigen-presenting cells, like e.g. B cells.

In particular, the carrier formed by cationic components allows considerably to vary its peptide or polyimine content and thus to modulate its biophysical/biochemical properties, particularly the cationic properties of the carrier, quite easily and fast, e.g. by incorporating as cationic components the same or different cationic peptide(s) or polyimines and, optionally, further polymer(s) and optionally adding other components into the carrier.

Accordingly, the carrier of the complex may comprise different (short) cationic or polycationic peptides, proteins or polyimines selected from cationic or polycationic peptides, proteins or (non-peptidic) polyimines as defined above, optionally together with further polymers or components as defined herein.

Additionally, the carrier of the complex as defined above, more preferably at least one of the different (short) cationic or polycationic peptides or (non-peptidic) polymers forming basis for the carrier, may be, modified with at least one further component. Alternatively, the carrier as such may be modified with at least one further component.

To allow modification of a cationic or polycationic peptide or a (non-peptidic) polymer as defined above, each of the components of the carrier may also contain at least one further functional moiety, which allows attaching such further components as defined herein. Such functional moieties may be selected from functionalities which allow the attachment of further components, e.g. functionalities as defined herein, e.g. by amide formation (e.g. carboxylic acids, sulphonic acids, amines, etc.), by Michael addition (e.g maleinimide moieties, $\alpha,\beta$ unsatured carbonyls, etc.), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow $S_n$-type substitution reactions (e.g halogenalkans, thiols, alcohols, amines, hydrazines, hydrazides, sulphonic acid esters, oxyphosphonium salts) or other chemical moieties which can be utilized in the attachment of further components.

According to a particularly preferred embodiment, the further component, which may be contained in the carrier or which may be used to modify the different (short) cationic or polycationic peptides or (non-peptidic) polymers forming basis for the carrier of the complex is an amino acid component (AA), which may e.g. modify the biophysical/biochemical properties of the carrier as defined herein. According to the present invention, the amino acid component (AA) comprises a number of amino acids preferably in a range of about 1 to 100, preferably in a range of about 1 to 50, more preferably selected from a number comprising 1, 2, 3, 4, 5, 6, 7, 8, 9, 10, 11, 12, 13, 14 or 15-20, or may be selected from a range formed by any two of the afore mentioned values. In this context the amino acids of amino acid component (AA) can be chosen independently from each other. For example, if in the carrier two or more (AA) components are present they can be the same or can be different from each other.

In this context, the amino acid component (AA) may be provided with functionalities as already described above for the other components of the carrier, which allow binding of the amino acid component (AA) to any of components of the carrier.

Thus, the amino acid component (AA) may be bound to further components of the carrier. Binding of the amino acid component (AA) to the other component of the carrier may be preferably carried out by using an amid-chemistry as defined herein. If desired or necessary, the other terminus of the amino acid component (AA), e.g. the N- or C-terminus, may be used to couple another component, e.g. a ligand L. For this purpose, the other terminus of the amino acid component (AA) preferably comprises or is modified to comprise a further functionality, e.g. an alkyn-species (see above), which may be used to add the other component via e.g. click-chemistry. If the ligand is bound via an acid-labile bond, the bond is preferably cleaved off in the endosome and the carrierpresents amino acid component (AA) at its surface.

The amino acid component (AA) may occur as a further component of the carrier as defined above, e.g. as a linker between cationic components e.g. as a linker between one cationic peptide and a further cationic peptide, as a linker between one cationic polymer and a further cationic polymer, as a linker between one cationic peptide and a cationic polymer, all preferably as defined herein, or as an additional component of the carrier, e.g. by binding the amino acid component (AA) to the carrier or a component thereof, e.g. via side chains, or via further moieties as defined herein, wherein the amino acid component (AA) is preferably accordingly modified.

According to a further and particularly preferred alternative, the amino acid component (AA) may be used to modify the carrier, particularly the content of cationic components in the carrier as defined above.

In this context it is preferable, that the content of cationic components in the carrier is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 30% to 100%, more preferably in the range of about 50% to 100%, even preferably in the range of about 70% to 100%, e.g. 70, 80, 90 or 100%, or in a range formed by any two of the afore mentioned values, provided, that the content of all components in the carrier is 100%.

In the context of the present invention, the amino acid component (AA) may be selected from the following alternatives.

According to a first alternative, the amino acid component (AA) may be an aromatic amino acid component (AA). The incorporation of aromatic amino acids or sequences as amino aromatic acid component (AA) into the carrier of the present invention enables a different (second) binding of the carrier to the nucleic acid due to interactions of the aromatic amino acids with the bases of the nucleic acid cargo in contrast to the binding thereof by cationic charged sequences of the carrier molecule to the phosphate backbone. This interaction may occur e.g. by intercalations or by minor or major groove binding. This kind of interaction is not prone to decompaction by anionic complexing partners (e.g. Heparin, Hyaluronic acids) which are found mainly in the extracellular matrix *in vivo* and is also less susceptible to salt effects.

For this purpose, the amino acids in the aromatic amino acid component (AA) may be selected from either the same or different aromatic amino acids e.g. selected from Trp, Tyr, or Phe.

Additionally, the aromatic amino acid component (AA) may contain or represent at least one proline, which may serve as a structure breaker of longer sequences of Trp, Tyr and Phe in the aromatic amino acid component (AA), preferably two, three or more prolines.

According to a second alternative, the amino acid component (AA) may be a hydrophilic (and preferably non-charged polar) amino acid component (AA). The incorporation of hydrophilic (and preferably noncharged polar) amino acids or sequences as amino hydrophilic (and preferably non charged polar) acid component (AA) into the carrier of the present invention enables a more flexible binding to the nucleic acid cargo. This leads to a more effective compaction of the nucleic acid cargo and hence to a better protection against nucleases and unwanted decompaction. It also allows provision of a (long) carrier which exhibits a reduced cationic charge over the entire carrier and in this context to better adjusted binding properties, if desired or necessary.

For this purpose, the amino acids in the hydrophilic (and preferably noncharged polar) amino acid component (AA) may be selected from either the same or different hydrophilic (and preferably non charged polar) amino acids e.g. selected from Thr, Ser, Asn or Gin.

Additionally, the hydrophilic (and preferably non-charged polar) amino acid component (AA) may contain at least one proline, which may serve as a structure breaker of longer sequences of Ser, Thr and Asn in the hydrophilic (and preferably non-charged polar) amino acid component (AA), preferably two, three or more prolines.

According to a third alternative, the amino acid component (AA) may be a lipohilic amino acid component (AA). The incorporation of lipohilic amino acids or sequences as amino lipohilic acid component (AA) into the carrier of the present invention enables a stronger compaction of the nucleic acid cargo and/or the carrier and its nucleic acid cargo when forming a complex. This is particularly due to interactions of one or more polymer strands of the carrier, particularly of lipophilic sections of lipohilic amino acid component (AA) and the nucleic acid cargo. This interaction will preferably add an additional stability to the complex between the carrier and its nucleic acid cargo. This stabilization may somehow be compared to a sort of non covalent crosslinking between different polymer strands. Especially in aqueous environment this interaction is typically strong and provides a significant effect.

For this purpose, the amino acids in the lipophilic amino acid component (AA) may be selected from either the same or different lipophilic amino acids e.g. selected from Leu, Val, Ile, Ala, Met.

Additionally, the lipophilic amino acid component (AA) may contain at least one proline, which may serve as a structure breaker of longer sequences of Leu, Val, lie, Ala and Met in the lipophilic amino acid component (AA), preferably two, three or more prolines.

Finally, according to a fourth alternative, the amino acid component (AA) may be a weak basic amino acid component (AA). The incorporation of weak basic amino acids or sequences as weak basic amino acid component (AA) into the carrier of the present invention may serve as a proton sponge and facilitates endosomal escape (also called endosomal release) (proton sponge effect). Incorporation of such a weak basic amino acid component (AA) preferably enhances transfection efficiency.

For this purpose, the amino acids in the weak basic amino acid component (AA) may be selected from either the same or different weak amino acids e.g. selected from histidine or aspartate (aspartic acid).

Additionally, the weak basic amino acid component (AA) may contain at least one proline, which may serve as a structure breaker of longer sequences of histidine or aspartate (aspartic acid) in the weak basic amino acid component (AA), preferably two, three or more prolines.

According to a fifth alternative, the amino acid component (AA) may be a signal peptide or signal sequence, a localization signal or sequence, a nuclear localization signal or sequence (NLS), an antibody, a cell penetrating peptide, (e.g. TAT), etc. Preferably, such an amino acid component (AA) is bound to the carrier. In this context, a signal peptide, a localization signal or sequence or a nuclear localization signal or sequence (NLS), may be used to direct the complex to specific target cells (e.g. hepatocytes or antigen-presenting cells) and preferably allows a translocalization of the complex to a specific target, e.g. into the cell, into the nucleus, into the endosomal compartment, sequences for the mitochondrial matrix, localisation sequences for the plasma membrane, localisation sequences for the Golgi apparatus, the nucleus, the cytoplasm and the cytosceleton, etc. Such signal peptide, a localization signal or sequence or a nuclear localization signal may be used for the transport of any of the herein defined nucleic acids, preferably an RNA or a DNA, more preferably an shRNA or a pDNA, e.g. into the endosome or into the cytoplasm. Without being limited thereto, such a signal peptide, a localization signal or sequence or a nuclear localization signal may comprise, e.g., localisation sequences for the endoplasmic reticulum. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulins as defined herein, signal sequences of the invariant chain of immunoglobulins or antibodies as defined herein, signal sequences of Lamp1, Tapasin, Erp57, Calreticulin, Calnexin, and further membrane associated proteins or of proteins associated with the endoplasmic reticulum (ER) or the endosomal-lysosomal compartment. Such an additional component may be bound e.g. to a cationic component or to any other component of the carrier as defined herein. Preferably, this signal peptide, localization signal or sequence or nuclear localization signal or sequence (NLS), is bound to the carrier or to another component of the carrier using an acid-labile bond, preferably via a side chain of any of components of the carrier, which allows to detach or release the additional component at lower pH-values, e.g. at physiological pH-values as defined herein.

Additionally, according to another alternative, the amino acid component (AA) may be a functional peptide or protein, which may modulate the functionality of the carrier accordingly. Such functional peptides or proteins as the amino acid component (AA) preferably comprise any peptides or proteins as defined herein, e.g. as defined below as therapeutically active proteins. According to one alternative, such further functional peptides or proteins may comprise so called cell penetrating peptides (CPPs) or cationic peptides for transportation. Particularly preferred are CPPs, which induce a pH-mediated conformational change in the endosome and lead to an improved release of the carrier (in complex with a nucleic acid) from the endosome by insertion into the lipid layer of the liposome. These cell penetrating peptides (CPPs) or cationic peptides for transportation, may include, without being limited thereto protamine, nucleoline, spermine or spermidine, oligo- or poly-L-lysine (PLL), basic polypeptides, oligo or poly-arginine, cell penetrating peptides (CPPs), chimeric CPPs, such as Transportan, or MPG peptides, HIV-binding peptides, Tat, HIV-1 Tat (HIV), Tat-derived peptides, members of the penetratin family, e.g. Penetratin, Antennapedia-derived peptides (particularly from *Drosophila antennapedia*), pAntp, pIsl, etc., antimicrobial-derived CPPs e.g. Buforin-2, Bac715-24, SynB, SynB(1), pVEC, hCT-derived peptides, SAP, MAP, KALA, PpTG20, Loligomere, FGF, Lactoferrin, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc. Such an amino acid component (AA) may also be bound to any component of the carrier as defined herein. The binding to any of the components of the carrier may be accomplished using an acid-labile bond, preferably via a side chain of any of components of the carrier which allows to detach or release the additional component at lower pH-values, e.g. at physiological pH-values as defined herein.

According to a last alternative, the amino acid component (AA) may consist of any peptide or protein which can execute any favourable function in the cell. Particularly preferred are peptides or proteins selected from therapeutically active proteins or peptides, from antigens, e.g. tumour antigens (= antigens associated with a cancer or tumour disease), pathogenic antigens (= antigens associated with infectious disease) (animal antigens, viral antigens, protozoan antigens, bacterial antigens), allergic antigens (= antigens associated with allergy or allergic disease), autoimmune antigens (=antigens associated with autoimmune disease), or further antigens, from, from antibodies, from immunostimulatory proteins or peptides, from antigen-specific T-cell receptors, from antigen-specific B cell receptors, or from any other protein or peptide suitable for a specific (therapeutic) application as defined below. Particularly preferred are peptide epitopes from the at least one antigen (an antigen from a pathogen associated with infectious disease; an antigen associated with allergy or allergic disease; an antigen associated with autoimmune disease; or an antigen associated with a cancer or tumour disease) as comprised as second ingredient in the inventive pharmaceutical composition, as defined herein.

The amino acid component (AA) is preferably not covalently attached to the carrier component. In particular, the amino acid component (AA) is preferably not covalently attached to the carrier component if the amino acid component (AA) is ovalbumin or a fragment of ovalbumin. Preferably, the amino acid component is not ovalbumin or a fragment of ovalbumin, such as the ovalbumin-derived peptide SIINFEKL (SEQ ID NO: 103) or ISQAVHAAHAEINE (SEQ ID NO: 104). Preferably, the amino acid component is not derived from mouse mastocytoma, in particular is preferably not the mouse mastocytoma P815-derived peptide P1A LPYLGWLVF (SEQ ID NO: 105). Preferably, the amino acid component is not derived from *Plasmodium yoelii,* in particular is preferably not derived from the circumsporozoite protein of *Plasmodium yoelii,* such as the CSP-peptide SYVPSAEQI (SEQ ID NO: 106). Preferably, the amino acid component is not derived from *Listeria monocytgenes,* in particular, not from listeriolysin O 91-99, such as the LLO-peptide GYKDGNEYI (SEQ ID NO: 107). Preferably, the amino acid component is not derived from the melanocyte stimulating hormone receptor (MC1R), in particular is not the MC1R-peptide WGPFFLHL (SEQ ID NO: 108).

Due to the peptidic nature of the amino acid component also the definition of peptide, protein, or fragment, variant and derivative thereof applies accordingly and are explicitly encompassed.

Furthermore, said (AA) components may be prepared by all methods known to a person of ordinary skill or by recombinant peptide or protein production or by peptide synthesis as described herein.

The carrier may comprise at least one of the above mentioned cationic or polycationic peptides, proteins or polyimines and additional polymers or further components, e.g. (AA), wherein any of the above alternatives may be combined with each other.

According to another embodiment, the carrier of the complex or single components thereof, e.g. of the above mentioned cationic or polycationic peptides, proteins or polyimines and additional polymers or further components, e.g. (AA), may be further modified with a ligand, preferably a carbohydrate, more preferably a sugar, even more preferably mannose. Preferably this ligand is bound to the carrier or to a component of the carrier e.g. via Michael addition. These ligands may be used to direct the complex to specific target cells (e.g. hepatocytes or antigen-presenting cells). In this context mannose is particular preferred as ligand in the case that dendritic cells are the target especially for vaccination or adjuvant purposes.

The complex additionally comprises as a cargo at least one nucleic acid molecule, which is an RNA. The RNA may be selected e.g. from any (single-stranded or double-stranded) RNA, preferably, without being limited thereto, a (short) RNA oligonucleotide ((short) oligoribonucleotide), a coding RNA, a messenger RNA (mRNA), an immunostimulatory RNA (isRNA), a small interfering RNA (siRNA), an antisense RNA, a micro RNA, a small nuclear RNA (snRNA), a small-hairpin (sh) RNA or riboswitches, ribozymes or aptamers. The nucleic acid molecule of the complex may also be a ribosomal RNA (rRNA), a transfer RNA (tRNA), a messenger RNA (mRNA), or a viral RNA (vRNA). More preferably, the nucleic acid molecule of the complex is a (linear) single-stranded RNA, even more preferably an mRNA or an immunostimulatory RNA. In the context of the present invention, an mRNA is typically an RNA, which is composed of several structural elements, e.g. an optional 5'-CAP structure, an optional 5'-UTR region, an upstream positioned ribosomal binding site followed by a coding region, an optional 3'-UTR region, which may be followed by a poly-A tail (and/or a poly-C-tail). An mRNA may occur as a mono-, di-, or even multicistronic RNA, i.e. a RNA which carries the coding sequences of one, two or more proteins or peptides. Such coding sequences in di-, or even multicistronic mRNA may be separated by at least one IRES sequence, e.g. as defined herein. In the context of the present invention, such a nucleic acid molecule may also be any suitable nucleic acid, which may additionally be comprised by the complex of the pharmaceutical composition, selected e.g. from any (single-stranded or double-stranded) DNA, preferably, without being limited thereto, e.g. genomic DNA, single-stranded DNA molecules, double-stranded DNA molecules, coding DNA, DNA primers, DNA probes, immunostimulatory DNA, a (short) DNA oligonucleotide ((short) oligodesoxyribonucleotides), or may be selected e.g. from any PNA (peptide nucleic acid).

Furthermore, the nucleic acid molecule of the complex may be a single- or a double-stranded nucleic acid molecule (which may also be regarded as a nucleic acid (molecule) due to non-covalent association of two single-stranded nucleic acid(s) (molecules)) or a partially double-stranded or partially single stranded nucleic acid, which are at least partially self complementary (both of these partially double-stranded or partially single stranded nucleic acid molecules are typically formed by a longer and a shorter single-stranded nucleic acid molecule or by two single stranded nucleic acid molecules, which are about equal in length, wherein one single-stranded nucleic acid molecule is in part complementary to the other single-stranded nucleic acid molecule and both thus form a double-stranded nucleic acid molecule in this region, i.e. a partially double-stranded or partially single stranded nucleic acid (molecule). Preferably, the nucleic acid (molecule) may be a single-stranded nucleic acid molecule. Furthermore, the nucleic acid (molecule) may be a circular or linear nucleic acid molecule, preferably a linear nucleic acid molecule.

According to one alternative, the nucleic acid molecule of the complex may be a coding RNA or, additionally comprised by the complex, a coding DNA. Such a coding DNA or RNA may be any DNA or RNA as defined herein. Preferably, such a coding DNA or RNA may be a single- or a double-stranded DNA or RNA, more preferably a single-stranded DNA or RNA, and/or a circular or linear DNA or RNA, more preferably a linear DNA or RNA. Even more preferably, the coding DNA or RNA may be a (linear) single-stranded DNA or RNA. Most preferably, the nucleic acid molecule according to the present invention may be a ((linear) single-stranded) messenger RNA (mRNA). Such an mRNA may occur as a mono-, di-, or even multicistronic RNA, i.e. an RNA which carries the coding sequences of one, two or more proteins or peptides. Such coding sequences in di-, or even multicistronic mRNA may be separated by at least one IRES sequence, e.g. as defined herein.

### Coding nucleic acids:

The nucleic acid molecule of the complex may encode a protein or a peptide, which may be selected, without being restricted thereto, e.g. from therapeutically active proteins or peptides, from antigens, e.g. tumour antigens (= antigens associated with a cancer or tumour disease), pathogenic antigens (= antigens associated with infectious disease) (animal antigens, viral antigens, protozoan antigens, bacterial antigens), allergic antigens (= antigens associated with allergy or allergic disease), autoimmune antigens (=antigens associated with autoimmune disease), or further antigens, from, from antibodies, from immunostimulatory proteins or peptides, from antigen-specific T-cell receptors, from antigen-specific B cell receptors, or from any other protein or peptide suitable for a specific (therapeutic) application, wherein the coding nucleic acid may be transported into a cell, a tissue or an organism and the protein may be expressed subsequently in this cell, tissue or organism. In this context, the coding nucleic acid may additionally code for a signal peptide as defined herein.

*a) Therapeutically active proteins*

In the context of the present invention, therapeutically active proteins or peptides may be encoded by the nucleic acid molecule of the herein defined complex. Therapeutically active proteins are defined herein as proteins which have an effect on healing, prevent prophylactically or treat therapeutically a disease, preferably as defined herein, or are proteins of which an individual is in need of. These may be selected from any naturally or synthetically designed occurring recombinant or isolated protein known to a skilled person from the prior art. Without being restricted thereto therapeutically active proteins may comprise proteins, capable of stimulating or inhibiting the signal transduction in the cell, e.g. cytokines, lymphokines, monokines, growth factors, receptors, signal transduction molecules, transcription factors, etc; anticoagulants; antithrombins; antiallergic proteins; apoptotic factors or apoptosis related proteins, therapeutic active enzymes and any protein or peptide connected with any acquired disease or any hereditary disease or favourable for the treatment of any acquired disease or any hereditary disease.

A therapeutically active protein, which may be encoded by the nucleic acid molecule of the herein defined complex, may also be an adjuvant protein. In this context, an adjuvant protein is preferably to be understood as any protein, which is capable to elicit an innate immune response as defined herein. Preferably, such an innate immune response comprises activation of a pattern recognition receptor, such as e.g. a receptor selected from the Toll-like receptor (TLR) family, including e.g. a Toll like receptor selected from human TLR1 to TLR10 or from murine Toll like receptors TLR1 to TLR13. More preferably, the adjuvant protein is selected from human adjuvant proteins or from pathogenic adjuvant proteins, selected from the group consisting of, without being limited thereto, bacterial proteins, protozoan proteins, viral proteins, or fungal proteins, animal proteins, in particular from bacterial adjuvant proteins. In addition, nucleic acids encoding human proteins involved in adjuvant effects (e.g. ligands of pattern recognition receptors, pattern recognition receptors, proteins of the signal transduction pathways, transcription factors or cytokines) may be used as well.

*b) Antigens*

The nucleic acid molecule of the herein defined complex may alternatively encode an antigen. In the context of the present invention, antigens as encoded by the nucleic acid molecule of the herein defined complex typically comprise any antigen, antigenic epitope or antigenic peptide, falling under the above definition, more preferably protein and peptide antigens, e.g. tumour antigens, allergenic antigens, auto-immune self-antigens, pathogenic antigens, etc. In particular antigens as encoded by the nucleic acid molecule of the herein defined complex may be antigens generated outside the cell, more typically antigens not derived from the host organism (e.g. a human) itself (i.e. non-self antigens) but rather derived from host cells outside the host organism, e.g. viral antigens, bacterial antigens, fungal antigens, protozoological antigens, animal antigens, allergenic antigens, etc. Allergenic antigens (allergy antigens) are typically antigens, which cause an allergy in a human and may be derived from either a human or other sources. Additionally, antigens as encoded by the nucleic acid molecule of the herein defined complex may be furthermore antigens generated inside the cell, the tissue or the body. Such antigens include antigens derived from the host organism (e.g. a human) itself, e.g. tumour antigens, self-antigens or auto-antigens, such as auto-immune self-antigens, etc., but also (non-self) antigens as defined herein, which have been originally been derived from host cells outside the host organism, but which are fragmented or degraded inside the body, tissue or cell, e.g. by (protease) degradation, metabolism, etc. In this context, an antigen as encoded by the nucleic acid cargo comprised in the complex is defined as described below for the at least one antigen, the second ingredient of the inventive pharmaceutical composition.

Particularly preferred in this context is, that the antigen or a fragment, variant and/or derivative thereof encoded by the nucleic acid cargo is the same antigen as the at least one protein or peptide antigen as defined herein as comprised in the inventive pharmaceutical composition as second ingredient. In alternative embodiments, however, the antigen or a fragment, variant and/or derivative thereof encoded by the nucleic acid cargo is an antigen different from the at least one protein or peptide antigen as defined herein as comprised in the inventive pharmaceutical composition as second ingredient. In the specific case that an antigen is encoded by the nucleic acid cargo, the nucleic acid molecule together with the carrier serves as adjuvant or imunostimulating agent to induce an unspecific innate immune response, whereas the encoded protein or peptide antigen which is expressed by the nucleic acid cargo serves as antigen to induce an antigen-specific adaptive immune response.

c) *Antibodies*

According to a further alternative, the nucleic acid molecule of the herein defined complex may encode an antibody or an antibody fragment. According to the present invention, such an antibody may be selected from any antibody, e.g. any recombinantly produced or naturally occurring antibodies, known in the art, in particular antibodies suitable for therapeutic, diagnostic or scientific purposes, or antibodies which have been identified in relation to specific cancer diseases. Herein, the term "antibody" is used in its broadest sense and specifically covers monoclonal and polyclonal antibodies (including agonist, antagonist, and blocking or neutralizing antibodies) and antibody species with polyepitopic specificity. According to the invention, the term "antibody" typically comprises any antibody known in the art (e.g. IgM, IgD, IgG, IgA and IgE antibodies), such as naturally occurring antibodies, antibodies generated by immunization in a host organism, antibodies which were isolated and identified from naturally occurring antibodies or antibodies generated by immunization in a host organism and recombinantly produced by biomolecular methods known in the art, as well as chimeric antibodies, human antibodies, humanized antibodies, bispecific antibodies, intrabodies, i.e. antibodies expressed in cells and optionally localized in specific cell compartments, and fragments and variants of the aforementioned antibodies. In general, an antibody consists of a light chain and a heavy chain both having variable and constant domains. The light chain consists of an N-terminal variable domain, $V_L$, and a C-terminal constant domain, $C_L$. In contrast, the heavy chain of the IgG antibody, for example, is comprised of an N-terminal variable domain, $V_H$, and three constant domains, $C_H1$, $C_H2$ und $C_H3$.

In the context of the present invention, antibodies as encoded by the nucleic acid molecule of the herein defined complex may preferably comprise full-length antibodies, i.e. antibodies composed of the full heavy and full light chains, as described above. However, derivatives of antibodies such as antibody fragments, variants or derivatives, as defined herein, may also be encoded by the nucleic acid molecule of the herein defined complex. Antibody fragments are preferably selected from Fab, Fab', $F(ab')_2$, Fc, Facb, pFc', Fd and Fv fragments of the aforementioned (full-length) antibodies. In general, antibody fragments are known in the art. For example, a Fab ("fragment, antigen binding") fragment is composed of one constant and one variable domain of each of the heavy and the light chain. The two variable domains bind the epitope on specific antigens. The two chains are connected via a disulfide linkage. A scFv ("single chain variable fragment") fragment, for example, typically consists of the variable domains of the light and heavy chains. The domains are linked by an artificial linkage, in general a polypeptide linkage such as a peptide composed of 15-25 glycine, proline and/or serine residues.

In the present context it is preferable that the different chains of the antibody or antibody fragment are encoded by a multicistronic nucleic acid molecule. Alternatively, the different strains of the antibody or antibody fragment are encoded by several monocistronic nucleic acid(s) (sequences).

## siRNA:

According to a further alternative, the nucleic acid molecule of the herein defined complex may be in the form of dsRNA, preferably siRNA. A dsRNA, or a siRNA, is of interest particularly in connection with the phenomenon of RNA interference. The *in vitro* technique of RNA interference (RNAi) is based on double-stranded RNA molecules (dsRNA), which trigger the sequence-specific suppression of gene expression (Zamore (2001) Nat. Struct. Biol. 9: 746-750; Sharp (2001) Genes Dev. 5:485-490: Hannon (2002) Nature 41: 244-251). In the transfection of mammalian cells with long dsRNA, the activation of protein kinase R and RnaseL brings about unspecific effects, such as, for example, an interferon response (Stark et al. (1998) Annu. Rev. Biochem. 67: 227-264; He and Katze (2002) Viral Immunol. 15: 95-119). These unspecific effects are avoided when shorter, for example 21- to 23-mer, so-called siRNA (small interfering RNA), is used, because unspecific effects are not triggered by siRNA that is shorter than 30 bp (Elbashir et al. (2001) Nature 411: 494-498).

The nucleic acid molecule of the herein defined complex may thus be a double-stranded RNA (dsRNA) having a length of from 17 to 29, preferably from 19 to 25, and preferably is at least 90%, more preferably 95% and especially 100% (of the nucleotides of a dsRNA) complementary to a section of the nucleic acid molecule of a (therapeutically relevant) protein or antigen described (as active ingredient) hereinbefore or of any further protein as described herein, either a coding or a non-coding section, preferably a coding section. Such a (section of the) nucleic acid molecule may be termed herein a "target sequence" and may be any nucleic acid molecule as defined herein, preferably a genomic DNA, a cDNA, a RNA, e.g. an mRNA, etc. 90% complementary means that with a length of a dsRNA described herein of, for example, 20 nucleotides, the dsRNA contains not more than 2 nucleotides showing no complementarity with the corresponding section of the target sequence. The sequence of the double-stranded RNA used according to the invention is, however, preferably wholly complementary in its general structure with a section of the target sequence. In this context the nucleic acid molecule of the complex may be a dsRNA having the general structure 5'-($N_{17-29}$)-3', preferably having the general structure 5'-($N_{19-25}$)-3', more preferably having the general structure 5'-($N_{19-24}$)-3', or yet more preferably having the general structure 5'-($N_{21-23}$)-3', wherein for each general structure each N is a (preferably different) nucleotide of a section of the target sequence, preferably being selected from a continuous number of 17 to 29 nucleotides of a section of the target sequence, and being present in the general structure 5'-($N_{17-29}$)-3' in their natural order. In principle, all the sections having a length of from 17 to 29, preferably from 19 to 25, base pairs that occur in the target sequence can serve for preparation of a dsRNA as defined herein. Equally, dsRNAs used as nucleic acid molecule of the complex can also be directed against nucleotide sequences of a (therapeutically relevant) protein or antigen described (as active ingredient) herein before that do not lie in the coding region, in particular in the 5' non-coding region of the target sequence, for example, therefore, against non-coding regions of the target sequence having a regulatory function. The target sequence of the dsRNA used as nucleic acid molecule of the complex can therefore lie in the translated and untranslated region of the target sequence and/or in the region of the control elements of a protein or antigen described hereinbefore. The target sequence for a dsRNA used as the nucleic acid molecule of the complex can also lie in the overlapping region of untranslated and translated sequence; in particular, the target sequence can comprise at least one nucleotide upstream of the start triplet of the coding region, e.g. of a genomic DNA, a cDNA, a RNA, or an mRNA, etc.

**Immunostimulatory nucleic acids:**

a) Immunostimulatory CpG nucleic acids:

According to another alternative, the nucleic acid molecule of the herein defined complex may be in the form of a(n) (immunostimulatory) CpG nucleic acid, i.e. CpG-RNA and, optionally additionally, an CpG-DNA, which preferably induces an innate immune response. A CpG-RNA or CpG-DNA used according to the invention can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid used according to the invention is preferably in the form of CpG-RNA, more preferably in the form of single-stranded CpG-RNA (ss CpG-RNA). Also preferably, such CpG nucleic acids have a length as described above. Preferably, at least one of the CpG motifs is unmethylated. Preferably, the CpG motifs are unmethylated.

In a preferred embodiment, the complex does not comprise a CpG-DNA consisting of the sequence 5'TCCATGACGTTCCTGACGTT-3' (SEQ ID NO: 102), in particular if the protein or peptide antigen or amino acid component (AA) is ovalbumin or a fragment of ovalbumin. In a further preferred embodiment, the CpG nucleic acid is not a sequence comprising SEQ ID NO: 102. In some embodiments of the present invention, the complex (A) does not comprise a CpG-DNA, preferably does not comprise a CpG nucleic acid. In some embodiments of the present invention, the pharmaceutical composition does not comprise a CpG-DNA, preferably does not comprise a CpG nucleic acid.

b) Immunostimulatory RNA (isRNA):

Likewise, according to a further alternative, the (immunostimulatory) nucleic acid molecule of the complex may be in the form of an immunostimulatory RNA (isRNA), which preferably elicits an innate immune response. Such an immunostimulatory RNA may be any (double-stranded or single-stranded) RNA, e.g. a coding RNA, as defined herein. Preferably, the immunostimulatory RNA may be a single-stranded, a double-stranded or a partially double-stranded RNA, more preferably a single-stranded RNA, and/or a circular or linear RNA, more preferably a linear RNA. More preferably, the immunostimulatory RNA may be a (linear) single-stranded RNA. Even more preferably, the immunostimulatory RNA may be a (long) (linear) single-stranded) non-coding RNA. In this context it is particular preferred that the isRNA carries a triphosphate at its 5'-end which is the case for *in vitro* transcribed RNA. An immunostimulatory RNA may also occur as a short RNA oligonucleotide as defined herein. An immunostimulatory RNA as used herein may furthermore be selected from any class of RNA molecules, found in nature or being prepared synthetically, and which can induce an innate immune response and may support an adaptive immune response induced by an antigen. In this context, an immune response may occur in various ways. A substantial factor for a suitable (adaptive) immune response is the stimulation of different T-cell sub-populations. T-lymphocytes are typically divided into two sub-populations, the T-helper 1 (Th1) cells and the T-helper 2 (Th2) cells, with which the immune system is capable of destroying intracellular (Th1) and extracellular (Th2) pathogens (e.g. antigens). The two Th cell populations differ in the pattern of the effector proteins (cytokines) produced by them. Thus, Th1 cells assist the cellular immune response by activation of macrophages and cytotoxic T-cells. Th2 cells, on the other hand, promote the humoral immune response by stimulation of B-cells for conversion into plasma cells and by formation of antibodies (e.g. against antigens). The Th1/Th2 ratio is therefore of great importance in the induction and maintenance of an adaptive immune response. In connection with the present invention, the Th1/Th2 ratio of the (adaptive) immune response is preferably shifted in the direction towards the cellular response (Th1 response) and a cellular immune response is thereby induced. According to one example, the innate immune system which may support an adaptive immune response may be activated by ligands of Toll-like receptors (TLRs). TLRs are a family of highly conserved pattern recognition receptor (PRR) polypeptides that recognize pathogen-associated molecular patterns (PAMPs) and play a critical role in innate immunity in mammals. Currently at least thirteen family members, designated TLR1 - TLR13 (Toll-like receptors: TLR1, TLR2, TLR3, TLR4, TLR5, TLR6, TLR7, TLR8, TLR9, TLR10, TLR11, TLR12 or TLR13), have been identified. Furthermore, a number of specific TLR ligands have been identified. It was e.g. found that unmethylated bacterial DNA and synthetic analogues thereof (CpG DNA) are ligands for TLR9 (Hemmi H et al. (2000) Nature 408:740-5; Bauer S et al. (2001) Proc NatlAcadSci USA 98, 9237-42). Furthermore, it has been reported that ligands for certain TLRs include certain nucleic acid molecules and that certain types of RNA are immunostimulatory in a sequence-independent or sequence-dependent manner, wherein these various immunostimulatory RNAs may e.g. stimulate TLR3, TLR7, or TLR8, or intracellular receptors such as RIG-I, MDA-5, etc. E.g. Lipford *et al.* determined certain G,U-containing oligoribonucleotides as immunostimulatory by acting via TLR7 and TLR8 (see WO 03/086280). The immunostimulatory G,U-containing oligoribonucleotides described by Lipford *et al.* were believed to be derivable from RNA sources including ribosomal RNA, transfer RNA, messenger RNA, and viral RNA.

The immunostimulatory RNA (isRNA) used as the nucleic acid molecule of the herein defined complex may thus comprise any RNA sequence known to be immunostimulatory, including, without being limited thereto, RNA sequences representing and/or encoding ligands of TLRs, preferably selected from human family members TLR1 - TLR10 or murine family members TLR1 - TLR13, more preferably selected from (human) family members TLR1 - TLR10, even more preferably from TLR7 and TLR8, ligands for intracellular receptors for RNA (such as RIG-I or MDA-5, etc.) (see e.g. Meylan, E., Tschopp, J. (2006). Toll-like receptors and RNA helicases: two parallel ways to trigger antiviral responses. Mol. Cell 22, 561-569), or any other immunostimulatory RNA sequence. Furthermore, (classes of) immunostimulatory RNA molecules, used as the nucleic acid molecule of the complex may include any other RNA capable of eliciting an innate immune response. Without being limited thereto, such an immunostimulatory RNA may include ribosomal RNA (rRNA), transfer RNA (tRNA), messenger RNA (mRNA), and viral RNA (vRNA), preferably the immunostimulatory RNA is a non-coding RNA. Such an immunostimulatory RNA may comprise a length of 1000 to 5000, of 500 to 5000, of 5 to 5000, or of 5 to 1000, 5 to 500, 5 to 250, of 5 to 100, of 5 to 50 or of 5 to 30 nucleotides.

According to a particularly preferred embodiment, such immunostimulatory nucleic acid sequences are preferably RNA preferably consisting of or comprising a nucleic acid sequence of formula (II) or (III):

$$\mathbf{G_l X_m G_n}\text{ ,} \qquad \textbf{(formula (II))}$$

wherein:

G is guanosine, uridine or an analogue of guanosine or uridine;
X is guanosine, uridine, adenosine, thymidine, cytidine or an analogue of the above-mentioned nucleotides;
l is an integer from 1 to 40, wherein when l = 1 G is guanosine or an analogue thereof, when l > 1 at least 50% of the nucleotides are guanosine or an analogue thereof;
m is an integer and is at least 3; wherein when m = 3 X is uridine or an analogue thereof, when m > 3 at least 3 successive uridines or analogues of uridine occur;
n is an integer from 1 to 40, wherein when n = 1 G is guanosine or an analogue thereof, when n > 1 at least 50% of the nucleotides are guanosine or an analogue thereof.

$$\mathbf{C_l X_m C_n}\text{,} \qquad \textbf{(formula (III))}$$

wherein:

C is cytidine, uridine or an analogue of cytidine or uridine;
X is guanosine, uridine, adenosine, thymidine, cytidine or an analogue of the above-mentioned nucleotides;
l is an integer from 1 to 40,
wherein
when l = 1 C is cytidine or an analogue thereof,
when l > 1 at least 50% of the nucleotides are cytidine or an analogue thereof;
m is an integer and is at least 3;
wherein
when m = 3 X is uridine or an analogue thereof,
when m > 3 at least 3 successive uridines or analogues of uridine occur;
n is an integer from 1 to 40,
wherein
when n = 1 C is cytidine or an analogue thereof,
when n > 1 at least 50% of the nucleotides are cytidine or an analogue thereof.

The nucleic acids of formula (II) or (III), which may be used as the nucleic acid cargo of the complex may be relatively short nucleic acid molecules with a typical length of approximately from 5 to 100 (but may also be longer than 100 nucleotides for specific embodiments, e.g. up to 200 nucleotides), from 5 to 90 or from 5 to 80 nucleotides, preferably a length of approximately from 5 to 70, more preferably a length of approximately from 8 to 60 and, more preferably a length of approximately from 15 to 60 nucleotides, more preferably from 20 to 60, most preferably from 30 to 60 nucleotides. If the nucleic acid of the nucleic acid cargo complex has a maximum length of e.g. 100 nucleotides, m will typically be <=98. The number of nucleotides G in the nucleic acid of formula (II) is determined by l or n. l and n, independently of one another, are each an integer from 1 to 40, wherein when l or n = 1 G is guanosine or an analogue thereof, and when l or n > 1 at least 50% of the nucleotides are guanosine or an analogue thereof. For example, without implying any limitation, when l or n = 4 $G_l$ or $G_n$ can be, for example, a GUGU, GGUU, UGUG, UUGG, GUUG, GGGU, GGUG, GUGG, UGGG or GGGG, etc.; when l or n = 5 $G_l$ or $G_n$ can be, for example, a GGGUU, GGUGU, GUGGU, UGGGU, UGGUG, UGUGG, UUGGG, GUGUG, GGGGU, GGGUG, GGUGG, GUGGG, UGGGG, or GGGGG, etc.; etc. A nucleotide adjacent to $X_m$ in the nucleic acid of formula (II) according to the invention is preferably not a uridine. Similarly, the number of nucleotides C in the nucleic acid of formula (III) according to the invention is determined by l or n. l and n, independently of one another, are each an integer from 1 to 40, wherein when l or n = 1 C is cytidine or an analogue thereof, and when l or n > 1 at least 50% of the nucleotides are cytidine or an analogue thereof. For example, without implying any limitation, when l or n = 4, $C_l$ or $C_n$ can be, for example, a CUCU, CCUU, UCUC, UUCC, CUUC, CCCU, CCUC, CUCC, UCCC or CCCC, etc.; when l or n = 5 $C_l$ or $C_n$ can be, for example, a CCCUU, CCUCU, CUCCU, UCCCU, UCCUC, UCUCC, UUCCC, CUCUC, CCCCU, CCCUC, CCUCC, CUCCC, UCCCC, or CCCCC, etc.; etc. A nucleotide adjacent to $X_m$ in the nucleic acid of formula (III) according to the invention is preferably not a uridine. Preferably, for formula (II), when l or n > 1, at least 60%, 70%, 80%, 90% or even 100% of the nucleotides are guanosine or an analogue thereof, as defined above. The remaining nucleotides to 100% (when guanosine constitutes less than 100% of the nucleotides) in the flanking sequences $G_l$ and/or $G_n$ are uridine or an analogue thereof, as defined hereinbefore. Also preferably, l and n, independently of one another, are each an integer from 2 to 30, more preferably an integer from 2 to 20 and yet more preferably an integer from 2 to 15. The lower limit of l or n can be varied if necessary and is at least 1, preferably at least 2, more preferably at least 3, 4, 5, 6, 7, 8, 9 or 10. This definition applies correspondingly to formula (III).

According to a particularly preferred embodiment, a nucleic acid according to any of formulas (II) or (III) above, which may be used as nucleic acid molecule of the complex, may be selected from a sequence consisting of or comprising any of the following sequences:

- GGUUUUUUUUUUUUUUUGGG (SEQ ID NO: 1);
- GGGGGUUUUUUUUUUGGGGG (SEQ ID NO: 2);
- GGGGGUUUUUUUUUUUUUUUUUUUUUUUUUUUUUUGGGGG (SEQ ID NO: 3);
- GUGUGUGUGUGUUUUUUUUUUUUUUUGUGUGUGUGUGU (SEQ ID NO: 4);
- GGUUGGUUGGUUUUUUUUUUUUUUUGGUUGGUUGGUU (SEQ ID NO: 5);
- GGGGGGGGGUUUGGGGGGGG (SEQ ID NO: 6);
- GGGGGGGGUUUUGGGGGGGG (SEQ ID NO: 7);
- GGGGGGGUUUUUUGGGGGGG (SEQ ID NO: 8);
- GGGGGGGUUUUUUUGGGGGG (SEQ ID NO: 9);
- GGGGGGUUUUUUUUGGGGGG (SEQ ID NO: 10);
- GGGGGGUUUUUUUUUGGGGG (SEQ ID NO: 11);
- GGGGGGUUUUUUUUUUGGGG (SEQ ID NO: 12);
- GGGGGUUUUUUUUUUUGGGG (SEQ ID NO: 13);
- GGGGGUUUUUUUUUUUUGGG (SEQ ID NO: 14);
- GGGGUUUUUUUUUUUUUGGG (SEQ ID NO: 15);
- GGGGUUUUUUUUUUUUUUGG (SEQ ID NO: 16);
- GGUUUUUUUUUUUUUUUUGG (SEQ ID NO: 17);
- GUUUUUUUUUUUUUUUUUUG (SEQ ID NO: 18);
- GGGGGGGGGGUUUGGGGGGGGG (SEQ ID NO: 19);
- GGGGGGGGGUUUUGGGGGGGGG (SEQ ID NO: 20);
- GGGGGGGGUUUUUUGGGGGGGG (SEQ ID NO: 21);
- GGGGGGGGUUUUUUUGGGGGGG (SEQ ID NO: 22);
- GGGGGGGUUUUUUUUGGGGGGG (SEQ ID NO: 23);
- GGGGGGGUUUUUUUUUGGGGGG (SEQ ID NO: 24);
- GGGGGGGUUUUUUUUUUGGGGG (SEQ ID NO: 25);
- GGGGGGUUUUUUUUUUUGGGGG (SEQ ID NO: 26);
- GGGGGGUUUUUUUUUUUUGGGG (SEQ ID NO: 27);
- GGGGGUUUUUUUUUUUUUGGGG (SEQ ID NO: 28);
- GGGGGUUUUUUUUUUUUUUGGG (SEQ ID NO: 29);
- GGGUUUUUUUUUUUUUUUUGGG (SEQ ID NO: 30);

- GGUUUUUUUUUUUUUUUUUUGG (SEQ ID NO: 31);
- GGGGGGGGGGGUUUGGGGGGGGGG (SEQ ID NO: 32);
- GGGGGGGGGGUUUUGGGGGGGGGG (SEQ ID NO: 33);
- GGGGGGGGGUUUUUUGGGGGGGGG (SEQ ID NO: 34);
- GGGGGGGGGUUUUUUUGGGGGGGG (SEQ ID NO: 35);
- GGGGGGGGUUUUUUUUGGGGGGGG (SEQ ID NO: 36);
- GGGGGGGGUUUUUUUUUGGGGGGG (SEQ ID NO: 37);
- GGGGGGGGUUUUUUUUUUGGGGGG (SEQ ID NO: 38);
- GGGGGGGUUUUUUUUUUUGGGGGG (SEQ ID NO: 39);
- GGGGGGGUUUUUUUUUUUUGGGGG (SEQ ID NO: 40);
- GGGGGGUUUUUUUUUUUUUGGGGG (SEQ ID NO: 41);
- GGGGGGUUUUUUUUUUUUUUGGGG (SEQ ID NO: 42);
- GGGGUUUUUUUUUUUUUUUUGGGG (SEQ ID NO: 43);
- GGGUUUUUUUUUUUUUUUUUUGGG (SEQ ID NO: 44);
- GUUUUUUUUUUUUUUUUUUUUUUUUUUUUUUG (SEQ ID NO: 45);
- GGUUUUUUUUUUUUUUUUUUUUUUUUUUUUUUGG (SEQ ID NO: 46);
- GGGUUUUUUUUUUUUUUUUUUUUUUUUUUUUUUGGG (SEQ ID NO: 47);
- GGGGUUUUUUUUUUUUUUUUUUUUUUUUUUUUUUGGG (SEQ ID NO: 48);
- GGGGGUUUUUUUUUUUUUUUUUUUUUUUUUUUUUUGGGG (SEQ ID NO: 49);
- GGGGGGUUUUUUUUUUUUUUUUUUUUUUUUUUUUUUGGGGG (SEQ ID NO: 50);
- GGGGGGGUUUUUUUUUUUUUUUUUUUUUUUUUUUUUUGGGGGG (SEQ ID NO: 51);
- GGGGGGGGUUUUUUUUUUUUUUUUUUUUUUUUUUUUUUGGGGGGG (SEQ ID NO: 52);
- GGGGGGGGGUUUUUUUUUUUUUUUUUUUUUUUUUUUUUUGGGGGGGG (SEQ ID NO: 53);
- GGUUUGG (SEQ ID NO: 54);
- GGUUUUGG (SEQ ID NO: 55);
- GGUUUUUGG (SEQ ID NO: 56);
- GGUUUUUUGG (SEQ ID NO: 57);
- GGUUUUUUUGG (SEQ ID NO: 58);
- GGUUUUUUUUGG (SEQ ID NO: 59);
- GGUUUUUUUUUGG (SEQ ID NO: 60);
- GGUUUUUUUUUUGG (SEQ ID NO: 61);
- GGUUUUUUUUUUUGG (SEQ ID NO: 62);
- GGUUUUUUUUUUUUGG (SEQ ID NO: 63);
- GGUUUUUUUUUUUUUGG (SEQ ID NO: 64);
- GGUUUUUUUUUUUUUUGG (SEQ ID NO: 65);
- GGUUUUUUUUUUUUUUUGG (SEQ ID NO: 66);
- GGGUUUGGG (SEQ ID NO: 67);
- GGGUUUUGGG (SEQ ID NO: 68);
- GGGUUUUUGGG (SEQ ID NO: 69);
- GGGUUUUUUGGG (SEQ ID NO: 70);
- GGGUUUUUUUGGG (SEQ ID NO: 71);
- GGGUUUUUUUUGGG (SEQ ID NO: 72);
- GGGUUUUUUUUUGGG (SEQ ID NO: 73);
- GGGUUUUUUUUUUGGG (SEQ ID NO: 74);
- GGGUUUUUUUUUUUGGG (SEQ ID NO: 75);
- GGGUUUUUUUUUUUUGGG (SEQ ID NO: 76);
- GGGUUUUUUUUUUUUUGGG (SEQ ID NO: 77);
- GGGUUUUUUUUUUUUUUUGGGUUUUUUUUUUUUUUUGGGUUUUUU UUUUUUUUUGGG (SEQ ID NO: 78);
- GGGUUUUUUUUUUUUUUUGGGGGUUUUUUUUUUUUUUUGGG (SEQ ID NO: 79);
- GGGUUUGGGUUUGGGUUUGGGUUUGGGUUUGGGUUUGGGUUUGGGU UUGGG (SEQ ID NO: 80);
- GGUUUUUUUUUUUUUUUGGG (short GU-rich, SEQ ID NO: 81)

or

- CCCUUUUUUUUUUUUUUUUCCCUUUUUUUUUUUUUUUUCCCUUUUUUUUUUUUUUUCCC (SEQ ID NO: 82)
- CCCUUUCCCUUUCCCUUUCCCUUUCCCUUUCCCUUUCCCUUUCCCUUUCCC (SEQ ID NO: 83)
- CCCUUUUUUUUUUUUUUUUCCCCCCUUUUUUUUUUUUUUUUCCC (SEQ ID NO: 84)

or from a sequence having at least 60%, 70%, 80%, 90%, or even 95% sequence identity with any of these sequences.

According to a further particularly preferred embodiment, such immunostimulatory nucleic acid sequences, particularly isRNA, consist of or comprise a nucleic acid of formula (IV) or (V):

$$(N_uG_lX_mG_nN_v)_a,\qquad \textbf{(formula (IV))}$$

wherein:

G is guanosine (guanine), uridine (uracil) or an analogue of guanosine (guanine) or uridine (uracil), preferably guanosine (guanine) or an analogue thereof;
X is guanosine (guanine), uridine (uracil), adenosine (adenine), thymidine (thymine), cytidine (cytosine), or an analogue of these nucleotides (nucleosides), preferably uridine (uracil) or an analogue thereof;
N is a nucleic acid sequence having a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides);
a is an integer from 1 to 20, preferably from 1 to 15, most preferably from 1 to 10;
l is an integer from 1 to 40, wherein when l = 1, G is guanosine (guanine) or an analogue thereof,
when l > 1, at least 50% of these nucleotides (nucleosides) are guanosine (guanine) or an analogue thereof;
m is an integer and is at least 3; wherein when m = 3, X is uridine (uracil) or an analogue thereof, and
when m > 3, at least 3 successive uridines (uracils) or analogues of uridine (uracil) occur;
n is an integer from 1 to 40, wherein when n = 1, G is guanosine (guanine) or an analogue thereof,
when n > 1, at least 50% of these nucleotides (nucleosides) are guanosine (guanine) or an analogue thereof;
u,v may be independently from each other an integer from 0 to 50, preferably wherein when u = 0, v ≥ 1, or
when v = 0, u ≥ 1;

wherein the nucleic acid molecule of formula (IV) has a length of at least 50 nucleotides, preferably of at least 100 nucleotides, more preferably of at least 150 nucleotides, even more preferably of at least 200 nucleotides and most preferably of at least 250 nucleotides.

$$(N_uC_lX_mC_nN_v)_a,\qquad \textbf{(formula (V))}$$

wherein:

C is cytidine (cytosine), uridine (uracil) or an analogue of cytidine (cytosine) or uridine (uracil), preferably cytidine (cytosine) or an analogue thereof;
X is guanosine (guanine), uridine (uracil), adenosine (adenine), thymidine (thymine), cytidine (cytosine) or an analogue of the above-mentioned nucleotides (nucleosides), preferably uridine (uracil) or an analogue thereof;
N is each a nucleic acid sequence having independent from each other a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides);
a is an integer from 1 to 20, preferably from 1 to 15, most preferably from 1 to 10;
l is an integer from 1 to 40,
wherein when l = 1, C is cytidine (cytosine) or an analogue thereof,
when l > 1, at least 50% of these nucleotides (nucleosides) are cytidine (cytosine) or an analogue thereof;
m is an integer and is at least 3;
wherein when m = 3, X is uridine (uracil) or an analogue thereof,
when m > 3, at least 3 successive uridines (uracils) or analogues of uridine (uracil) occur;
n is an integer from 1 to 40,
wherein when n = 1, C is cytidine (cytosine) or an analogue thereof,

when $n > 1$, at least 50% of these nucleotides (nucleosides) are cytidine (cytosine) or an analogue thereof.

u, v may be independently from each other an integer from 0 to 50, preferably wherein when $u = 0$, $v \geq 1$, or when $v = 0$, $u \geq 1$;

wherein the nucleic acid molecule of formula (V) according to the invention has a length of at least 50 nucleotides, preferably of at least 100 nucleotides, more preferably of at least 150 nucleotides, even more preferably of at least 200 nucleotides and most preferably of at least 250 nucleotides.

For formula (V), any of the definitions given above for elements N (i.e. $N_u$ and $N_v$) and X ($X_m$), particularly the core structure as defined above, as well as for integers a, l, m, n, u and v, similarly apply to elements of formula (V) correspondingly, wherein in formula (V) the core structure is defined by $C_lX_mC_n$. The definition of bordering elements $N_u$ and $N_v$ is identical to the definitions given above for $N_u$ and $N_v$.

According to a very particularly preferred embodiment, the nucleic acid molecule according to formula (IV) comprises, preferably consists of, e.g. any of the following sequences:

UAGCGAAGCUCUUGGACCUAGGUUUUUUUUUUUUUUUGGGUGCGUUCCUAGAA
GUACACG (SEQ ID NO: 85)

UAGCGAAGCUCUUGGACCUAGGUUUUUUUUUUUUUUUGGGUGCGUUCCUAGAA
GUACACGAUCGCUUCGAGAACCUGGAUCCAAAAAAAAAAAAAAACCCACGCAAGGA
UCUUCAUGUGC (SEQ ID NO: 86)

GGGAGAAAGCUCAAGCUUGGAGCAAUGCCCGCACAUUGAGGAAACCGAGUUGCAU
AUCUCAGAGUAUUGGCCCCCGUGUAGGUUAUUCUUGACAGACAGUGGAGCUUAU
UCACUCCCAGGAUCCGAGUCGCAUACUACGGUACUGGUGACAGACCUAGGUCGUC
AGUUGACCAGUCCGCCACUAGACGUGAGUCCGUCAAAGCAGUUAGAUGUUACACU
CUAUUAGAUC (SEQ ID NO: 87)

GGGAGAAAGCUCAAGCUUGGAGCAAUGCCCGCACAUUGAGGAAACCGAGUUGCAU
AUCUCAGAGUAUUGGCCCCCGUGUAGGUUAUUCUUGACAGACAGUGGAGCUUAU
UCACUCCCAGGAUCCGAGUCGCAUACUACGGUACUGGUGACAGACCUAGGUCGUC
AGUUGACCAGUCCGCCACUAGACGUGAGUCCGUCAAAGCAGUUAGAUGUUACACU
CUAUUAGAUCUCGGAUUACAGCUGGAAGGAGCAGGAGUAGUGUUCUUGCUCUAA
GUACCGAGUGUGCCCAAUACCCGAUCAGCUUAUUAACGAACGGCUCCUCCUCUUA
GACUGCAGCGUAAGUGCGGAAUCUGGGGAUCAAAUUACUGACUGCCUGGAUUAC
CCUCGGACAUAUAACCUUGUAGCACGCUGUUGCUGUAUAGGUGACCAACGCCCAC
UCGAGUAGACCAGCUCUCUUAGUCCGGACAAUGAUAGGAGGCGCGGUCAAUCUAC
UUCUGGCUAGUUAAGAAUAGGCUGCACCGACCUCUAUAAGUAGCGUGUCCUCUA
G (SEQ ID NO: 88)

GGGAGAAAGCUCAAGCUUGGAGCAAUGCCCGCACAUUGAGGAAACCGAGUUGCAUAUCUCAGAGUAUUGGCCCCCGUGUAGGUUAUUCUUGACAGACAGUGGAGCUUAUUCACUCCCAGGAUCCGAGUCGCAUACUACGGUACUGGUGACAGACCUAGGUCGUCAGUUGACCAGUCCGCCACUAGACGUGAGUCCGUCAAAGCAGUUAGAUGUUACACUCUAUUAGAUCUCGGAUUACAGCUGGAAGGAGCAGGAGUAGUGUUCUUGCUCUAAGUACCGAGUGUGCCCAAUACCCGAUCAGCUUAUUAACGAACGGCUCCUCCUCUUAGACUGCAGCGUAAGUGCGGAAUCUGGGGAUCAAAUUACUGACUGCCUGGAUUACCCUCGGACAUAUAACCUUGUAGCACGCUGUUGCUGUAUAGGUGACCAACGCCCACUCGAGUAGACCAGCUCUCUUAGUCCGGACAAUGAUAGGAGGCGCGGUCAAUCUACUUCUGGCUAGUUAAGAAUAGGCUGCACCGACCUCUAUAAGUAGCGUGUCCUCUAGAGCUACGCAGGUUCGCAAUAAAAGCGUUGAUUAGUGUGCAUAGAACAGACCUCU

UAUUCGGUGAAACGCCAGAAUGCUAAAUUCCAAUAACUCUUCCCAAAACGCGUACGGCCGAAGACGCGCGCUUAUCUUGUGUACGUUCUCGCACAUGGAAGAAUCAGCGGGCAUGGUGGUAGGGCAAUAGGGGAGCUGGGUAGCAGCGAAAAAGGGCCCCUGCGCACGUAGCUUCGCUGUUCGUCUGAAACAACCCGGCAUCCGUUGUAGCGAUCCCGUUAUCAGUGUUAUUCUUGUGCGCACUAAGAUUCAUGGUGUAGUCGACAAUAACAGCGUCUUGGCAGAUUCUGGUCACGUGCCCUAUGCCCGGGCUUGUGCCUCUCAGGUGCACAGCGAUACUUAAAGCCUUCAAGGUACUCGACGUGGGUACCGAUUCGUGACACUUCCUAAGAUUAUUCCACUGUGUUAGCCCCGCACCGCCGACCUAAACUGGUCCAAUGUAUACGCAUUCGCUGAGCGGAUCGAUAAUAAAAGCUUGAAUU (SEQ ID NO: 89)

GGGAGAAAGCUCAAGCUUAUCCAAGUAGGCUGGUCACCUGUACAACGUAGCCGGUAUUUUUUUUUUUUUUUUUUUUUUGACCGUCUCAAGGUCCAAGUUAGUCUGCCUAUAAAGGUGCGGAUCCACAGCUGAUGAAAGACUUGUGCGGUACGGUUAAUCUCCCCUUUUUUUUUUUUUUUUUUUUUUAGUAAAUGCGUCUACUGAAUCCAGCGAUGAUGCUGGCCCAGAUC (SEQ ID NO: 90)

GGGAGAAAGCUCAAGCUUAUCCAAGUAGGCUGGUCACCUGUACAACGUAGCCGGU
AUUUUUUUUUUUUUUUUUUUUUUUGACCGUCUCAAGGUCCAAGUUAGUCUGCCU
AUAAAGGUGCGGAUCCACAGCUGAUGAAAGACUUGUGCGGUACGGUUAAUCUCC
CCUUUUUUUUUUUUUUUUUUUUUUAGUAAAUGCGUCUACUGAAUCCAGCGAUGA
UGCUGGCCCAGAUCUUCGACCACAAGUGCAUAUAGUAGUCAUCGAGGGUCGCCU
UUUUUUUUUUUUUUUUUUUUUUGGCCCAGUUCUGAGACUUCGCUAGAGACUAC
AGUUACAGCUGCAGUAGUAACCACUGCGGCUAUUGCAGGAAAUCCCGUUCAGGU
UUUUUUUUUUUUUUUUUUUUUCCGCUCACUAUGAUUAAGAACCAGGUGGAGUGU
CACUGCUCUCGAGGUCUCACGAGAGCGCUCGAUACAGUCCUUGGAAGAAUCUUU
UUUUUUUUUUUUUUUUUUUGUGCGACGAUCACAGAGAACUUCUAUUCAUGCAG
GUCUGCUCUA (R722A or isRNA722A; SEQ ID NO: 91)

GGGAGAAAGCUCAAGCUUAUCCAAGUAGGCUGGUCACCUGUACAACGUAGCCGGU
AUUUUUUUUUUUUUUUUUUUUUUUGACCGUCUCAAGGUCCAAGUUAGUCUGCCU
AUAAAGGUGCGGAUCCACAGCUGAUGAAAGACUUGUGCGGUACGGUUAAUCUCC
CCUUUUUUUUUUUUUUUUUUUUUUAGUAAAUGCGUCUACUGAAUCCAGCGAUGA

UGCUGGCCCAGAUCUUCGACCACAAGUGCAUAUAGUAGUCAUCGAGGGUCGCCU
UUUUUUUUUUUUUUUUUUUUUUGGCCCAGUUCUGAGACUUCGCUAGAGACUAC
AGUUACAGCUGCAGUAGUAACCACUGCGGCUAUUGCAGGAAAUCCCGUUCAGGU
UUUUUUUUUUUUUUUUUUUUUCCGCUCACUAUGAUUAAGAACCAGGUGGAGUGU
CACUGCUCUCGAGGUCUCACGAGAGCGCUCGAUACAGUCCUUGGAAGAAUCUUU
UUUUUUUUUUUUUUUUUUUGUGCGACGAUCACAGAGAACUUCUAUUCAUGCAG
GUCUGCUCUAG (R722B or isRNA722B; SEQ ID NO: 101)

GGGAGAAAGCUCAAGCUUAUCCAAGUAGGCUGGUCACCUGUACAACGUAGCCGGU AUUUUUUUUUUUUUUUUUUUUUUGACCGUCUCAAGGUCCAAGUUAGUCUGCCU AUAAAGGUGCGGAUCCACAGCUGAUGAAAGACUUGUGCGGUACGGUUAAUCUCC CCUUUUUUUUUUUUUUUUUUUUUAGUAAAUGCGUCUACUGAAUCCAGCGAUGA UGCUGGCCCAGAUCUUCGACCACAAGUGCAUAUAGUAGUCAUCGAGGGUCGCCU UUUUUUUUUUUUUUUUUUUUUGGCCCAGUUCUGAGACUUCGCUAGAGACUAC AGUUACAGCUGCAGUAGUAACCACUGCGGCUAUUGCAGGAAAUCCCGUUCAGGU UUUUUUUUUUUUUUUUUUUUCCGCUCACUAUGAUUAAGAACCAGGUGGAGUGU CACUGCUCUCGAGGUCUCACGAGAGCGCUCGAUACAGUCCUUGGAAGAAUCUUU UUUUUUUUUUUUUUUUUUUGUGCGACGAUCACAGAGAACUUCUAUUCAUGCAG GUCUGCUCUAGAACGAACUGACCUGACGCCUGAACUUAUGAGCGUGCGUAUUUU UUUUUUUUUUUUUUUUUUUCCUCCCAACAAAUGUCGAUCAAUAGCUGGGCUGU UGGAGACGCGUCAGCAAAUGCCGUGGCUCCAUAGGACGUGUAGACUUCUAUUUU UUUUUUUUUUUUUUUUUUCCCGGGACCACAAAUAAUAUUCUUGCUUGGUUGGGC GCAAGGGCCCCGUAUCAGGUCAUAAACGGGUACAUGUUGCACAGGCUCCUUUUU UUUUUUUUUUUUUUUUUUCGCUGAGUUAUUCCGGUCUCAAAAGACGGCAGACG UCAGUCGACAACACGGUCUAAAGCAGUGCUACAAUCUGCCGUGUUCGUGUUUUU UUUUUUUUUUUUUUUGUGAACCUACACGGCGUGCACUGUAGUUCGCAAUUCAU AGGGUACCGGCUCAGAGUUAUGCCUUGGUUGAAAACUGCCCAGCAUACUUUUUU UUUUUUUUUUUUUUCAUAUUCCCAUGCUAAGCAAGGGAUGCCGCGAGUCAUGU UAAGCUUGAAUU (SEQ ID NO: 92)

or a nucleic acid sequence having at least 60%, preferably at least 70%, preferably at least 80%, more preferably at least 90%, and most preferably at least 95% identity to any of the above defined sequences.

According to another very particularly preferred embodiment, the nucleic acid molecule according to formula (V) comprises, preferably consists of, e.g. any of the following sequences:

UAGCGAAGCUCUUGGACCUACCUUUUUUUUUUUUUUCCCUGCGUUCCUAGAAGU ACACG (SEQ ID NO: 93)

or

UAGCGAAGCUCUUGGACCUACCUUUUUUUUUUUUUUUCCCUGCGUUCCUAGAAG UACACGAUCGCUUCGAGAACCUGGAUGGAAAAAAAAAAAAAAAGGACGCAAGGAU CUUCAUGUGC (SEQ ID NO: 94)

or a nucleic acid sequence having at least 60%, preferably at least 70%, preferably at least 80%, more preferably at least 90%, and most preferably at least 95% identity to any of the above defined sequences.

In a further preferred embodiment, the nucleic acid molecule of the herein defined complex may also occur in the form of a "modified nucleic acid" as defined herein.

According to a first embodiment, the nucleic acid molecule of the herein defined complex may be provided as a "stabilized nucleic acid", i.e. as a stabilized RNA and, optionally additionally, as a stabilized DNA, more preferably as a RNA that is essentially resistant to *in vivo* degradation (e.g. by an exo- or endo-nuclease) as defined herein.

According to another embodiment, the nucleic acid cargo of the herein defined complex may be modified as defined herein, and/or stabilized, especially if the nucleic acid molecule is in the form of a coding nucleic acid e.g. an mRNA, by modifying the G/C content of the nucleic acid molecule, particularly an mRNA, preferably of the coding region thereof as defined herein.

Nucleic acid molecules used herein as cargo comprised in the complex as defined herein may be prepared using any method known in the art, including the methods for nucleic acid synthesis as defined herein.

Furthermore, the present invention explicitly encloses variants and fragments of nucleic acid molecules as defined herein comprised as nucleic acid cargo in the complex.

Particularly preferred nucleic acid cargo molecules in the context of the present invention are nucleic acid molecules comprising, preferably consisting of, a nucleic acid sequence according to SEQ ID NO. 91 or 101 or a sequence which is at least 60%, preferably at least 70%, preferably at least 80%, more preferably at least 90%, and most preferably at least 95% identical to SEQ ID NO. 91 or 101.

Furthermore, in the complex, the cationic and/or polycationic components of the carrier as defined herein and the nucleic acid cargo are provided in an N/P-ratio of 0.4 to 0.9, such as in the range of 0.4 to 0.6. Most preferably, the N/P ratio lies in a ratio range between 0.5 and 0.9. In this context, the N/P ratio is a measure of the ionic charge of the cationic (side chain) component(s) of the carrier or of the carrier as such. In particular, if the cationic properties of the cationic component(s) are generated mostly by nitrogens (e.g. of the amino acid side chains), the N/P ratio expresses the ratio of basic nitrogen atoms to phosphate residues in the nucleotide backbone, considering that (side chain) nitrogen atoms in the cationic component of the carrier contribute to positive charges and phosphate of the phosphate backbone of the nucleic acid contribute to the negative charge. Generally, one phosphate provides one negative charge, e.g. one nucleotide in the cargo nucleic acid molecule provides one negative charge. A formula is given in the Examples. The N/P-ratio is defined as the nitrogen/phosphate ratio (N/P-ratio) of the entire complex. This is typically illustrative for the content/amount of cationic components, in the carrier and characteristic for the content/amount of nucleic acids bound or complexed in the complex. It may be calculated on the basis that, for example, 1 $\mu$g RNA typically contains about 3 nmol phosphate residues, provided that RNA exhibits a statistical distribution of bases. Additionally, 1 nmol peptide typically contains about x nmol nitrogen residues, dependent on the number of (cationic) amino acids and the pH of the solution (or environment).

The N/P ratio significantly influences the surface charge of the resulting complex. Thus it is preferable that the resulting complex is negatively charged. The surface charge of the resulting complex can be indicated as Zetapotential which may be measured by Doppler electrophoresis method using a Zetasizer Nano (Malvern Instruments, Malvern, UK) as described herein.

The complex as used in the present invention, such as for use as an adjuvant, is preferably capable of triggering a non-antigen-specific, (innate) immune reaction (as provided by the innate immune system), preferably in an immunostimulating manner. An immune reaction can generally be brought about in various ways. An important factor for a suitable immune response is the stimulation of different T-cell sub-populations. T-lymphocytes typically differentiate into two sub-populations, the T-helper 1 (Th1) cells and the T-helper 2 (Th2) cells, with which the immune system is capable of destroying intracellular (Th1) and extracellular (Th2) pathogens (e.g. antigens). The two Th cell populations differ in the pattern of effector proteins (cytokines) produced by them. Thus, Th1 cells assist the cellular immune response by activation of macrophages and cytotoxic T-cells. Th2 cells, on the other hand, promote the humoral immune response by stimulation of B-cells for conversion into plasma cells and by formation of antibodies (e.g. against antigens). The Th1/Th2 ratio is therefore of great importance in the immune response. In connection with the present invention, the Th1/Th2 ratio of the immune response is preferably displaced by the adjuvant or immunostimulating agent, in particular the complex, in the direction towards the Th1 response, and therefore IgG2a antibodies and a cellular immune response are predominantly induced. As described above, the complex can induce an unspecific innate immune response, which may allow the support of a specific adaptive immune response elicited by the antigen.

**Determination of the (innate) immunostimulatory or adjuvant capacity of a component in the inventive pharmaceutical composition:**

For the determination of the immunostimulatory capacity of an immunostimulating agent or adjuvant (in particular of a complex as used in the present invention) several methods are known in the art and may be used. E.g., in vitro methods are advantageous to utilize for compounds as to their capacity to induce cytokines, which are (exclusively or at least typically) part of the innate immune system and thereby (as an additional arm of the immune system) typically improve the induction of an antigen-specific immune response caused by an antigen. For this purpose, e.g. PBMCs may be isolated from blood samples and stimulated with the particular immunostimulating agent or adjuvant. After incubation, secretion of the desired cytokines (e.g. as a reaction of an activation of the PAMP receptors) being typically part of the innate immune system (and not of the antigen-specific immune system) is determined by ELISA. These selected cytokines may be used in the art as determinants of the induction of an innate immune response in the body. In this context, the secretion of TNF-alpha and IFN-alpha is preferably measured to determine the unspecific (innate immune response) evoked by a compound or complex. Especially, IFN-alpha plays an important role in the induction of an unspecific immune response after viral infection and can be used as an indicator of induction of a Th1-shifted adaptive immune response, which is particularly preferred in the context of the treatment of cancer or tumour diseases and specific infectious diseases, like e.g. Influenza or RSV. Accordingly, it is particularly preferred that the immunostimulatory compound or complex tested in the screening assay, induces the secretion of e.g. IFN-alpha. Such a compound or complex may then be applied e.g. for the use as an immunostimulating agent (triggering the unspecific (innate) immune response) in vaccination therapies, particularly in the context of peptide or protein antigens which predominantly induce a Th2-shifted immune response.

IFN-alpha is part of the family of type I interferons. Type I interferons (IFN) are pleiotropic cytokines that are essential for supporting anti-viral immune responses. They induce apoptosis of virus-infected cells and cellular resistance to viral infection, in addition to activating natural killer (NK) and T cells. Type I interferons have effects on a large set of cytokines and chemokines that i.a. influence immunocyte maturation, homing, effector functions and apoptosis. Typically, a major role of IFN-alpha is the induction of a priming state affecting the production and regulation of other mediators, including cytokines. For example, IFN-alpha signalling upregulates IFN-alpha production by dendritic cells (DCs) and T cells and thereby favours the induction and maintenance of Th1 cells. Shifting of an immune response in direction of a Th1 immune response may become particularly important, once protein or peptide vaccines are used, because these vaccines usually induce a Th2-based immune response which consequently prevents or decreases the induction of cytotoxic T cells.

Therefore, it is preferred that a compound or complex to be used as an adjuvant in the context of the present invention may preferably have the property of shifting an antigen-specific immune response caused by a antigen to a Th1-based immune response. The direction of an immune response induced by an antigen is usually measured by determination of the induction of several subtypes of antigen-specific antibodies and the induction of antigen-specific cytotoxic $CD8^+$ T cells. In this context, the subtype antibody IgG1 represents the induction of a Th2-based immune response and the induction of the subtype antibody IgG2a and the induction of cytotoxic T cells represent the induction of a Th1-based immune response. The induction of antigen-specific antibodies is typically determined by measurement of the antibody titer in the blood of the vaccinee by ELISA. The induction of antigen-specific cytotoxic T cells is typically determined by measurement of IFN-gamma secretion in splenocytes after stimulation with antigen-specific peptides or proteins by ELISPOT. In this context, the induction of IFN-gamma secretion provides evidence that antigen-specific cytotoxic T cells are present in the spleen and which can specifically attack cells that present epitopes of the antigen on MHC I molecules on their surface.

For the determination of beneficial properties of an adjuvant, *in vivo* vaccinations are typically performed. Therewith, it is possible to investigate, if the adjuvant or immunostimulatory compound or complex improves an antigen-specific immune response caused by the vaccine or antigen and, furthermore, if it can shift an antigen-specific immune response in the desired direction to display adjuvant properties. Particularly, in the induction of an anti-tumoral immune response the induction of a Th1-shifted immune response, especially the induction of cytotoxic T cells is believed to play a major role, because the induction of antigen-specific cytotoxic T cells are believed to represent an indispensable prerequisite for the successful combat of a tumour.

Accordingly, the methods to screen for, test and/or investigate compound or complexes which exhibit properties as adjuvants are well known in the art and may readily be applied e.g. by ELISA tests measuring the immune response elicited by the tested compounds/complexes.

As a second ingredient the inventive pharmaceutical composition comprises at least one peptide or protein antigen selected from an antigen from a pathogen associated with infectious disease; an antigen associated with allergy or allergic disease; an antigen associated with autoimmune disease; or an antigen associated with a cancer or tumour disease, or in each case a fragment, variant and/or derivative of said antigen.

It is also disclosed that the at least one antigen can be provided as nucleic acid coding for the at least one antigen, or as antigenic cells, antigenic cellular fragments, cellular fractions; cell wall components, modified, attenuated or inactivated (e.g. chemically or by irradiation) pathogens (virus, bacteria etc.) comprising the at least one antigen.

The pharmaceutical composition of the invention comprises the peptide or protein antigen, or a functional fragment, variant and/or derivative of said peptide or protein antigen.

In specific embodiments, said peptide or protein antigen may be comprised in a preparation of an inactivated or attenuated pathogen (e.g. virus) or may be comprised in an antigenic cell preparation.

In other embodiments, said peptide or protein antigen is a recombinant expressed peptide or protein (peptide or protein manufactured by recombinant peptide or protein production, as defined herein) or a synthesized peptide (peptide manufactured by peptide synthesis, as defined herein).

a) Antigens from a pathogen associated with infectious disease:

Antigens from a pathogen associated with infectious disease are derived from a pathogen which is associated with the induction of an infectious disease. Said antigen is a peptide or protein antigen, or a functional fragment, variant and/or derivative of said peptide or protein antigen, and/or is comprised in, provided as and/or derived from (e.g. a preparation of) inactivated or attenuated said pathogen, (e.g. a virus such as any one described herein). In this context, the peptide or protein antigen may be comprised in provided as and/or derived from (e.g. a preparation of) an attenuated or inactivated pathogen (e.g. a virus such as any one described herein) associated with infectious disease.

In alternative embodiments of all aspects of the invention, an antigen (e.g. a peptide or protein antigen) used in the present invention is not one comprised in (e.g. a preparation of) inactivated or attenuated virus (such as any one described herein, or any pathogen described herein); and/or is one that is not provided as (e.g. a preparation of) inactivated or attenuated said virus or pathogen; and/or is one that is not derived from (e.g. a preparation of) inactivated or attenuated said virus or pathogen. For example, the antigen used in any aspect of the present invention may be, or may be provided as, an isolated and/or purified protein or peptide antigen. As will be understood by the person of ordinary skill, an isolated (and/or purified) antigen includes such antigens that are present (or provided) in a (starting) composition that has less than about 40%, 30%, 20%, 10%, 5%, 2% or 1% non-desired or specified other components such as other proteins/peptides or impurities.

In particular embodiments, the (e.g. protein or peptide) antigen used in the present invention is a recombinant antigen, for example one that is prepared using recombinant production, such as using those methodologies described herein. In alternative embodiments, the (e.g. protein or peptide) antigen used in the present invention is a synthetic antigen, for example one that is prepared using peptide synthesis, such as using those methodologies described herein.

Antigens from a pathogen associated with infectious disease are selected from antigens from the pathogens Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia and other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheriae, Coxiella burnetii, Crimean-Congo hemorrhagic fever virus, Cryptococcus neoformans, Cryptosporidium genus, Cytomegalovirus, Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and Enterovirus 71 (EV71), Epidermophyton spp, Epstein-Barr Virus (EBV), Escherichia coli 0157:H7, 0111 and 0104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenzae, Helicobacter pylori, Henipavirus (Hendra virus Nipah virus), Hepatitis A Virus, Hepatitis B Virus, Hepatitis C Virus, Hepatitis D Virus, Hepatitis E Virus, Herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV-7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family, Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, rhinoviruses, Rickettsia akari, Rickettsia genus, Rickettsia prowazekii, Rickettsia rickettsii, Rickettsia typhi, Rift Valley fever virus, Rotavirus, Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yellow fever virus, Yersinia enterocolitica, Yersinia pestis, and Yersinia pseudotuberculosis.

In this context particularly preferred are antigens from the pathogens selected from Influenza, Rabies virus, Hepatitis B virus, human Papilloma virus (hPV), Bacillus anthracis, respiratory syncytial virus (RSV), herpes simplex virus (HSV), and Mycobacterium tuberculosis.

Furthermore, the antigen from a pathogen associated with infectious disease may be selected from the following antigens: Outer membrane protein A OmpA, biofilm associated protein Bap, transport protein MucK (Acinetobacter baumannii, Acinetobacter infections)); variable surface glycoprotein VSG, microtubule-associated protein MAPP15, trans-sialidase TSA (Trypanosoma brucei, African sleeping sickness (African trypanosomiasis)); HIV p24 antigen, HIV Eenvelope proteins (Gp120, Gp41, Gp160), polyprotein GAG, negative factor protein Nef, trans-activator of transcription Tat (HIV (Human immunodeficiency virus), AIDS (Acquired immunodeficiency syndrome)); galactose-inhibitable adherence protein GIAP, 29 kDa antigen Eh29, Gal/GalNAc lectin, protein CRT, 125 kDa immunodominant antigen, protein M17, adhesin ADH112, protein STIRP (Entamoeba histolytica, Amoebiasis); Major surface proteins 1-5 (MSP1a, MSP1b, MSP2, MSP3, MSP4, MSP5), type IV secreotion system proteins (VirB2, VirB7, VirB11, VirD4) (Anaplasma genus, Anaplasmosis); protective Antigen PA, edema factor EF, lethal facotor LF, the S-layer homology proteins SLH (Bacillus anthracis, Anthrax); acranolysin, phospholipase D, collagen-binding protein CbpA (Arcanobacterium haemolyticum, Arcanobacterium haemolyticum infection); nucleocapsid protein NP, glycoprotein precursor GPC, glycoprotein GP1, glycoprotein GP2 (Junin virus, Argentine hemorrhagic fever); chitin-protein layer proteins, 14 kDa suarface antigen A14, major sperm protein MSP, MSP polymerization-organizing protein MPOP, MSP fiber protein 2 MFP2, MSP polymerization-activating kinase MPAK, ABA-1-like protein ALB, protein ABA-1, cuticulin CUT-1 (Ascaris lumbricoides, Ascariasis); 41 kDa allergen Asp v13, allergen Asp f3, major conidial surface protein rodlet A, protease Pep1p, GPI-anchored protein Gel1p, GPI-anchored protein Crf1p (Aspergillus genus, Aspergillosis); family VP26 protein, VP29 protein (Astroviridae, Astrovirus infection); Rhoptry-associated protein 1 RAP-1, merozoite surface antigens MSA-1, MSA-2 (a1, a2, b, c), 12D3, 11C5, 21B4, P29, variant erythrocyte surface antigen VESA1, Apical Membrane Antigen 1 AMA-1 (Babesia genus, Babesiosis); hemolysin, enterotoxin C, PXO1-51, glycolate oxidase, ABC-transporter, penicillin-bingdn protein, zinc transporter family protein, pseudouridine synthase Rsu, plasmid replication protein RepX, oligoendopeptidase F, prophage membrane protein, protein HemK, flagellar antigen H, 28.5-kDa cell surface antigen (Bacillus cereus, Bacillus cereus infection); large T antigen LT, small T antigen, capsid protein VP1, capsid protein VP2 (BK virus, BK virus infection); 29 kDa-protein, caspase-3-like antigens, glycoproteins (Blastocystis hominis, Blastocystis hominis infection); yeast surface adhesin WI-1 (Blastomyces dermatitidis, Blastomycosis); nucleoprotein N, polymerase L, matrix protein Z, glycoprotein GP (Machupo virus, Bolivian hemorrhagic fever); outer surface protein A OspA, outer surface protein OspB, outer surface protein OspC, decorin binding protein A DbpA, decorin binding protein B DbpB, flagellar filament 41 kDa core protein Fla, basic membrane protein A precursor BmpA (Immunodominant antigen P39), outer surface 22 kDa lipoprotein precursor (antigen IPLA7), variable surface lipoprotein vlsE (Borrelia genus, Borrelia infection); Botulinum neurotoxins BoNT/A1, BoNT/A2, BoNT/A3, BoNT/B, BoNT/C, BoNT/D, BoNT/E, BoNT/F, BoNT/G, recombinant botulinum toxin F Hc domain FHc (Clostridium botulinum, Botulism (and Infant botulism)); nucleocapsid, glycoprotein precursor (Sabia virus, Brazilian hemorrhagic fever); copper/Zinc superoxide dismutase SodC, bacterioferritin Bfr, 50S ribosomal protein RplL, OmpA-like transmembrane domain-containing protein Omp31, immunogenic 39-kDa protein M5 P39, zinc ABC transporter periplasmic zinc-bnding protein znuA, periplasmic immunogenic protein Bp26, 30S ribosomal protein S12 RpsL, glyceraldehyde-3-phosphate dehydrogenase Gap, 25 kDa outer-membrane immunogenic protein precursor Omp25, invasion protein B lalB, trigger factor Tig, molecular chaperone DnaK, putative peptidyl-prolyl cis-trans isomerase SurA, lipoprotein Omp19, outer membrane protein MotY Omp16, conserved outer membrane protein D15, malate dehydrogenase Mdh, component of the Type-IV secretion system (T4SS) VirJ, lipoprotein of unknown function BAB1_0187 (Brucella genus, Brucellosis); members of the ABC transporter family (LolC, OppA, and PotF), putative lipoprotein releasing system transmembrane protein LolC/E, flagellin FliC, Burkholderia intracellular motility A BimA, bacterial Elongation factor-Tu EF-Tu, 17 kDa OmpA-like protein, boaA coding protein, boaB coding protein (Burkholderia cepacia and other Burkholderia species, Burkholderia infection); mycolyl-transferase Ag85A, heat-shock protein Hsp65, protein TB10.4, 19 kDa antigen, protein PstS3, heat-shock protein Hsp70 (Mycobacterium ulcerans, Buruli ulcer); norovirus major and minor viral capsid proteins VP1 and VP2, genome polyprotein, Sapoviurus capsid protein VP1, protein Vp3, geome polyprotein (Caliciviridae family, Calicivirus infection (Norovirus and Sapovirus)); major outer membrane protein PorA, flagellin FlaA, surface antigen CjaA, fibronectin binding protein CadF, aspartate/glutamate-binding ABC transporter protein Peb1A, protein FspA1, protein FspA2 (Campylobacter genus, Campylobacteriosis); glycolytic enzyme enolase, secreted aspartyl proteinases SAP1-10, glycophosphatidylinositol (GPI)-linked cell wall protein, protein Hyr1, complement receptor 3-related protein CR3-RP, adhesin Als3p, heat shock protein 90 kDa hsp90, cell surface hydrophobicity protein CSH (usually Candida albicans and other Candida species, Candidiasis); 17-kDa antigen, protein P26, trimeric autotransporter adhesins TAAs, Bartonella adhesin A BadA, variably expressed outer-membrane proteins Vomps, protein Pap3, protein HbpA, envelope-associated protease HtrA, protein OMP89, protein GroEL, protein LalB, protein OMP43, dihydrolipoamide succinyltransferase SucB (Bartonella henselae, Cat-scratch disease); amastigote surface protein-2, amastigote-specific surface protein SSP4, cruzipain, trans-sialidase TS, trypomastigote surface glycoprotein TSA-1, complement regulatory protein CRP-10, protein G4, protein G2, paraxonemal rod protein PAR2, paraflagellar rod component Par1, mucin-Associated Surface Proteins MPSP (Trypanosoma cruzi, Chagas Disease (American trypanosomiasis)); envelope glycoproteins (gB, gC, gE, gH, gI, gK, gL) (Varicella zoster virus (VZV), Chickenpox); major outer membrane protein MOMP, probable outer membrane protein PMPC, outer membrane complex protein B OmcB, heat shock proteins Hsp60 HSP10, protein IncA, proteins from the type III secretion system, ribonucleotide reductase small chain protein NrdB, plasmid protein Pgp3, chlamydial outer protein N CopN, antigen CT521, antigen CT425, antigen CT043, antigen TC0052, antigen TC0189, antigen TC0582, antigen TC0660, antigen TC0726, antigen TC0816, antigen TC0828 (Chlamydia trachomatis, Chlamydia); low calcium response protein E LCrE, chlamydial outer protein N CopN, serine/threonine-protein kinase PknD, acyl-carrier-protein S-malonyltransferase FabD, single-stranded DNA-binding protein Ssb, major outer membrane protein MOMP, outer membrane protein 2 Omp2, polymorphic membrane protein family (Pmp1, Pmp2, Pmp3, Pmp4, Pmp5, Pmp6, Pmp7, Pmp8, Pmp9, Pmp10, Pmp11, Pmp12, Pmp13, Pmp14, Pmp15, Pmp16, Pmp17, Pmp18, Pmp19, Pmp20, Pmp21) (Chlamydophila pneumoniae, Chlamydophila pneumoniae infection); cholera toxin B CTB, toxin coregulated pilin A TcpA, toxin coregulated pilin TcpF, toxin co-regulated pilus biosynthesis ptrotein F TcpF, cholera enterotoxin subunit A, cholera enterotoxin subunit B, Heat-stable enterotoxin ST, mannose-sensitive hemagglutinin MSHA, outer membrane protein U Porin ompU, Poring B protein, polymorphic membrane protein-D (Vibrio cholerae, Cholera); propionyl-CoA carboxylase PCC, 14-3-3 protein, prohibitin, cysteine proteases, glutathione transferases, gelsolin, cathepsin L proteinase CatL, Tegumental Protein 20.8 kDa TP20.8, tegumental protein 31.8 kDa TP31.8, lysophosphatidic acid phosphatase LPAP, (Clonorchis sinensis, Clonorchiasis); surface layer proteins SLPs, glutamate dehydrogenase antigen GDH, toxin A, toxin B, cysteine protease Cwp84, cysteine protease Cwp13, cysteine protease Cwp19, Cell Wall Protein CwpV, flagellar protein FliC, flagellar protein FliD (Clostridium difficile, Clostridium difficile infection); rhinoviruses: capsid proteins VP1, VP2, VP3, VP4; coronaviruses: sprike proteins S, envelope proteins E, membrane proteins M, nucleocapsid proteins N (usually rhinoviruses and coronaviruses, Common cold (Acute viral rhinopharyngitis; Acute coryza)); prion protein Prp (CJD prion, Creutzfeldt-Jakob disease (CJD)); envelope protein Gc, envelope protein Gn, nucleocapsid proteins (Crimean-Congo hemorrhagic fever virus, Crimean-Congo hemorrhagic fever (CCHF)); virulence-associated DEAD-box RNA helicase VAD1, galactoxylomannan-protein GalXM, glucuronoxylomannan GXM, mannoprotein MP (Cryptococcus neoformans, Cryptococcosis); acidic ribosomal protein P2 CpP2, mucin antigens Muc1, Muc2, Muc3 Muc4, Muc5, Muc6, Muc7, surface adherence protein CP20, surface adherence protein CP23, surface protein CP12, surface protein CP21, surface protein CP40, surface protein CP60, surface protein CP15, surface-associated glycopeptides gp40, surface-associated glycopeptides gp15, oocyst wall protein AB, profilin PRF, apyrase (Cryptosporidium genus, Cryptosporidiosis); fatty acid and retinol binding protein-1 FAR-1, tissue inhibitor of metalloproteinase TIMP (TMP), cysteine proteinase ACEY-1, cysteine proteinase ACCP-1, surface antigen Ac-16, secreted protein 2 ASP-2, metalloprotease 1 MTP-1, aspartyl protease inhibitor API-1, surface-associated antigen SAA-1, adult-specific secreted factor Xa serine protease inhibitor anticoagulant AP, cathepsin D-like aspartic protease ARR-1 (usually Ancylostoma braziliense; multiple other parasites, Cutaneous larva migrans (CLM)); cathepsin L-like proteases, 53/25-kDa antigen, 8kDa family members, cysticercus protein with a marginal trypsin-like activity TsAg5, oncosphere protein TSOL18, oncosphere protein TSOL45-1A, lactate dehydrogenase A LDHA, lactate dehydrogenase B LDHB (Taenia solium, Cysticercosis); pp65 antigen, membrane protein pp15, capsid-proximal tegument protein pp150, protein M45, DNA polymerase UL54, helicase UL105, glycoprotein gM, glycoprotein gN, glcoprotein H, glycoprotein B gB, protein UL83, protein UL94, protein UL99 (Cytomegalovirus, Cytomegalovirus infection); capsid protein C, premembrane protein prM, membrane protein M, envelope protein E (domain I, domain II, domain II), protein NS1, protein NS2A, protein NS2B, protein NS3, protein NS4A, protein 2K, protein NS4B, protein NS5 (Dengue viruses (DEN-1, DEN-2, DEN-3 and DEN-4)-Flaviviruses, Dengue fever); 39 kDa protein (Dientamoeba fragilis, Dientamoebiasis); diphtheria toxin precursor Tox, diphteria toxin DT, pilin-specific sortase SrtA, shaft pilin protein SpaA, tip pilin protein SpaC, minor pilin protein SpaB, surface-associated protein DIP1281 (Corynebacterium diphtheriae, Diphtheria); glycoprotein GP, nucleoprotein NP, minor matrix protein VP24, major matrix protein VP40, transcription activator VP30, polymerase cofactor VP35, RNA polymerase L (Ebolavirus (EBOV), Ebola hemorrhagic fever); prion protein (vCJD prion, Variant Creutzfeldt-Jakob disease (vCJD, nvCJD)); UvrABC system protein B, protein Flp1, protein Flp2, protein Flp3, protein TadA, hemoglobin receptor HgbA, outer membrane protein TdhA, protein CpsRA, regulator CpxR, protein SapA, 18 kDa antigen, outer membrane protein NcaA, protein LspA, protein LspA1, protein LspA2, protein LspB, outer membrane component DsrA, lectin DltA, lipoprotein Hlp, major outer membrane protein OMP, outer membrane protein OmpA2 (Haemophilus ducreyi, Chancroid); aspartyl protease 1 Pep1, phospholipase B PLB, alpha-mannosidase 1 AMN1, glucanosyltransferase GEL1, urease URE, peroxisomal matrix protein Pmp1, proline-rich antigen Pra, humal T-cell reative protein TcrP (Coccidioides immitis and Coccidioides posadasii, Coccidioidomycosis); allergen Tri r 2, heat shock protein 60 Hsp60, fungal actin Act, antigen Tri r2, antigen Tri r4, antigen Tri t1, protein IV, glycerol-3-phosphate dehydrogenase Gpd1, osmosensor HwSho1A, osmosensor HwSho1B, histidine kinase HwHhk7B, allergen Mala s 1, allergen Mala s 11, thioredoxin Trx Mala s 13, allergen Mala f, allergen Mala s (usually Trichophyton spp, Epidermophyton spp., Malassezia spp., Hortaea werneckii, Dermatophytosis); protein EG95, protein EG10, protein EG18, protein EgA31, protein EM18, antigen EPC1, antigen B, antigen 5, protein P29, protein 14-3-3, 8-kDa protein, myophilin, heat shock protein 20 HSP20, glycoprotein GP-89, fatty acid binding protein FAPB (Echinococcus genus, Echinococcosis); major surface protein 2 MSP2, major surface protein 4 MSP4, MSP variant SGV1, MSP variant SGV2, outer membrane protein OMP, outer membrande protein 19 OMP-19, major antigenic protein MAP1, major antigenic protein MAP1-2, major antigenic protein MAP1B, major antigenic protein MAP1-3, Erum2510 coding protein, protein GroEL, protein GroES, 30-kDA major outer membrane proteins, GE 100-kDa protein, GE 130-kDa protein, GE 160-kDa protein (Ehrlichia genus, Ehrlichiosis); secreted antigen SagA, sagA-like proteins SalA and SalB, collagen adhesin Scm, surface proteins Fms1 (EbpA(fm), Fms5 (EbpB(fm), Fms9 (EpbC(fm) and Fms10, protein EbpC(fm), 96 kDa immunoprotective glycoprotein G1 (Enterococcus genus, Enterococcus infection); genome polyprotein, polymerase 3D, viral capsid protein VP1, viral capsid protein VP2, viral capsid protein VP3, viral capsid protein VP4, protease 2A, protease 3C (Enterovirus genus, Enterovirus infection); outer membrane proteins OM, 60 kDa outer membrane protein, cell surface antigen OmpA, cell surface antigen OmpB (sca5), 134 kDa outer membrane protein, 31 kDa outer membrane protein, 29.5 kDa outer membrane protein, cell surface protein SCA4, cell surface protein Adr1 (RP827), cell surface protein Adr2 (RP828), cell surface protein SCA1, Invasion protein invA, cell division protein fts, secretion proteins sec 0family, virulence proteins virB, tlyA, tlyC, parvulin-like protein Plp, preprotein translocase SecA, 120-kDa surface protein antigen SPA, 138 kD complex antigen, major 100-kD protein (protein I), intracytoplasmic protein D, protective surface protein antigen SPA (Rickettsia prowazekii, Epidemic typhus); Epstein-Barr nuclear antigens (EBNA-1, EBNA-2, EBNA-3A, EBNA-3B, EBNA-3C, EBNA-leader protein (EBNA-LP)), latent membrane proteins (LMP-1, LMP-2A, LMP-2B), early antigen EBV-EA, membrane antigen EBV-MA, viral capsid antigen EBV-VCA, alkaline nuclease EBV-AN, glycoprotein H, glycoprotein gp350, glycoprotein gp110, glycoprotein gp42, glycoprotein gHgL, glycoprotein gB (Epstein-Barr Virus (EBV), Epstein-Barr Virus Infectious Mononucleosis); cpasid protein VP2, capsid protein VP1, major protein NS1 (Parvovirus B19, Erythema infectiosum (Fifth disease)); pp65 antigen, glycoprotein 105, major capsid protein, envelope glycoprotein H, protein U51 (Human herpesvirus 6 (HHV-6) and Human herpesvirus 7 (HHV-7), Exanthem subitum); thioredoxin-glutathione reductase TGR, cathepsins L1 and L2, Kunitz-type protein KTM, leucine aminopeptidase LAP, cysteine proteinase Fas2, saposin-like protein-2 SAP-2, thioredoxin peroxidases TPx, Prx-1, Prx-2, cathepsin I cysteine proteinase CL3, protease cathepsin L CL1, phosphoglycerate kinase PGK, 27-kDa secretory protein, 60 kDa protein HSP35alpha, glutathione transferase GST, 28.5 kDa tegumental antigen 28.5 kDa TA, cathepsin B3 protease CatB3, Type I cystatin stefin-1, cathepsin L5, cathepsin L1g and cathepsin B, fatty acid binding protein FABP, leucine aminopeptidases LAP (Fasciola hepatica and Fasciola gigantica, Fasciolosis); prion protein (FFI prion, Fatal familial insomnia (FFI)); venom allergen homolog-like protein VAL-1, abundant larval transcript ALT-1, abundant larval transcript ALT-2, thioredoxin peroxidase TPX, vespid allergen homologue VAH, thiordoxin peroxidase 2 TPX-2, antigenic protein SXP (peptides N, N1, N2, and N3), activation associated protein-1 ASP-1, Thioredoxin TRX, transglutaminase BmTGA, glutathione-S-transferases GST, myosin, vespid allergen homologue VAH, 175 kDa collagenase, glyceraldehyde-3-phosphate dehydrogenase GAPDH, cuticular collagen Col-4, secreted larval acidic proteins SLAPs, chitinase CHI-1, maltose binding protein MBP, glycolytic enzyme fructose-1,6-bisphosphate aldolase Fba, tropomyosin TMY-1, nematode specific gene product OvB20, onchocystatin CPI-2, Cox-2 (Filarioidea superfamily, Filariasis); phospholipase C PLC, heat-labile enterotoxin B, Iota toxin component Ib, protein CPE1281 , pyruvate ferredoxin oxidoreductase, elongation factor G EF-G, perfringolysin O Pfo, glyceraldehyde-3-phosphate dehydrogenase GapC, Fructose-bisphosphate aldolase Alf2, clostridium perfringens enterotoxin CPE, alpha toxin AT, alpha toxoid ATd, epsilon-toxoid ETd, protein HP, large cytotoxin TpeL, endo-beta-N-acetylglucosaminidase Naglu, phosphoglyceromutase Pgm (Clostridium perfringens, Food poisoning by Clostridium perfringens); leukotoxin lktA, adhesion FadA, outer membrane protein RadD, high-molecular weight arginine-binding protein (Fusobacterium genus, Fusobacterium infection); phospholipase C PLC, heat-labile enterotoxin B, Iota toxin component Ib, protein CPE1281, pyruvate ferredoxin oxidoreductase, elongation factor G EF-G, perfringolysin O Pfo, glyceraldehyde-3-phosphate dehydrogenase GapC, fructose-bisphosphate aldolase Alf2, clostridium perfringens enterotoxin CPE, alpha toxin AT, alpha toxoid ATd, epsilon-toxoid ETd, protein HP, large cytotoxin TpeL, endo-beta-N-acetylglucosaminidase Naglu, phosphoglyceromutase Pgm (usually Clostridium perfringens; other Clostridium species, Gas gangrene (Clostridial myonecrosis)); lipase A, lipase B, peroxidase Dec1 (Geotrichum candidum, Geotrichosis); prion protein (GSS prion, Gerstmann-Sträussler-Scheinker syndrome (GSS)); cyst wall proteins CWP1, CWP2, CWP3, variant surface protein VSP, VSP1, VSP2, VSP3, VSP4, VSP5, VSP6, 56 kDa antigen, pyruvate ferredoxin oxidoreductase PFOR, alcohol dehydrogenase E ADHE, alpha-giardin, alpha8-giardin, alpha1-guiardin, beta-giardin, cystein proteases, glutathione-S-transferase GST, arginine deiminase ADI, fructose-1,6-bisphosphat aldolase FBA, Giardia trophozoite antigens GTA (GTA1, GTA2), ornithine carboxyl transferase OCT, striated fiber-asseblin-like protein SALP, uridine phosphoryl-like protein UPL, alpha-tubulin, beta-tubulin (Giardia intestinalis, Giardiasis); members of the ABC transporter family (LolC, OppA, and PotF), putative lipoprotein releasing system transmembrane protein LolC/E, flagellin FliC, Burkholderia intracellular motility A BimA, bacterial Elongation factor-Tu EF-Tu, 17 kDa OmpA-like protein, boaA coding protein (Burkholderia mallei, Glanders); cyclophilin CyP, 24 kDa third-stage larvae protien GS24, excretion-secretion products ESPs (40, 80, 120 and 208 kDa) (Gnathostoma spinigerum and Gnathostoma hispidum, Gnathostomiasis); pilin proteins, minor pilin-associated subunit pilC, major pilin subunit and variants pilE, pilS, phase variation protein porA, Porin B PorB, protein TraD, Neisserial outer membrane antigen H.8, 70kDa antigen, major outer membrane protein PI, outer membrane proteins PIA and PIB, W antigen, surface protein A NspA, transferrin binding protein TbpA, transferrin binding protein TbpB , PBP2, mtrR coding protein, ponA coding protein, membrane permease FbpBC, FbpABC protein system, LbpAB proteins, outer membrane protein Opa, outer membrane transporter FetA, iron-repressed regulator MpeR (Neisseria gonorrhoeae, Gonorrhea); outer membrane protein A OmpA, outer membrane protein C OmpC, outer membrane protein K17 OmpK17 (Klebsiella granulomatis, Granuloma inguinale (Donovanosis)); fibronectin-binding protein Sfb, fibronectin/fibrinogen-binding protein FBP54, fibronectin-binding protein FbaA, M protein type 1 Emm1, M protein type 6 Emm6, immunoglobulin-binding protein 35 Sib35, Surface protein R28 Spr28, superoxide dismutase SOD, C5a peptidase ScpA, antigen I/II AgI/II, adhesin AspA, G-related alpha2-macroglobulin-binding protein GRAB, surface fibrillar protein M5 (Streptococcus pyogenes, Group A streptococcal infection); C protein β antigen, arginine deiminase proteins, adhesin BibA, 105 kDA protein BPS, surface antigens c, surface antigens R, surface antigens X, trypsin-resistant protein R1, trypsin-resistant protein R3, trypsin-resistant protein R4, surface immunogenic protein Sip, surface protein Rib, Leucine-rich repeats protein LrrG, serine-rich repeat protein Srr-2, C protein alpha-antigen Bca, Beta antigen Bag, surface antigen Epsilon, alpha-like protein ALP1, alpha-like protein ALP5 surface antigen delta, alpha-like protein ALP2, alpha-like protein ALP3, alpha-like protein ALP4, Cbeta protein Bac (Streptococcus agalactiae, Group B streptococcal infection); transferrin-binding protein 2 Tbp2, phosphatase P4, outer membrane protein P6, peptidoglycan-associated lipoprotein Pal, protein D, protein E, adherence and penetration protein Hap, outer membrane protein 26 Omp26, outer membrane protein P5 (Fimbrin), outer membrane protein D15, outer membrane protein OmpP2, 5'-nucleotidase NucA, outer membrane protein P1, outer membrane protein P2, outer membrane lipoprotein Pcp, Lipoprotein E, outer membrane protein P4, fuculokinase FucK, [Cu,Zn]-superoxide dismutase SodC, protease HtrA, protein O145, alpha-galactosylceramide (Haemophilus influenzae, Haemophilus influenzae infection); polymerase 3D, viral capsid protein VP1, viral capsid protein VP2, viral capsid protein VP3, viral capsid protein VP4, protease 2A, protease 3C (Enteroviruses, mainly Coxsackie A virus and Enterovirus 71 (EV71), Hand, foot and mouth disease (HFMD)); RNA polymerase L, protein L, glycoprotein Gn, glycoprotein Gc, nucleocapsid protein S, envelope glycoprotein G1, nucleoprotein NP, protein N, polyprotein M (Sin Nombre virus, Hantavirus, Hantavirus Pulmonary Syndrome (HPS)); heat shock protein HspA, heat shock protein HspB, citrate synthase GltA, protein UreB, heat shock protein Hsp60, neutrophil-activating protein NAP, catalase KatA, vacuolating cytotoxin VacA, urease alpha UreA, urease beta Ureb, protein Cpn10, protein groES, heat shock protein Hsp10, protein MopB, cytotoxicity-associated 10 kDa protein CAG, 36 kDa antigen, beta-lactamase HcpA, Beta-lactamase HcpB (Helicobacter pylori, Helicobacter pylori infection); integral membrane proteins, aggregation-prone proteins, O-antigen, toxin-antigens Stx2B, toxin-antigen Stx1B, adhesion-antigen fragment Int28, protein EspA, protein EspB, Intimin, protein Tir, protein IntC300, protein Eae (Escherichia coli O157:H7, O111 and O104:H4, Hemolytic-uremic syndrome (HUS)); RNA polymerase L, protein L, glycoprotein Gn, glycoprotein Gc, nucleocapsid protein S, envelope glycoprotein G1, nucleoprotein NP, protein N, polyprotein M (Bunyaviridae family, Hemorrhagic fever with renal syndrome (HFRS)); glycoprotein G, matrix protein M, nucleoprotein N, fusion protein F, polymerase L, protein W, proteinC, phosphoprotein p, non-structural protein V (Henipavirus (Hendra virus Nipah virus), Henipavirus infections); polyprotein, glycoproten Gp2, hepatitis A surface antigen HBAg, protein 2A, virus protein VP1, virus protein VP2, virus protein VP3, virus protein VP4, protein P1B, protein P2A, protein P3AB, protein P3D (Hepatitis A Virus, Hepatitis A); hepatitis B surface antigen HBsAg, Hepatitis B core antigen HbcAg, polymerase, protein Hbx, preS2 middle surface protein, surface protein L, large S protein, virus protein VP1, virus protein VP2, virus protein VP3, virus protein VP4 (Hepatitis B Virus, Hepatitis B); envelope glycoprotein E1 gp32 gp35 , envelope glycoprotein E2 NS1 gp68 gp70, capsid protein C , core protein Core, polyprotein, virus protein VP1, virus protein VP2, virus protein VP3, virus protein VP4, antigen G, protein NS3, protein NS5A, (Hepatitis C Virus, Hepatitis C); virus protein VP1, virus protein VP2, virus protein VP3, virus protein VP4, large hepaptitis delta antigen, small hepaptitis delta antigen (Hepatitis D Virus, Hepatitis D); virus protein VP1, virus protein VP2, virus protein VP3, virus protein VP4, capsid protein E2 (Hepatitis E Virus, Hepatitis E); glycoprotein L UL1, uracil-DNA glycosylase UL2, protein UL3, protein UL4, DNA replication protein UL5, portal protein UL6, virion maturation protein UL7, DNA helicase UL8, replication origin-binding protein UL9, glycoprotein M UL10, protein UL11, alkaline exonuclease UL12, serine-threonine protein kinase UL13, tegument protein UL14, terminase UL15, tegument protein UL16, protein UL17, capsid protein VP23 UL18, major capsid protein VP5 UL19, membrane protein UL20, tegument protein UL21, Glycoprotein H (UL22), Thymidine Kinase UL23, protein UL24, protein UL25, capsid protein P40 (UL26, VP24, VP22A),gGlycoprotein B (UL27), ICP18.5 protein (UL28), major DNA-binding protein ICP8 (UL29), DNA polymerase UL30, nuclear matrix protein UL31, envelope glycoprotein UL32, protein UL33, inner nuclear membrane protein UL34, capsid protein VP26 (UL35), large tegument protein UL36, capsid assembly protein UL37, VP19C protein (UL38), ribonucleotide reductase (Large subunit) UL39, ribonucleotide reductase (Small subunit) UL40, tegument protein/virion host shutoff VHS protein (UL41), DNA polymerase processivity factor UL42, membrane protein UL43, glycoprotein C (UL44), membrane protein UL45, tegument proteins VP11/12 (UL46), tegument protein VP13/14 (UL47), virion maturation protein VP16 (UL48, Alpha-TIF), envelope protein UL49, dUTP diphosphatase UL50, tegument protein UL51, DNA helicase/primase complex protein UL52, glycoprotein K (UL53), transcriptional regulation protein IE63 (ICP27, UL54), protein UL55, protein UL56, viral replication protein ICP22 (IE68, US1), protein US2, serine/threonine-protein kinase US3, glycoprotein G (US4),gGlycoprotein J (US5), glycoprotein D (US6),glycoprotein I (US7), glycoprotein E (US8), tegument protein US9, capsid/tegument protein US10, Vmw21 protein (US11), ICP47 protein (IE12, US12), major transcriptional activator ICP4 (IE175, RS1), E3 ubiquitin ligase ICP0 (IE110), latency-related protein 1 LRP1, latency-related protein 2 LRP2, neurovirulence factor RL1 (ICP34.5), latency-associated transcript LAT (Herpes simplex virus 1 and 2 (HSV-1 and HSV-2), Herpes simplex); heat shock protein Hsp60, cell surface protein H1C, dipeptidyl peptidase type IV DppIV, M antigen, 70 kDa protein, 17 kDa histone-like protein (Histoplasma capsulatum, Histoplasmosis); fatty acid and retinol binding protein-1 FAR-1, tissue inhibitor of metalloproteinase TIMP (TMP), cysteine proteinase ACEY-1, cysteine proteinase ACCP-1, surface antigen Ac-16, secreted protein 2 ASP-2, metalloprotease 1 MTP-1, aspartyl protease inhibitor API-1, surface-associated antigen SAA-1, surface-associated antigen SAA-2, adult-specific secreted factor Xa, serine protease inhibitor anticoagulant AP, cathepsin D-like aspartic protease ARR-1, glutathione S-transferase GST, aspartic protease APR-1, acetylcholinesterase AChE (Ancylostoma duodenale and Necator americanus, Hookworm infection); protein NS1, protein NP1, protein VP1, protein VP2, protein VP3 (Human bocavirus (HBoV), Human bocavirus infection); major surface protein 2 MSP2, major surface protein 4 MSP4, MSP variant SGV1, MSP variant SGV2, outer membrane protein OMP, outer membrande protein 19 OMP-19, major antigenic protein MAP1, major antigenic protein MAP1-2, major antigenic protein MAP1B, major antigenic protein MAP1-3, Erum2510 coding protein, protein GroEL, protein GroES, 30-kDA major outer membrane proteins, GE 100-kDa protein, GE 130-kDa protein, GE 160-kDa protein (Ehrlichia ewingii, Human ewingii ehrlichiosis); major surface proteins 1-5 (MSP1a, MSP1b, MSP2, MSP3, MSP4, MSP5), type IV secreotion system proteins VirB2, VirB7, VirB11, VirD4 (Anaplasma phagocytophilum, Human granulocytic anaplasmosis (HGA)); protein NS1, small hydrophobic protein NS2, SH protein, fusion protein F, glycoprotein G, matrix protein M, matrix protein M2-1, matrix protein M2-2, phosphoprotein P, nucleoprotein N, polymerase L (Human metapneumovirus (hMPV), Human metapneumovirus infection); major surface protein 2 MSP2, major surface protein 4 MSP4, MSP variant SGV1, MSP variant SGV2, outer membrane protein OMP, outer membrande protein 19 OMP-19, major antigenic protein MAP1, major antigenic protein MAP1-2, major antigenic protein MAP1B, major antigenic protein MAP1-3, Erum2510 coding protein, protein GroEL, protein GroES, 30-kDA major outer membrane proteins, GE 100-kDa protein, GE 130-kDa protein, GE 160-kDa protein (Ehrlichia chaffeensis, Human monocytic ehrlichiosis); replication protein E1, regulatory protein E2, protein E3, protein E4, protein E5, protein E6, protein E7, protein E8, major capsid protein L1, minor capsid protein L2 (Human papillomavirus (HPV), Human papillomavirus (HPV) infection); fusion protein F, hemagglutinin-neuramidase HN, glycoprotein G, matrix protein M, phosphoprotein P, nucleoprotein N, polymerase L (Human parainfluenza viruses (HPIV), Human parainfluenza virus infection); "hemagglutinin HA, neuraminidase NA, nucleoprotein NP, matrix protein M1, matrix protein M2, protein NS1, polymerase complex PA, PB1, PB2, nuclear export protein NEP; ; " (Orthomyxoviridae family, Influenza (flu)); genome polyprotein, protein E, protein M, capsid protein C (Japanese encephalitis virus, Japanese encephalitis); RTX toxin, type IV pili, major pilus subunit PilA, regulatory transcription factors PilS and PilR, protein sigma54, outer membrane proteins (Kingella kingae, Kingella kingae infection); prion protein (Kuru prion, Kuru); nucleoprotein N, polymerase L, matrix protein Z, glycoprotein GP (Lassa virus, Lassa fever); peptidoglycan-associated lipoprotein PAL, 60 kDa chaperonin Cpn60 (groEL, HspB), type IV pilin PilE, outer membrane protein MIP, major outer membrane protein MompS, zinc metalloproteinase MSP (Legionella pneumophila, Legionellosis (Legionnaires' disease, Pontiac fever)); P4 nuclease, protein WD, ribonucleotide reductase M2, surface membrane glycoprotein Pg46, cysteine proteinase CP, glucose-regulated protein 78 GRP-78, stage-specific S antigen-like protein A2, ATPase F1, beta-tubulin, heat shock protein 70 Hsp70, KMP-11, glycoprotein GP63, protein BT1, nucleoside hydrolase NH, cell surface protein B1, ribosomal protein P1-like protein P1, sterol 24-c-methyltransferase SMT, LACK protein, histone H1, SPB1 protein, thiol specific antioxidant TSA, protein antigen STI1, signal peptidase SP, histone H2B, suface antigen PSA-2, cystein proteinase b Cpb (Leishmania genus, Leishmaniasis); major membrane protein I, serine-rich antigen-45 kDa, 10 kDa caperonin GroES, HSP kDa antigen, amino-oxononanoate synthase AONS, protein recombinase A RecA, Acetyl-/propionyl-coenzyme A carboxylase alpha, alanine racemase, 60 kDa chaperonin 2, ESAT-6-like protein EcxB (L-ESAT-6), protein Lsr2, protein ML0276, Heparin-binding hemagglutinin HBHA, heat-shock protein 65 Hsp65, mycP1 or ML0041 coding protein , htrA2 or ML0176 coding protein , htrA4 or ML2659 coding protein, gcp or ML0379 coding protein, clpC or ML0235 coding protein (Mycobacterium leprae and Mycobacterium lepromatosis, Leprosy); outer membrane protein LipL32, membrane protein LIC10258, membrane protein LP30, membrane protein LIC12238, Ompa-like protein Lsa66, surface protein LigA, surface protein LigB, major outer membrane protein OmpL1, outer membrane protein LipL41, protein LigAni, surface protein LcpA, adhesion protein LipL53, outer membrane protein UpL32, surface protein Lsa63, flagellin FlaB1, membran lipoprotein LipL21, membrane protein pL40, leptospiral surface adhesin Lsa27, outer membrane protein OmpL36, outer membrane protein OmpL37, outer membrane protein OmpL47, outer membrane protein OmpL54, acyltransferase LpxA (Leptospira genus, Leptospirosis); listeriolysin O precursor Hly (LLO), invasion-associated protein Iap (P60), Listeriolysin regulatory protein PrfA, Zinc metalloproteinase Mpl, Phosphatidylinositol-specific phospholipase C PLC (PlcA, PlcB), O-acetyltransferase Oat, ABC-transporter permease Im.G_1771, adhesion protein LAP, LAP receptor Hsp60, adhesin LapB, haemolysin listeriolysin O LLO, protein ActA, Internalin A InlA, protein InlB (Listeria monocytogenes, Listeriosis); outer surface protein A OspA, outer surface protein OspB, outer surface protein OspC, decorin binding protein A DbpA, decorin binding protein B DbpB, flagellar filament 41 kDa core protein Fla, basic membrane protein A BmpA (Immunodominant antigen P39), outer surface 22 kDa lipoprotein precursor (antigen IPLA7), variable surface lipoprotein vlsE (usually Borrelia burgdorferi and other Borrelia species, Lyme disease (Lyme borreliosis)); venom allergen homolog-like protein VAL-1, abundant larval transcript ALT-1, abundant larval transcript ALT-2, thioredoxin peroxidase TPX, vespid allergen homologue VAH, thiordoxin peroxidase 2 TPX-2, antigenic protein SXP (peptides N, N1, N2, and N3), activation associated protein-1 ASP-1, thioredoxin TRX, transglutaminase BmTGA, glutathione-S-transferases GST, myosin, vespid allergen homologue VAH, 175 kDa collagenase, glyceraldehyde-3-phosphate dehydrogenase GAPDH, cuticular collagen Col-4, Secreted Larval Acidic Proteins SLAPs, chitinase CHI-1, maltose binding protein MBP, glycolytic enzyme fructose-1,6-bisphosphate aldolase Fba, tropomyosin TMY-1, nematode specific gene product OvB20, onchocystatin CPI-2, protein Cox-2 (Wuchereria bancrofti and Brugia malayi, Lymphatic filariasis (Elephantiasis)); glycoprotein GP, matrix protein Z, polymerase L, nucleoprotein N (Lymphocytic choriomeningitis virus (LCMV), Lymphocytic choriomeningitis); thrombospondin-related anonymous protein TRAP, SSP2 Sporozoite surface protein 2, apical membrane antigen 1 AMA1, rhoptry membrane antigen RMA1, acidic basic repeat antigen ABRA, cell-traversal protein PF, protein Pvs25, merozoite surface protein 1 MSP-1, merozoite surface protein 2 MSP-2, ring-infected erythrocyte surface antigen RESALiver stage antigen 3 LSA-3, protein Eba-175, serine repeat antigen 5 SERA-5, circumsporozoite protein CS, merozoite surface protein 3 MSP3, merozoite surface protein 8 MSP8, enolase PF10, hepatocyte erythrocyte protein 17 kDa HEP17, erythrocyte membrane protein 1 EMP1, protein Kbetamerozoite surface protein 4/5 MSP 4/5heat shock protein Hsp90, glutamate-rich protein GLURP, merozoite surface protein 4 MSP-4, protein STARP, circumsporozoite protein-related antigen precursor CRA (Plasmodium genus, Malaria); nucleoprotein N, membrane-associated protein VP24, minor nucleoprotein VP30, polymerase cofactor VP35, polymerase L, matrix protein VP40, envelope glycoprotein GP (Marburg virus, Marburg hemorrhagic fever (MHF)); protein C, matrix protein M, phosphoprotein P, non-structural protein V, hemagglutinin glycoprotein H, polymerase L, nucleoprotein N, fusion protein F (Measles virus, Measles); members of the ABC transporter family (LolC, OppA, and PotF), putative lipoprotein releasing system transmembrane protein LolC/E, flagellin FliC, Burkholderia intracellular motility A BimA, bacterial Elongation factor-Tu EF-Tu, 17 kDa OmpA-like protein, boaA coding protein, boaB coding protein (Burkholderia pseudomallei, Melioidosis (Whitmore's disease)); pilin proteins, minor pilin-associated subunit pilC, major pilin subunit and variants pilE, pilS, phase variation protein porA, Porin B PorB, protein TraD, Neisserial outer membrane antigen H.8, 70kDa antigen, major outer membrane protein PI, outer membrane proteins PIA and PIB, W antigen, surface protein A NspA, transferrin binding protein TbpA, transferrin binding protein TbpB , PBP2, mtrR coding protein, ponA coding protein, membrane permease FbpBC, FbpABC protein system, LbpAB proteins, outer membrane protein Opa, outer membrane transporter FetA, iron-repressed regulator MpeR, factor H-binding protein fHbp, adhesin NadA, protein NhbA, repressor FarR (Neisseria meningitidis, Meningococcal disease); 66 kDa protein, 22 kDa protein (usually Metagonimus yokagawai, Metagonimiasis); polar tube proteins (34, 75, and 170 kDa in Glugea, 35, 55 and 150kDa in Encephalitozoon), kinesin-related protein, RNA polymerase II largest subunit, similar ot integral membrane protein YIPA, anti-silencing protein 1, heat shock transcription factor HSF, protein kinase, thymidine kinase, NOP-2 like nucleolar protein (Microsporidia phylum, Microsporidiosis); CASP8 and FADD-like apoptosis regulator, Glutathione peroxidase GPX1, RNA helicase NPH-II NPH2, Poly(A) polymerase catalytic subunit PAPL, Major envelope protein P43K, early transcription factor 70 kDa subunit VETFS, early transcription factor 82 kDa subunit VETFL, metalloendopeptidase G1-type, nucleoside triphosphatase I NPH1, replication protein A28-like MC134L, RNA polymease 7 kDa subunit RPO7 (Molluscum contagiosum virus (MCV), Molluscum contagiosum (MC)); matrix protein M, phosphoprotein P/V, small hydrophobic protein SH, nucleoprotein N, protein V, fusion glycoprotein F, hemagglutinin-neuraminidase HN, RNA polymerase L (Mumps virus, Mumps); Outer membrane proteins OM, cell surface antigen OmpA, cell surface antigen OmpB (sca5), cell surface protein SCA4, cell surface protein SCA1, intracytoplasmic protein D, crystalline surface layer protein SLP, protective surface protein antigen SPA (Rickettsia typhi, Murine typhus (Endemic typhus)); adhesin P1, adhesion P30, protein p116, protein P40, cytoskeletal protein HMW1, cytoskeletal protein HMW2, cytoskeletal protein HMW3, MPN152 coding protein, MPN426 coding protein, MPN456 coding protein, MPN-500coding protein (Mycoplasma pneumoniae, Mycoplasma pneumonia); NocA, Iron dependent regulatory protein, VapA, VapD, VapF, VapG, caseinolytic protease, filament tip-associated 43-kDa protein, protein P24, protein P61, 15-kDa protein, 56-kDa protein (usually Nocardia asteroides and other Nocardia species, Nocardiosis); venom allergen homolog-like protein VAL-1, abundant larval transcript ALT-1, abundant larval transcript ALT-2, thioredoxin peroxidase TPX, vespid allergen homologue VAH, thiordoxin peroxidase 2 TPX-2, antigenic protein SXP (peptides N, N1, N2, and N3), activation associated protein-1 ASP-1, Thioredoxin TRX, transglutaminase BmTGA, glutathione-S-transferases GST, myosin, vespid allergen homologue VAH, 175 kDa collagenase, glyceraldehyde-3-phosphate dehydrogenase GAPDH, cuticular collagen Col-4, Secreted Larval Acidic Proteins SLAPs, chitinase CHI-1, maltose binding protein MBP, glycolytic enzyme fructose-1,6-bisphosphate aldolase Fba, tropomyosin TMY-1, nematode specific gene product OvB20, onchocystatin CPI-2, Cox-2 (Onchocerca volvulus, Onchocerciasis (River blindness)); 43 kDa secreted glycoprotein, glycoprotein gp0, glycoprotein gp75, antigen Pb27, antigen Pb40, heat shock protein Hsp65, heat shock protein Hsp70, heat shock protein Hsp90, protein P10, triosephosphate isomerase TPI, N-acetyl-glucosamine-binding lectin Paracoccin, 28 kDa protein Pb28 (Paracoccidioides brasiliensis, Paracoccidioidomycosis (South American blastomycosis)); 28-kDa cruzipain-like cystein protease Pw28CCP (usually Paragonimus westermani and other Paragonimus species, Paragonimiasis); outer membrane protein OmpH, outer membrane protein Omp28, protein PM1539, protein PM0355, protein PM1417, repair protein MutL, protein BcbC, prtein PM0305, formate dehydrogenase-N, protein PM0698, protein PM1422, DNA gyrase, lipoprotein PlpE, adhesive protein Cp39, heme aquisition system receptor HasR, 39 kDa capsular protein, iron-regulated OMP IROMP, outer membrane protein OmpA87, fimbrial protein Ptf, fimbrial subunit protein PtfA, transferrin binding protein Tbpl, esterase enzyme MesA, Pasteurella multocida toxin PMT, adhesive protein Cp39 (Pasteurella genus, Pasteurellosis); "filamentous hemagglutinin FhaB, adenylate cyclase CyaA, pertussis toxin subunit 4 precursor PtxD, pertactin precursor Prn, toxin subunit 1 PtxA, protein Cpn60, protein brkA, pertussis toxin subunit 2 precursor PtxB, pertussis toxin subunit 3 precursor PtxC, pertussis toxin subunit 5 precursor PtxE, pertactin Prn, protein Fim2, protein Fim3; " (Bordetella pertussis, Pertussis (Whooping cough)); "F1 capsule antigen, virulence-associated V antigen, secreted effector protein LcrV, V antigen, outer membrane protease Pla,secreted effector protein YopD, putative secreted protein-tyrosine phosphatase YopH, needle complex major subunit YscF, protein kinase YopO, putative autotransporter protein YapF, inner membrane ABC-transporter YbtQ (Irp7), putative sugar binding protein YPO0612, heat shock protein 90 HtpG, putative sulfatase protein YdeN, outer-membrane lipoprotein carrier protein LolA, secretion chaperone YerA, putative lipoprotein YPO0420, hemolysin activator protein HpmB, pesticin/yersiniabactin outer membrane receptor Psn, secreted effector protein YopE, secreted effector protein YopF, secreted effector protein YopK, outer membrane protein YopN , outer membrane protein YopM, Coagulase/fibrinolysin precursor Pla ; " (Yersinia pestis, Plague); protein PhpA, surface adhesin PsaA, pneumolysin Ply, ATP-dependent protease Clp, lipoate-protein ligase LplA, cell wall surface anchored protein psrP, sortase SrtA, glutamyl-tRNA synthetase GltX, choline binding protein A CbpA, pneumococcal surface protein A PspA, pneumococcal surface protein C PspC, 6-phosphogluconate dehydrogenase Gnd, iron-binding protein PiaA, Murein hydrolase LytB, proteon LytC, protease A1 (Streptococcus pneumoniae, Pneumococcal infection); major surface protein B, kexin-like protease KEX1, protein A12, 55 kDa antigen P55, major surface glycoprotein Msg (Pneumocystis jirovecii, Pneumocystis pneumonia (PCP)); genome polyprotein, polymerase 3D, viral capsid protein VP1, viral capsid protein VP2, viral capsid protein VP3, viral capsid protein VP4, protease 2A, protease 3C (Poliovirus, Poliomyelitis); protein Nfa1, exendin-3, secretory lipase, cathepsin B-like protease, cysteine protease, cathepsin, peroxiredoxin, protein CrylAc (usually Naegleria fowleri, Primary amoebic meningoencephalitis (PAM)); agnoprotein, large T antigen, small T antigen, major capsid protein VP1, minor capsid protein Vp2 (JC virus, Progressive multifocal leukoencephalopathy); low calcium response protein E LCrE, chlamydial outer protein N CopN, serine/threonine-protein kinase PknD, acyl-carrier-protein S-malonyltransferase FabD, single-stranded DNA-binding protein Ssb, major outer membrane protein MOMP, outer membrane protein 2 Omp2, polymorphic membrane protein family (Pmp1, Pmp2, Pmp3, Pmp4, Pmp5, Pmp6, Pmp7, Pmp8, Pmp9, Pmp10, Pmp11, Pmp12, Pmp13, Pmp14, Pmp15, Pmp16, Pmp17, Pmp18, Pmp19, Pmp20, Pmp21) (Chlamydophila psittaci, Psittacosis); outer membrane protein P1, heat shock protein B HspB, peptide ABC transporter, GTP-binding protein, protein IcmB, ribonuclease R, phosphatas SixA, protein DsbD, outer membrane protein TolC, DNA-binding protein PhoB, ATPase DotB, heat shock protein B HspB, membrane protein Com1, 28 kDa protein, DNA-3-methyladenine glycosidase I, pouter membrane protein OmpH, outer membrane protein AdaA, glycine cleavage system T-protein (Coxiella burnetii, Q fever); nucleoprotein N, large structural protein L, phophoprotein P, matrix protein M, glycoprotein G (Rabies virus, Rabies); fusionprotein F, nucleoprotein N, matrix protein M, matrix protein M2-1, matrix protein M2-2, phophoprotein P, small hydrophobic protein SH, major surface glycoprotein G, polymerase L, non-structural protein 1 NS1, non-structural protein 2 NS2 (Respiratory syncytial virus (RSV), Respiratory syncytial virus infection); genome polyprotein, polymerase 3D, viral capsid protein VP1, viral capsid protein VP2, viral capsid protein VP3, viral capsid protein VP4, protease 2A, protease 3C (Rhinovirus, Rhinovirus infection); outer membrane proteins OM, cell surface antigen OmpA, cell surface antigen OmpB (sca5), cell surface protein SCA4, cell surface protein SCA1, protein PS120, intracytoplasmic protein D, protective surface protein antigen SPA (Rickettsia genus, Rickettsial infection); outer membrane proteins OM, cell surface antigen OmpA, cell surface antigen OmpB (sca5), cell surface protein SCA4, cell surface protein SCA1, intracytoplasmic protein D (Rickettsia akari, Rickettsialpox); envelope glycoprotein GP, polymerase L, nucleoprotein N, non-structural protein NSS (Rift Valley fever virus, Rift Valley fever (RVF)); outer membrane proteins OM, cell surface antigen OmpA, cell surface antigen OmpB (sca5), cell surface protein SCA4, cell surface protein SCA1, intracytoplasmic protein D (Rickettsia rickettsii, Rocky mountain spotted fever (RMSF)); "non-structural protein 6 NS6, non-structural protein 2 NS2, intermediate capsid protein VP6, inner capsid protein VP2, non-structural protein 3 NS3, RNA-directed RNA polymerase L, protein VP3, non-structural protein 1 NS1, non-structural protein 5 NS5, outer capsid glycoprotein VP7, non-structural glycoprotein 4 NS4, outer capsid protein VP4; ; " (Rotavirus, Rotavirus infection); polyprotein P200, glycoprotein E1, glycoprotein E2, protein NS2, capsid protein C (Rubella virus, Rubella); chaperonin GroEL (MopA), inositol phosphate phosphatase SopB, heat shock protein HslU, chaperone protein DnaJ, protein TviB, protein IroN, flagellin FliC, invasion protein SipC, glycoprotein gp43, outer membrane protein LamB, outer membrane protein PagC, outer membrane protein TolC, outer membrane protein NmpC, outer membrane protein FadL, transport protein SadA, transferase WgaP, effector proteins SifA, SteC, SseL, SseJ and SseF (Salmonella genus, Salmonellosis); "protein 14, non-structural protein NS7b, non-structural protein NS8a, protein 9b, protein 3a, nucleoprotein N, non-structural protein NS3b, non-structural protein NS6, protein 7a, non-structural protein NS8b, membrane protein M, envelope small membrane protein EsM, replicase polyprotein 1a, spike glycoprotein S, replicase polyprotein 1ab ; " (SARS coronavirus, SARS (Severe Acute Respiratory Syndrome)); serin protease, Atypical Sarcoptes Antigen 1 ASA1, glutathione S-transferases GST, cystein protease, serine protease, apolipoprotein (Sarcoptes scabiei, Scabies); glutathione S-transferases GST, paramyosin, hemoglbinase SM32, major egg antigen, 14 kDa fatty acid-binding protein Sm14, major larval surface antigen P37, 22,6 kDa tegumental antigen, calpain CANP, triphospate isomerase Tim, surface protein 9B, outer capsid protein VP2, 23 kDa integral membrane protein Sm23, Cu/Zn-superoxide dismutase, glycoprotein Gp, myosin (Schistosoma genus, Schistosomiasis (Bilharziosis)); 60 kDa chaperonin, 56 kDa type-specific antigen, pyruvate phosphate dikinase, 4-hydroxybenzoate octaprenyltransferase (Orientia tsutsugamushi, Scrub typhus); dehydrogenase GuaB, invasion protein Spa32, invasin IpaA, invasin IpaB, invasin IpaC, invasin IpaD, invasin IpaH, invasin IpaJ (Shigella genus, Shigellosis (Bacillary dysentery)); protein P53, virion protein US10 homolog, transcriptional regulator IE63, transcriptional transactivator IE62, protease P33, alpha trans-inducing factor 74 kDa protein, deoxyuridine 5'-triphosphate nucleotidohydrolase, transcriptional transactivator IE4, membrane protein UL43 homolog, nuclear phosphoprotein UL3 homolog, nuclear protein UL4 homolog, replication origin-binding protein, membrane protein 2, phosphoprotein 32, protein 57,DNA polymerase processivity factor, portal protein 54, DNA primase, tegument protein UL14 homolog, tegument protein UL21 homolog, tegument protein UL55 homolog,tripartite terminase subunit UL33 homolog,tripartite terminase subunit UL15 homolog, capsid-binding protein 44, virion-packaging protein 43 (Varicella zoster virus (VZV), Shingles (Herpes zoster)); truncated 3-beta hydroxy-5-ene steroid dehydrogenase homolog, virion membrane protein A13, protein A19, protein A31, truncated protein A35 homolog, protein A37.5 homolog, protein A47, protein A49, protein A51, semaphorin-like protein A43, serine proteinase inhibitor 1, serine proteinase inhibitor 2, serine proteinase inhibitor 3, protein A6, protein B15, protein C1, protein C5, protein C6, protein F7, protein F8, protein F9, protein F11, protein F14, protein F15, protein F16 (Variola major or Variola minor, Smallpox (Variola)); adhesin/glycoprotein gp70, proteases (Sporothrix schenckii, Sporotrichosis); heme-iron binding protein IsdB, collagen adhesin Cna, clumping factor A ClfA, protein MecA, fibronectin-binding protein A FnbA, enterotoxin type A EntA, enterotoxin type B EntB, enterotoxin type C EntC1, enterotoxin type C EntC2, enterotoxin type D EntD, enterotoxin type E EntE, Toxic shock syndrome toxin-1 TSST-1, Staphylokinase, Penicillin binding protein 2a PBP2a (MecA), secretory antigen SssA (Staphylococcus genus, Staphylococcal food poisoning); heme-iron binding protein IsdB, collagen adhesin Cna, clumping factor A ClfA, protein MecA, fibronectin-binding protein A FnbA, enterotoxin type A EntA, enterotoxin type B EntB, enterotoxin type C EntC1, enterotoxin type C EntC2, enterotoxin type D EntD, enterotoxin type E EntE, Toxic shock syndrome toxin-1 TSST-1, Staphylokinase, Penicillin binding protein 2a PBP2a (MecA), secretory antigen SssA (Staphylococcus genus, Staphylococcal infection); antigen Ss-IR, antigen NIE, strongylastacin, Na+-K+ ATPase Sseat-6, tropomysin SsTmy-1, protein LEC-5, 41 kDa aantigen P5, 41-kDa larval protein, 31-kDa larval protein, 28-kDa larval protein (Strongyloides stercoralis, Strongyloidiasis); glycerophosphodiester phosphodiesterase GlpQ (Gpd), outer membrane protein TmpB, protein Tp92, antigen TpF1, repeat protein Tpr, repeat protein F TprF, repeat protein G TprG, repeat protein I TprI, repeat protein J TprJ, repeat protein K TprK, treponemal membrane protein A TmpA, lipoprotein, 15 kDa Tpp15, 47 kDa membrane antigen, miniferritin TpF1, adhesin Tp0751, lipoprotein TP0136, protein TpN17, protein TpN47, outer membrane protein TP0136, outer membrane protein TP0155, outer membrane protein TP0326, outer membrane protein TP0483, outer membrane protein TP0956 (Treponema pallidum, Syphilis); Cathepsin L-like proteases, 53/25-kDa antigen, 8kDa family members, cysticercus protein with a marginal trypsin-like activity TsAg5, oncosphere protein TSOL18, oncosphere protein TSOL45-1A, lactate dehydrogenase A LDHA, lactate dehydrogenase B LDHB (Taenia genus, Taeniasis); tetanus toxin TetX, tetanus toxin C TTC, 140 kDa S layer protein, flavoprotein beta-subunit CT3, phospholipase (lecithinase), phosphocarrier protein HPr (Clostridium tetani, Tetanus (Lockjaw)); genome polyprotein, protein E, protein M, capsid protein C (Tick-borne encephalitis virus (TBEV), Tick-borne encephalitis); 58-kDa antigen, 68-kDa antigens, Toxocara larvae excretory-secretory antigen TES, 32-kDa glycoprotein, glycoprotein TES-70, glycoprotein GP31, excretory-secretory antigen TcES-57, perienteric fluid antigen Pe, soluble extract antigens Ex, excretory/secretory larval antigens ES, antigen TES-120, polyprotein allergen TBA-1, cathepsin L-like cysteine protease c-cpl-1, 26-kDa protein (Toxocara canis or Toxocara cati, Toxocariasis (Ocular Larva Migrans (OLM) and Visceral Larva Migrans (VLM))); microneme proteins (MIC1, MIC2, MIC3, MIC4, MIC5, MIC6, MIC7, MIC8), rhoptry protein Rop2, rhoptry proteins (Rop1, Rop2, Rop3, Rop4, Rop5, Rop6, Rop7, Rop16, Rjop17), protein SR1,surface antigen P22, major antigen p24, major surface antigen p30, dense granule proteins (GRA1, GRA2, GRA3, GRA4, GRA5, GRA6, GRA7, GRA8, GRA9, GRA10), 28 kDa antigen, surface antigen SAG1, SAG2 related antigen, nucleoside-triphosphatase 1, nucleoside-triphosphatase 2, protein Stt3, HesB-like domain-containing protein, rhomboid-like protease 5, toxomepsin 1 (Toxoplasma gondii, Toxoplasmosis); 43 kDa secreted glycoprotein, 53 kDa secreted glycoprotein, paramyosin, antigen Ts21, antigen Ts87, antigen p46000, TSL-1 antigens, caveolin-1 CAV-1, 49 kDa newborn larva antigen, prosaposin homologue, serine protease, serine proteinase inhibitor, 45 -kDa glycoprotein Gp45 (Trichinella spiralis, Trichinellosis); Myb-like transcriptional factors (Myb1, Myb2, Myb3), adhesion protein AP23, adhesion protein AP33, adhesin protein AP33-3, adhesins AP51, adhesin AP65, adhesion protein AP65-1, alpha-actinin, kinesin-associated protein, teneurin, 62 kDa proteinase, subtilisin-like serine protease SUB1, cysteine proteinase gene 3 CP3, alpha-enolase Eno1, cysteine proteinase CP30, heat shock proteins (Hsp70, Hsp60), immunogenic protein P270, (Trichomonas vaginalis, Trichomoniasis); beta-tubulin, 47-kDa protein, secretory leucocyte-like proteinase-1 SLP-1, 50-kDa protein TT50, 17 kDa antigen, 43/47 kDa protein (Trichuris trichiura, Trichuriasis (Whipworm infection)); protein ESAT-6 (EsxA), 10 kDa filtrate antigen EsxB, secreted antigen 85-B FBPB, fibronectin-binding protein A FbpA (Ag85A), serine protease PepA, PPE family protein PPE18, fibronectin-binding protein D FbpD, immunogenic protein MPT64, secreted protein MPT51, catalase-peroxidase-peroxynitritase T KATG, periplasmic phosphate-binding lipoprotein PSTS3 (PBP-3, Phos-1), iron-regulated heparin binding hemagglutinin Hbha, PPE family protein PPE14, PPE family protein PPE68, protein Mtb72F, protein Apa, immunogenic protein MPT63, periplasmic phosphate-binding lipoprotein PSTS1 (PBP-1), molecular chaperone DnaK, cell surface lipoprotein Mpt83, lipoprotein P23, phosphate transport system permease protein pstA, 14 kDa antigen, fibronectin-binding protein C FbpC1, Alanine dehydrogenase TB43, Glutamine synthetase 1, ESX-1 protein, protein CFP10, TB10.4 protein, protein MPT83, protein MTB12, protein MTB8, Rpf-like proteins, protein MTB32, protein MTB39, crystallin, heat-shock protein HSP65, protein PST-S (usually Mycobacterium tuberculosis, Tuberculosis); outer membrane protein FobA, outer membrane protein FobB, intracellular growth locus IglC1, intracellular growth locus IglC2, aminotransferase Wbtl, chaperonin GroEL, 17 kDa major membrane protein TUL4, lipoprotein LpnA, chitinase family 18 protein, isocitrate dehydrogenase, Nif3 family protein, type IV pili glycosylation protein, outer membrane protein tolC, FAD binding family protein, type IV pilin multimeric outer membrane protein, two component sensor protein KdpD, chaperone protein DnaK, protein TolQ (Francisella tularensis, Tularemia); "MB antigen, urease, protein GyrA, protein GyrB, protein ParC, protein ParE, lipid associated membrane proteins LAMP, thymidine kinase TK, phospholipase PL-A1, phospholipase PL-A2, phospholipase PL-C, surface-expressed 96-kDa antigen; " (Ureaplasma urealyticum, Ureaplasma urealyticum infection); non-structural polyprotein, structural polyprotein, capsid protein CP, protein E1, protein E2, protein E3, protease P1, protease P2, protease P3 (Venezuelan equine encephalitis virus, Venezuelan equine encephalitis); glycoprotein GP, matrix protein Z, polymerase L, nucleoprotein N (Guanarito virus, Venezuelan hemorrhagic fever); polyprotein, protein E, protein M, capsid protein C, protease NS3, protein NS1, protein NS2A, protein AS2B, brotein NS4A, protein NS4B, protein NS5 (West Nile virus, West Nile Fever); cpasid protein CP, protein E1, protein E2, protein E3, protease P2 (Western equine encephalitis virus, Western equine encephalitis); genome polyprotein, protein E, protein M, capsid protein C, protease NS3, protein NS1, protein NS2A, protein AS2B, protein NS4A, protein NS4B, protein NS5 (Yellow fever virus, Yellow fever); putative Yop targeting protein YobB, effector protein YopD, effector protein YopE, protein YopH, effector protein YopJ, protein translocation protein YopK, effector protein YopT, protein YpkA, flagellar biosyntheses protein FlhA, peptidase M48, potassium efflux system KefA, transcriptional regulatoer RovA, adhesin Ifp, translocator portein LcrV, protein PcrV, invasin Inv, outer membrane protein OmpF-like porin, adhesin YadA, protein kinase C, phospholipase C1, protein PsaA, mannosyltransferase-like protein WbyK, protein YscU, antigen YPMa (Yersinia pseudotuberculosis, Yersinia pseudotuberculosis infection); effector protein YopB, 60 kDa chaperonin, protein WbcP, tyrosin-protein phosphatase YopH, protein YopQ, enterotoxin, Galactoside permease, reductaase NrdE, protein YasN, Invasin Inv, adhesin YadA, outer membrane porin F OmpF, protein UspA1, protein EibA, protein Hia, cell surface protein Ail, chaperone SycD, protein LcrD, protein LcrG, protein LcrV, protein SycE, protein YopE, regulator protein TyeA, protein YopM, protein YopN, protein YopO, protein YopT, protein YopD, protease ClpP, protein MyfA, protein FilA, and protein PsaA (Yersinia enterocolitica, Yersiniosis).

(in brackets are the particular pathogen of which the antigen(s) is/are derived and the infectious disease with which the antigen is associated)

In specific embodiments according to the present invention, following antigens of pathogens associated with infectious disease are particularly preferred:

- The Hemagglutinin (HA), the Neuraminidase (NA), the Nucleoprotein (NP), the M1 protein, the M2 protein, the NS1 protein, the NS2 protein (the NEP protein: nuclear export protein), the PA protein, the PB1 protein (polymerase basic 1 protein), the PB1-F2 protein and the PB2 protein of Influenza virus;
- The nucleoprotein (N), the phosphoprotein (P), the matrix protein (M), the glycoprotein (G), and the viral RNA polymerase (L), in each case of Rabies virus;
- the Hepatitis B surface antigen (HBsAg), the Hepatitis B core antigen (HbcAg), the Hepatitis B virus DNA polymerase, the HBx protein, the preS2 middle surface protein, the large S protein, the virus protein VP1, the virus protein VP2, the virus protein VP3, and the virus protein VP4, in each case of Hepatitis B virus;
- the E1 protein, the E2 protein, the E3 protein, the E4 protein, the E5 protein, the E6 protein, the E7 protein, the E8 protein, the L1 protein, and the L2 protein, in each case of human Papilloma virus (hPV);
- the protective antigen (PA), the edema factor (EF), the lethal factor (LF), and the S-layer homology proteins (SLH), in each case of Bacillus anthracis;
- the Fusion (F) protein, the nucleocapsid (N) protein, the phosphoprotein (P), the matrix (M) protein, the glycoprotein (G), the large protein (L; RNA polymerase), the non-structural protein 1 (NS1), the non-structural protein 2 (NS2), the small hydrophobic (SH) protein, the elongation factor M2-1, and the transcription regulation protein M2-2, in each case of respiratory syncytial virus (RSV);
- the Glycoprotein L (UL1), the Uracil-DNA glycosylase UL2, the UL3 protein, the UL4 protein, the DNA replication protein UL5, the Portal protein UL6, the Virion maturation protein UL7, the DNA helicase UL8, the Replication origin-binding protein UL9, the Glycoprotein M (UL10), the UL11 protein, the Alkaline exonuclease UL12, the Serine-threonine protein kinase UL13, the Tegument protein UL14, the Terminase (UL15), the Tegument protein UL16, the UL17 protein , the Capsid protein VP23 (UL18), the Major capsid protein VP5 (UL19), the Membrane protein UL20, the Tegument protein UL21, the Glycoprotein H (UL22), the Thymidine Kinase UL23, the UL24 protein, the UL25 protein, the Capsid protein P40 (UL26, VP24, VP22A), the Glycoprotein B (UL27), the ICP18.5 protein (UL28), the Major DNA-binding protein ICP8 (UL29), the DNA polymerase UL30, the Nuclear matrix protein UL31, the Envelope glycoprotein UL32, the UL33 protein, the Inner nuclear membrane protein UL34, the Capsid protein VP26 (UL35), the Large tegument protein UL36, the Capsid assembly protein UL37, the VP19C protein (UL38), the Ribonucleotide reductase (Large subunit) UL39, the Ribonucleotide reductase (Small subunit) UL40, the Tegument protein/Virion host shutoff VHS protein (UL41), the DNA polymerase processivity factor UL42, the Membrane protein UL43, the Glycoprotein C (UL44), the Membrane protein UL45, the Tegument proteins VP11/12 (UL46), the Tegument protein VP13/14 (UL47), the Virion maturation protein VP16 (UL48, Alpha-TIF), the Envelope protein UL49, the dUTP diphosphatase UL50, the Tegument protein UL51, the DNA helicase/primase complex protein UL52, the Glycoprotein K (UL53), the Transcriptional regulation protein IE63 (ICP27, UL54), the UL55 protein, the UL56 protein, the Viral replication protein ICP22 (IE68, US1), the US2 protein, the Serine/threonine-protein kinase US3, the Glycoprotein G (US4), the Glycoprotein J (US5), the Glycoprotein D (US6), the Glycoprotein I (US7), the Glycoprotein E (US8), the Tegument protein US9, the Capsid/Tegument protein US10, the Vmw21 protein (US11), the ICP47 protein (IE12, US12), the Major transcriptional activator ICP4 (IE175, RS1), the E3 ubiquitin ligase ICP0 (IE110), the Latency-related protein 1 (LRP1), the Latency-related protein 2 (LRP2), the Neurovirulence factor RL1 (ICP34.5), and the Latency-associated transcript (LAT), in each case of Herpes simplex virus (HSV); or

- the ESAT-6 protein, the ESX-1 protein, the CFP10 protein, the TB10.4 protein, the MPT63 protein, the MPT64 protein, the MPT83 protein, the MTB12 protein, the MTB8 protein, the AG85A protein, the AG85B protein, the Rpf-like proteins, the KATG protein, the PPE18 protein, the MTB32 protein, the MTB39 protein, the Crystallin, the HSP65 protein, the PST-S protein, and the HBHA protein, the 10 kDa filtrate antigen EsxB, the serine protease PepA, the fibronectin-binding protein D FbpD, the secreted protein MPT51, the periplasmic phosphate-binding lipoprotein PSTS1 (PBP-1), the periplasmic phosphate-binding lipoprotein PSTS3 (PBP-3, Phos-1), the PPE family protein PPE14, the PPE family protein PPE68, the protein MTB72F, the molecular chaperone DnaK, the cell surface lipoprotein MPT83, the lipoprotein P23, the Phosphate transport system permease protein PstA, the 14 kDa antigen, the fibronectin-binding protein C FbpC1, the Alanine dehydrogenase TB43, and the Glutamine synthetase 1, in each case of Mycobacterium tuberculosis.

b) Antigens associated with allergy or allergic disease (allergenic antigens or allergens):

According to another alternative, one further class of antigens comprises allergenic antigens. Such allergenic antigens may be selected from antigens derived from different sources, e.g. from animals, plants, fungi, bacteria, etc. Sources of allergens in this context include e.g. grasses, pollens, molds, drugs, or numerous environmental triggers, etc. Allergenic antigens typically belong to different classes of compounds, such as nucleic acids and their fragments, proteins or peptides and their fragments, carbohydrates, polysaccharides, sugars, lipids, phospholipids, etc. Of particular interest in the context of the present invention are protein or peptide antigens and their fragments or epitopes, or nucleic acids and their fragments, particularly nucleic acids and their fragments, encoding such protein or peptide antigens and their fragments or epitopes.

In alternative embodiments, said antigen is a peptide or protein antigen, or a fragment, variant and/or derivative of said peptide or protein antigen, such as a peptide or protein antigen comprised in a preparation extracted from said source. In alternative embodiments, a peptide or protein antigen used in the present invention is not one comprised in a preparation extracted from said source, and/or is one that is not obtained from a preparation extracted from said source.

Antigens associated with allergy or allergic diseases (allergens) are preferably derived from a source selected from the list consisting of: Acarus spp (Aca s 1, Aca s 10, Aca s 10.0101, Aca s 13, Aca s 13.0101, Aca s 2, Aca s 3, Aca s 7, Aca s 8), Acanthocybium spp (Aca so 1), Acanthocheilonema spp (Aca v 3, Aca v 3.0101), Acetes spp (Ace ja 1), Actinidia spp (Act a 1, Act c 1, Act c 10, Act c 10.0101, Act c 2, Act c 4, Act c 5, Act c 5.0101, Act c 8, Act c 8.0101, Act c Chitinase, Act d 1, Act d 1.0101, Act d 10, Act d 10.0101, Act d 10.0201, Act d 11, Act d 11.0101, Act d 2, Act d 2.0101, Act d 3, Act d 3.0101, Act d 3.02, Act d 4, Act d 4.0101, Act d 5, Act d 5.0101, Act d 6, Act d 6.0101, Act d 7, Act d 7.0101, Act d 8, Act d 8.0101, Act d 9, Act d 9.0101, Act d Chitinase, Act e 1, Act e 5), Acyrthosiphon spp (Acy pi 7, Acy pi 7.0101, Acy pi 7.0102), Adenia spp (Ade v RIP), Aedes spp (Aed a 1, Aed a 1.0101, Aed a 2, Aed a 2.0101, Aed a 3, Aed a 3.0101, Aed a 4, Aed a 7, Aed a 7.0101, Aed a 7.0102, Aed a 7.0103, Aed a 7.0104, Aed a 7.0105, Aed a 7.0106, Aed a 7.0107, Aed a 7.0108, Aed a 7.0109, Aed a 7.0110, Aed a 7.0111, Aed al 1, Aed al 3, Aed al 37kD, Aed v 37kD, Aed v 63kD), Aegilops spp (Aeg ta 28, Aeg ta alpha_Gliadin, Aeg um 28, Aeg un 28), Aethaloperca spp (Aet ro 1), Agropyron spp (Agr c 7), Agrostis spp (Agr ca 1, Agr ca 5, Agr g 1, Agr g 4, Agr s 5), Agrobacterium spp (Agr sp CP4 EPSPS), Ailuropoda spp (Ail me Phosvitin, Ail me TCTP), Aix spp (Aix ga 1, Aix sp 1), Aleuroglyphus spp (Ale o 1, Ale o 10, Ale o 10.0101, Ale o 10.0102, Ale o 13, Ale o 14, Ale o 2, Ale o 20, Ale o 3, Ale o 5, Ale o 7, Ale o 8, Ale o 9), Allium spp (All a 3, All a Alliin lyase, All c 3, All c 30kD, All c 4, All c Alliin lyase, All p Alliin lyase, All s Alliin lyase), Alnus spp (Aln g 1, Aln g 1.0101, Aln g 1/Bet v 1/Cor a 1 TPC7, Aln g 1/Bet v 1/Cor a 1 TPC9, Aln g 2, Aln g 4, Aln g 4.0101), Alopochen spp (Alo ae 1), Alopecurus spp (Alo p 1, Alo p 5), Alternaria spp (Alt a 1, Alt a 1.0101, Alt a 1.0102, Alt a 10, Alta 10.0101, Alt a 12, Alt a 12.0101, Alt a 13, Alt a 13.0101, Alt a 2, Alt a 3, Alt a 3.0101, Alt a 4, Alt a 4.0101, Alt a 5, Alt a 5.0101, Alt a 6, Alt a 6.0101, Alt a 7, Alt a 7.0101, Alt a 70kD, Alt a 8, Alt a 8.0101, Alt a 9, Alt a MnSOD, Alt a NTF2, Alt a TCTP, Alt ar 1, Alt arg 1, Alt b 1, Alt bl 1, Alt br 1, Alt c 1, Alt ca 1, Alt ce 1, Alt ch 1, Alt ci 1, Alt co 1, Alt cr 1, Alt ct 1, Alt cu 1, Alt cy 1, Alt d 1, Alt du 1, Alt e 1, Alt et 1, Alt eu 1, Alt ga 1, Alt gr 1, Alt j 1, Alt l 1, Alt lo 1, Alt m 1, Alt me 1, Alt mi 1, Alt mo 1, Alt o 1, Alt p 1, Alt ph 1, Alt po 1, Alt ps 1, Alt r 1, Alt s 1, Alt se 1, Alt sm 1, Alt so 1, Alt su 1, Alt t 1, Alt te 1, Alt to 1), Amaranthus spp (Ama r 2, Ama r 2.0101, Ama v 2, Ama v 2.0101, Ama v 2.0201), Ambrosia spp (Amb a 1, Amb a 1.0101, Amb a 1.0201, Amb a 1.0202, Amb a 1.0301, Amb a 1.0302, Amb a 1.0303, Amb a 1.0304, Amb a 1.0305, Amb a 1.0401, Amb a 1.0402, Amb a 1.0501, Amb a 1.0502, Amb a 10, Amb a 10.0101, Amb a 3, Amb a 3.0101, Amb a 4, Amb a 4.0101, Amb a 5, Amb a 5.0101, Amb a 6, Amb a 6.0101, Amb a 7, Amb a 7.0101, Amb a 8, Amb a 8.0101, Amb a 8.0102, Amb a 9, Amb a 9.0101, Amb a 9.0102, Amb a CPI, Amb p 1, Amb p 5, Amb p 5.0101, Amb p 5.0201, Amb t 5, Amb t 5.0101, Amb t 8), Ammothea spp (Amm h 7, Amm h 7.0101), Anadara spp (Ana br 1), Ananas spp (Ana c 1, Ana c 1.0101, Ana c 2, Ana c 2.0101, Ana c 2.0101 (MUXF3)), Anas spp (Ana ca 1), Anarhichas spp (Ana [ 1), Anacardium spp (Ana o 1, Ana o 1.0101, Ana o 1.0102, Ana o 2, Ana o 2.0101, Ana o 3, Ana o 3.0101), Anas spp (Ana p 1, Ana p 2, Ana p 3), Anguilla spp (Ang a 1, Ang j 1), Anisakis spp (Ani s 1, Ani s 1.0101, Ani s 10, Ani s 10.0101, Ani s 11, Ani s 11.0101, Ani s 12, Ani s 12.0101, Ani s 2, Ani s 2.0101, Ani s 24kD, Ani s 3, Ani s 3.0101, Ani s 4, Ani s 4.0101, Ani s 5, Ani s 5.0101, Ani s 6, Ani s 6.0101, Ani s 7, Ani s 7.0101, Ani s 8, Ani s 8.0101, Ani s 9, Ani s 9.0101, Ani s CCOS3, Ani s Cytochrome B, Ani s FBPP, Ani s NADHDS4L, Ani s NARaS, Ani s PEPB, Ani s Troponin), Annona spp (Ann c Chitinase), Anopheles spp (Ano da 17, Ano da 17.0101, Ano da 27, Ano da 27.0101, Ano da 7, Ano da 7.0101, Ano g 7, Ano g 7.0101), Anser spp (Ans a 1, Ans a 2, Ans a 3, Ans in 1), Anthoxanthum spp (Ant o 1, Ant o 1.0101, Ant o 12, Ant o 13, Ant o 2, Ant o 4, Ant o 5, Ant o 6, Ant o 7), Apis spp (Api c 1, Api c 1.0101, Api c 10, Api c 2, Api c 4, Api d 1, Api d 1.0101, Api d 4, Api fl 4), Apium spp (Api g 1, Api g 1.0101, Api g 1.0201, Api g 2, Api g 2.0101, Api g 3, Api g 3.0101, Api g 4, Api g 4.0101, Api g 5, Api g 5.0101, Api g 6, Api g 6.0101), Apis spp (Api m 1, Api m 1.0101, Api m 10, Api m 10.0101, Api m 11, Api m 11.0101, Api m 11.0201, Api m 13kD, Api m 2, Api m 2.0101, Api m 3, Api m 3.0101, Api m 4, Api m 4.0101, Api m 5, Api m 5.0101, Api m 6, Api m 6.0101, Api m 7, Api m 7.0101, Api m 8, Api m 8.0101, Api m 9, Api m 9.0101, Api m A1-A2, Api m A1-A2-A3, Api m Apalbumin 1, Api m Apalbumin 2, Api me 1, Api me 4), Arachis spp (Ara d 2, Ara d 6, Ara f 3, Ara f 4, Ara h 1, Ara h 1.0101, Ara h 10, Ara h 10.0101, Ara h 10.0102, Ara h 11, Ara h 11.0101, Ara h 2, Ara h 2.0101, Ara h 2.0102, Ara h 2.0201, Ara h 2.0202, Ara h 3, Ara h 3.0101, Ara h 4, Ara h 4.0101, Ara h 5, Ara h 5.0101, Ara h 6, Ara h 6.0101, Ara h 7, Ara h 7.0101, Ara h 7.0201, Ara h 7.0202, Ara h 8, Ara h 8.0101, Ara h 8.0201, Ara h 9, Ara h 9.0101, Ara h 9.0201, Ara h Agglutinin, Ara h Oleosin 18kD, Ara i 2, Ara i 6), Arabidopsis spp (Ara t 3, Ara t 8, Ara t GLP), Archosargus spp (Arc pr 1), Archaeopotamobius spp (Arc s 8, Arc s 8.0101), Aequipecten spp (Arg i 1), Argas spp (Arg r 1, Arg r 1.0101), Ariopsis spp (Ari fe 1), Armoracia spp (Arm r HRP), Arrhenatherum spp (Arr e 1, Arr e 5), Artemisia spp (Art a 1, Art ap 1), Artemia spp (Art fr 1, Art fr 1.0101, Art fr 5, Art fr 5.0101), Arthrobacter spp (Art gl CO), Achorion spp (Art gy 7), Artocarpus spp (Art h 17kD, Art h 4), Arthrospira spp (Art pl beta_Phycocyanin), Artemisia spp (Art v 1, Art v 1.0101, Art v 1.0102, Art v 1.0103, Art v 1.0104, Art v 1.0105, Art v 1.0106, Art v 1.0107, Art v 2, Art v 2.0101, Art v 3, Art v 3.0101, Art v 3.0201, Art v 3.0202, Art v 3.0301, Art v 4, Art v 4.0101, Art v 4.0201, Art v 47kD, Art v 5, Art v 5.0101, Art v 6, Art v 6.0101, Art v 60kD), Arthroderma spp (Art va 4), Ascaris spp (Asc l 3, Asc l 3.0101, Asc l 3.0102, Asc l 34kD, Asc s 1, Asc s 1.0101, Asc s 3, Asc s 3.0101, Asc s GST), Aspergillus spp (Asp aw Glucoamylase, Asp c 22, Asp f 1, Asp f 1.0101, Asp f 10, Asp f 10.0101, Asp f 11, Asp f 11.0101, Asp f12, Asp f12.0101, Asp f 13, Asp f13.0101, Asp f15, Asp f 15.0101, Asp f 16, Asp f 16.0101, Asp f 17, Asp f 17.0101, Asp f 18, Asp f 18.0101, Asp f 2, Asp f 2.0101, Asp f 22, Asp f 22.0101, Asp f 23, Asp f 23.0101, Asp f 27, Asp f 27.0101, Asp f 28, Asp f 28.0101, Asp f 29, Asp f 29.0101, Asp f 3, Asp f 3.0101, Asp f 34, Asp f 34.0101, Asp f 4, Asp f 4.0101, Asp f 5, Asp f 5.0101, Asp f 56kD, Asp f 6, Asp f 6.0101, Asp f 7, Asp f 7.0101, Asp f 8, Asp f 8.0101, Asp f 9, Asp f 9.0101, Asp f AfCalAp, Asp f AT_V, Asp f Catalase, Asp f Chitosanase, Asp f CP, Asp f DPPV, Asp f FDH, Asp f gamma_Actin, Asp f Glucosidase, Asp f GPI, Asp f GST, Asp f GT, Asp f IAO, Asp f IPMI, Asp f LPL1, Asp f LPL3, Asp f Mannosidase, Asp f MDH, Asp f PL, Asp f PUP, Asp f RPS3, Asp f SXR, Asp fl 13, Asp fl 13.0101, Asp fl 18, Asp fl 2, Asp fl 21, Asp fl 3, Asp fl 4, Asp fl 7, Asp fl 8, Asp fl 9, Asp me Seaprose, Asp n 14, Asp n 14.0101, Asp n 18, Asp n 18.0101, Asp n 25, Asp n 25.0101, Asp n 30, Asp n Glucoamylase, Asp n Hemicellulase, Asp n Pectinase, Asp o 13, Asp o 13.0101, Asp o 21, Asp o 21.0101, Asp o 3, Asp o 4, Asp o 7, Asp o 8, Asp o Lactase, Asp o Lipase, Asp oc 13, Asp r 1, Asp sa AP, Asp sp Glucoamylase, Asp sp Glucoseoxidase, Asp sp PL, Asp sp PME, Asp sy 13, Asp v 13, Asp v 13.0101, Asp v Catalase A, Asp v Enolase, Asp v GAPDH, Asp v MDH, Asp v SXR), Asparagus spp (Aspa o 1, Aspa o 1.01, Aspa o 1.02, Aspa o 17kD, Aspa o 4), Aspergillus spp (Aspe ni 2, Aspe ni 3, Aspe ni 4, Aspe ni 7, Aspe ni 8, Aspe ni 9), Avena spp (Ave s 1, Ave s 12, Ave s 13, Ave s 2, Ave s 4, Ave s 5, Ave s 7), Babylonia spp (Bab ja 1), Bacillus spp (Bac al Subtilisin, Bac cl Subtilisin, Bac l Subtilisin, Bac li aA, Bac li Subtilisin), Bactrocera spp (Bac ol 27, Bac ol 27.0101), Bacillus spp (Bac sp aA1, Bac sp aA3, Bac sp Decarboxylase, Bac st amyM, Bac su Subtilisin, Bac t Cry1Ab, Bac t Cry1Fa, Bac t Cry3Bb1, Bac t Cry9c), Bagre spp (Bag ma 1), Balistes spp (Bal ca 1), Balanus spp (Bal r 1, Bal r 1.0101), Beauveria spp (Bea b Ald, Bea b Enol, Bea b f2, Bea b Hex), Bertholletia spp (Ber e 1, Ber e 1.0101, Ber e 2, Ber e 2.0101), Beryx spp (Ber sp 1), Betula spp (Bet ab 1, Bet al 1, Bet ch 1, Bet co 1, Bet da 1, Bet gr 1, Bet hu 1, Bet le 1, Bet me 1, Bet n 1, Bet p 1, Bet pa 1, Bet po 1, Bet pu 1, Bet pu 2, Bet pu 4, Bet pu 6, Bet pu 7, Bet sc 1, Bet ut 1, Bet v 1, Bet v 1 B1-B1-B1, Bet v 1 fv Mal 4x, Bet v 1.0101, Bet v 1.0102, Bet v 1.0103, Bet v 1.0201, Bet v 1.0301, Bet v 1.0401, Bet v 1.0402, Bet v 1.0501, Bet v 1.0601, Bet v 1.0602, Bet v 1.0701, Bet v 1.0801, Bet v 1.0901, Bet v 1.1001, Bet v 1.1101, Bet v 1.1201, Bet v 1.1301, Bet v 1.1401, Bet v 1.1402, Bet v 1.1501, Bet v 1.1502, Bet v 1.1601, Bet v 1.1701, Bet v 1.1801, Bet v 1.1901, Bet v 1.2001, Bet v 1.2101, Bet v 1.2201, Bet v 1.2301, Bet v 1.2401, Bet v 1.2501, Bet v 1.2601, Bet v 1.2701, Bet v 1.2801, Bet v 1.2901, Bet v 1.3001, Bet v 1.3101, Bet v 2, Bet v 2.0101, Bet v 3, Bet v 3.0101, Bet v 4, Bet v 4.0101, Bet v 6, Bet v 6.0101, Bet v 6.0102, Bet v 7, Bet v 7.0101, Bet v 8, Bet v Glucanase), Beta spp (Beta v 1, Beta v 1.0101, Beta v 2, Beta v 2.0101), Blattella spp (Bla g 1, Bla g 1.0101, Bla g 1.0102, Bla g 1.0103, Bla g 1.0201, Bla g 1.0202, Blag 2, Blag 2.0101, Blag 2.0201, Blag 36kD, Blag 4, Bla g 4.0101, Bla g 4.0201, Bla g 5, Bla g 5.0101, Bla g 5.0201, Bla g 6, Bla g 6.0101, Bla g 6.0201, Bla g 6.0301, Bla g 7, Bla g 7.0101, Bla g 8, Bla g 8.0101, Bla g 9, Bla g Enolase, Bla g GSTD1, Bla g RACK1, Bla g TPI, Bla g Trypsin, Bla g Vitellogenin), Blatta spp (Bla o 1, Bla o 7), Blomia spp (Blo t 1, Blo t 1.0101, Blo t 1.0201, Blo t 10, Blo t 10.0101, Blo t 10.0102, Blo t 11, Blo t 11.0101, Blo t 12, Blo t 12.0101, Blo t 12.0102, Blo t 13, Blo t 13.0101, Blo t 14, Blo t 15, Blo t 18, Blo t 19, Blo t 19.0101, Blo t 2, Blo t 2.0101, Blo t 2.0102, Blo t 2.0103, Blo t 20, Blo t 21, Blo t 21.0101, Blo t 3, Blo t 3.0101, Blo t 4, Blo t 4.0101, Blo t 5, Blo t 5.0101, Blo t 6, Blo t 6.0101, Blo t 7, Blo t 8, Blo t 9, Blo t HSP70), Bombus spp (Bom ar 4, Bom hy 4, Bom p 1, Bom p 1.0101, Bom p 2, Bom p 3, Bom p 4, Bom p 4.0101, Bom t 1, Bom t 1.0101, Bom t 4, Bom t 4.0101), Bombyx spp (Bomb m 1, Bomb m 1.0101, Bomb m 7, Bomb m 7.0101, Bomb m 7.0102, Bomb m 7.0103, Bomb m 7.0104, Bomb m 7.0105, Bomb m 7.0106), Boophilus spp (Boo m 1, Boo m 7, Boo m 7.0101), Bos spp (Bos d 2, Bos d 2.0101, Bos d 2.0102, Bos d 2.0103, Bos d 3, Bos d 3.0101, Bos d 4, Bos d 4.0101, Bos d 5, Bos d 5.0101, Bos d 5.0102, Bos d 6, Bos d 6 (MDA), Bos d 6.0101, Bos d 7, Bos d 7.0101, Bos d 8, Bos d 8 alphaS1, Bos d 8 alphaS2, Bos d 8 beta, Bos d 8 kappa, Bos d alpha2I, Bos d alpha2I.0101, Bos d Chymosin, Bos d Fibrin, Bos d Gelatin, Bos d HG, Bos d Insulin, Bos d Lactoferrin, Bos d Lactoperoxidase, Bos d Myoglobin, Bos d OBP, Bos d OSCP, Bos d Phosvitin, Bos d PLA2, Bos d PRVB, Bos d Thrombin, Bos d TI, Bos gr ALA, Bos gr Myoglobin), Bothrops spp (Bot as 1, Bot at 1), Bouteloua spp (Bou g 1), Biting spp (Bov ov 1), Brama spp (Bra du 1), Brassica spp (Braj 1, Bra j 1.0101, Bra n 1, Bra n 1.0101, Bra n 4, Bra n 7, Bra n 8, Bra n PG, Bra ni 8, Bra o 3, Bra o 3.0101, Bra r 1, Bra r 1.0101, Bra r 2, Bra r 2.0101, Bra r 3, Bra r 4, Bra r 7), Bromus spp (Bro a 1, Bro a 4), Brosme spp (Bro br 1), Bromus spp (Bro i 1, Bro i 5, Bro i 7), Brugia spp (Bru m 3, Bru m 3.0101, Bru m Bm33), Bubalus spp (Bub b ALA, Bub b BLG, Bub b Casein, Bub b Casein alphaS1, Bub b Casein alphaS2, Bub b Casein beta, Bub b Casein kappa), Caenorhabditis spp (Cae b 3, Cae b 3.0101, Cae br 3, Cae br 3.0101, Cae e 3, Cae e 3.0101, Cae e 3.0102, Cae re 13, Cae re 13.0101), Cajanus spp (Caj c 1), Caligus spp (Cal cl 1, Cal cl 1.0101, Cal cl 1.0102), Calamus spp (Cal le 1), Callinectes spp (Cal s 2), Camelus spp (Cam d ALA, Cam d Casein, Cam d Casein alphaS1, Cam d Casein alphaS2, Cam d Casein beta, Cam d Casein kappa), Camponotus spp (Cam fl 7, Cam fl 7.0101), Canis spp (Can f 1, Can f 1.0101, Can f 2, Can f 2.0101, Can f 3, Can f 3.0101, Can f 4, Can f 4.0101, Can f 5, Can f 5.0101, Can f 6, Can f 6.0101, Can f Feld1-like, Can f Homs2-like, Can f Phosvitin, Can f TCTP), Canthidermis spp (Can ma 1), Cancer spp (Can mg 2, Can p 1), Cannabis spp (Can s 3), Candida spp (Cand a 1, Cand a 1.0101, Cand a 3, Cand a 3.0101, Cand a CAAP, Cand a CyP, Cand a Enolase, Cand a FPA, Cand a MnSOD, Cand a PGK, Cand b 2, Cand b 2.0101, Cand b FDH, Cand r Lipase), Capsicum spp (Cap a 1, Cap a 1.0101, Cap a 17kD, Cap a 2, Cap a 2.0101, Cap a 30kD, Cap a Glucanase, Cap ch 17kD), Caprella spp (Cap e 1), Capra spp (Cap h ALA, Cap h BLG, Cap h Casein, Cap h Casein alphaS1, Cap h Casein alphaS2, Cap h Casein beta, Cap h Casein kappa, Cap h GSA), Capitulum spp (Cap m 1), Carassius spp (Car au 1), Carpinus spp (Car b 1, Car b 1.0101, Car b 1.0102, Car b 1.0103, Car b 1.0104, Car b 1.0105, Car b 1.0106, Car b 1.0107, Car b 1.0108, Car b 1.0109, Car b 1.0110, Car b 1.0111, Car b 1.0112, Car b 1.0113, Car b 1.0201, Car b 1.0301, Car b 1.0302, Car b 2, Car b 4), Caranx spp (Car cr 1), Carya spp (Car i 1, Car i 1.0101, Car i 2, Car i 4, Car i 4.0101), Carcinus spp (Car ma 2), Caryota spp (Car mi 2), Carica spp (Car p 1, Car p Chitinase, Car p Chymopapain, Car p Endoproteinase), Castanea spp (Cas c 24kD, Cas s 1, Cas s 1.0101, Cas s 1.0102, Cas s 1.0103, Cas s 2, Cas s 5, Cas s 5.0101, Cas s 8, Cas s 8.0101, Cas s 9, Cas s 9.0101), Catharanthus spp (Cat r 1, Cat r 1.0101, Cat r 17kD, Cat r 2), Caulolatilus spp (Cau ch 1), Cavia spp (Cav p 1, Cav p 1.0101, Cav p 2, Cav p 2.0101, Cav p 3, Cav p 3.0101, Cav p Gelatin, Cav p GSA), Centropristis spp (Cen s 1), Cephalopholis spp (Cep so 1), Charybdis spp (Cha f 1, Cha f 1.0101), Chaetodipterus spp (Cha fa 1), Chamaecyparis spp (Cha o 1, Cha o 1.0101, Cha o 2, Cha o 2.0101), Chenopodium spp (Che a 1, Che a 1.0101, Che a 2, Che a 2.0101, Che a 3, Che a 3.0101), Chironomus spp (Chi k 1, Chi k 10, Chi k 10.0101), Chinchilla spp (Chi l 21kD_a, Chi l 21 kD_b), Chionoecetes spp (Chi o 1, Chi o 1.0101, Chi o 2, Chi o 4, Chi o 6, Chi o alpha_Actin, Chi o SERCA), Chironomus spp (Chi t 1, Chi t 1.0101, Chi t 1.0201, Chi t 2, Chi t 2.0101, Chi t 2.0102, Chi t 3, Chi t 3.0101, Chi t 4, Chi t 4.0101, Chi t 5, Chi t 5.0101, Chi t 6, Chi t 6.0101, Chi t 6.0201, Chi t 7, Chi t 7.0101, Chi t 8, Chi t 8.0101, Chi t 9, Chi t 9.0101), Chlamys spp (Chi n 1), Chloephaga spp (Chi pi 1), Chortoglyphus spp (Cho a 10), Chrysomela spp (Chr tr 7, Chr tr 7.0101), Cicer spp (Cic a 2S Albumin, Cic a Albumin), Cichorium spp (Cic i 1), Cimex spp (Cim I Nitrophorin), Citrus spp (Cit l 1, Cit l 3, Cit l 3.0101), Citrullus spp (Cit la 2, Cit la MDH, Cit la TPI), Citrus spp (Cit r 3, Cit r 3.0101, Cit s 1, Cit s 1.0101, Cit s 2, Cit s 2.0101, Cit s 3, Cit s 3.0101, Cit s 3.0102, Cit s IFR), Cladosporium spp (Cla c 14, Cla c 14.0101, Cla c 9, Cla c 9.0101, Cla h 1, Cla h 10, Cla h 10.0101, Cla h 12, Cla h 12.0101, Cla h 2, Cla h 2.0101, Cla h 42kD, Cla h 5, Cla h 5.0101, Cla h 6, Cla h 6.0101, Cla h 7, Cla h 7.0101, Cla h 8, Cla h 8 CSP, Cla h 8.0101, Cla h 9, Cla h 9.0101, Cla h abH, Cla h GST, Cla h HCh1, Cla h HSP70, Cla h NTF2, Cla h TCTP), Clostridium spp (Clo hi Collagenase, Clo t Toxoid), Clupea spp (Clu h 1, Clu h 1.0101, Clu h 1.0201, Clu h 1.0301), Cocos spp (Coc n 2, Coc n 4, Coc n 5), Coccidioides spp (Coc po 8), Coffea spp (Cof a 1, Cof a 1.0101), Columba spp (Col l PSA), Coprinus spp (Cop c 1, Cop c 1.0101, Cop c 2, Cop c 2.0101, Cop c 3, Cop c 3.0101, Cop c 4, Cop c 5, Cop c 5.0101, Cop c 6, Cop c 7, Cop c 7.0101), Corylus spp (Cor a 1, Cor a 1.0101, Cor a 1.0102, Cor a 1.0103, Cor a 1.0104, Cor a 1.0201, Cor a 1.0301, Cor a 1.0401, Cor a 1.0402, Cor a 1.0403, Cor a 1.0404, Cor a 10, Cor a 10.0101, Cor a 11, Cor a 11.0101, Cor a 12, Cor a 12.0101, Cor a 13, Cor a 13.0101, Cor a 14, Cor a 14.0101, Cor a 2, Cor a 2.0101, Cor a 2.01 02, Cor a 8, Cor a 8.0101, Cor a 9, Cor a 9.0101), Corynebacterium spp (Cor d Toxoid), Corylus spp (Cor he 1), Coryphaena spp (Cor hi 1), Coriandrum spp (Cor s 1, Cor s 11kD, Cor s 2), Cotoneaster spp (Cot l 3), Crangon spp (Cra c 1, Cra c 1.0101, Cra c 2, Cra c 2.0101, Cra c 4, Cra c 4.0101, Cra c 5, Cra c 5.0101, Cra c 6, Cra c 6.0101, Cra c 8, Cra c 8.0101), Crassostrea spp (Cra g 1), Cricetus spp (Cri c HSA), Crivellia spp (Cri pa 1), Crocus spp (Cro s 1, Cro s 1.0101, Cro s 2, Cro s 2.0101, Cro s 3, Cro s 3.01, Cro s 3.02), Cryptomeria spp (Cry j 1, Cry j 1.0101, Cry j 1.0102, Cry j 1.0103, Cry j 2, Cry j 2.0101, Cry j 2.0102, Cry j 3, Cry j 3.1, Cry j 3.2, Cry j 3.3, Cry j 3.4, Cry j 3.5, Cry j 3.6, Cry j 3.7, Cry j 3.8, Cry j 4, Cry j AP, Cry j Chitinase, Cry j CPA9, Cry j IFR, Cry j LTP, Cry j P1-P2), Cryphonectria spp (Cry p AP), Ctenocephalides spp (Cte f 1, Cte f 1.0101, Cte f 2, Cte f 2.0101, Cte f3, Cte f3.0101), Ctenopharyngodon spp (Cte id 1), Cucumis spp (Cuc m 1, Cuc m 1.0101, Cuc m 2, Cuc m 2.0101, Cuc m 3, Cuc m 3.0101, Cuc m Lec17, Cuc m MDH), Cucurbita spp (Cuc ma 18kD, Cuc ma 2, Cuc p 2, Cuc p AscO), Cucumis spp (Cuc s 2), Culicoides spp (Cul n 1, Cul n 10, Cul n 11, Cul n 2, Cul n 3, Cul n 4, Cul n 5, Cul n 6, Cul n 7, Cul n 8, Cul n 9, Cul n HSP70), Culex spp (Cul q 28kD, Cul q 35kD, Cul q 7, Cul q 7.0101, Cul q 7.0102), Culicoides spp (Cul so 1), Cuminum spp (Cum c 1, Cum c 2), Cupressus spp (Cup a 1, Cup a 1.0101, Cup a 1.02, Cup a 2, Cup a 3, Cup a 4, Cup a 4.0101, Cup s 1, Cup s 1.0101, Cup s 1.0102, Cup s 1.0103, Cup s 1.0104, Cup s 1.0105, Cup s 3, Cup s 3.0101, Cup s 3.0102, Cup s 3.0103, Cup s 8), Cochliobolus spp (Cur l 1, Cur l 1.0101, Cur l 2, Cur l 2.0101, Cur l 3, Cur l 3.0101, Cur l 4, Cur l 4.0101, Cur l ADH, Cur l GST, Cur l MnSOD, Cur l Oryzin, Cur l Trx, Cur l ZPS1), Cyanochen spp (Cya cy 1), Cynoscion spp (Cyn ar 1), Cynosurus spp (Cyn cr 1, Cyn cr 5), Cynodon spp (Cyn d 1, Cyn d 1.0101, Cyn d 1.0102, Cyn d 1.0103, Cyn d 1.0104, Cyn d 1.0105, Cyn d 1.0106, Cyn d 1.0107, Cyn d 1.0201, Cyn d 1.0202, Cyn d 1.0203, Cyn d 1.0204, Cyn d 10, Cyn d 11, Cyn d 12, Cyn d 12.0101, Cyn d 13, Cyn d 15, Cyn d 15.0101, Cyn d 2, Cyn d 22, Cyn d 22.0101, Cyn d 23, Cyn d 23.0101, Cyn d 24, Cyn d 24.0101, Cyn d 4, Cyn d 5, Cyn d 6, Cyn d 7, Cyn d 7.0101), Cynoscion spp (Cyn ne 1), Cynomys spp (Cyn sp Lipocalin), Cyprinus spp (Cyp c 1, Cyp c 1.01, Cyp c 1.02), Daboia spp (Dab ru 1), Dactylis spp (Dac g 1, Dac g 1.01, Dac g 1.0101, Dac g 1.02, Dac g 12, Dac g 13, Dac g 2, Dac g 2.0101, Dac g 3, Dac g 3.0101, Dac g 4, Dac g 4.0101, Dac g 5, Dac g 5.0101, Dac g 7), Dama spp (Dam d CSA), Danio spp (Dan re 1, Dan re 2, Dan re alpha2I, Dan re CK), Dasyatis spp (Das ak 1, Das am 1, Das sa 1), Daucus spp (Dau c 1, Dau c 1.0101, Dau c 1.0102, Dau c 1.0103, Dau c 1.0104, Dau c 1.0105, Dau c 1.0201, Dau c 1.0301, Dau c 3, Dau c 4, Dau c 4.0101, Dau c CyP), Decapterus spp (Dec ru 1), Dendronephthya spp (Den n 1, Den n 1.0101), Dermatophagoides spp (Der f 1, Der f 1.0101, Der f 1.0102, Der f 1.0103, Der f 1.0104, Der f 1.0105, Der f 1.0106, Der f 1.0107, Der f 1.0108, Der f 1.0109, Der f 1.0110, Der f 10, Der f 10.0101, Der f 10.0102, Der f 11, Der f 11.0101, Der f 13, Der f 13.0101, Der f 14, Der f 14.0101, Der f 15, Der f 15.0101, Der f 16, Der f 16.0101, Der f 17, Der f 17.0101, Der f 18, Der f 18.0101, Derf 2, Der f 2.0101, Der f 2.0102, Der f 2.0103, Der f 2.0104, Der f 2.0105, Der f 2.0106, Der f 2.0107, Der f 2.0108, Der f 2.0109, Der f 2.0110, Der f 2.0111, Der f 2.0112, Der f 2.0113, Der f 2.0114, Der f 2.0115, Der f 2.0116, Der f 2.0117, Der f 20, Der f 21, Der f 22, Der f 22.0101, Der f 3, Der f 3.0101, Der f 4, Der f 5, Der f 6, Der f 6.0101, Der f 7, Der f 7.0101, Der f 8, Der f 9, Der f HSP70), Dermanyssus spp (Der g 10, Der g 10.0101), Dermatophagoides spp (Der m 1, Der m 1.0101, Der p 1, Der p 1.0101, Der p 1.0102, Der p 1.0103, Der p 1.0104, Der p 1.0105, Der p 1.0106, Der p 1.0107, Der p 1.0108, Der p 1.0109, Der p 1.0110, Der p 1.0111, Der p 1.0112, Der p 1.0113, Der p 1.0114, Der p 1.0115, Der p 1.0116, Der p 1.0117, Der p 1.0118, Der p 1.0119, Der p 1.0120, Der p 1.0121, Der p 1.0122, Der p 1.0123, Der p 1.0124, Der p 10, Der p 10.0101, Der p 10.0102, Der p 10.0103, Der p 11, Der p 11.0101, Der p 13, Der p 14, Der p 14.0101, Der p 15, Der p 18, Der p 2, Der p 2.0101, Der p 2.0102, Der p 2.0103, Der p 2.0104, Der p 2.0105, Der p 2.0106, Der p 2.0107, Der p 2.0108, Der p 2.0109, Der p 2.0110, Der p 2.0111, Der p 2.0112, Der p 2.0113, Der p 2.0114, Der p 2.0115, Der p 20, Der p 20.0101, Der p 21, Der p 21.0101, Der p 23, Der p 23.0101, Der p 3, Der p 3.0101, Der p 4, Der p 4.0101, Der p 5, Der p 5.0101, Der p 5.0102, Der p 6, Der p 6.0101, Der p 7, Der p 7.0101, Der p 8, Der p 8.0101, Der p 9, Der p 9.0101, Der p 9.0102, Der p P1-P2, Der p P2-P1, Der s 1, Der s 2, Der s 3), Dianthus spp (Dia c RIP), Dicranopteris spp (Dic l 2S Albumin), Diospyros spp (Dio k 17kD, Dio k 4, Dio k IFR), Dioscorea spp (Dio p TSP), Diplodus spp (Dip ho 1), Distichlis spp (Dis s 1, Dis s 7), Ditrema spp (Dit te 1), Dolichovespula spp (Dol a 1, Dol a 2, Dol a 5, Dol a 5.0101), Dolichos spp (Dol b Agglutinin), Dolichovespula spp (Dol m 1, Dol m 1.0101, Dol m 1.02, Dol m 2, Dol m 2.0101, Dol m 5, Dol m 5.0101, Dol m 5.02), Drosophila spp (Dro an 7, Dro an 7.0101, Dro er 7, Dro er 7.0101, Dro er 7.0102, Dro gr 7, Dro gr 7.0101, Dro gr 7.0102, Dro m 7, Dro m 7.0101, Dro m 7.0102, Dro m 7.0103, Dro m 7.0104, Dro m 7.0105, Dro m 7.0106, Dro m 7.0107, Dro m 7.0108, Dro m 7.0109, Dro m 7.0110, Dro m 7.0111, Dro m 7.0112, Dro m 7.0113, Dro m 9, Dro m MnSOD, Dro mo 7, Dro mo 7.0101, Dro pp 7, Dro pp 7.0101, Dro se 7, Dro se 7.0101, Dro si 7, Dro si 7.0101, Dro si 7.0102, Dro vi 7, Dro vi 7.0101, Dro wi 7, Dro wi 7.0101, Dro y 7, Dro y 7.0101, Dro y 7.0102, Dro y 7.0103), Echium spp (Ech p Cytochrome C), Elaeis spp (Ela g 2, Ela g Bd31kD), Elops spp (Elo sa 1), Embellisia spp (Emb a 1, Emb i 1, Emb nz 1, Emb t 1), Engraulis spp (Eng e 1), Enteroctopus spp (Ent d 1), Epinephelus spp (Epi bl 1, Epi co 1, Epi fl 1, Epi mc 1, Epi mo 1), Epicoccum spp (Epi p 1, Epi p 1.0101, Epi p 12kD, Epi p GST), Epinephelus spp (Epi po 1, Epi un 1), Equisetum spp (Equ a 17kD), Equus spp (Equ as 4, Equ as DSA, Equ bu 4, Equ c 1, Equ c 1.0101, Equ c 2, Equ c 2.0101, Equ c 2.0102, Equ c 3, Equ c 3.0101, Equ c 4, Equ c 4.0101, Equ c 5, Equ c 5.0101, Equ c ALA, Equ c BLG, Equ c Casein, Equ c Casein beta, Equ c Casein kappa, Equ c PRVB, Equ he 4, Equ z ZSA), Erimacrus spp (Eri i 1, Eri i 1.0101, Eri i 1.0102), Eriocheir spp (Eri s 1, Eri s 1.0101, Eri s 2), Erwinia spp (Erw ch Asparaginase), Escherichia spp (Esc c Asparaginase, Esc c beta GAL), Esox spp (Eso l 1), Euphausia spp (Eup p 1, Eup p 1.0101), Euphasia spp (Eup s 1, Eup s 1.0101), Euroglyphus spp (Eur m 1, Eur m 1.0101, Eur m 1.0102, Eur m 1.0103, Eur m 10, Eur m 14, Eur m 14.0101, Eur m 2, Eur m 2.0101, Eur m 2.0102, Eur m 3, Eur m 3.0101, Eur m 4, Eur m 4.0101), Evynnis spp (Evy j 1), Fagopyrum spp (Fag e 1, Fag e 1.0101, Fag e 10kD, Fag e 19kD, Fag e 2, Fag e 2.0101, Fag e TI), Fagus spp (Fag s 1, Fag s 1.0101, Fag s 2, Fag s 4), Fagopyrum spp (Fag t 1, Fag t 10kD, Fag t 2, Fag t 2.0101), Felis spp (Fel d 1, Fel d 1.0101, Fel d 2, Fel d 2.0101, Fel d 3, Fel d 3.0101, Fel d 4, Fel d 4.0101, Fel d 5, Fel d 5.0101, Fel d 6, Fel d 6.0101, Fel d 7, Fel d 7.0101, Fel d 8, Fel d 8.0101, Fel d IgG), Fenneropenaeus spp (Fen c 1, Fen c 2, Fen me 1, Fen me 1.0101), Festuca spp (Fes e 1, Fes e 13, Fes e 4, Fes e 5, Fes e 7, Fes p 1, Fes p 13, Fes p 4, Fes p 4.0101, Fes p 5, Fes r 1, Fes r 5), Ficus spp (Fic c 17kD, Fic c 4, Fic c Ficin), Foeniculum spp (Foe v 1, Foe v 2), Forsythia spp (For s 1), Forcipomyia spp (For t 1, For t 1.0101, For t 2, For t 2.0101, For t 7, For t FPA, For t Myosin, For t TPI), Fragaria spp (Fra a 1, Fra a 1.0101, Fra a 3, Fra a 3.0101, Fra a 3.0102, Fra a 3.0201, Fra a 3.0202, Fra a 3.0203, Fra a 3.0204, Fra a 3.0301, Fra a 4, Fra a 4.0101, Fra c 1), Fraxinus spp (Fra e 1, Fra e 1.0101, Fra e 1.0102, Fra e 1.0201, Fra e 12, Fra e 2, Fra e 3, Fra e 9), Fragaria spp (Fra v 1), Fusarium spp (Fus c 1, Fus c 1.0101, Fus c 2, Fus c 2.0101, Fus c 3, Fus s 1, Fus s 45kD, Fus sp Lipase), Gadus spp (Gad c 1, Gad c 1.0101, Gad c APDH, Gad m 1, Gad m 1.0101, Gad m 1.0102, Gad m 1.0201, Gad m 1.0202, Gad m 45kD, Gad m Gelatin, Gad ma 1), Gallus spp (Gal d 1, Gal d 1.0101, Gal d 2, Gal d 2.0101, Gal d 3, Gal d 3.0101, Gal d 4, Gal d 4.0101, Gal d 5, Gal d 5.0101, Gal d 6, Gal d 6.0101, Gal d Apo I, Gal d Apo VI, Gal d GPI, Gal d HG, Gal d IgY, Gal d L-PGDS, Gal d Ovomucin, Gal d Phosvitin, Gal d PRVB, Gal la 4), Galleria spp (Gal m 18kD, Gal m 24kD), Gallus spp (Gal so 4), Gammarus spp (Gam s TM), Gelonium spp (Gel m RIP), Geothelphusa spp (Geo de 1), Glossina spp (Glo m 5, Glo m 5.0101, Glo m 7, Glo m 7.0101, Glo m 7.0102, Glo m 7.0103), Glycine spp (Gly a Bd30K, Gly ar Bd30K, Gly ca Bd30K, Gly cl Bd30K, Gly cu Bd30K, Gly cy Bd30K), Glycyphagus spp (Gly d 10, Gly d 10.0101, Gly d 13, Gly d 2, Gly d 2.0101, Gly d 2.0201, Gly d 2.03, Gly d 2/Lep d 2 L1, Gly d 2/Lep d 2 L2, Gly d 2/Lep d 2 L3, Gly d 2/Lep d 2 L4, Gly d 2/Lep d 2 R1, Gly d 2/Lep d 2 R2, Gly d 2/Lep d 2 R3, Gly d 2/Lep d 2 R4, Gly d 2/Lep d 2 R5, Gly d 20, Gly d 3, Gly d 5, Gly d 5.01, Gly d 5.02, Gly d 7, Gly d 8), Glycine spp (Gly f Bd30K, Gly l Bd30K, Gly m 1, Gly m 1.0101, Gly m 1.0102, Gly m 2, Gly m 2.0101, Gly m 2S Albumin, Gly m 3, Gly m 3.0101, Gly m 3.0102, Gly m 39kD, Gly m 4, Gly m 4.0101, Gly m 5, Gly m 5.0101, Gly m 5.0201, Gly m 5.0301, Gly m 5.0302, Gly m 50kD, Gly m 6, Gly m 6.0101, Gly m 6.0201, Gly m 6.0301, Gly m 6.0401, Gly m 6.0501, Gly m 68kD, Gly m Agglutinin, Gly m Bd28K, Gly m Bd30K, Gly m Bd60K, Gly m CPI, Gly m EAP, Gly m TI, Gly mi Bd30K, Gly s Bd30K, Gly t Bd30K, Gly to Bd30K), Gossypium spp (Gos h Vicilin), Haemophilus spp (Hae in P6), Haemaphysalis spp (Hae l 7, Hae l 7.0101, Hae q 7, Hae q 7.0101), Haliotis spp (Hal a 1, Hal d 1, Hal di 1, Hal di PM, Hal m 1, Hal m 1.0101, Hal r 1, Hal r 49kD, Hal ru 1), Harmonia spp (Har a 1, Har a 1.0101, Har a 2, Har a 2.0101), Harpegnathos spp (Har sa 7, Har sa 7.0101, Har sa 7.0102), Helianthus spp (Hel a 1, Hel a 1.0101, Hel a 2, Hel a 2.0101, Hel a 2S Albumin, Hel a 3, Hel a 3.0101, Hel a 4), Helix spp (Hel ap 1, Hel as 1, Hel as 1.0101), Heligmosomoides spp (Hel p 3, Hel p 3.0101), Helianthus spp (Hel tu 1), Hemanthias spp (Hem le 1), Hemifusus spp (Hem t 1), Heterodera spp (Het g 3, Het g 3.0101), Hevea spp (Hev b 1, Hev b 1.0101, Hev b 10, Hev b 10.0101, Hev b 10.0102, Hev b 10.0103, Hev b 11, Hev b 11.0101, Hev b 11.0102, Hev b 12, Hev b 12.0101, Hev b 13, Hev b 13.0101, Hev b 14, Hev b 14.0101, Hev b 2, Hev b 2.0101, Hev b 3, Hev b 3.0101, Hev b 4, Hev b 4.0101, Hev b 5, Hev b 5.0101, Hev b 6, Hev b 6.01, Hev b 6.02, Hev b 6.0202, Hev b 6.03, Hev b 7, Hev b 7.01, Hev b 7.02, Hev b 7.D2, Hev b 7.S2, Hev b 8, Hev b 8.0101, Hev b 8.0102, Hev b 8.0201, Hev b 8.0202, Hev b 8.0203, Hev b 8.0204, Hev b 9, Hev b 9.0101, Hev b Citrate binding Protein, Hev b GAPDH, Hev b HSP80, Hev b IFR, Hev b Proteasome subunit, Hev b Rotamase, Hev b SPI, Hev b Trx, Hev b UDPGP), Hexagrammos spp (Hex ot 1), Hippoglossus spp (Hip h 1), Hippoglossoides spp (Hip pl 1), Hippoglossus spp (Hip st 1), Hirudo spp (Hir me Hirudin), Holcus spp (Hol l 1, Hol l 1.0101, Hol l 1.0102, Hol l 2, Hol l 4, Hol l 5, Hol l 5.0101, Hol l 5.0201), Holocnemus spp (Hol pl 9, Hol pl Hemocyanin), Homarus spp (Hom a 1, Hom a 1.0101, Hom a 1.0102, Hom a 1.0103, Hom a 3, Hom a 3.0101, Hom a 4, Hom a 6, Hom a 6.0101, Hom g 1, Hom g 2), Homo spp (Hom s 1, Hom s 1.0101, Hom s 2, Hom s 2.0101, Hom s 3, Hom s 3.0101, Hom s 4, Hom s 4.0101, Hom s 5, Hom s 5.0101, Hom s AAT, Hom s ACTH, Hom s Adalimumab, Hom s ALA, Hom s alpha_Actin, Hom s alpha-Galactosidase, Hom s APDH, Hom s Arylsulfatase B, Hom s Casein, Hom s CyP A, Hom s CyP B, Hom s CyP C, Hom s DSF70, Hom s DSG3, Hom s eIF6, Hom s Etanercept, Hom s Factor IX, Hom s Factor VII, Hom s Factor VIII, Hom s G-CSF, Hom s Glucocerebrosidase, Hom s Glucosidase, Hom s HLA-DR-alpha, Hom s HSA, Hom s Iduronidase, Hom s Idursulfase, Hom s IgA, Hom s Insulin, Hom s Lactoferrin, Hom s Laminin gamma_2, Hom s MnSOD, Hom s Oxytocin, Hom s P2, Hom s Phosvitin, Hom s Profilin, Hom s PSA, Hom s RP1, Hom s TCTP, Hom s TL, Hom s TPA, Hom s TPO, Hom s Transaldolase, Hom s Trx, Hom s Tubulin-alpha, Hom s/Mus m Basiliximab, Hom s/Mus m Cetuximab, Hom s/Mus m Cetuximab (Gal-Gal), Hom s/Mus m Infliximab, Hom s/Mus m Natalizumab, Hom s/Mus m Omalizumab, Hom s/Mus m Palivizumab, Hom s/Mus m Rituximab, Hom s/Mus m Tocilizumab, Hom s/Mus m Trastuzumab), Hoplostethus spp (Hop a 1), Hordeum spp (Hor v 1, Hor v 12, Hor v 12.0101, Hor v 13, Hor v 14, Hor v 15, Hor v 15.0101, Hor v 16, Hor v 16.0101, Hor v 17, Hor v 17.0101, Hor v 18kD, Hor v 2, Hor v 21, Hor v 21.0101, Hor v 28, Hor v 33, Hor v 4, Hor v 5, Hor v 5.0101, Hor v BDAI, Hor v BTI), Humicola spp (Hum in Cellulase), Humulus spp (Hum j 1, Hum j 1.0101, Hum j 10kD, Hum j 2), Huso spp (Hus h 1), Hylocereus spp (Hyl un LTP), Hymenocephalus spp (Hym st 1), Hyperoglyphe spp (Hyp by 1), Hypophthalmichthys spp (Hyp mo 1), Hypophthalmichthy spp (Hyp no 1), Ictalurus spp (Ict fu 1, Ict p 1), Imperata spp (Imp c 4, Imp c 5, Imp c VIIIe1), Ixodes spp (Ixo r 2, Ixo sc 7, Ixo sc 7.0101), Jasus spp (Jas la 1, Jas la 1.0101, Jas la 1.0102), Juglans spp (Jug ca 1, Jug ca 2, Jug ci 1, Jug ci 2, Jug n 1, Jug n 1.0101, Jug n 2, Jug n 2.0101, Jug r 1, Jug r 1.0101, Jug r 2, Jug r 2.0101, Jug r 3, Jug r 3.0101, Jug r 4, Jug r 4.0101, Jug r 5), Juniperus spp (Jun a 1, Jun a 1.0101, Jun a 1.0102, Jun a 2, Jun a 2.0101, Jun a 3, Jun a 3.0101, Jun c 1, Jun o 1, Jun o 4, Jun o 4.0101, Jun r 3, Jun r 3.1, Jun r 3.2, Jun v 1, Jun v 1.0101, Jun v 1.0102, Jun v 3, Jun v 3.0101, Jun v 3.0102, Jun v 4), Katsuwonus spp (Kat p 1), Kyphosus spp (Kyp se 1), Lachnolaimus spp (Lac ma 1), Lachesis spp (Lac mu 1), Lactuca spp (Lac s 1, Lac s 1.0101), Lagocephalus spp (Lag la 1), Larus spp (Lar a 1, Lar a 2, Lar a 3), Larimichthys spp (Lar po 1), Lates spp (Lat c 1), Lateolabrax spp (Lat ja 1), Lathyrus spp (Lat oc Agglutinin), Leiostomus spp (Lei xa 1), Lens spp (Len c 1, Len c 1.0101, Len c 1.0102, Len c 1.0103, Len c 2, Len c 2.0101, Len c 3, Len c 3.0101, Len c Agglutinin), Leopardus spp (Leo p 1), Lepidoglyphus spp (Lep d 10, Lep d 10.0101, Lep d 12, Lep d 13, Lep d 13.0101, Lep d 2, Lep d 2.0101, Lep d 2.0102, Lep d 2.0201, Lep d 2.0202, Lep d 3, Lep d 39kD, Lep d 5, Lep d 5.0101, Lep d 5.0102, Lep d 5.0103, Lep d 7, Lep d 7.0101, Lep d 8, Lep d alpha Tubulin), Lepomis spp (Lep gi 1), Leptomelanosoma spp (Lep i 1), Lepomis spp (Lep ma 1), Lepisma spp (Lep s 1, Lep s 1.0101, Lep s 1.0102), Lepeophtheirus spp (Lep sa 1, Lep sa 1.0101, Lep sa 1.0102, Lep sa 1.0103), Leptailurus spp (Lep se 1), Lepidorhombus spp (Lep w 1, Lep w 1.0101), Lethocerus spp (Let in 7, Let in 7.0101, Let in 7.0102), Leuciscus spp (Leu ce 1), Lewia spp (Lew in 1), Ligustrum spp (Lig v 1, Lig v 1.0101, Lig v 1.0102, Lig v 2), Lilium spp (Lil l 2, Lil l PG), Limanda spp (Lim fe 1), Limnonectes spp (Lim m 1), Limulus spp (Lim p 1, Lim p 1.0101, Lim p 2, Lim p LPA), Liposcelis spp (Lip b 1, Lip b 1.0101), Litchi spp (Litc 1, Lit c 1.0101, Lit c IFR, Lit c TPI), Lithobates spp (Lit ca 1), Litopenaeus spp (Lit se 1, Lit v 1, Lit v 1.0101, Lit v 2, Lit v 2.0101, Lit v 3, Lit v 3.0101, Lit v 4, Lit v 4.0101), Filiaria spp (Loa lo 3, Loa lo 3.0101), Lobotes spp (Lob su 1), Locusta spp (Loc m 7, Loc m 7.0101), Loligo spp (Lol b 1, Lol e 1), Lolium spp (Lol m 2, Lol m 5, Lol p 1, Lol p 1.0101, Lol p 1.0102, Lol p 1.0103, Lol p 10, Lol p 11, Lol p 11.0101, Lol p 12, Lol p 13, Lol p 2, Lol p 2.0101, Lol p 3, Lol p 3.0101, Lol p 4, Lol p 4.0101, Lol p 5, Lol p 5.0101, Lol p 5.0102, Lol p 7, Lol p CyP, Lol p FT, Lol p Legumin), Lonomia spp (Lon o 7, Lon o 7.0101), Lophodytes spp (Lop cu 1), Lophonetta spp (Lop sp 1), Lupinus spp (Lup a 1, Lup a alpha_Conglutin, Lup a delta_Conglutin, Lup a gamma_Conglutin, Lup an 1, Lup an 1.0101, Lup an alpha_Conglutin, Lup an delta_Conglutin, Lup an gamma_Conglutin, Lup l 17kD), Lutjanus spp (Lut a 1, Lut c 1, Lut cy 1, Lut gr 1, Lut gu 1, Lut jo 1), Lutraria spp (Lut p 1), Lutjanus spp (Lut pu 1, Lut sy 1), Lycopersicon spp (Lyc e 1, Lyc e 1.0101, Lyc e 11S Globulin, Lyc e 2, Lyc e 2.0101, Lyc e 2.0102, Lyc e 3, Lyc e 3.0101, Lyc e 4, Lyc e 4.0101, Lyc e ARP60S, Lyc e Chitinase, Lyc e Glucanase, Lyc e Peroxidase, Lyc e PG, Lyc e PME, Lyc e PR23, Lyc e Vicilin), Maconellicoccus spp (Mac h 7, Mac h 7.0101), Macruronus spp (Mac ma 1, Mac n 1), Maclura spp (Mac po 17kD), Macrobrachium spp (Mac ro 1, Mac ro 1.0101, Mac ro Hemocyanin), Macropus spp (Macr s Gelatin), Malus spp (Mal d 1, Mal d 1.0101, Mal d 1.0102, Mal d 1.0103, Mal d 1.0104, Mal d 1.0105, Mal d 1.0106, Mal d 1.0107, Mal d 1.0108, Mal d 1.0109, Mal d 1.0201, Mal d 1.0202, Mal d 1.0203, Mal d 1.0204, Mal d 1.0205, Mal d 1.0206, Mal d 1.0207, Mal d 1.0208, Mal d 1.0301, Mal d 1.0302, Mal d 1.0303, Mal d 1.0304, Mal d 1.0401, Mal d 1.0402, Mal d 1.0403, Mal d 2, Mal d 2.0101, Mal d 3, Mal d 3.0101, Mal d 3.0102, Mal d 3.0201, Mal d 3.0202, Mal d 3.0203, Mal d 4, Mal d 4.0101, Mal d 4.0102, Mal d 4.0201, Mal d 4.0202, Mal d 4.0301, Mal d 4.0302), Malpighia spp (Mal g 4, Mal g Hevein), Malus spp (Mal p 1), Malassezia spp (Mala f 2, Mala f 2.0101, Mala f 3, Mala f 3.0101, Mala f 4, Mala f 4.0101, Mala g 10, Mala s 1, Mala s 1.0101, Mala s 10, Mala s 10.0101, Mala s 11, Mala s 11.0101, Mala s 12, Mala s 12.0101, Mala s 13, Mala s 13.0101, Mala s 5, Mala s 5.0101, Mala s 6, Mala s 6.0101, Mala s 7, Mala s 7.0101, Mala s 8, Mala s 8.0101, Mala s 9, Mala s 9.0101), Manihot spp (Man e 5, Man e 5.0101, Man e FPA, Man e GAPDH), Mangifera spp (Man i 1, Man i 14kD, Man i 2, Man i 3, Man i 3.01, Man i 3.02, Man i Chitinase), Marsupenaeus spp (Mar j 1, Mar j 1.0101, Mar j 2, Mar j 4), Matricaria spp (Mat c 17kD), Mecopoda spp (Mec e 7), Megalobrama spp (Meg am 2, Meg am CK), Megathura spp (Meg c Hemocyanin), Megalops spp (Meg sp 1), Melanogrammus spp (Mel a 1), Meleagris spp (Mel g 1, Mel g 2, Mel g 3, Mel g PRVB, Mel g TSA), Melicertus spp (Mel l 1), Menticirrhus spp (Men am 1), Mercurialis spp (Mer a 1, Mer a 1.0101), Merluccius spp (Mer ap 1, Mer au 1, Mer bi 1, Mer ca 1, Mer ga 1, Mer hu 1), Merlangius spp (Mer me 1), Merluccius spp (Mer mr 1, Mer pa 1, Mer po 1, Mer pr 1, Mer se 1), Meriones spp (Mer un 23kD), Metarhizium spp (Met a 30), Metapenaeopsis spp (Met ba 1), Metapenaeus spp (Met e 1, Met e 1.0101, Met e 2), Metasequoia spp (Met gl 2), Metapenaeus spp (Met j 1, Met j 2), Metanephrops spp (Met ja 1), Metapenaeopsis spp (Met la 1), Metanephrops spp (Met t 2), Micromesistius spp (Mic po 1), Micropogonias spp (Mic un 1), Mimachlamys spp (Mim n 1), Momordica spp (Mom c RIP), Morus spp (Mor a 17kD, Mor a 4), Morone spp (Mor am 1), Morus spp (Mor n 3, Mor n 3.0101), Morone spp (Mor sa 1, Mor sc 1), Mugil spp (Mug c 1), Muraenolepis spp (Mur mi 1), Musa spp (Mus a 1, Mus a 1.0101, Mus a 2, Mus a 2.0101, Mus a 3, Mus a 3.0101, Mus a 4, Mus a 4.0101, Mus a 5, Mus a 5.0101, Mus a 5.0102), Mus spp (Mus m 1, Mus m 1.0101, Mus m 1.0102, Mus m 2, Mus m Gelatin, Mus m IgG, Mus m MSA, Mus m Muromonab, Mus m Phosvitin), Mustela spp (Mus p 17kD), Musa spp (Mus xp 1, Mus xp 2, Mus xp 5), Mycteroperca spp (Myc bo 1, Myc mi 1, Myc ph 1), Myceliophthora spp (Myc sp Laccase), Myrmecia spp (Myr p 1, Myr p 1.0101, Myr p 2, Myr p 2.0101, Myr p 2.0102, Myr p 3, Myr p 3.0101), Mytilus spp (Myt e 1, Myt g 1, Myt g PM), Myzus spp (Myz p 7, Myz p 7.0101), Nemorhedus spp (Nae go Hya), Necator spp (Nec a Calreticulin), Nemipterus spp (Nem vi 1), Neosartorya spp (Neo fi 1, Neo fi 22), Neochen spp (Neo ju 1), Neoscona spp (Neo n 7, Neo n 7.0101), Nephelium spp (Nep I GAPDH), Nephrops spp (Nep n 1, Nep n DF9), Neptunea spp (Nep po 1, Nep po 1.0101), Nicotiana spp (Nic t 8, Nic t Osmotin, Nic t Villin), Nimbya spp (Nim c 1, Nim s 1), Nippostrongylus spp (Nip b Ag1), Nycticebus spp (Nyc c 1), Octopus spp (Oct f 1, Oct l 1, Oct v 1, Oct v 1.0101, Oct v PM), Ocyurus spp (Ocy ch 1), Olea spp (Ole e 1, Ole e 1.0101, Ole e 1.0102, Ole e 1.0103, Ole e 1.0104, Ole e 1.0105, Ole e 1.0106, Ole e 1.0107, Ole e 10, Ole e 10.0101, Ole e 11, Ole e 11.0101, Ole e 11.0102, Ole e 12, Ole e 13, Ole e 2, Ole e 2.0101, Ole e 3, Ole e 3.0101, Ole e 36kD, Ole e 4, Ole e 4.0101, Ole e 5, Ole e 5.0101, Ole e 6, Ole e 6.0101, Ole e 7, Ole e 7.0101, Ole e 8, Ole e 8.0101, Ole e 9, Ole e 9.0101), Ommastrephes spp (Omm b 1, Omm b 1.0101), Oncorhynchus spp (Onc ke 1, Onc ke 18 kD, Onc ke alpha2l, Onc ke Vitellogenin, Onc m 1, Onc m 1.0101, Onc m 1.0201, Onc m alpha2l, Onc m Protamine, Onc m Vitellogenin, Onc ma 1, Onc ma FPA, Onc ma FSA, Onc ma TPI, Onc n 1), Onchocerca spp (Onc o 3, Onc o 3.0101), Oncorhynchus spp (Onc ts 1), Onchocerca spp (Onc v 3, Onc v 3.0101), Oratosquilla spp (Ora o 1, Ora o 1.0101), Oreochromis spp (Ore a 1, Ore mo 1, Ore mo 2, Ore mo FPA, Ore mo SCAF7145, Ore ni 1, Ore ni 18kD, Ore ni 45kD), Ornithonyssus spp (Orn sy 10, Orn sy 10.0101, Orn sy 10.0102), Oryctolagus spp (Ory c 1, Ory c 1.0101, Ory c 2, Ory c Casein, Ory c Phosvitin, Ory c RSA), Oryza spp (Ory s 1, Ory s 1.0101, Ory s 11, Ory s 12, Ory s 12.0101, Ory s 13, Ory s 14, Ory s 17kD, Ory s 19kD, Ory s 2, Ory s 23, Ory s 3, Ory s 7, Ory s aA_TI, Ory s GLP52, Ory s GLP63, Ory s Glyoxalase I, Ory s NRA), Ostrya spp (Ost c 1, Ost c 1.0101), Ovis spp (Ovi a ALA, Ovi a BLG, Ovi a Casein, Ovi a Casein alphaS1, Ovi a Casein alphaS2, Ovi a Casein beta, Ovi a Casein kappa, Ovi a Phosvitin, Ovi a SSA), Pachycondyla spp (Pac c 3), Pagrus spp (Pag m 1, Pag pa 1), Pampus spp (Pam ar 1, Pam c 1), Pandalus spp (Pan b 1, Pan b 1.0101), Pangasius spp (Pan bo 1), Pandalus spp (Pan e 1, Pan e 1.0101, Pan e 4), Panulirus spp (Pan h 1, Pan hy 1), Pangasius spp (Pan hy 18kD, Pan hy 45kD), Panulirus spp (Pan j 1), Panthera spp (Pan l 1, Pan o 1, Pan p 1), Panulirus spp (Pan s 1, Pan s 1.0101), Panthera spp (Pan t 1), Pan spp (Pan tr TCTP), Papaver spp (Pap s 17kD, Pap s 2, Pap s 34kD), Papilio spp (Pap xu 7, Pap xu 7.0101, Pap xu 7.0102), Paralichthys spp (Par a 1), Parasilurus spp (Par as 1, Par c 1), Paralithodes spp (Par c 1.0101, Par c 1.0102, Par f 1), Parthenium spp (Par h 1), Parietaria spp (Par j 1, Par j 1.0101, Par j 1.0102, Par j 1.0103, Par j 1.0201, Par j 2, Par j 2.0101, Par j 2.0102, Par j 3, Par j 3.0101, Par j 3.0102, Par j 4, Par j 4.0101, Par j J1-J2), Paralichthys spp (Par le 1), Parietaria spp (Par m 1, Par o 1, Par o 1.0101), Paralichthys spp (Par ol 1, Par ol alpha2l), Parahucho spp (Par pe Vitellogenin), Passiflora spp (Pas e Chitinase, Pas e Hevein), Paspalum spp (Pas n 1, Pas n 1.0101, Pas n 13), Patinopecten spp (Pat y 1), Pediculus spp (Ped h 7, Ped h 7.0101), Penaeus spp (Pen a 1, Pen a 1.0101, Pen a 1.0102, Pen a 1.0102 (103-117), Pen a 1.0102 (109-123), Pen a 1.0102 (1-15), Pen a 1.0102 (115-129), Pen a 1.0102 (121-135), Pen a 1.0102 (127-141), Pen a 1.0102 (13-27), Pen a 1.0102 (133-147), Pen a 1.0102 (139-153), Pen a 1.0102 (145-159)), Farfantepenaeus spp (Pen a 1.0102 (151-165)), Penaeus spp (Pen a 1.0102 (157-171), Pen a 1.0102 (163-177), Pen a 1.0102 (169-183), Pen a 1.0102 (175-189), Pen a 1.0102 (181-195), Pen a 1.0102 (187-201), Pen a 1.0102 (193-207), Pen a 1.0102 (19-33), Pen a 1.0102 (199-213), Pen a 1.0102 (205-219), Pen a 1.0102 (211-225), Pen a 1.0102 (217-231), Pen a 1.0102 (223-237), Pen a 1.0102 (229-243)), Farfantepenaeus spp (Pen a 1.0102 (235-249)), Penaeus spp (Pen a 1.0102 (241-255), Pen a 1.0102 (247-261), Pen a 1.0102 (253-267), Pen a 1.0102 (25-39), Pen a 1.0102 (259-273), Pen a 1.0102 (265-279), Pen a 1.0102 (270-284), Pen a 1.0102 (31-45), Pen a 1.0102 (37-51), Pen a 1.0102 (43-57), Pen a 1.0102 (49-63)), Farfantepenaeus spp (Pen a 1.0102 (55-69)), Penaeus spp (Pen a 1.0102 (61-75), Pen a 1.0102 (67-81), Pen a 1.0102 (7-21), Pen a 1.0102 (73-87), Pen a 1.0102 (79-93), Pen a 1.0102 (85-99), Pen a 1.0102 (91-105), Pen a 1.0102 (97-111), Pen a 1.0103), Penicillium spp (Pen b 13, Pen b 13.0101, Pen b 26, Pen b 26.0101, Pen c 1, Pen c 13, Pen c 13.0101, Pen c 18, Pen c 19, Pen c 19.0101, Pen c 2, Pen c 22, Pen c 22.0101, Pen c 24, Pen c 24.0101, Pen c 3, Pen c 3.0101, Pen c 30, Pen c 30.0101, Pen c 32, Pen c 32.0101, Pen c MnSOD, Pen ch 13, Pen ch 13.0101, Pen ch 18, Pen ch 18.0101, Pen ch 20, Pen ch 20.0101, Pen ch 31, Pen ch 31.0101, Pen ch 33, Pen ch 33.0101, Pen ch 35, Pen ch 35.0101, Pen ch MnSOD), Penaeus spp (Pen i 1, Pen i 1.0101, Pen m 1, Pen m 1.0101, Pen m 1.0102, Pen m 2, Pen m 2.0101, Pen m 3, Pen m 3.0101, Pen m 4, Pen m 4.0101, Pen m 6, Pen m 6.0101), Penicillium spp (Pen o 18, Pen o 18.0101), Penaeus spp (Pena o 1, Pena o 1.0101), Periplaneta spp (Per a 1, Per a 1.0101, Per a 1.0102, Per a 1.0103, Per a 1.0104, Per a 1.0105, Per a 1.0201, Per a 10, Per a 10.0101, Per a 2, Per a 3, Per a 3.0101, Per a 3.0201, Per a 3.0202, Per a 3.0203, Per a 4, Per a 5, Per a 6, Per a 6.0101, Per a 7, Per a 7.0101, Per a 7.0102, Per a 7.0103, Per a 9, Per a 9.0101, Per a Cathepsin, Per a FABP, Per a Trypsin, Per f 1, Per f 7, Per f 7.0101), Perna spp (Per v 1), Persea spp (Pers a 1, Pers a 1.0101, Pers a 4), Petroselinum spp (Pet c 1, Pet c 2, Pet c 3), Phalaris spp (Pha a 1, Pha a 1.0101, Pha a 5, Pha a 5.0101, Pha a 5.02, Pha a 5.03, Pha a 5.04), Phaseolus spp (Pha v 3, Pha v 3.0101, Pha v 3.0201, Pha v aAI, Pha v aAI.0101, Pha v Chitinase, Pha v PHA, Pha v Phaseolin), Phleum spp (Phl p 1, Phl p 1.0101, Phl p 1.0102, Phl p 11, Phl p 11.0101, Phl p 12, Phl p 12.0101, Phl p 12.0102, Phl p 12.0103, Phl p 13, Phl p 13.0101, Phl p 2, Phl p 2.0101, Phl p 3, Phl p 3.0101, Phl p 3.0102, Phl p 4, Phl p 4.0101, Phl p 4.0102, Phl p 4.0201, Phl p 4.0202, Phl p 4.0203, Phl p 4.0204, Phl p 5, Phl p 5.0101, Phl p 5.0102, Phl p 5.0103, Phl p 5.0104, Phl p 5.0105, Phl p 5.0106, Phl p 5.0107, Phl p 5.0108, Phl p 5.0109, Phl p 5.0201, Phl p 5.0202, Phl p 5.0203, Phl p 5.0204, Phl p 5.0205, Phl p 5.0206, Phl p 5.0207, Phl p 6, Phl p 6.0101, Phl p 6.0102, Phl p 7, Phl p 7.0101, Phl p P1-P2-P5-P6, Phl p P2-P6, Phl p P5-P1, Phl p P6-P2), Phoenix spp (Pho d 2, Pho d 2.0101, Pho d 40kD, Pho d 90kD), Phodopus spp (Pho s 21kD), Phoma spp (Pho t 1), Phragmites spp (Phr a 1, Phr a 12, Phr a 13, Phr a 4, Phr a 5), Phytolacca spp (Phy a RIP), Pimpinella spp (Pim a 1, Pim a 2), Pinna spp (Pin a 1), Piper spp (Pip n 14kD, Pip n 28kD), Pisum spp (Pis s 1, Pis s 1.0101, Pis s 1.0102, Pis s 2, Pis s 2.0101, Pis s 5, Pis s Agglutinin, Pis s Albumin), Pistacia spp (Pis v 1, Pis v 1.0101, Pis v 2, Pis v 2.0101, Pis v 2.0201, Pis v 3, Pis v 3.0101, Pis v 4, Pis v 4.0101, Pis v 5, Pis v 5.0101), Platanus spp (Pla a 1, Pla a 1.0101, Pla a 2, Pla a 2.0101, Pla a 3, Pla a 3.0101, Pla a 8), Platichthys spp (Pla f 1), Plantago spp (Pla l 1, Pla l 1.0101, Pla l 1.0102, Pla l 1.0103, Pla l Cytochrome C), Platanus spp (Pla oc 1, Pla or 1, Pla or 1.0101, Pla or 2, Pla or 2.0101, Pla or 3, Pla or 3.0101, Pla or 4, Pla or CyP, Pla r 1), Plectropomus spp (Pie ar 1), Pleospora spp (Pie h 1), Plectropomus spp (Pie le 1), Plodia spp (Plo i 1, Plo i 1.0101, Plo i 2, Plo i 2.0101), Poa spp (Poa p 1, Poa p 1.0101, Poa p 10, Poa p 12, Poa p 13, Poa p 2, Poa p 4, Poa p 5, Poa p 5.0101, Poa p 6, Poa p 7), Polistes spp (Pol a 1, Pol a 1.0101, Pol a 2, Pol a 2.0101, Pol a 5, Pol a 5.0101, Pol d 1, Pol d 1.0101, Pol d 1.0102, Pol d 1.0103, Pol d 1.0104, Pol d 4, Pol d 4.0101, Pol d 5, Pol d 5.0101, Pol e 1, Pol e 1.0101, Pol e 2, Pol e 4, Pol e 4.0101, Pol e 5, Pol e 5.0101, Pol f 5, Pol f 5.0101, Pol g 1, Pol g 1.0101, Pol g 2, Pol g 4, Pol g 5, Pol g 5.0101, Pol he MLT, Pol m 5, Pol m 5.0101), Polypedilum spp (Pol n 1), Pollicipes spp (Pol po 1), Pollachius spp (Pol vi 1), Polybia spp (Poly p 1, Poly p 1.0101, Poly p 2, Poly p 5, Poly s 5, Poly s 5.0101), Pomatomus spp (Pom sa 1), Pongo spp (Pon ab HSA), Pontastacus spp (Pon l 4, Pon l 4.0101, Pon l 7, Pon l 7.0101), Portunus spp (Por s 1, Por s 1.0101, Por s 1.0102, Por tr 1, Por tr 1.0101), Protortonia spp (Pro ca 38kD), Procumbarus spp (Pro cl 1, Pro cl 1.0101, Pro cl 21kD), Prosopis spp (Pro j 20kD), Prunus spp (Pru ar 1, Pru ar 1.0101, Pru ar 3, Pru ar 3.0101, Pru av 1, Pru av 1.0101, Pru av 1.0201, Pru av 1.0202, Pru av 1.0203, Pru av 2, Pru av 2.0101, Pru av 3, Pru av 3.0101, Pru av 4, Pru av 4.0101, Pru c 1, Pru d 1, Pru d 2, Pru d 3, Pru d 3.0101, Pru d 4, Pru du 1, Pru du 2, Pru du 2S Albumin, Pru du 3, Pru du 3.0101, Pru du 4, Pru du 4.0101, Pru du 4.0102, Pru du 5, Pru du 5.0101, Pru du 6, Pru du 6.0101, Pru du 6.0201, Pru du Conglutin, Pru p 1, Pru p 1.0101, Pru p 2, Pru p 2.0101, Pru p 2.0201, Pru p 2.0301, Pru p 3, Pru p 3.0101, Pru p 3.0102, Pru p 4, Pru p 4.0101, Pru p 4.0201, Pru sa 3), Psilocybe spp (Psi c 1, Psi c 1.0101, Psi c 2, Psi c 2.0101), Psoroptes spp (Pso o 1, Pso o 10, Pso o 10.0101, Pso o 11, Pso o 13, Pso o 14, Pso o 2, Pso o 21, Pso o 3, Pso o 5, Pso o 7), Puma spp (Pum c 1), Punica spp (Pun g 3), Pyrus spp (Pyr c 1, Pyr c 1.0101, Pyr c 3, Pyr c 3.0101, Pyr c 4, Pyr c 4.0101, Pyr c 5, Pyr c 5.0101, Pyr py 2), Quercus spp (Que a 1, Que a 1.0101, Que a 1.0201, Que a 1.0301, Que a 1.0401, Que a 2, Que a 4), Rachycentron spp (Rac ca 1), Rana spp (Ran e 1, Ran e 1.0101, Ran e 2, Ran e 2.0101), Ranina spp (Ran ra 1), Rangifer spp (Ran t BLG), Rattus spp (Rat n 1, Rat n 1.0101, Rat n Casein, Rat n Gelatin, Rat n IgG, Rat n Phosvitin, Rat n RSA, Rat n Transferrin), Rhizomucor spp (Rhi m AP), Rhizopus spp (Rhi nv Lipase, Rhi o Lipase), Rhomboplites spp (Rho au 1), Rhodotorula spp (Rho m 1, Rho m 1.0101, Rho m 2, Rho m 2.0101), Ricinus spp (Ric c 1, Ric c 1.0101, Ric c 2, Ric c 3, Ric c 8, Ric c RIP), Rivulus spp (Riv ma 1), Robinia spp (Rob p 2, Rob p 4, Rob p Glucanase), Rosa spp (Ros r 3), Roystonea spp (Roy e 2), Rubus spp (Rub i 1, Rub i 1.0101, Rub i 3, Rub i 3.0101, Rub i Chitinase, Rub i CyP), Saccharomyces spp (Sac c Carboxypeptidase Y, Sac c CyP, Sac c Enolase, Sac c Glucosidase, Sac c Invertase, Sac c MnSOD, Sac c P2, Sac c Profilin), Salvelinus spp (Sal f 1), Salsola spp (Sal k 1, Sal k 1.0101, Sal k 1.0201, Sal k 1.0301, Sal k 1.0302, Sal k 2, Sal k 2.0101, Sal k 3, Sal k 3.0101, Sal k 4, Sal k 4.0101, Sal k 4.0201, Sal k 5, Sal k 5.0101), Salvelinus spp (Sal le Vitellogenin), Salmo spp (Sal s 1, Sal s 1.0101, Sal s 1.0201, Sal s 2, Sal s 2.0101, Sal s Gelatin), Sambucus spp (Sam n 1), Sander spp (San lu 1), Saponaria spp (Sap o RIP), Sardinops spp (Sar m 1), Sarkidiornis spp (Sar ml 1), Sardina spp (Sar p 1), Sarcoptes spp (Sar s 1, Sar s 14, Sar s 3, Sar s GST, Sar s PM), Sardinops spp (Sar sa 1, Sar sa 1.0101), Schistosoma spp (Sch j GST, Sch j PM, Sch j Sj22, Sch j Sj67, Sch ma Sm20, Sch ma Sm21, Sch ma Sm22, Sch ma Sm31), Sciaenops spp (Sci oc 1), Scomber spp (Sco a 1), Scombermorus spp (Sco ca 1), Scomberomorus spp (Sco g 1), Scomber spp (Sco j 1, Sco ma 1, Sco s 1), Scolopendra spp (Sco y 7, Sco y 7.0101), Scylla spp (Scy o 1, Scy o 1.0101, Scy o 2, Scy pa 1, Scy pa 2, Scy s 1, Scy s 1.0101, Scy s 2), Sebastes spp (Seb fa 1, Seb in 1, Seb m 1, Seb m 1.0101, Seb m 1.0201), Secale spp (Sec c 1, Sec c 12, Sec c 13, Sec c 2, Sec c 20, Sec c 20.0101, Sec c 20.0201, Sec c 28, Sec c 3, Sec c 4, Sec c 4.0101, Sec c 4.0201, Sec c 5, Sec c 5.0101, Sec c aA_TI, Sec c aA_TI.0101), Senecio spp (Sen j MDH, Sen j PL), Sepia spp (Sep e 1, Sep e 1.0101), Sepioteuthis spp (Sep l 1, Sep l 1.0101), Sepia spp (Sep m 1), Seriola spp (Ser d 1, Ser la 1), Sergestes spp (Ser lu 1), Seriola spp (Ser q 1, Ser ri 1), Sesamum spp (Ses i 1, Ses i 1.0101, Ses i 2, Ses i 2.0101, Ses i 3, Ses i 3.0101, Ses i 4, Ses i 4.0101, Ses i 5, Ses i 5.0101, Ses i 6, Ses i 6.0101, Ses i 7, Ses i 7.0101, Ses i 8), Shigella spp (Shi bo GST, Shi dy GST), Simulia spp (Sim vi 1, Sim vi 2, Sim vi 3, Sim vi 4, Sim vi 70kD), Sinapis spp (Sin a 1, Sin a 1.0101, Sin a 1.0104, Sin a 1.0105, Sin a 1.0106, Sin a 1.0107, Sin a 1.0108, Sin a 2, Sin a 2.0101, Sin a 3, Sin a 3.0101, Sin a 4, Sin a 4.0101), Sinonovacula spp (Sin c 1, Sin c 1.0101), Solenopsis spp (Sol g 2, Sol g 2.0101, Sol g 3, Sol g 3.0101, Sol g 4, Sol g 4.0101, Sol g 4.0201, Sol i 1, Sol i 1.0101, Sol i 2, Sol i 2.0101, Sol i 3, Sol i 3.0101, Sol i 4, Sol i 4.0101), Solenocera spp (Sol me 1), Solenopsis spp (Sol r 1, Sol r 2, Sol r 2.0101, Sol r 3, Sol r 3.0101, Sol s 2, Sol s 2.0101, Sol s 3, Sol s 3.0101, Sol s 4), Solea spp (Sol so 1, Sol so TPI), Solanum spp (Sola t 1, Sola t 1.0101, Sola t 2, Sola t 2.0101, Sola t 3, Sola t 3.0101, Sola t 3.0102, Sola t 4, Sola t 4.0101, Sola t 8, Sola t Glucanase), Sorghum spp (Sor b 1, Sor h 1, Sor h 1.0101, Sor h 12, Sor h 7), Sparus spp (Spa a 1), Sphyrna spp (Sph ti 1), Spirulina spp (Spi mx beta_Phycocyanin), Spinacia spp (Spi o 2, Spi o RuBisCO), Squilla spp (Squ ac 1, Squ ac 1.0101, Squ o 1, Squ o 1.0101), Staphylococcus spp (Sta a FBP, Sta a SEA, Sta a SEB, Sta a SEC, Sta a SED, Sta a SEE, Sta a TSST), Stachybotrys spp (Sta c 3, Sta c 3.0101, Sta c Cellulase, Sta c Hemolysin, Sta c SchS34, Sta c Stachyrase A), Stemphylium spp (Ste b 1, Ste c 1, Ste v 1), Stolephorus spp (Sto i 1), Struthio spp (Str c 1, Str c 2, Str c 3), Streptococcus spp (Str dy Streptokinase), Streptomyces spp (Str g Pronase), Streptococcus spp (Str pn PspC), Strongylocentrotus spp (Str pu 18kD, Str pu Vitellogenin), Streptococcus spp (Str py SPEA, Str py SPEC, Str py Streptokinase), Strongyloides spp (Str st 45kD), Streptomyces spp (Str v PAT), Styela spp (Sty p 1), Suidasia spp (Sui m 1, Sui m 13, Sui m 2, Sui m 3, Sui m 5, Sui m 5.01, Sui m 5.02, Sui m 5.03, Sui m 6, Sui m 7, Sui m 8, Sui m 9), Sus spp (Sus s ACTH, Sus s ALA, Sus s Amylase, Sus s BLG, Sus s Casein, Sus s Casein alphaS1, Sus s Casein alphaS2, Sus s Casein beta, Sus s Casein kappa, Sus s Gelatin, Sus s HG, Sus s Insulin, Sus s Lipase, Sus s Pepsin, Sus s Phosvitin, Sus s PRVB, Sus s PSA, Sus s TCTP), Syntelopodeuma spp (Syn y 7, Syn y 7.0101), Syringa spp (Syr v 1, Syr v 1.0101, Syr v 1.0102, Syr v 1.0103, Syr v 2, Syr v 3, Syr v 3.0101), Tabanus spp (Tab y 1, Tab y 1.0101, Tab y 2, Tab y 2.0101, Tab y 5, Tab y 5.0101), Tadorna spp (Tad ra 1), Talaromyces spp (Tal st 22, Tal st 3, Tal st 8), Taraxacum spp (Tar o 18kD), Taxodium spp (Tax d 2), Tegenaria spp (Teg d Hemocyanin), Teladorsagia spp (Tel ci 3), Thaumetopoea spp (Tha p 1, Tha p 1.0101, Tha p 2, Tha p 2.0101), Theragra spp (The c 1), Thermomyces spp (The I Lipase, The sp Lipase, The sp Xylanase), Thunnus spp (Thu a 1, Thu a 1.0101, Thu a Collagen, Thu al 1, Thu at 1, Thu o 1, Thu o Collagen), Thuja spp (Thu oc 3, Thu p 1), Thunnus spp (Thu t 1, Thu to 1), Thyrsites spp (Thy at 1), Thyrophygus spp (Thy y 7, Thy y 7.0101), Todarodes spp (Tod p 1, Tod p 1.0101, Tod p 1.0102), Toxoptera spp (Tox c 7, Tox c 7.0101), Toxocara spp (Tox ca TES120, Tox ca TES26, Tox ca TES30), Toxoplasma spp (Tox g HSP70), Trachypenaeus spp (Tra c 1), Trachinotus spp (Tra ca 1), Trachurus spp (Tra j 1, Tra j Gelatin, Tra tr Gelatin), Triticum spp (Tri a 1, Tri a 10kD, Tri a 12, Tri a 12.0101, Tri a 12.0102, Tri a 12.0103, Tri a 12.0104, Tri a 13, Tri a 14, Tri a 14.0101, Tri a 14.0201, Tri a 15, Tri a 15.0101, Tri a 18, Tri a 18.0101, Tri a 19, Tri a 19.0101, Tri a 2, Tri a 21, Tri a 21.0101, Tri a 23kd, Tri a 25, Tri a 25.0101, Tri a 26, Tri a 26.0101, Tri a 27, Tri a 27.0101, Tri a 28, Tri a 28.0101, Tri a 29, Tri a 29.0101, Tri a 29.0201, Tri a 3, Tri a 30, Tri a 30.0101, Tri a 31, Tri a 31.0101, Tri a 32, Tri a 32.0101, Tri a 33, Tri a 33.0101, Tri a 34, Tri a 34.0101, Tri a 35, Tri a 35.0101, Tri a 36, Tri a 36.0101, Tri a 37, Tri a 37.0101, Tri a 4, Tri a 4.0101, Tri a 4.0201, Tri a 5, Tri a 7, Tri a aA_SI, Tri a alpha_Gliadin, Tri a bA, Tri a Bd36K, Tri a beta_Gliadin, Tri a Chitinase, Tri a CM16, Tri a DH, Tri a Endochitinase, Tri a gamma_Gliadin, Tri a Germin, Tri a Gliadin, Tri a GST, Tri a LMW Glu, Tri a LMW-GS B16, Tri a LMW-GS P42, Tri a LMW-GS P73, Tri a LTP2, Tri a omega2_Gliadin, Tri a Peroxidase, Tri a Peroxidase 1, Tri a SPI, Tri a TLP, Tri a Tritin, Tri a XI), Tritirachium spp (Tri al Proteinase K), Tribolium spp (Tri ca 17, Tri ca 17.0101, Tri ca 7, Tri ca 7.0101), Trichostrongylus spp (Tri co 3, Tri co 3.0101), Trichophyton spp (Tri eq 4), Trigonella spp (Tri fg 1, Tri fg 2, Tri fg 3, Tri fg 4), Trichosanthes spp (Tri k RIP), Trichiurus spp (Tri le 1), Triticum spp (Tri m Peroxidase), Trichophyton spp (Tri me 2, Tri me 4), Trisetum spp (Tri p 1, Tri p 5), Trichinella spp (Tri ps 3, Tri ps 3.0101), Trichophyton spp (Tri r 2, Tri r 2.0101, Tri r 4, Tri r 4.0101), Trichoderma spp (Tri rs Cellulase), Triticum spp (Tri s 14), Trichophyton spp (Tri sc 2, Tri sc 4, Tri so 2), Trichinella spp (Tri sp 3, Tri sp 3.0101, Tri sp 3.0102, Tri sp 3.0103, Tri sp 3.0104, Tri sp 3.0105, Tri sp 3.0106), Trichophyton spp (Tri t 1, Tri t 1.01 01, Tri t 4, Tri t 4.0101), Triticum spp (Tri td 14, Tri td aA_TI), Trichoderma spp (Tri v Cellulase), Trichophyton spp (Tri ve 4), Triatoma spp (Tria p 1, Tria p 1.0101), Triplochiton spp (Trip s 1), Turbo spp (Tur c 1, Tur c PM), Tyrophagus spp (Tyr p 1, Tyr p 10, Tyr p 10.0101, Tyr p 10.0102, Tyr p 13, Tyr p 13.0101, Tyr p 2, Tyr p 2.0101, Tyr p 24, Tyr p 24.0101, Tyr p 3, Tyr p 3.0101, Tyr p 4, Tyr p 5, Tyr p 5.01, Tyr p 5.02, Tyr p 5.03, Tyr p 7, Tyr p alpha Tubulin), Ulocladium spp (Ulo a 1, Ulo at 1, Ulo b 1, Ulo c 1, Ulo co 1, Ulo cu 1, Ulo mu 1, Ulo ob 1, Ulo se 1, Ulo su 1, Ulo tu 1), Uncia spp (Unc u 1), Urophycis spp (Uro te 1), Vaccinium spp (Vac m 3), Varroa spp (Var j 13kD), Venerupis spp (Ven ph 1, Ven ph 1.0101), Vespula spp (Ves f 1, Ves f 2, Ves f 5, Ves f 5.0101, Ves g 1, Ves g 2, Ves g 5, Ves g 5.0101, Ves m 1, Ves m 1.0101, Ves m 2, Ves m 2.0101, Ves m 5, Ves m 5.0101, Ves m MLT, Ves p 1, Ves p 2, Ves p 5, Ves p 5.0101, Ves s 1, Ves s 1.0101, Ves s 2, Ves s 5, Ves s 5.0101, Ves v 1, Ves v 1.0101, Ves v 2, Ves v 2.0101, Ves v 2.0201, Ves v 3, Ves v 3.0101, Ves v 5, Ves v 5.0101, Ves v 5-Pol a 5, Ves vi 5, Ves vi 5.0101), Vespa spp (Vesp c 1, Vesp c 1.0101, Vesp c 2, Vesp c 5, Vesp c 5.0101, Vesp c 5.0102, Vesp m 1, Vesp m 1.0101, Vesp m 5, Vesp m 5.0101, Vesp ma 1, Vesp ma 2, Vesp ma 5, Vesp ma MLT, Vesp v MLT), Vigna spp (Vig r 1, Vig r 1.0101, Vig r 17kD, Vig r 5, Vig r 8S Globulin, Vig r Albumin, Vig r beta-Conglycinin), Vitis spp (Vit v 1, Vit v 1.0101, Vit v 4, Vit v 5, Vit v Glucanase, Vit v TLP), Xiphias spp (Xip g 1, Xip g 1.0101, Xip g 25kD), Zea spp (Zea m 1, Zea m 1.0101, Zea m 11, Zea m 12, Zea m 12.0101, Zea m 12.0102, Zea m 12.0103, Zea m 12.0104, Zea m 12.0105, Zea m 13, Zea m 14, Zea m 14.0101, Zea m 14.0102, Zea m 2, Zea m 20S, Zea m 22, Zea m 25, Zea m 25.0101, Zea m 27kD Zein, Zea m 3, Zea m 4, Zea m 5, Zea m 50kD Zein, Zea m 7, Zea m Chitinase, Zea m G1, Zea m G2, Zea m PAO, Zea m Zm13), Zeus spp (Zeu fa 1), Ziziphus spp (Ziz m 1, Ziz m 1.0101), Zoarces spp (Zoa a ISP III), Zygophyllum spp (Zyg f 2).

In this context, the terms in brackets indicate the particular preferred allergens from the particular source.

Most preferably the antigen associated with allergy or allergic disease is preferably derived from a source selected from the list consisting of grass pollen (e.g. pollen of rye), tree pollen (e.g. pollen of hazel, birch, alder, ash), flower pollen, herb pollen (e.g. pollen of mugwort), dust mite (e.g. Der f 1, Der p 1, Eur m 1, Der m 1 Der f 2, Der p 2, Eur m 2, Tyr p 2, Lep d 2), mold (e.g. allergens of Acremonium, Aspergillus, Cladosporium, Fusarium, Mucor, Penicillium, Rhizopus, Stachybotrys, Trichoderma, or Alternaria), animals (e.g Fel d1, Fel d 2, Fel d3, or Fel d4 of cats), food (e.g. allergens of fish (e.g. bass, cod, flounder), seafood (e.g. crab, lobster, shrimps), egg, wheat, nuts (e.g. peanuts, almonds, cashews, walnuts), soya, milk, etc.) or insect venom (e.g. allergens from the venom of wasps, bees, hornets, ants, mosquitos, or ticks).

c) Antigens associated with autoimmune disease:

Antigens associated with autoimmune disease are preferably selected from autoantigens asscociated with autoimmune diseases selected from Addison disease (autoimmune adrenalitis, Morbus Addison), alopecia areata, Addison's anemia (Morbus Biermer), autoimmune hemolytic anemia (AIHA), autoimmune hemolytic anemia (AIHA) of the cold type (cold hemagglutinine disease, cold autoimmune hemolytic anemia (AIHA) (cold agglutinin disease), (CHAD)), autoimmune hemolytic anemia (AIHA) of the warm type (warm AIHA, warm autoimmune haemolytic anemia (AIHA)), autoimmune hemolytic Donath-Landsteiner anemia (paroxysmal cold hemoglobinuria), antiphospholipid syndrome (APS), atherosclerosis, autoimmune arthritis, arteriitis temporalis, Takayasu arteriitis (Takayasu's disease, aortic arch disease), temporal arteriitis/giant cell arteriitis, autoimmune chronic gastritis, autoimmune infertility, autoimmune inner ear disease (AIED), Basedow's disease (Morbus Basedow), Bechterew's disease (Morbus Bechterew, ankylosing spondylitis, spondylitis ankylosans), Behcet's syndrome (Morbus Behcet), bowel disease including autoimmune inflammatory bowel disease (including colitis ulcerosa (Morbus Crohn, Crohn's disease), cardiomyopathy, particularly autoimmune cardiomyopathy, idiopathic dilated cardiomyopathy (DCM), celiac sprue dermatitis (gluten mediated enteropathia), chronic fatigue immune dysfunction syndrome (CFIDS), chronic inflammatory demyelinating polyneuropathy (CIDP), chronic polyarthritis, Churg-Strauss syndrome, cicatricial pemphigoid, Cogan syndrome, CREST syndrome (syndrom with Calcinosis cutis, Raynaud phenomenon, motility disorders of the esophagus, sklerodaktylia and teleangiectasia), Crohn's disease (Morbus Crohn, colitis ulcerosa), dermatitis herpetiformis during, dermatologic autoimmune diseases, dermatomyositis, Diabetes, Diabetes mellitus Type 1 (type I diabetes, insuline dependent Diabetes mellitus), Diabetes mellitus Type 2 (type II diabetes), essential mixed cryoglobulinemia, essential mixed cryoglobulinemia, fibromyalgia, fibromyositis, Goodpasture syndrome (anti-GBM mediated glomerulonephritis), graft versus host disease, Guillain-Barré syndrome (GBM, Polyradikuloneuritis), haematologic autoimmune diseases, Hashimoto thyroiditis, hemophilia, acquired hemophilia, hepatitis, autoimmune hepatitis, particularly autoimmune forms of chronic hepatitis, idiopathic pulmonary fibrosis (IPF), idiopathic thrombocytopenic purpura, Immuno-thrombocytopenic purpura (Morbus Werlhof; ITP), IgA nephropathy, infertility, autoimmune infertility, juvenile rheumatoid arthritis (Morbus Still, Still syndrome), Lambert-Eaton syndrome, lichen planus, lichen sclerosus, lupus erythematosus, systemic lupus erythematosus (SLE), lupus erythematosus (discoid form), Lyme arthritis (Lyme disease, borrelia arthritis), Ménierè's disease (Morbus Ménierè); mixed connective tissue disease (MCTD), multiple sclerosis (MS, encephalomyelitis disseminate, Charcot's disease), Myasthenia gravis (myasthenia, MG), myosits, polymyositis, neural autoimmune diseases, neurodermitis, pemphigus vulgaris, bullous pemphigoid, scar forming pemphigoid; polyarteriitis nodosa (periarteiitis nodosa), polychondritis (panchondritis), polyglandular (autoimmune) syndrome (PGA syndrome, Schmidt's syndrome), Polymyalgia rheumatica, primary agammaglobulinemia, primary biliary cirrhosis PBC, primary autoimmune cholangitis), progressive systemic sclerosis (PSS), Psoriasis, Psoriasis vulgaris, Raynaud's phenomena, Reiter's syndrome (Morbus Reiter, urethral conjunctive synovial syndrome)), rheumatoid arthritis (RA, chronic polyarthritis, rheumatic disease of the joints, rheumatic fever), sarcoidosis (Morbus Boeck, Besnier-Boeck-Schaumann disease), stiff-man syndrome, Sclerodermia, Scleroderma, Sjögren's syndrome, sympathetic ophtalmia; Transient gluten intolerance, transplanted organ rejection, uveitis, autoimmune uveiitis, Vasculitis, Vitiligo, (leucoderma, piebold skin), and Wegner's disease (Morbus Wegner, Wegner's granulomatosis).

Particularly preferred in this context are autoantigens selected from:

- myelin basic protein (MBP), proteolipid protein (PLP), and myelin oligodendrocyte glycoprotein (MOG), in each case associated with multiple sclerosis (MS);
- CD44, preproinsulin, proinsulin, insulin, glutamic acid decaroxylase (GAD65), tyrosine phosphatase-like insulinoma antigen 2 (IA2), zinc transporter ((ZnT8), and heat shock protein 60 (HSP60), in each case associated with diabetes Typ I;
- interphotoreceptor retinoid-binding protein (IRBP) associated with autoimmune uveitis;
- acetylcholine receptor AchR, and insulin-like growth factor-1 receptor (IGF-1 R), in each case associated with Myasthenia gravis;
- M-protein from beta-hemolytic streptocci (pseudo-autoantigen) associated with Rheumatic Fever;
- Macrophage migration inhibitory factor associated with Arthritis;

- Ro/La RNP complex, alpha- and beta-fodrin, islet cell autoantigen, poly(ADP)ribose polymerase (PARP), NuMA, NOR-90, Ro60 autoantigen, and p27 antigen, in each case associated with Sjögren's syndrome;
- Ro60 autoantigen, low-density lipoproteins, Sm antigens of the U-1 small nuclear ribonucleoprotein complex (B/B', D1, D2, D3, E, F, G), and RNP ribonucleoproteins, in each case associated with lupus erythematosus;
- oxLDL, beta(2)GPI, HSP60/65, and oxLDL/beta(2)GPI, in each case associated with Atherosclerosis;
- cardiac beta(1)-adrenergic receptor associated with idiopathic dilated cardiomyopathy (DCM);
- histidyl-tRNA synthetase (HisRS) associated with myositis;
- topoisomerase I associated with scleroderma disease.

Furthermore, in other embodiments, said antigen is associated with the respective autoimmune disease, like e.g. IL-17, heat shock proteins, and/or any idiotype pathogenic T cell or chemokine receptor which is expressed by immune cells involved in the autoimmune response in said autoimmune disease (such as any autoimmune diseases described herein).

d) Antigens associated with a cancer or tumour disease ("Tumour antigens"):

"Tumour antigens" in this context are antigens which are preferably located on the surface of the (tumour) cell. Tumour antigens may also be selected from proteins, which are overexpressed in tumour cells compared to a normal cell. Furthermore, tumour antigens also include antigens expressed in cells which are (were) not themselves (or originally not themselves) degenerated but are associated with the supposed tumour. Antigens which are connected with tumour-supplying vessels or (re)formation thereof, in particular those antigens which are associated with neovascularization, e.g. growth factors, such as VEGF, bFGF etc., are also included herein. Antigens connected with a tumour furthermore include antigens from cells or tissues, typically embedding the tumour. Further, some substances (usually proteins or peptides) are expressed in patients suffering (knowingly or not-knowingly) from a cancer disease and they occur in increased concentrations in the body fluids of said patients. These substances are also referred to as "tumour antigens", however they are not antigens in the stringent meaning of an immune response inducing substance. The class of tumour antigens can be divided further into tumour-specific antigens (TSAs) and tumour-associated-antigens (TAAs). TSAs can only be presented by tumour cells and never by normal "healthy" cells. They typically result from a tumour specific mutation. TAAs, which are more common, are usually presented by both tumour and healthy cells. These antigens are recognized and the antigen-presenting cell can be destroyed by cytotoxic T cells. Additionally, tumour antigens can also occur on the surface of the tumour in the form of, e.g., a mutated receptor. In this case, they can be recognized by antibodies. Particular preferred tumour antigens are selected from the group consisting of 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PAP, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGF, VEGFR1, VEGFR-2/FLK-1, and WT1. Such tumour antigens preferably may be selected from the group consisting of p53, CA125, EGFR, Her2/neu, hTERT, PAP, MAGE-A1, MAGE-A3, Mesothelin, MUC-1, NY-ESO-1, GP100, MART-1, Tyrosinase, PSA, PSCA, PSMA VEGF, VEGFR1, VEGFR2, Ras, CEA or WT1, and more preferably from PAP, NY-ESO-1, MAGE-A3, WT1, and MUC-1.

In this context, and for certain embodiments of all aspects of the present invention, the antigen associated with a cancer or tumour disease, does not include (x) an idiotype immunoglobulin (an idiotype antibody or an idiotype B cell receptor); or (y) an idiotype T cell receptor, and optionally is not a fragment, variant and/or derivative of such antigen.

Furthermore, the antigen, such as the protein or peptide antigen, is preferably not covalently attached to the carrier component. In particular, the antigen is preferably not covalently attached to the carrier component if the antigen is ovalbumin or a fragment of ovalbumin.The at least one antigen provided as protein or peptide antigen is not the model antigen Ovalbumine or the Ovalbumine derived peptide SIINFEKL (SEQ ID NO: 103).Preferably, it is not the Ovalbumine derived peptide ISQAVHAAHAEINE (SEQ ID NO: 104). Preferably, the amino acid component is not derived from mouse mastocytoma, in particular is preferably not the mouse mastocytoma P815-derived peptide P1A LPYLGWLVF (SEQ ID NO: 105). Preferably, the antigen is not derived from *Plasmodium yoelii,* in particular it is preferably not derived from the circumsporozoite protein of *Plasmodium yoelii.* For example, in some embodiments, the antigen is not the CSP-peptide SYVPSAEQI (SEQ ID NO: 106). Preferably, the antigen is not derived from *Listeria monocytgenes,* in particular, not from listeriolysin O 91-99. For example, in some embodiments, the antigen is not the LLO-peptide GYKDGNEYI (SEQ ID NO: 107). Preferably, the antigen is not derived from the melanocyte stimulating hormone receptor (MC1R). For example, in some embodiments, the antigen is not the MC1 R-peptide WGPFFLHL (SEQ ID NO: 108).

The at least one antigen is provided as a protein or peptide, or a functional fragment, variant and/or derivative of said protein or peptide antigen. It is also disclosed that the at least one antigen in the inventive pharmaceutical composition can be encoded by a nucleic acid, e.g. a DNA (e.g. a plasmid DNA or viral DNA), or an RNA (e.g. an mRNA or a viral RNA). In certain embodiments, said protein or peptide antigen (or functional fragment, variant and/or derivative of said protein or peptide antigen) is comprised in, provided as or derived from a defined sample, for example a sample having a known number and or composition of components. For example, said protein or peptide antigen is not comprised in; or is not provided as; or is not derived from, in each case a mixture of (e.g. undefined) other components, such as a mixture being a preparation of inactivated or attenuated virus or pathogen (such as, in either case, any one describe herein). For example, the antigen used in any aspect of the present invention may be, or may be provided as, an isolated and/or purified protein or peptide antigen. As will be understood by the person of ordinary skill, an isolated (and/or purified) antigen includes such antigens that are present (or provided) in a (starting) composition that has less than about 40%, 30%, 20%, 10%, 5%, 2% or 1% non-desired or specified other components such as other proteins/peptides or impurities.

Protein or peptide antigens can, for example, be prepared as follows.

Protein or peptide antigens as described above, can be prepared using recombinant production methods, such as those described herein, or e.g. with the aid of molecular biology methods known to the person of ordinary skill. Such an antigen can be described, as applicable, as a "recombinant protein antigen" and/or a "recombinant peptide antigen".

Alternatively, a protein or peptide as described above (e.g fragments, domains, epitopes or protein antigens and/or peptide analogues) can be prepared using peptide synthesis methods such as those described herein, or e.g. with other methodologies known to the person of ordinary skill. Such an antigen can be described, as applicable, as a "synthetic protein antigen" and/or a "synthetic peptide antigen".

According to the invention, the peptide or protein antigen or functional fragment, variant and/or derivative thereof is provided as a separate component of the inventive pharmaceutical composition. In this case the at least one protein or peptide antigen is not part of the complex or in other words: in this case the complex does not include the at least one antigen. Furthermore, in a further alternative embodiment a protein or peptide antigen is provided as component of the carrier of the complex and at least one additional protein or peptide antigen (the same or a different) is provided as a separate component of the inventive pharmaceutical composition which is not part of the complex.It is also disclosed that the at least one protein or peptide antigen can be provided as component of the complex. In this case the peptide or protein antigen can be added to the complex during the complexation step c) of the method of preparing of the complex as described herein. Thus, the peptide or protein antigen is integrated in the complex.

The pharmaceutical composition of the invention may additionally comprise the at least one antigen (or a functional fragment, variant and/or derivative thereof) in the form of nucleic acids coding for the at least one antigen (or functional fragments, variants and/or derivatives thereof).

In this context, the nucleic acids coding for the at least one antigen (or functional fragments, variants and/or derivatives thereof) are defined as disclosed above for the nucleic acid cargo comprised in the complex used as an adjuvant in the inventive pharmaceutical composition. Therefore, also functional fragments, variants, derivatives and modifications of a nucleic acid as defined herein are explicitly encompassed.

The at least one antigen (or a functional fragment, variant and/or derivative thereof) if additionally provided in the inventive pharmaceutical composition in the form of nucleic acids coding for the at least one antigen (or fragments, variants and/or derivatives thereof), can be prepared with all methods for nucleic acid synthesis known for a skilled person. Particularly preferred are methods for nucleic acid synthesis as defined herein.

Two alternatives exist. The first alternative provides the nucleic acid coding for the at least one antigen as part of the complex (e.g. as nucleic acid cargo molecule) and the second alternative provides the nucleic acid coding for the at least one antigen as separate component of the inventive pharmaceutical composition. Thus, in this case the nucleic acid coding for the at least one antigen is not part of the complex.

In a further embodiment of the present invention, the at least one antigen (or a functional fragment, variant and/or derivative thereof) coded by a nucleic acid can additionally be provided as part of the (adjuvant) complex (e.g. as nucleic acid cargo coding for the at least one antigen) and additionally an antigen coded by a nucleic acid can be provided as a separate component which is not part of the complex.

The invention further provides the alternative that at least one antigen is provided as a nucleic acid (as part of the complex or as an additional component) and that at least one additional antigen is provided as protein or peptide antigen (as part of the complex or not).

As a further embodiment, the at least one antigen if provided as protein or peptide or, optionally additionally, as a nucleic acid coding for the at least one antigen may further comprise or code for a signal peptide as defined herein.

As a further ingredient, the pharmaceutical composition may comprise at least one additional pharmaceutically active component. A pharmaceutically active component in this connection is a compound that has a therapeutic effect to heal, ameliorate or prevent a particular indication, preferably tumour or cancer diseases, autoimmune disease, allergies or infectious diseases. Such compounds include, without implying any limitation, peptides or proteins, preferably as defined herein, nucleic acids, preferably as defined herein, (therapeutically active) low molecular weight organic or inorganic compounds (molecular weight less than 5000, preferably less than 1000), sugars, antigens or antibodies, preferably as defined herein, therapeutic agents already known in the prior art, antigenic cells, antigenic cellular fragments, cellular fractions; cell wall components (e.g. polysaccharides), modified, attenuated or de-activated (e.g. chemically or by irradiation) pathogens (virus, bacteria etc.), adjuvants, preferably as defined herein, etc.

Furthermore, the inventive pharmaceutical composition may comprise a pharmaceutically acceptable carrier and/or vehicle. In the context of the present invention, a pharmaceutically acceptable carrier typically includes the liquid or non-liquid basis of the pharmaceutical composition. If the pharmaceutical composition is provided in liquid form, the carrier will typically be pyrogen-free water; isotonic saline or buffered (aqueous) solutions, e.g phosphate, citrate etc. buffered solutions. The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person.

However, one or more compatible solid or liquid fillers or diluents or encapsulating compounds may be used as well for the pharmaceutical composition, which are suitable for administration to a patient to be treated. The term "compatible" as used here means that these constituents of the pharmaceutical composition are capable of being mixed with the complex as defined herein in such a manner that no interaction occurs which would substantially reduce the pharmaceutical effectiveness of the pharmaceutical composition under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

According to a specific embodiment, the inventive pharmaceutical composition may comprise an (additional) adjuvant. In this context, an adjuvant may be understood as any compound, which is suitable to initiate or increase an immune response of the innate immune system, i.e. a non-specific immune response. With other words, when administered, the pharmaceutical composition typically elicits an innate immune response due to the adjuvant, optionally contained therein. Such an adjuvant may be selected from any adjuvant known to a skilled person and suitable for the present case, i.e. supporting the induction of an innate immune response in a mammal.

The inventive pharmaceutical composition may be administered orally, parenterally, by inhalation spray, topically, rectally, nasally, buccally, vaginally or via an implanted reservoir. The term parenteral as used herein includes subcutaneous, intravenous, intramuscular, intra-articular, intra-nodal, intra-synovial, intrasternal, intrathecal, intrahepatic, intralesional, intracranial, transdermal, intradermal, intrapulmonal, intraperitoneal, intracardial, intraarterial, and sublingual injection or infusion techniques.

Preferably, the inventive pharmaceutical composition may be administered by parenteral injection, more preferably by subcutaneous, intravenous, intramuscular, intra-articular, intra-nodal, intra-synovial, intrasternal, intrathecal, intrahepatic, intralesional, intracranial, transdermal, intradermal, intrapulmonal, intraperitoneal, intracardial, intraarterial, and sublingual injection or via infusion techniques. Particularly preferred is intradermal, subcutaneous and intramuscular injection. Sterile injectable forms of the pharmaceutical compositions may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation of the pharmaceutical composition.

The inventive pharmaceutical composition as defined herein may also be administered orally in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient, i.e. the complex, is combined with emulsifying and suspending agents. If desired, certain sweetening, flavoring or coloring agents may also be added.

The inventive pharmaceutical composition may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the pharmaceutical composition may be formulated in a suitable ointment, containing the complex suspended or dissolved in one or more carriers. Carriers for topical administration include, but are not limited to, mineral oil, liquid petrolatum, white petrolatum, propylene glycol, polyoxyethylene, polyoxypropylene compound, emulsifying wax and water. Alternatively, the pharmaceutical composition can be formulated in a suitable lotion or cream. In the context of the present invention, suitable carriers include, but are not limited to, mineral oil, sorbitan monostearate, polysorbate 60, cetyl esters wax, cetearyl alcohol, 2-octyldodecanol, benzyl alcohol and water.

The inventive pharmaceutical composition typically comprises a "safe and effective amount" of the components of the pharmaceutical composition, particularly of the complex as defined herein or the nucleic acid as such. As used herein, a "safe and effective amount" means an amount of the complex as such that is sufficient to significantly induce a positive modification of a disease or disorder as defined herein. At the same time, however, a "safe and effective amount" is small enough to avoid serious side-effects and to permit a sensible relationship between advantage and risk. The determination of these limits typically lies within the scope of sensible medical judgment. A "safe and effective amount" of the components of the pharmaceutical composition, particularly of the complex or of the at least one antigen as defined herein, will furthermore vary in connection with the particular condition to be treated and also with the age and physical condition of the patient to be treated, the body weight, general health, sex, diet, time of administration, rate of excretion, drug combination, the activity of the complex or of the antigen, the severity of the condition, the duration of the treatment, the nature of the accompanying therapy, of the particular pharmaceutically acceptable carrier used, and similar factors, within the knowledge and experience of the accompanying doctor. The pharmaceutical composition may be used for human and also for veterinary medical purposes, preferably for human medical purposes, as a pharmaceutical composition in general or as a vaccine.

The inventive pharmaceutical composition can additionally contain one or more auxiliary substances in order to increase its immunogenicity or immunostimulatory capacity, if desired. A synergistic action of the (adjuvant) complex as defined herein and of an auxiliary substance, which may be optionally contained in the inventive pharmaceutical composition as defined herein, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CSF, which allow an immune response to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH.

Further additives which may be included in the inventive pharmaceutical composition are emulsifiers, such as, for example, Tween®; wetting agents, such as, for example, sodium lauryl sulfate; colouring agents; taste-imparting agents, pharmaceutical carriers; tablet-forming agents; stabilizers; antioxidants; preservatives.

The inventive pharmaceutical composition can also additionally contain any further compound, which is known to be immunostimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

The inventive pharmaceutical composition can also additionally or alternatively contain an immunostimulatory RNA, i.e. an RNA derived from an immunostimulatory RNA, which triggers or increases an (innate) immune response. Preferably, such an immunostimulatory RNA may be in general be as defined hereinbefore.

Another class of compounds, which may be added, in some embodiments, to the inventive pharmaceutical composition in this context, may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytidine/guanosine dinucleotide sequence(s) (CpG motif(s)). According to a first preferred alternative, at least one CpG motif contained in these sequences, that is to say the C (cytosine) and the G (guanine) of the CpG motif, is unmethylated. All further cytidines or guanosines optionally contained in these sequences can be either methylated or unmethylated. According to a further preferred alternative, however, the C (cytidine) and the G (guanosine) of the CpG motif can also be present in methylated form.

In the context of the present invention, the nucleic acid cargo in the complex comprised in the inventive pharmaceutical composition is as defined above. More preferably, the nucleic acid of the complex, preferably contained in the pharmaceutical composition, is typically an immunostimulatory nucleic acid as defined herein, e.g. an immunostimulatory RNA (isRNA), preferably an isRNA. Alternatively, the nucleic acid of the complex, preferably contained in the pharmaceutical composition, is a coding nucleic acid as defined herein, preferably an mRNA, more preferably encoding an adjuvant protein preferably as defined herein. Additionally, the complex may comprise a CpG-DNA or a coding cDNA. In this context, the complex, typically initiates an innate immune response in the patient to be treated.

In a specific embodiment in this context, it is preferred that an adjuvant protein is a component of the complex and, preferably, of the carrier.

The present disclosure also relates to a method of preparing a pharmaceutical composition of the invention, said method comprising the steps of: (i) providing at least one complex as defined anywhere herein; (ii) providing an antigen as defined anywhere herein; and (iii) combining said complex and said antigen. The combining step of (iii) may occur briefly before administration to a patient (such as about 1, 5, 15, 30 or 60 minutes prior to, up to 72 hours before, said administration), or may occur during manufacture of said pharmaceutical composition. The respective person of ordinary skill (e.g. a doctor or health professional, or a manufacturer) will be aware of the routine methodologies suitable for such combining step.

A method of preparing the complex as defined herein may comprise the following steps:

a) providing at least one cationic protein or peptide as defined herein and/or at least one cationic or polycationic polymer and optionally at least one amino acid component (AA) as defined herein,
b) providing at least one nucleic acid molecule as defined herein, preferably in the above mentioned ratios,
c) mixing the components provided in steps a) and b), as defined herein,
d) optionally purifying the complex obtained according to step c), preferably using a method as defined herein,
e) optionally lyophilization of the complex obtained according to step c) or d).

As described herein in a step a) of the method of preparing the complex, at least one cationic or polycationic protein or peptide as defined herein and/or at least one cationic or polycationic polymer as defined herein are provided, e.g. in the ratios indicated above. These components are mixed in step c) with the nucleic acid molecule provided in step b), to obtain a carrier complexed to the nucleic acid molecule as defined herein.

In this context, different carriers, particularly different peptides and/or different polymers, may be selected in step a). In this context, the selection of different component(s) of the carrier is typically dependent upon the desired properties of the final carrier and the desired cationic strength of the final carrier. Accordingly, the content of cationic components, may furthermore be "diluted" or modified in step a) e.g. by introducing an amino acid component (AA) as defined herein, preferably in the above defined ratios. Thereby, a modified carrier may be obtained, wherein the cationic character of the unmodified carrier typically remains in the limitations as defined herein. The properties of the final carrier may thus be adjusted as desired with properties of components (AA) by inserting amino acid component (AA) as defined herein in step a).

In step c), the at least one cationic or polycationic protein or peptide as defined herein and/or at least one cationic or polycationic polymer as defined herein, and, optionally, at least one amino acid component (AA) and the at least one nucleic acid as defined herein, are typically contained in an acidic or neutral milieu. Such a acidic or neutral milieu typically exhibits a pH range of about 5 to about 8, e.g. a pH range of about 6 to about 8, or e.g. a pH range of about 6 to about 7, e.g. about 6.5, 7, or 7.5 or any range selected from any two of these or the aforementioned values.

Furthermore, the temperature of the solution in step c) is typically in a range of about 5°C to about 60°C, e.g. in a range of about 15°C to about 40°C, or e.g. in a range of about 20°C to about 30°C, or or e.g. in a range of about 20°C to about 25°C, e.g. about 25°C.

According to one alternative, the complex may additionally be modified with a component (AA) as defined herein.

According to a first example, a component (AA) (e.g. a ligand) is attached to the cationic component prior to providing the cationic component in step a) via any functionality as defined herein. This component (AA) or (e.g. a ligand) is preferably attached to the cationic component at one terminus of these components.

Alternatively, a component (AA) or (e.g. a ligand) can be bound to the complex after step c) via any functionality as defined herein.

According to step c) of the method of preparing the complex as described herein, at least one nucleic acid molecule as defined herein is mixed with the cationic components provided in step b), e.g. in the above mentioned ratios. The N/P ratios are e.g. as indicated above.

(AA) components as defined above can also be incorporated into the complex without covalent linkage. Thereby these (AA) components are typically not covalently linked and included non-covalently in the complex as a further component. Particularly preferred in this context is the incorporation of the at least one antigen or a fragment, variant and/or derivative thereof, provided as protein or peptid in the complex as (AA) component.

According to a further step d) of the method of preparing the complex as described herein, the complex obtained according to step c) is optionally purified. Purification may occur by using chromatographic methods, such as HPLC, FPLC, GPS, dialysis, etc.

According to a further step e) of the method of preparing the complex as described herein, the complex obtained according to step c) or d) is optionally lyophilized. For this purpose any suitable cryoprotectant or lyoprotectant may be added to the complex obtained in step c) or d).

The method of preparing the complex as defined herein is particularly suitable to adapt the chemical properties of the desired complex due to specific selection of its components of the carrier.

According to a further aspect, the present invention also provides kits, particularly kits of parts, comprising as components alone or in combination with optional further ingredients, and including (as a first component):

(a) a complex as defined according to any one of claims 1 to 3 and 9 to 12; and
(b) at least one protein or peptide antigen or a functional fragment, variant and/or derivative thereof as defined according to any one of claims 1 and 4 to 8.

The present invention provides kits, particularly kits of parts, comprising as components alone or in combination with optional further ingredients, and including (as a first component):

(A) a complex, comprising:

a) at least one cationic and/or polycationic peptide or protein or a polyimine as a carrier,
and
b) at least one nucleic acid molecule as a cargo, wherein the at least one nucleic acid molecule is RNA,
wherein the cationic and/or polycationic component of the carrier and the nucleic acid molecule comprised as cargo in said complex are provided in an N/P ratio in the range of 0.4-0.9,
and

(B) at least one protein or peptide antigen that is selected from the group consisting of:

(i) an antigen from a pathogen associated with infectious disease;
(ii) an antigen associated with allergy or allergic disease;
(iii) an antigen associated with autoimmune disease; and
(iv) an antigen associated with a cancer or tumour disease
or

a functional fragment, variant and/or derivative of said protein or peptide antigen,
wherein the at least one protein or peptide antigen is provided as a separate component of the pharmaceutical composition and wherein component (B) is not ovalbumine or the ovalbumine-derived peptide SIINFEKL,
in each case as defined anywhere herein, and optionally technical instructions with information on the administration and dosage of the complex and the at least one antigen.

Such kits, preferably kits of parts, may be applied, e.g., for any of the applications or uses as defined herein. Such kits, when occurring as a kit of parts, may further contain each component of inventive pharmaceutical composition in a different part of the kit.

In certain embodiments of the kits of the present invention, the antigen is comprised in a vaccine.

The present invention furthermore provides several applications and uses of the inventive pharmaceutical composition (e.g. the adjuvanted vaccine) or of kits or kits of parts comprising same as defined anywhere herein.

The present disclosure also provides a method for transfecting and/or treating a cell, a tissue or an organism, thereby applying or administering the inventive pharmaceutical composition particularly for therapeutic purposes. In this context, typically after preparing the pharmaceutical composition, the pharmaceutical composition is typically administered to a cell, a tissue or an organism, e.g. using any of the administration modes as described herein. The method for transfecting and/or treating a cell may be carried out *in vitro, in vivo* or *ex vivo.*

Furthermore, the present invention provides the use of a pharmaceutical composition or of kits or kits of parts in each case as defined anywhere herein, in therapy and/or as a medicament, preferably as a vaccine such as an adjuvanted vaccine.

In this aspect of the present invention, particularly preferred is the use of the inventive pharmaceutical composition or of the kits or kits of parts comprising same as defined herein in the treatment of infectious diseases, allergies or allergic diseases, autoimmune diseases and cancer or tumour diseases, in each case as defined anywhere herein.

In this context, infectious diseases are preferably viral, bacterial or protozoological infectious diseases. Such infectious diseases, preferably (viral, bacterial or protozoological) infectious diseases, are typically selected from the list consisting of Acinetobacter infections, African sleeping sickness (African trypanosomiasis), AIDS (Acquired immunodeficiency syndrome), Amoebiasis, Anaplasmosis, Anthrax, Appendicitis, Arcanobacterium haemolyticum infections, Argentine hemorrhagic fever, Ascariasis, Aspergillosis, Astrovirus infections, Athlete’s foot, Babesiosis, Bacillus cereus infections, Bacterial meningitis, Bacterial pneumonia, Bacterial vaginosis (BV), Bacteroides infections, Balantidiasis, Baylisascaris infections, Bilharziosis, BK virus infections, Black piedra, Blastocystis hominis infections, Blastomycosis, Bolivian hemorrhagic fever, Borrelia infectionss (Borreliosis), Botulism (and Infant botulism), Bovine tapeworm, Brazilian hemorrhagic fever, Brucellosis, Burkholderia infections, Buruli ulcer, Calicivirus infections (Norovirus and Sapovirus), Campylobacteriosis, Candidiasis (Candidosis), Canine tapeworm infections, Cat-scratch disease, Chagas Disease (American trypanosomiasis), Chancroid, Chickenpox, Chlamydia infections, Chlamydia trachomatis infections, Chlamydophila pneumoniae infections, Cholera, Chromoblastomycosis, Climatic bubo, Clonorchiasis, Clostridium difficile infections, Coccidioidomycosis, Cold, Colorado tick fever (CTF), Common cold (Acute viral rhinopharyngitis; Acute coryza), Condyloma acuminata, Conjunctivitis, Creutzfeldt-Jakob disease (CJD), Crimean-Congo hemorrhagic fever (CCHF), Cryptococcosis, Cryptosporidiosis, Cutaneous larva migrans (CLM), Cutaneous Leishmaniosis, Cyclosporiasis, Cysticercosis, Cytomegalovirus infections, Dengue fever, Dermatophytosis, Dientamoebiasis, Diphtheria, Diphyllobothriasis, Donavanosis, Dracunculiasis, Early summer meningoencephalitis (FSME), Ebola hemorrhagic fever, Echinococcosis, Ehrlichiosis, Enterobiasis (Pinworm infections), Enterococcus infections, Enterovirus infections, Epidemic typhus, Epiglottitis, Epstein-Barr Virus Infectious Mononucleosis, Erythema infectiosum (Fifth disease), Exanthem subitum, Fasciolopsiasis, Fasciolosis, Fatal familial insomnia (FFI), Fifth disease, Filariasis, Fish poisoning (Ciguatera), Fish tapeworm, Flu, Food poisoning by Clostridium perfringens, Fox tapeworm, Free-living amebic infections, Fusobacterium infections, Gas gangrene, Geotrichosis, Gerstmann-Sträussler-Scheinker syndrome (GSS), Giardiasis, Glanders, Gnathostomiasis, Gonorrhea, Granuloma inguinale (Donovanosis), Group A streptococcal infections, Group B streptococcal infections, Haemophilus influenzae infections, Hand foot and mouth disease (HFMD), Hantavirus Pulmonary Syndrome (HPS), Helicobacter pylori infections, Hemolytic-uremic syndrome (HUS), Hemorrhagic fever with renal syndrome (HFRS), Henipavirus infections, Hepatitis A, Hepatitis B, Hepatitis C, Hepatitis D, Hepatitis E, Herpes simplex, Herpes simplex type I, Herpes simplex type II, Herpes zoster, Histoplasmosis, Hollow warts, Hookworm infections, Human bocavirus infections, Human ewingii ehrlichiosis, Human granulocytic anaplasmosis (HGA), Human metapneumovirus infections, Human monocytic ehrlichiosis, Human papillomavirus (HPV) infections, Human parainfluenza virus infections, Hymenolepiasis, Influenza, Isosporiasis, Japanese encephalitis, Kawasaki disease, Keratitis, Kingella kingae infections, Kuru, Lambliasis (Giardiasis), Lassa fever, Legionellosis (Legionnaires’ disease, Pontiac fever), Leishmaniasis, Leprosy, Leptospirosis, Lice, Listeriosis, Lyme borreliosis, Lyme disease, Lymphatic filariasis (Elephantiasis), Lymphocytic choriomeningitis, Malaria, Marburg hemorrhagic fever (MHF), Marburg virus, Measles, Melioidosis (Whitmore’s disease), Meningitis, Meningococcal disease, Metagonimiasis, Microsporidiosis, Miniature tapeworm, Miscarriage (prostate inflammation), Molluscum contagiosum (MC), Mononucleosis, Mumps, Murine typhus (Endemic typhus), Mycetoma, Mycoplasma hominis, Mycoplasma pneumonia, Myiasis, Nappy/diaper dermatitis, Neonatal conjunctivitis (Ophthalmia neonatorum), Neonatal sepsis (Chorioamnionitis), Nocardiosis, Noma, Norwalk virus infections, Onchocerciasis (River blindness), Osteomyelitis, Otitis media, Paracoccidioidomycosis (South American blastomycosis), Paragonimiasis, Paratyphus, Pasteurellosis, Pediculosis capitis (Head lice), Pediculosis corporis (Body lice), Pediculosis pubis (Pubic lice, Crab lice), Pelvic inflammatory disease (PID), Pertussis (Whooping cough), Pfeiffer’s glandular fever, Plague, Pneumococcal infections, Pneumocystis pneumonia (PCP), Pneumonia, Polio (childhood lameness), Poliomyelitis, Porcine tapeworm, Prevotella infections, Primary amoebic meningoencephalitis (PAM), Progressive multifocal leukoencephalopathy, Pseudo-croup, Psittacosis, Q fever, Rabbit fever, Rabies, Rat-bite fever, Reiter's syndrome, Respiratory syncytial virus infections (RSV), Rhinosporidiosis, Rhinovirus infections, Rickettsial infections, Rickettsialpox, Rift Valley fever (RVF), Rocky mountain spotted fever (RMSF), Rotavirus infections, Rubella, Salmonella paratyphus, Salmonella typhus, Salmonellosis, SARS (Severe Acute Respiratory Syndrome), Scabies, Scarlet fever, Schistosomiasis (Bilharziosis), Scrub typhus, Sepsis, Shigellosis (Bacillary dysentery), Shingles, Smallpox (Variola), Soft chancre, Sporotrichosis, Staphylococcal food poisoning, Staphylococcal infections, Strongyloidiasis, Syphilis, Taeniasis, Tetanus, Three-day fever, Tick-borne encephalitis, Tinea barbae (Barber's itch), Tinea capitis (Ringworm of the Scalp), Tinea corporis (Ringworm of the Body), Tinea cruris (Jock itch), Tinea manuum (Ringworm of the Hand), Tinea nigra, Tinea pedis (Athlete's foot), Tinea unguium (Onychomycosis), Tinea versicolor (Pityriasis versicolor), Toxocariasis (Ocular Larva Migrans (OLM) and Visceral Larva Migrans (VLM)), Toxoplasmosis, Trichinellosis, Trichomoniasis, Trichuriasis (Whipworm infections), Tripper, Trypanosomiasis (sleeping sickness), Tsutsugamushi disease, Tuberculosis, Tularemia, Typhus, Typhus fever, Ureaplasma urealyticum infections, Vaginitis (Colpitis), Variant Creutzfeldt-Jakob disease (vCJD, nvCJD), Venezuelan equine encephalitis, Venezuelan hemorrhagic fever, Viral pneumonia, Visceral Leishmaniosis, Warts, West Nile Fever, Western equine encephalitis, White piedra (Tinea blanca), Whooping cough, Yeast fungus spots, Yellow fever, Yersinia pseudotuberculosis infections, Yersiniosis, and Zygomycosis.

Allergies or allergic diseases are preferably selected from pollen allergy (allergy against grass pollen, tree pollen (e.g. pollen of hazel, birch, alder, ash), flower pollen, herb pollen (e.g. pollen of mugwort)), dust mite allergy, mold allergy (e.g. allergy against Acremonium, Aspergillus, Cladosporium, Fusarium, Mucor, Penicillium, Rhizopus, Stachybotrys, Trichoderma, or Alternaria), pet allergy (allergy against animals; e.g against cats, dogs, horses), food allergy (e.g. allergy against fish (e.g. bass, cod, flounder), seafood (e.g. crab, lobster, shrimps), egg, wheat, nuts (e.g. peanuts, almonds, cashews, walnuts), soya, milk, etc.) or insect bite allergy (allergy against insect venom, e.g. venom of wasps, bees, hornets, ants, mosquitos, or ticks).

According to another specific embodiment, diseases as defined herein comprise autoimmune diseases as defined in the following. Autoimmune diseases are preferably selected from Addison disease (autoimmune adrenalitis, Morbus Addison), alopecia areata, Addison's anemia (Morbus Biermer), autoimmune hemolytic anemia (AIHA), autoimmune hemolytic anemia (AIHA) of the cold type (cold hemagglutinine disease, cold autoimmune hemolytic anemia (AIHA) (cold agglutinin disease), (CHAD)), autoimmune hemolytic anemia (AIHA) of the warm type (warm AIHA, warm autoimmune haemolytic anemia (AIHA)), autoimmune hemolytic Donath-Landsteiner anemia (paroxysmal cold hemoglobinuria), antiphospholipid syndrome (APS), atherosclerosis, autoimmune arthritis, arteriitis temporalis, Takayasu arteriitis (Takayasu's disease, aortic arch disease), temporal arteriitis/giant cell arteriitis, autoimmune chronic gastritis, autoimmune infertility, autoimmune inner ear disease (AIED), Basedow's disease (Morbus Basedow), Bechterew's disease (Morbus Bechterew, ankylosing spondylitis, spondylitis ankylosans), Behcet's syndrome (Morbus Behcet), bowel disease including autoimmune inflammatory bowel disease (including colitis ulcerosa (Morbus Crohn, Crohn's disease), cardiomyopathy, particularly autoimmune cardiomyopathy, idiopathic dilated cardiomyopathy (DCM), celiac sprue dermatitis (gluten mediated enteropathia), chronic fatigue immune dysfunction syndrome (CFIDS), chronic inflammatory demyelinating polyneuropathy (CIDP), chronic polyarthritis, Churg-Strauss syndrome, cicatricial pemphigoid, Cogan syndrome, CREST syndrome (syndrom with Calcinosis cutis, Raynaud phenomenon, motility disorders of the esophagus, sklerodaktylia and teleangiectasia), Crohn's disease (Morbus Crohn, colitis ulcerosa), dermatitis herpetiformis during, dermatologic autoimmune diseases, dermatomyositis, Diabetes, Diabetes mellitus Type 1 (type I diabetes, insuline dependent Diabetes mellitus), Diabetes mellitus Type 2 (type II diabetes), essential mixed cryoglobulinemia, essential mixed cryoglobulinemia, fibromyalgia, fibromyositis, Goodpasture syndrome (anti-GBM mediated glomerulonephritis), graft versus host disease, Guillain-Barré syndrome (GBM, Polyradikuloneuritis), haematologic autoimmune diseases, Hashimoto thyroiditis, hemophilia, acquired hemophilia, hepatitis, autoimmune hepatitis, particularly autoimmune forms of chronic hepatitis, idiopathic pulmonary fibrosis (IPF), idiopathic thrombocytopenic purpura, Immuno-thrombocytopenic purpura (Morbus Werlhof; ITP), IgA nephropathy, infertility, autoimmune infertility, juvenile rheumatoid arthritis (Morbus Still, Still syndrome), Lambert-Eaton syndrome, lichen planus, lichen sclerosus, lupus erythematosus, systemic lupus erythematosus (SLE), lupus erythematosus (discoid form), Lyme arthritis (Lyme disease, borrelia arthritis), Ménierè's disease (Morbus Ménierè); mixed connective tissue disease (MCTD), multiple sclerosis (MS, encephalomyelitis disseminate, Charcot's disease), Myasthenia gravis (myasthenia, MG), myosits, polymyositis, neural autoimmune diseases, neurodermitis, pemphigus vulgaris, bullous pemphigoid, scar forming pemphigoid; polyarteriitis nodosa (periarteiitis nodosa), polychondritis (panchondritis), polyglandular (autoimmune) syndrome (PGA syndrome, Schmidt's syndrome), Polymyalgia rheumatica, primary agammaglobulinemia, primary biliary cirrhosis PBC, primary autoimmune cholangitis), progressive systemic sclerosis (PSS), Psoriasis, Psoriasis vulgaris, Raynaud's phenomena, Reiter's syndrome (Morbus Reiter, urethral conjunctive synovial syndrome)), rheumatoid arthritis (RA, chronic polyarthritis, rheumatic disease of the joints, rheumatic fever), sarcoidosis (Morbus Boeck, Besnier-Boeck-Schaumann disease), stiff-man syndrome, Sclerodermia, Scleroderma, Sjögren's syndrome, sympathetic ophtalmia; Transient gluten intolerance, transplanted organ rejection, uveitis, autoimmune uveiitis, Vasculitis, Vitiligo, (leucoderma, piebold skin), and Wegner's disease (Morbus Wegner, Wegner's granulomatosis).

Furthermore, cancer or tumor diseases are preferably selected from melanomas, malignant melanomas, colon carcinomas, lymphomas, sarcomas, blastomas, renal carcinomas, gastrointestinal tumors, gliomas, prostate tumors, bladder cancer, rectal tumors, stomach cancer, oesophageal cancer, pancreatic cancer, liver cancer, mammary carcinomas (= breast cancer), uterine cancer, cervical cancer, acute myeloid leukaemia (AML), acute lymphoid leukaemia (ALL), chronic myeloid leukaemia (CML), chronic lymphocytic leukaemia (CLL), hepatomas, various virus-induced tumors such as, for example, papilloma virus-induced carcinomas (e.g. cervical carcinoma = cervical cancer), adenocarcinomas, herpes virus-induced tumors (e.g. Burkitt's lymphoma, EBV-induced B-cell lymphoma), heptatitis B-induced tumors (hepatocell carcinomas), HTLV-1- and HTLV-2-induced lymphomas, acoustic neuroma, lung carcinomas (= lung cancer = bronchial carcinoma), small-cell lung carcinomas, pharyngeal cancer, anal carcinoma, glioblastoma, rectal carcinoma, astrocytoma, brain tumors, retinoblastoma, basalioma, brain metastases, medulloblastomas, vaginal cancer, pancreatic cancer, testicular cancer, Hodgkin's syndrome, meningiomas, Schneeberger disease, hypophysis tumor, Mycosis fungoides, carcinoids, neurinoma, spinalioma, Burkitt's lymphoma, laryngeal cancer, renal cancer, thymoma, corpus carcinoma, bone cancer, non-Hodgkin's lymphomas, urethral cancer, CUP syndrome, head/neck tumors, oligodendroglioma, vulval cancer, intestinal cancer, colon carcinoma, oesophageal carcinoma (= oesophageal cancer), wart involvement, tumors of the small intestine, craniopharyngeomas, ovarian carcinoma, genital tumors, ovarian cancer (= ovarian carcinoma), pancreatic carcinoma (= pancreatic cancer), endometrial carcinoma, liver metastases, penile cancer, tongue cancer, gall bladder cancer, leukaemia, plasmocytoma, lid tumor, prostate cancer (= prostate tumors), etc..

The present invention provides a complex as defined herein and claimed by claim 15 for use in therapy in combination with at least one protein or peptide antigen or a functional fragment, variant and/or derivative thereof, in each case as defined anywhere herein, particularly for the treatment of infectious diseases, allergies or allergic diseases, autoimmune diseases and cancer or tumour diseases as defined above.

The present invention provides at least one protein or peptide antigen or a functional fragment, variant and/or derivative thereof, as defined herein and claimed by claim 16, for use in therapy in combination with a complex as defined anywhere herein,

particularly for the treatment of infectious diseases, allergies or allergic diseases, autoimmune diseases and cancer or tumour diseases as defined above.

In certain embodiments of such aspects of the present invention, the protein or peptide antigen is comprised in a vaccine, such as a commercially available vaccine.

In this context, "in combination" means that the different components (the complex and the at least one peptide or protein antigen or a functional fragment, variant and/or derivative thereof) can be provided together in the same composition, or can be formulated separately in different compositions, i.e. one composition comprising or representing the complex as defined herein, and one further composition comprising the at least one protein or peptide antigen, or a functional fragment, variant and/or derivative thereof as defined herein. If provided in different compositions the complex and the at least one protein or peptide antigen or a functional fragment, variant and/or derivative thereof may be administered separated in time (in a time-staggered manner) and/or may be administered at different administration sites and/or via different administration routes. This means that the complex may be administered e.g. prior, concurrent or subsequent to the at least one protein or peptide antigen, or a functional fragment, variant and/or derivative thereof, or vice versa. Subsequent administration includes that each component used in the therapy is administered within about 48 hours, 24 hours, 12 hours, 8 hours, 6 hours, 4 hours, 2 hours, 1 hour, 30 mins, 15 mins or 5 mins of each other.

The present disclosure provides a pharmaceutical package, including:

(A) a complex as defined herein; and

(B) instructions describing the use of said complex in therapy in combination with at least one antigen or fragment, variant and/or derivative thereof as defined anywhere herein.

The present invention provides a pharmaceutical package according to claim 18. The pharmaceutical package according to the present invention comprises at least one protein or peptide antigen or a functional fragment, variant and/or derivative thereof as defined anywhere herein.

Furthermore, the present disclosure provides a pharmaceutical package, including:

(A) at least one antigen or fragment, variant and/or derivative thereof, in each case as defined anywhere herein; and

(B) instructions describing the use of said antigen or fragment, variant and/or derivative thereof in therapy in combination with a complex as defined anywhere herein.

The pharmaceutical package may further comprise a complex as defined anywhere herein.

In this context, the invention furthermore provides the use of the components included in the above defined pharmaceutical packages in the treatment of the particular disease (indication) selected from an infectious disease, an allergy or allergic disease, an autoimmune disease or a cancer or tumour disease as defined above. The respective disease may be one as described anywhere herein.

In the present invention, if not otherwise indicated, different features of alternatives and embodiments may be combined with each other, where suitable. Furthermore, the term "comprising" shall not be construed as meaning "consisting of", if not specifically mentioned. However, in the context of the present invention, term "comprising" may be substituted with the term "consisting of", where suitable.

In some further embodiments, it may be preferred that the pharmaceutical composition may comprise no further component than the components A) and B), preferably no other mRNA component (other than comprised by the components A)), preferably the pharmaceutical compositon may not comprise any mRNA at all.

In some further embodiments, it may be preferred, provided the pharmaceutical composition comprises mRNA (other than nucleic acid of component (A)), the mRNA may not be a mRNA encoding a peptide or antigen according to B), further preferred the mRNA may not be a mRNA encoding Ovalbumin, PSMA, Luciferase or STEAP.

In some further embodiments, it may be preferred, provided the pharmaceutical composition contains a mRNA (other than nucleic acid of component (A)), particularly mRNA encoding a peptide or antigen according to (B), and/or mRNA encoding Ovalbumin, PSMA, Luciferase or STEAP, the mRNA may not be complexed with protamin, preferably not in a ratio of 2:1 or 4:1 or between 2:1 and 4:1.

In some further embodiments, it may be preferred that the claimed pharmaceutical composition may not be used for treatment of pancreas carcinoma or non-small cell lung carcinoma.

In some further embodiments, it may be preferred, provided the pharmaceutical composition comprises mRNA (other than nucleic acid of component (A)), that the mRNA may not be a free mRNA.

In some further embodiments, it may be preferred, provided the pharmaceutical composition comprises mRNA (other than nucleic acid of component (A)), that the mRNA may not be complexed with protamine.

In some further embodiments, it may be preferred, provided the pharmaceutical composition comprises free mRNA, that the mRNA may not encode for a therapeutically active protein and may not encode for an antibody and may not encode for an antigen.

In some further embodiments, it may be preferred that with respect to component (A) of the inventive pharmaceutical composition, that the carrier protein may not be protamine.

In some further embodiments, it may be preferred, provided that the carrier protein of component (A) is protamine, the carrier protein is not present in a ratio of 1:2 or 1:4 with respect to the nucleic acid of component (A).

In some further embodiments, it may be preferred, that with respect to component (A) the nucleic acid is not an mRNA.

In some further embodiments, it may be preferred, provided the nucleic acid of component (A) is an mRNA, that the mRNA may not encode Ovalbumin, PSMA, Luciferase or STEAP.

In some further embodiments, it may be preferred, provided the nucleic acid, i.e. b), of the component (A) is mRNA; that the mRNA is not a free mRNA, but is exclusively compexed with the carrier protein of a).

In some further embodiments, it may be preferred, that the carrier may not be a carrier formed by disulfide-crosslinked cationic and/or polycationic components.

In some further embodiments, it may be preferred, that the pharmaceutical composition may not comprise a cationic peptide formed by disulfide-crosslinked cationic and/or polycationic components.

In some further embodiments, component (B) is not a fragment of ovalbumin. Preferably, the pharmaceutical composition, the kit, or the pharmaceutical package according to the present invention does not comprise ovalbumin or a fragment of ovalbumin or a nucleic acid sequence coding for ovalbumin or for a fragment of ovalbumin.

## Figures:

The following Figures are intended to illustrate the invention further. They are not intended to limit the subject matter of the invention thereto.

**Figure 1:** shows the secretion of hIFNa cytokine *(in vitro)* in hPBMCs after stimulation with complexes formed by the long non-coding GU-rich isRNA R722 as nucleic acid cargo and Lipofectamine® as carrier in a mass ratio of 4:1, 2:1, 1:1, 1:2 and 1:4 (w/w) (R722/Lipofectamine). As can be seen, the negatively charged complexes (R722/Lipofectamine 4:1 and 2:1 (w/w)) lead to a higher increase of hIFNa cytokine release in hPBMCs compared to positively charged complexes (R722/Lipofectamine 1:1, 1:2 and 1:4 (w/w)), the nucleic acid cargo alone or the carrier alone. The respective zetapotentials of the different formulations were assessed and are shown in the Table below:

| **Ratio** | **4:1** | **2:1** | **1:1** | **1:2** | **1:4** |
|---|---|---|---|---|---|

(continued)

| | | | | | |
|---|---|---|---|---|---|
| **Zetapetential (mV)** | **-29.8** | **-17.2** | **+0.09** | **+32.5** | **+33.1** |

**Figure 2:** shows the secretion of hIFNa cytokine *(in vitro)* in hPBMCs after stimulation with complexes formed by the long non-coding GU-rich isRNA R722 as nucleic acid cargo and polyethylenimine (PEI) as carrier in a N/P ratio of 0.25, 0.5, 2.5, and 5. As can be seen, the negatively charged complexes (R722/PEI N/P 0.25 and N/P 0.5) lead to a much higher increase of hIFNa cytokine release in hPBMCs compared to positively charged complexes (R722/PEI N/P 2.5 and N/P 5), the nucleic acid cargo alone or the carrier alone.

**Figure 3:** shows the (*in vivo*) effect of the addition of complexes formed by the long non-coding GU-rich isRNA R722 as nucleic acid cargo and polyethylenimine (PEI) as carrier in a N/P ratio of 0.5 or 5, or of complexes formed by the long non-coding GU-rich isRNA R722 as nucleic acid cargo and Lipofectamine® as carrier in a mass ratio of 4:1 or 1:2 (w/w) or of complexes formed by the long non-coding GU-rich isRNA R722 as nucleic acid cargo and the cationic peptide CR12C as carrier in a mass ratio of 2:1 (w/w) to the seasonal influenza vaccine Influvac® (Season 2010/2011) for the use as an adjuvant on the induction of Influenza (Influvac)-specific IgG2a antibodies.

For this purpose 5 female Balb/c mice per group were vaccinated two times in two weeks with 0.1 µg Influvac® (Season 2010/2011) combined with 15 µg R722 complexed with the indicated amount of PEI, Lipofectamine®, or CR12C. For comparison mice were injected with Influvac® or buffer alone. 7 days after the last vaccination sera were prepared and the induction of Influvac®-specific IgG2a antibodies was measured.

As can be seen, the negatively charged complexes (R722/PEI N/P 0.5, R722/Lipofectamine 4:1 and R722/CR12C 2:1) strongly increase the B-cell response compared to the vaccine Influvac® alone and the combination of the vaccine Influvac® with positively charged complexes (R722/PEI N/P 5 and R722/Lipofectamine 1:2), which proves the beneficial adjuvant properties of the negatively charged complexes, particularly in regard to the induction of a Th1-shifted immune response.

**Figure 4:** shows the secretion of hIFNa cytokine *(in vitro)* in hPBMCs after stimulation with complexes formed by the long non-coding GU-rich isRNA R722 as nucleic acid cargo and the cationic peptide CR12C in a N/P ratio of 5.5, 5.0, 4.4, 3.9, 3.3, 2.7, 2.2, 1.6, 1.0, 0.55, 0.28, 0.18, 0.14, 0.11, 0.09, 0.08, 0.07, 0.06, and 0.05. As can be seen, the negatively charged complexes (R722/CR12C N/P 0.55, 0.28, 0.18, 0.14, 0.11, 0.09, 0.08, 0.07, 0.06, and 0.05) lead to a much higher increase of hIFNa cytokine release in hPBMCs compared to positively charged complexes (R722/CR12C N/P 5.5, 5.0, 4.4, 3.9, 3.3, 2.7, 2.2, 1.6, and 1.0), the nucleic acid cargo alone or the carrier alone.

**Figure 5:** shows the uptake of negatively charged complexes formed by the fluourescent labelled long non-coding GU-rich isRNA R722 as cargo and the cationic peptide CR12C in a mass ratio of 2:1 in different cell types.

For this purpose hPBMCs were transfected with the negatively charged complexes and 3h after transfection the cells were sorted by FACS analysis in CD3+ and CD19+ cells.

As can be seen the negatively charged complexes were dominantly uptaken into CD19+ cells.

**Figure 6:** shows the uptake of positively charged complexes formed by the fluourescent labelled long non-coding GU-rich isRNA R722 as cargo and the cationic peptide CR12C in a mass ratio of 1:2 in different cell types.

For this purpose hPBMCs were transfected with the positively charged complexes and 3h after transfection the cells were sorted by FACS analysis in CD3+ and CD19+ cells.

As can be seen the positively charged complexes were dominantly uptaken into CD3+ cells.

**Figure 7:** shows the secretion of hIFNa cytokine *(in vitro)* in hPBMCs after stimulation with complexes formed by the CpG DNA oligo 2261 as nucleic acid cargo and the cationic peptides CR12C or R12 at a w/w ratio nucleic acid/peptide of 2. As can be seen, these negatively charged complexes (CpG 2261/CR12C and CpG 2261/R12) lead to a much higher amount of hIFNa cytokine release in hPBMCs compared to the nucleic acid cargo CpG 2261 alone.

## Examples:

The following examples are intended to illustrate the invention further. They are not intended to limit the subject matter of the invention thereto.

### 1. Reagents:

Carrier:

$R_{12}$: Arg-Arg-Arg-Arg-Arg-Arg-Arg Arg-Arg-Arg-Arg-Arg ($Arg_{12}$) (SEQ ID NO 97)

(continued)

CR$_{12}$C: Cys-Arg-Arg-Arg-Arg-Arg-Arg-Arg-Arg-Arg-Arg-Arg-Arg-Cys (Cys-Arg$_{12}$-Cys) (SEQ ID NO. 98)

bPEI 25kDa (Sigma Aldrich)
Lipofectamine2000® (Life Technologies)

Nucleic acids as cargo of the complex:

| | |
|---|---|
| R722A: | long non-coding isGU-rich RNA (SEQ ID NO. 91) |
| R722B: | long non-coding isGU-rich RNA (SEQ ID NO. 101) |
| CpG 2216: | CpG oligonucleotide GGGGGACGATCGTCGGGGGG (SEQ ID NO. 99) |

Experiments indicating the use of nucleic acid molecule R722 have been performed with the sequences R722A and/or R722B.

Antigens:

Influvac® (Season 2010/2011) (Abbott Arzneimittel GmbH)

**2. Preparation of nucleic acid sequences :**

For the present examples nucleic acid sequences as indicated in example 1 were prepared and used for formation of the polymerized complexes or for non-polymerized carrier cargo complexes for comparison. These complexes were used for in vitro and in vivo transaction, for in vitro immunostimulation and for particle characterizations.

According to a first preparation, the DNA sequences, coding for the corresponding RNA sequence R722 were prepared. The sequence of the corresponding RNA is shown in the sequence listing (SEQ ID NO: 91).

The CpG 2216 oligonucleotides were prepared by automatic solid-phase synthesis by means of phosphoramidite chemistry. The sequence is shown in the sequence listing (SEQ ID NO: 99).

**3. *In vitro* transcription:**

The respective DNA plasmid prepared according to Example 2 for R722 was transcribed *in vitro* using T7-Polymerase (T7-Opti mRNA Kit, CureVac, Tübingen, Germany) following the manufactures instructions. Subsequently the mRNA was purified using PureMessenger® (CureVac, Tübingen, Germany).

**4. Synthesis of complexes:**

The nucleic acid sequences defined above in Example 1 were mixed with the carrier as defined in Example 1. Therefore, the indicated amount of nucleic acid sequence was mixed with the respective carrier in mass ratios or N/P ratios as indicated, thereby forming a complex. Afterwards the resulting solution was adjusted with injection solution (e.g. RiLa) to a final volume of 50 µl and incubated for 30 min at room temperature.

N/P ratio = is a measure of the ionic charge of the cationic component of the carrier or of the carrier as such. In the case that the cationic properties of the cationic components are provided by nitrogen atoms the N/P ratio is the ratio of basic nitrogen atoms to phosphate residues, considering that nitrogen atoms confer to positive charges and phosphate of the phosphate backbone of the nucleic acid confers to the negative charge.

N/P is preferably calculated by the following formula:

$$N/P = \frac{\text{pmol [RNA]* ratio*cationic AS}}{\mu\text{g RNA*3*1000}}$$

As an example the RNA R722 according to SEQ ID NO: 91 was applied, which has a molecular weight of 186 kDa. Therefore, 1 μg R722 RNA confers to 5.38 pmol RNA.

### 5. Cytokine stimulation in hPBMCs:

HPBMC cells from peripheral blood of healthy donors were isolated using a Ficoll gradient and washed subsequently with 1xPBS (phophate-buffered saline). The cells were then seeded on 96-well microtiter plates ($200x10^3$/well). The hPBMC cells were incubated for 24 h with 10 μl of the complex from Example 3 containing the indicated amount of nucleic acid in X-VIVO 15 Medium (BioWhittaker). The immunostimulatory effect was measured by detecting the cytokine production of the hPBMCs (Interferon alpha). Therefore, ELISA microtiter plates (Nunc Maxisorb) were incubated over night (o/n) with binding buffer (0.02% NaN3, 15 mM $Na_2CO_3$, 15 mM $NaHCO_3$, pH 9.7), additionally containing a specific cytokine antibody. Cells were then blocked with 1×PBS, containing 1% BSA (bovine serum albumin). The cell supernatant was added and incubated for 4 h at 37°C. Subsequently, the microtiter plate was washed with 1×PBS, containing 0.05% Tween-20 and then incubated with a Biotin-labelled secondary antibody (BD Pharmingen, Heidelberg, Germany). Streptavidin-coupled horseraddish peroxidase was added to the plate. Then, the plate was again washed with 1×PBS, containing 0.05% Tween-20 and ABTS (2,2'-azino-bis(3-ethyl-benzthiazoline-6-sulfonic acid) was added as a substrate. The amount of cytokine was determined by measuring the absorption at 405 nm (OD 405) using a standard curve with recombinant cytokines (BD Pharmingen, Heidelberg, Germany) with the Sunrise ELISA-Reader from Tecan (Crailsheim, Germany). The respective results are shown in Fig. 1, 2, 4, and 7.

### 6. Zetapotential measurements:

The Zeta potential of the complexes was evaluated by the laser Doppler electrophoresis method using a Zetasizer Nano (Malvern Instruments, Malvern, UK). The measurement was performed at 25°C and a scattering angle of 173° was used. The results are shown in Table 1:

Table 1

| Complex | N/P or mass ratio | Zeta potential |
|---|---|---|
| R722/CR12C | 2:1 (w/w) (N/P ratio 0.9) | -2.8 mV |
| R722/CR12C | N/P 5.5 | + 24.7 mV |
| R722/CR12C | N/P 4.4 | + 20.1 mV |
| R722/CR12C | N/P 2.2 | + 1.89 mV |
| R722/CR12C | N/P 1.0 | -1.9 mV |
| R722/CR12C | N/P 0.55 | -6.31 mV |
| R722/CR12C | N/P 0.28 | -7.7 mV |
| R722/CR12C | N/P 0.18 | -19.3 mV |
| R722/Lipofectamine | 4:1 (w/w) | -29.8 mV |
| R722/Lipofectamine | 1:1 (w/w) | + 0.1 mV |
| R722/Lipofectamine | 1:4 (w/w) | +27.5 mV |
| R722/PEI | N/P 0.25 | - 6.6 mV |
| R722/PEI | N/P 2.5 | +22.4 mV |
| R722/PEI | N/P 5 | +25 mV |

### 7. Immunization experiments:

a) Immunization with seasonal influenza vaccine:

For immunization the seasonal influenza vaccine Influvac® (comprises inactivated influenza virus strains as recommended by the WHO; season 2010/2011) (0.1 μg/dose) was combined with 15 μg R722 complexed with the indicated amount of PEI, Lipofectamine®, or CR12C. 5 female Balb/c mice per group were vaccinated two times in two weeks. For comparison mice were injected with Influvac® or buffer alone. 7 days after the last vaccination sera were prepared and the induction of Influvac®-specific IgG2a antibodies was measured. The results of this induction of antibodies upon vaccination with an inventive pharmaceutical composition are shown in Fig 3.

b) Immunization with Ovalbumine or SIINFEKL (reference example):

For immunization the vaccines Ovalbumine protein (OVA) (5 μg) or Ovalbumin-specific peptide SIINFEKL (50 μg) are combined with the complexes R722/$R_{12}$ (30 μg R722 / 15 μg $R_{12}$) (in a mass ratio of 2:1 w/w), R722/Lipofectamine (30 μg R722 / 15 μg Lipofectamine) (in a mass ratio of 2:1 w/w), R722/PEI (in a N/P ratio of 0.5), as adjuvant and injected intradermally into female C57BL/6 mice (7 mice per group for tumour challenge and 5 mice per group for detection of an immune response). The vaccination was repeated 2 times in 2 weeks. For comparison mice were injected alone with the antigens.

**8. Detection of an antigen-specific immune response (B-cell immune response):**

a) Detection of antibodies directed against seasonal influenza virus strains:

Detection of an antigen specific immune response (B-cell immune response) was carried out by detecting Influenza virus specific IgG2a antibodies. Therefore, blood samples were taken from vaccinated mice 7 days after last vaccination and sera were prepared. MaxiSorb plates (Nalgene Nunc International) were coated with Influvac® season 2010/2011 (at 5 μg/ml) containing the same viral Influenza antigens as the Influenza vaccine used for vaccination. After blocking with 1×PBS containing 0.05% Tween-20 and 1% BSA the plates were incubated with diluted mouse serum. Subsequently a biotin-coupled secondary antibody (Anti-mouse-IgG2a Pharmingen) was added. After washing, the plate was incubated with Horseradish peroxidase-streptavidin and subsequently the conversion of the ABTS substrate (2,2'-azino-bis(3-ethyl-benzthiazoline-6-sulfonic acid) was measured to determine the induction of IgG2a antibodies. The results of this induction of antibodies upon vaccination with an inventive pharmaceutical composition are shown in Fig 3.

b) Detection of antibodies directed against Ovalbumine (reference example):

Detection of an antigen specific immune response (B-cell immune response) is carried out by detecting antigen specific antibodies. Therefore, blood samples are taken from vaccinated mice 5 days after the last vaccination and sera are prepared. MaxiSorb plates (Nalgene Nunc International) are coated with *Gallus gallus* ovalbumine protein. After blocking with 1×PBS containing 0.05% Tween-20 and 1% BSA the plates are incubated with diluted mouse serum. Subsequently a biotin-coupled secondary antibody (Anti-mouse-IgG2a Pharmingen) is added. After washing, the plate is incubated with Horseradish peroxidase-streptavidin and subsequently the conversion of the ABTS substrate (2,2'-azino-bis(3-ethyl-benzthiazoline-6-sulfonic acid) is measured.

**9. Detection of an antigen specific cellular immune response by ELISPOT:**

a) Detection of cytotoxic T cell response directed against Ovalbumine (reference example):

5 days after the last vaccination mice are sacrificed, the spleens were removed and the splenocytes are isolated. For detection of IFNgamma a coat multiscreen plate (Millipore) is incubated overnight with coating buffer (0.1 M Carbonat-Bicarbonat Buffer pH 9.6, 10.59 g/l $Na_2CO_3$, 8.4g/l $NaHCO_3$) comprising antibody against IFNγ (BD Pharmingen, Heidelberg, Germany). The next day 1x $10^6$ cells/well are added and re-stimulated with 1 μg/well of relevant peptide (SIINFEKL of ovalbumine); irrelevant peptide (Connexin = control peptide) or buffer without peptide. Afterwards the cells are incubated for 24h at 37°C. The next day the plates are washed 3 times with PBS, once with water and once with PBS/0.05% Tween-20 and afterwards incubated with a biotin-coupled secondary antibody for 11-24h at 4°C. Then the plates are washed with PBS/0.05% Tween-20 and incubated for 2h at room temperature with alkaline phosphatase coupled to streptavidin in blocking buffer. After washing with PBS/0.05% Tween-20 the substrate (5-Bromo-4-Cloro-3-Indolyl Phosphate/Nitro Blue Tetrazolium Liquid Substrate System from Sigma Aldrich, Taufkirchen, Germany) is added to the plate and the conversion of the substrate can be detected visually. The reaction is then stopped by washing the plates with water. The dried plates are then read out by an ELISPOT plate reader. For visualization of the spot levels the numbers are corrected by background subtraction.

**10. Tumour challenge:**

One week after the last vaccination 1x$10^6$ E.G7-OVA cells (tumour cells which stably express ovalbumine) are implanted subcutaneously in the vaccinated mice. Tumour growth is monitored by measuring the tumour size in 3 dimensions using a calliper.

**11. Study of the uptake of complexes:**

The uptake of negatively or positively charged complexes formed by the fluorescent labelled long non-coding GU-rich isRNA R722 as cargo and the cationic peptide CR12C in a mass ratio of 2:1 or 1:2 (w/w) were measured by FACS analysis in different cell types. Therefore [200000] hPBMCs were transfected with the complexes containing 5 μg RNA and 3h after transfection the cells were stained by fluorescent Antibodies recognizing CD19, CD3 and CD8 and sorted by FACS analysis in CD3+ and CD19+ cells. The results of this uptake study are shown in Fig. 5.

## Claims

**1.** A pharmaceutical composition comprising:

(A) a complex, comprising:

a) at least one cationic and/or polycationic peptide or protein or a polyimine as a carrier, and
b) at least one nucleic acid molecule as a cargo, wherein the at least one nucleic acid molecule is RNA,

wherein the cationic and/or polycationic component of the carrier and the nucleic acid molecule comprised as cargo in said complex are provided in an N/P ratio in the range of 0.4-0.9,
and
(B) at least one protein or peptide antigen that is selected from the group consisting of:

(i) an antigen from a pathogen associated with infectious disease;
(ii) an antigen associated with allergy or allergic disease;
(iii) an antigen associated with autoimmune disease; and
(iv) an antigen associated with a cancer or tumour disease

or
a functional fragment, variant and/or derivative of said protein or peptide antigen,
wherein the at least one protein or peptide antigen is provided as a separate component of the pharmaceutical composition and wherein component (B) is not ovalbumine or the ovalbumine-derived peptide SIINFEKL.

**2.** The pharmaceutical composition of claim 1, wherein the at least one nucleic acid molecule is single-stranded RNA, preferably an mRNA.

**3.** The pharmaceutical composition of any one of claims 1 or 2, wherein the N/P ratio is in the range of 0.5-0.9.

**4.** The pharmaceutical composition of any one of claims 1 to 3, wherein said protein or peptide antigen is from a pathogen selected from the list consisting of: Influenza virus, Rabies virus, Hepatitis B virus, human Papilloma virus (hPV), Bacillus anthracis, Respiratory syncytial virus (RSV), Herpes simplex virus (HSV), and Mycobacterium tuberculosis.

**5.** The pharmaceutical composition of any one of claims 1-4, wherein said protein or peptide antigen is selected from the list consisting of:

- The Hemagglutinin (HA), the Neuraminidase (NA), the Nucleoprotein (NP), the M1 protein, the M2 protein, the NS1 protein, the NS2 protein (the NEP protein: nuclear export protein), the PA protein, the PB1 protein (polymerase basic 1 protein), the PB1-F2 protein and the PB2 protein of Influenza virus;
- the nucleoprotein (N), the phosphoprotein (P), the matrix protein (M), the glycoprotein (G), and the viral RNA polymerase (L), in each case of Rabies virus;
- the Hepatitis B surface antigen (HBsAg), the Hepatitis B core antigen (HbcAg), the Hepatitis B virus DNA polymerase, the HBx protein, the preS2 middle surface protein, the large S protein, the virus protein VP1, the virus protein VP2, the virus protein VP3, and the virus protein VP4, in each case of Hepatitis B virus;
- the E1 protein, the E2 protein, the E3 protein, the E4 protein, the E5 protein, the E6 protein, the E7 protein, the E8 protein, the L1 protein, and the L2 protein, in each case of human Papilloma virus (hPV);
- the protective antigen (PA), the edema factor (EF), the lethal factor (LF), and the S-layer homology proteins (SLH), in each case of Bacillus anthracis;
- the Fusion (F) protein, the nucleocapsid (N) protein, the phosphoprotein (P), the matrix (M) protein, the glycoprotein (G), the large protein (L; RNA polymerase), the non-structural protein 1 (NS1), the non-structural protein 2 (NS2), the small hydrophobic (SH) protein, the elongation factor M2-1, and the transcription regulation protein M2-2, in each case of respiratory syncytial virus (RSV);
- the Glycoprotein L (UL1), the Uracil-DNA glycosylase UL2, the UL3 protein, the UL4 protein, the DNA replication protein UL5, the Portal protein UL6, the Virion maturation protein UL7, the DNA helicase UL8, the Replication origin-binding protein UL9, the Glycoprotein M (UL10), the UL11 protein, the Alkaline exonuclease UL12, the Serine-threonine protein kinase UL13, the Tegument protein UL14, the Terminase (UL15), the Tegument protein UL16, the UL17 protein , the Capsid protein VP23 (UL18), the Major capsid protein VP5 (UL19), the Membrane protein UL20, the Tegument protein UL21, the Glycoprotein H (UL22), the Thymidine Kinase UL23, the UL24 protein, the UL25 protein, the Capsid protein P40 (UL26, VP24, VP22A), the Glycoprotein B (UL27), the ICP18.5 protein (UL28), the Major DNA-binding protein ICP8 (UL29), the DNA polymerase UL30, the Nuclear matrix protein UL31, the Envelope glycoprotein UL32, the UL33 protein, the Inner nuclear membrane protein UL34, the Capsid protein VP26 (UL35), the Large tegument protein UL36, the Capsid assembly protein UL37, the VP19C protein (UL38), the Ribonucleotide reductase (Large subunit) UL39, the Ribonucleotide reductase (Small subunit) UL40, the Tegument protein/Virion host shutoff VHS protein (UL41), the DNA polymerase processivity factor UL42, the Membrane protein UL43, the Glycoprotein C (UL44), the Membrane protein UL45, the Tegument proteins VP11/12 (UL46), the Tegument protein VP13/14 (UL47), the Virion maturation protein VP16 (UL48, Alpha-TIF), the Envelope protein UL49, the dUTP diphosphatase UL50, the Tegument protein UL51, the DNA helicase/primase complex protein UL52, the Glycoprotein K (UL53), the Transcriptional regulation protein IE63 (ICP27, UL54), the UL55 protein, the UL56 protein, the Viral replication protein ICP22 (IE68, US1), the US2 protein, the Serine/threonine-protein kinase US3, the Glycoprotein G (US4), the Glycoprotein J (US5), the Glycoprotein D (US6), the Glycoprotein I (US7), the Glycoprotein E (US8), the Tegument protein US9, the Capsid/Tegument protein US10, the Vmw21 protein (US11), the ICP47 protein (IE12, US12), the Major transcriptional activator ICP4 (IE175, RS1), the E3 ubiquitin ligase ICP0 (IE110), the Latency-related protein 1 (LRP1), the Latency-related protein 2 (LRP2), the Neurovirulence factor RL1 (ICP34.5), and the Latency-associated transcript (LAT), in each case of Herpes simplex virus (HSV); and
- the ESAT-6 protein, the ESX-1 protein, the CFP10 protein, the TB10.4 protein, the MPT63 protein, the MPT64 protein, the MPT83 protein, the MTB12 protein, the MTB8 protein, the AG85A protein, the AG85B protein, the Rpf-like proteins, the KATG protein, the PPE18 protein, the MTB32 protein, the MTB39 protein, the Crystallin, the HSP65 protein, the PST-S protein, and the HBHA protein, the 10 kDa filtrate antigen EsxB, the serine protease PepA, the fibronectin-binding protein D FbpD, the secreted protein MPT51, the periplasmic phosphate-binding lipoprotein PSTS1 (PBP-1), the periplasmic phosphate-binding lipoprotein PSTS3 (PBP-3, Phos-1), the PPE family protein PPE14, the PPE family protein PPE68, the protein MTB72F, the molecular chaperone DnaK, the cell surface lipoprotein MPT83, the lipoprotein P23, the Phosphate transport system permease protein PstA, the 14 kDa antigen, the fibronectin-binding protein C FbpC1, the Alanine dehydrogenase TB43, and the Glutamine synthetase 1, in each case of Mycobacterium tuberculosis.

**6.** The pharmaceutical composition of any one of claims 1-3, wherein said protein or peptide antigen is associated with allergy or allergic disease and is derived from a source selected from the list consisting of: grass pollen, tree pollen, flower pollen, herb pollen, dust mite, mold, animals, food, and insect venom.

**7.** The pharmaceutical composition of any one of claims 1-3, wherein said protein or peptide antigen is associated with autoimmune disease and is selected from the list consisting of:

- myelin basic protein (MBP), proteolipid protein (PLP), and myelin oligodendrocyte glycoprotein (MOG), in each case associated with multiple sclerosis (MS);
- CD44, preproinsulin, proinsulin, insulin, glutamic acid decaroxylase (GAD65), tyrosine phosphatase-like insulinoma antigen 2 (IA2), zinc transporter (ZnT8), and heat shock protein 60 (HSP60), in each case associated with diabetes Typ I;
- interphotoreceptor retinoid-binding protein (IRBP) associated with autoimmune uveitis;
- acetylcholine receptor AchR, and insulin-like growth factor-1 receptor (IGF-1 R), in each case associated with Myasthenia gravis;
- M-protein from beta-hemolytic streptocci (pseudo-autoantigen) associated with Rheumatic Fever;
- Macrophage migration inhibitory factor associated with Arthritis;
- Ro/La RNP complex, alpha- and beta-fodrin, islet cell autoantigen, poly(ADP)ribose polymerase (PARP), NuMA, NOR-90, Ro60 autoantigen, and p27 antigen, in each case associated with Sjögren's syndrome;
- Ro60 autoantigen, low-density lipoproteins, Sm antigens of the U-1 small nuclear ribonucleoprotein complex (B/B', D1, D2, D3, E, F, G), and RNP ribonucleoproteins, in each case associated with lupus erythematosus;
- oxLDL, beta(2)GPI, HSP60/65, and oxLDL/beta(2)GPI, in each case associated with Atherosclerosis;
- cardiac beta(1)-adrenergic receptor associated with idiopathic dilated cardiomyopathy (DCM);
- histidyl-tRNA synthetase (HisRS) associated with myositis;
- topoisomerase I associated with scleroderma;
- IL-17; and
- heat shock proteins.

**8.** The pharmaceutical composition of any one of claims 1-3, wherein said protein or peptide antigen is associated with a cancer or tumour disease and is selected from the list consisting of: p53, CA125, EGFR, Her2/neu, hTERT, PAP, MAGE-A1, MAGE-A3, Mesothelin, MUC-1, NY-ESO-1, GP100, MART-1, Tyrosinase, PSA, PSCA, PSMA VEGF, VEGFR1, VEGFR2, Ras, CEA and WT1.

**9.** The pharmaceutical composition of any one of claims 1 to 8, wherein said complex is for use as an adjuvant.

**10.** The pharmaceutical composition of any one of claims 1 to 9, wherein said nucleic acid molecule cargo is an immunostimulatory RNA (isRNA).

**11.** The pharmaceutical composition of any one of claims 1 to 10, wherein the cationic or polycationic peptides or proteins comprise polyarginine and/or polylysine peptides and at least one cysteine residue.

**12.** The pharmaceutical composition of any one of claims 1 to 10, wherein said cationic peptides are selected from peptides according to formula (I)

$$(Arg)_l;(Lys)_m;(His)_n;(Orn)_o;(Xaa)_x,$$

wherein

l + m + n +o + x = 3-100, and
l, m, n or o = independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100, provided that the overall content of Arg, Lys, His and Orn represents at least 10% of all amino acids of the cationic peptide; and Xaa is any amino acid selected from native (= naturally occurring) or non-native amino acids except of Arg, Lys, His or Orn; and
x = any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the cationic peptide,

wherein $(Arg)_l$; $(Lys)_m$; $(His)_n$; $(Orn)_o$; and x are as defined above; Xaa is any amino acid selected from native (= naturally occurring) or non-native amino acids except of Arg, Lys, His, Orn; provided that the overall content of Arg (Arginine), Lys (Lysine), His (Histidine) and Orn (Ornithine) represents at least 10% of all amino acids of the oligopeptide.

**13.** A kit or kit of parts comprising:

(a) a complex as defined according to any one of claims 1 to 3 and 9 to 12; and
(b) at least one protein or peptide antigen or a functional fragment, variant and/or derivative thereof as defined according to any one of claims 1 and 4 to 8.

**14.** The pharmaceutical composition of any one of claims 1 to 12, or the kit or kit of parts of claim 13, for use as a vaccine.

**15.** A complex as defined according to any one of claims 1 to 3 and 9 to 12, for use in therapy in combination with at least one protein or peptide antigen or a functional fragment, variant and/or derivative thereof as defined according to any one of claims 1 and 4 to 8.

**16.** A protein or peptide antigen or a functional fragment, variant and/or derivative thereof as defined according to any one of claims 1 and 4 to 8 for use in therapy in combination with a complex as defined according to any one of claims 1 to 3 and 9 to 12.

**17.** The complex of claim 15 for use in the therapy of; or the protein or peptide antigen or a functional fragment, variant and/or derivative thereof of claim 16 for use in the therapy of:

(i) infectious disease;
(ii) allergy or allergic disease;
(iii) autoimmune disease; or
(iv) cancer or tumour disease.

**18.** A pharmaceutical package, including:

(a) a complex as defined according to any one of claims 1 to 3 and 9 to 12; and
(b) instructions describing the use of said complex in therapy in combination with at least one protein or peptide antigen or a functional fragment, variant and/or derivative thereof as defined according to any one of claims 1 and 4 to 8,

wherein the pharmaceutical package further includes at least one protein or peptide antigen or a functional fragment, variant and/or derivative thereof as defined according to any one of claims 1 and 4 to 8.

**19.** The pharmaceutical package of claim 18, wherein said instructions further describe the use in the therapy of:

(i) infectious disease;
(ii) allergy or allergic disease;
(iii) autoimmune disease; or
(iv) cancer or tumour disease.

**Patentansprüche**

**1.** Pharmazeutische Zusammensetzung umfassend:

(A) einen Komplex umfassend:

a) mindestens ein kationisches und/oder polykationisches Peptid oder Protein oder ein Polyimin als Träger und
b) mindestens ein Nukleinsäuremolekül als Fracht, wobei das mindestens eine Nukleinsäuremolekül RNA ist,

wobei die kationische und/oder polykationische Komponente des Trägers und das Nukleinsäuremolekül, das als Fracht in dem Komplex enthalten ist, in einem N/P-Verhältnis im Bereich von 0,4-0,9 bereitgestellt werden, und
(B) mindestens ein Protein- oder Peptidantigen, das ausgewählt ist aus der Gruppe bestehend aus:

(i) einem Antigen eines mit einer Infektionskrankheit assoziierten Erregers;
(ii) einem Antigen, das mit einer Allergie oder allergischen Erkrankung assoziiert ist
(iii) einem mit einer Autoimmunerkrankung assoziierten Antigen; und
(iv) einem mit einer Krebs- oder Tumorerkrankung assoziierten Antigen

oder
einem funktionellen Fragment, einer Variante und/oder einem Derivat des Protein- oder Peptidantigens,
wobei das mindestens eine Protein- oder Peptidantigen als separate Komponente der pharmazeutischen Zusammensetzung bereitgestellt wird und wobei Komponente (B) nicht Ovalbumin oder das von Ovalbumin abgeleitete Peptid SIINFEKL ist.

2. Pharmazeutische Zusammensetzung nach Anspruch 1, wobei das mindestens eine Nukleinsäuremolekül eine einzelsträngige RNA, vorzugsweise eine mRNA, ist.

3. Die pharmazeutische Zusammensetzung nach einem der Ansprüche 1 oder 2, wobei das N/P-Verhältnis im Bereich von 0,5-0,9 liegt.

4. Pharmazeutische Zusammensetzung nach einem der Ansprüche 1 bis 3, wobei das Protein- oder Peptidantigen von einem Pathogen stammt, das ausgewählt ist aus der Liste bestehend aus: Influenzavirus, Tollwutvirus, Hepatitis-B-Virus, humanes Papillomavirus (hPV), Bacillus anthracis, Respiratorisches Syncytial-Virus (RSV), Herpes-simplex-Virus (HSV) und Mycobacterium tuberculosis.

5. Pharmazeutische Zusammensetzung nach einem der Ansprüche 1-4, wobei das Protein- oder Peptidantigen ausgewählt ist aus der Liste bestehend aus:

- dem Hämagglutinin (HA), der Neuraminidase (NA), dem Nukleoprotein (NP), dem M1-Protein, dem M2-Protein, dem NS1-Protein, dem NS2-Protein (dem NEP-Protein: Kernexportprotein("nuclear export protein")), dem PA-Protein, dem PB1-Protein (Polymerase-Basic-1-Protein), dem PB1-F2-Protein und dem PB2-Protein von Influenzavirus;
- dem Nukleoprotein (N), dem Phosphoprotein (P), dem Matrixprotein (M), dem Glykoprotein (G) und der viralen RNA-Polymerase (L), jeweils von Tollwutvirus;
- dem Hepatitis-B-Oberflächenantigen (HBsAg), dem Hepatitis-B-Kernantigen (HbcAg), der Hepatitis-B-Virus-DNA-Polymerase, dem HBx-Protein, dem mittleren Oberflächenprotein preS2, dem großen S-Protein, dem Virusprotein VP1, dem Virusprotein VP2, dem Virusprotein VP3 und dem Virusprotein VP4, jeweils von Hepatitis-B-Virus;
- dem E1-Protein, dem E2-Protein, dem E3-Protein, dem E4-Protein, dem E5-Protein, dem E6-Protein, dem E7-Protein, dem E8-Protein, dem L1-Protein und dem L2-Protein, jeweils von humanem Papillomavirus (hPV);
- dem protektiven Antigen (PA), dem Ödemfaktor (EF), dem Letalfaktor (LF) und den S-Layer-Homology-Proteinen (SLH), jeweils von Bacillus anthracis;
- dem Fusionsprotein (F), dem Nukleokapsidprotein (N), dem Phosphoprotein (P), dem Matrixprotein (M), dem Glykoprotein (G), dem großen Protein (L; RNA-Polymerase), dem Nicht-Strukturprotein 1 (NS1), dem Nicht-Strukturprotein 2 (NS2), dem kleinen hydrophoben Protein (SH), dem Elongationsfaktor M2-1 und dem Transkriptionsregulationsprotein M2-2, jeweils von Respiratorischem Syncytial-Virus (RSV);
- dem Glykoprotein L (UL1), der Uracil-DNA-Glykosylase UL2, dem UL3-Protein, dem UL4-Protein, dem DNA-Replikationsprotein UL5, dem Portalprotein UL6, dem Virionreifungsprotein UL7, der DNA-Helikase UL8, dem Replikationsursprung-bindenden Protein UL9, dem Glykoprotein M (UL10), dem UL11-Protein, der alkalischen Exonuklease UL12, der Serin-Threonin-Proteinkinase UL13, dem Tegument-Protein UL14, der Terminase (UL15), dem Tegument-Protein UL16, dem UL17-Protein , dem Capsid-Protein VP23 (UL18), dem Major-Capsid-Protein VP5 (UL19), dem Membran-Protein UL20, dem Tegumentprotein UL21, dem Glykoprotein H (UL22), der Thymidinkinase UL23, dem UL24-Protein, dem UL25-Protein, dem Capsidprotein P40 (UL26, VP24, VP22A), dem Glykoprotein B (UL27), dem ICP18. 5-Protein (UL28), dem Major-DNA-Bindungsprotein ICP8 (UL29), der DNA-Polymerase UL30, dem Kernmatrixprotein UL31, dem Hüllglykoprotein UL32, dem UL33-Protein, dem inneren Kernmembranprotein UL34, dem Capsid-Protein VP26 (UL35), dem großen Tegument-Protein UL36, dem Capsid-Assembly-Protein UL37, dem VP19C-Protein (UL38), der Ribonukleotidreduktase (große Untereinheit) UL39, der Ribonukleotid-Reduktase (Kleine Untereinheit) UL40, dem Tegumentprotein/Virion-Host-Shutoff-VHS-Protein (UL41), dem DNA-Polymerase-Prozessivitätsfaktor UL42, dem Membranprotein UL43, dem Glykoprotein C (UL44), dem Membranprotein UL45, den Tegumentproteinen VP11/12 (UL46), dem Tegumentprotein VP13/14 (UL47), dem Virionreifungsprotein VP16 (UL48, Alpha-TIF), dem Hüllprotein UL49, der dUTP-Diphosphatase UL50, dem Tegument-Protein UL51, dem DNA-Helikase/Primase-Komplex-Protein UL52, dem Glykoprotein K (UL53), dem Transkriptionsregulationsprotein IE63 (ICP27, UL54), dem UL55-Protein, dem UL56-Protein, dem viralen Replikationsprotein ICP22 (IE68, US1), dem US2-Protein, der Serin/Threonin-Proteinkinase US3, dem Glykoprotein G (US4), dem Glykoprotein J (US5), dem Glykoprotein D (US6), dem Glykoprotein I (US7), dem Glykoprotein E (US8), dem Tegumentprotein US9, dem Capsid/Tegumentprotein US10, dem Vmw21-Protein (US11), dem ICP47-Protein (IE12, US12), dem Major-Transkriptionsaktivator ICP4 (IE175, RS1), der E3-Ubiquitin-Ligase ICPO (IE110), dem Latency-related protein 1 (LRP1), dem Latency-related protein 2 (LRP2), dem Neurovirulenzfaktor RL1 (ICP34. 5) und dem Latenz-assoziierten Transkript (LAT), jeweils von Herpes-simplex-Virus (HSV); und
- dem ESAT-6-Protein, dem ESX-1 -Protein, dem CFP10-Protein, dem TB10. 4-Protein, dem MPT63-Protein, dem MPT64-Protein, dem MPT83-Protein, dem MTB12-Protein, dem MTB8-Protein, dem AG85A-Protein, dem AG85B-Protein, den Rpf-like-Proteinen, dem KATG-Protein, dem PPE18-Protein, dem MTB32-Protein, dem MTB39-Protein, dem Crystallin, dem HSP65-Protein, dem PST-S-Protein und dem HBHA-Protein, dem 10 kDa-Filtrat-Antigen EsxB, der Serinprotease PepA, dem Fibronektin-bindenden Protein D FbpD, dem sekretierten Protein MPT51, dem periplasmatischen Phosphat-bindenden Lipoprotein PSTS1 (PBP-1), dem periplasmatischen phosphatbindenden Lipoprotein PSTS3 (PBP-3, Phos-1), dem PPE-Familienprotein PPE14, dem PPE-Familienprotein PPE68, dem Protein MTB72F, dem molekularen Chaperon DnaK, dem Zelloberflächenlipoprotein MPT83, dem Lipoprotein P23, dem Phosphat-Transportsystem-Permease-Protein PstA, dem 14 kDa-Antigen, dem Fibronektin-bindenden Protein C FbpC1, der Alanin-Dehydrogenase TB43 und der Glutamin-Synthetase 1, jeweils von Mycobacterium tuberculosis.

**6.** Pharmazeutische Zusammensetzung nach einem der Ansprüche 1 bis 3, wobei das Protein- oder Peptidantigen mit einer Allergie oder allergischen Erkrankung assoziiert ist und von einer Quelle stammt, die ausgewählt ist aus: Gräserpollen, Baumpollen, Blütenpollen, Kräuterpollen, Staubmilben, Schimmelpilzen, Tieren, Nahrungsmitteln und Insektengift.

**7.** Pharmazeutische Zusammensetzung nach einem der Ansprüche 1 bis 3, wobei das Protein- oder Peptidantigen mit einer Autoimmunerkrankung assoziiert ist und ausgewählt ist aus der Liste bestehend aus:

- Myelin-Basic-Protein (MBP), Proteolipidprotein (PLP) und Myelin-Oligodendrozyten-Glykoprotein (MOG), jeweils assoziiert mit Multipler Sklerose (MS);
- CD44, Präproinsulin, Proinsulin, Insulin, Glutaminsäure-Decarboxylase (GAD65), Tyrosinphosphatase-ähnliches Insulinom-Antigen 2 (IA2), Zink-Transporter (ZnT8) und Hitzeschock-Protein 60 (HSP60), jeweils assoziiert mit Diabetes Typ I;
- Interphotorezeptor-Retinoid-bindendes Protein (IRBP), assoziiert mit Autoimmun-Uveitis;
- Acetylcholin-Rezeptor AchR und Insulin-like-Growth-Factor-1-Rezeptor (IGF-1R), jeweils assoziiert mit Myasthenia gravis;
- M-Protein aus beta-hämolytischen Streptokokken (Pseudo-Autoantigen), assoziiert mit Rheumatischem Fieber;
- Macrophage-Migration-Inhibitory-Factor assoziiert mit Arthritis;
- Ro/La-RNP-Komplex, Alpha- und Beta-Fodrin, Inselzell-Autoantigen, Poly(ADP)-Ribose-Polymerase (PARP), NuMA, NOR-90, Ro60-Autoantigen und p27-Antigen, jeweils assoziiert mit dem Sjögren-Syndrom;
- Ro60-Autoantigen, Low-Density-Lipoproteine, Sm-Antigene des U-1-Small-Nuclear-Ribonucleoprotein-Komplexes (B/B', D1, D2, D3, E, F, G) und RNP-Ribonukleoproteine, jeweils assoziiert mit Lupus erythematodes;
- oxLDL, beta(2)GPI, HSP60/65 und oxLDL/beta(2)GPI, jeweils assoziiert mit Atherosklerose;
- kardialer beta(1)-adrenerger Rezeptor assoziiert mit idiopathischer dilatativer Kardiomyopathie (DCM);
- Histidyl-tRNA-Synthetase (HisRS) assoziiert mit Myositis;
- Topoisomerase I assoziiert mit Sklerodermie;
- IL-17; und
- Hitzeschockproteine.

**8.** Pharmazeutische Zusammensetzung nach einem der Ansprüche 1 bis 3, wobei das Protein- oder Peptidantigen mit einer Krebs- oder Tumorerkrankung assoziiert ist und ausgewählt ist aus der Liste bestehend aus: p53, CA125, EGFR, Her2/neu, hTERT, PAP, MAGE-A1, MAGE-A3, Mesothelin, MUC-1, NY-ESO-1, GP100, MART-1, Tyrosinase, PSA, PSCA, PSMA, VEGF, VEGFR1, VEGFR2, Ras, CEA und WT1.

**9.** Pharmazeutische Zusammensetzung nach einem der Ansprüche 1 bis 8, wobei der Komplex zur Verwendung als Adjuvans bestimmt ist.

**10.** Pharmazeutische Zusammensetzung nach einem der Ansprüche 1 bis 9, wobei die Nukleinsäuremolekül-Fracht eine immunstimulatorische RNA (isRNA) ist.

**11.** Pharmazeutische Zusammensetzung nach einem der Ansprüche 1 bis 10, wobei die kationischen oder polykationischen Peptide oder Proteine Polyarginin- und/oder Polylysin-Peptide und mindestens einen Cystein-Rest umfassen.

**12.** Pharmazeutische Zusammensetzung nach einem der Ansprüche 1 bis 10, wobei die kationischen Peptide ausgewählt sind aus Peptiden gemäß der Formel (I)

$$(Arg)_l;(Lys)_m;(His)_n;(Orn)_o;(Xaa)_x,$$

wobei

l + m + n + o + x = 3-100, und
l, m, n oder o = unabhängig voneinander eine beliebige Zahl, ausgewählt aus 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 und 91-100, mit der Maßgabe, daß der Gesamtgehalt an Arg, Lys, His und Orn mindestens 10 % aller Aminosäuren des kationischen Peptids darstellt; und Xaa eine beliebige Aminosäure ist, ausgewählt aus nativen (= natürlich vorkommenden) oder nicht-nativen Aminosäuren mit Ausnahme von Arg, Lys, His oder Orn; und
x = eine beliebige Zahl, ausgewählt aus 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, mit der Maßgabe, daß der Gesamtgehalt an Xaa 90 % aller Aminosäuren des kationischen Peptids nicht überschreitet,

worin $(Arg)_l$; $(Lys)_m$; $(His)_n$; $(Orn)_o$; und x wie oben definiert sind; Xaa eine beliebige Aminosäure ist, ausgewählt aus nativen (= natürlich vorkommenden) oder nicht-nativen Aminosäuren außer Arg, Lys, His, Orn; mit der Maßgabe, dass der Gesamtgehalt an Arg (Arginin), Lys (Lysin), His (Histidin) und Orn (Ornithin) mindestens 10 % aller Aminosäuren des Oligopeptids ausmacht.

**13.** Kit oder Kit von Teilen, umfassend:

(a) einen Komplex wie nach einem der Ansprüche 1 bis 3 und 9 bis 12 definiert; und
(b) mindestens ein Protein- oder Peptidantigen oder ein funktionelles Fragment, eine funktionelle Variante und/oder ein funktionelles Derivat davon, wie nach einem der Ansprüche 1 und 4 bis 8 definiert.

**14.** Pharmazeutische Zusammensetzung nach einem der Ansprüche 1 bis 12 oder Kit oder Kit von Teilen nach Anspruch 13, zur Verwendung als Impfstoff.

**15.** Komplex, wie nach einem der Ansprüche 1 bis 3 und 9 bis 12 definiert, zur Verwendung in der Therapie in Kombination mit mindestens einem Protein- oder Peptidantigen oder einem funktionellen Fragment, einer funktionellen Variante und/oder einem funktionellen Derivat davon, wie nach einem der Ansprüche 1 und 4 bis 8 definiert.

**16.** Protein- oder Peptidantigen oder ein funktionelles Fragment, eine funktionelle Variante und/oder ein funktionelles Derivat davon, wie nach einem der Ansprüche 1 und 4 bis 8 definiert, zur Verwendung in der Therapie in Kombination mit einem Komplex, wie nach einem der Ansprüche 1 bis 3 und 9 bis 12 definiert.

**17.** Der Komplex nach Anspruch 15 zur Verwendung in der Therapie von; oder das Protein- oder Peptidantigen oder ein funktionelles Fragment, eine funktionelle Variante und/oder ein funktionelles Derivat davon nach Anspruch 16 zur Verwendung in der Therapie von:

(i) Infektionskrankheit;
(ii) Allergie oder allergischer Erkrankung;

(iii) Autoimmunerkrankung; oder
(iv) Krebs- oder Tumorerkrankung.

**18.** Pharmazeutische Packung, umfassend:

(a) einen Komplex, wie er nach einem der Ansprüche 1 bis 3 und 9 bis 12 definiert ist; und
(b) Anweisungen, die die Verwendung des Komplexes in der Therapie in Kombination mit mindestens einem Protein- oder Peptidantigen oder einem funktionellen Fragment, einer funktionellen Variante und/oder einem funktionellem Derivat davon, wie nach einem der Ansprüche 1 und 4 bis 8 definiert, beschreiben,

wobei die pharmazeutische Packung weiterhin mindestens ein Protein- oder Peptidantigen oder ein funktionelles Fragment, eine funktionelle Variante und/oder ein funktionelles Derivat davon, wie nach einem der Ansprüche 1 und 4 bis 8 definiert, enthält.

**19.** Pharmazeutische Packung nach Anspruch 18, wobei die Anweisungen weiterhin beschreiben die Verwendung in der Therapie von:

(i) Infektionskrankheit;
(ii) Allergie oder allergischer Erkrankung;
(iii) Autoimmunerkrankung; oder
(iv) Krebs- oder Tumorerkrankung.

**Revendications**

**1.** Composition pharmaceutique comprenant :

(A) un complexe, comprenant :

a) au moins un peptide ou une protéine ou une polyimine cationique et/ou polycationique comme support, et
b) au moins une molécule d'acide nucléique comme cargo, la au moins une molécule d'acide nucléique étant l'ARN,

le constituant cationique et/ou polycationique du support et la molécule d'acide nucléique comprise comme cargo dans ledit complexe étant fournis sous un rapport N/P situé dans la plage de 0,4 à 0,9, et
(B) au moins un antigène protéique ou peptidique qui est sélectionné dans le groupe constitué de :

(i) un antigène provenant d'un pathogène associé à une maladie infectieuse ;
(ii) un antigène associé à une allergie ou une maladie allergique ;
(iii) un antigène associé à une maladie auto-immune ; et
(iv) un antigène associé à un cancer ou une maladie tumorale

ou
un fragment, un variant et/ou un dérivé fonctionnel dudit antigène protéique ou peptidique,
le au moins un antigène protéique ou peptidique étant fourni sous la forme d'un constituant séparé de la composition pharmaceutique et le constituant (B) n'étant pas l'ovalbumine ni le peptide SIINFEKL dérivé de l'ovalbumine.

**2.** Composition pharmaceutique selon la revendication 1,
la au moins une molécule d'acide nucléique étant l'ARN simple brin, préférablement un ARNm.

**3.** Composition pharmaceutique selon l'une quelconque des revendications 1 ou 2, le rapport N/P se situant dans la plage de 0,5 à 0,9.

**4.** Composition pharmaceutique selon l'une quelconque des revendications 1 à 3, ledit antigène protéique ou peptidique provenant d'un pathogène sélectionné dans la liste constituée : du virus de la grippe, du virus de la rage, du virus de l'hépatite B, du virus du papillome humain (hPV), du *Bacillus anthracis,* du virus syncytial respiratoire (RSV), du virus de l'herpès simplex (HSV), et du *Mycobacterium tuberculosis.*

**5.** Composition pharmaceutique selon l'une quelconque des revendications 1 à 4, ledit antigène protéique ou peptidique étant sélectionné dans la liste constituée de :

- l'hémagglutinine (HA), la neuraminidase (NA), la nucléoprotéine (NP), la protéine M1, la protéine M2, la protéine NS1, la protéine NS2 (la protéine NEP : protéine d'exportation nucléaire), la protéine PA, la protéine PB1 (la protéine polymérase basique 1), la protéine PB1-F2 et la protéine PB2 du virus de la grippe ;
- la nucléoprotéine (N), la phosphoprotéine (P), la protéine de la matrice (M), la glycoprotéine (G), et l'ARN polymérase virale (L), dans chaque cas du virus de la rage ;
- l'antigène de surface de l'hépatite B (HBsAg), l'antigène du noyau de l'hépatite B (HbcAg), l'ADN polymérase du virus de l'hépatite B, la protéine HBx, la protéine de la surface moyenne preS2, la grande protéine S, la protéine virale VP1, la protéine virale VP2, la protéine virale VP3, et la protéine virale VP4, dans chaque cas du virus de l'hépatite B ;
- la protéine E1, la protéine E2, la protéine E3, la protéine E4, la protéine E5, la protéine E6, la protéine E7, la protéine E8, la protéine L1, et la protéine L2, dans chaque cas du virus du papillome humain (hPV) ;
- l'antigène protecteur (PA), le facteur de l'œdème (EF), le facteur létal (LF), et les protéines d'homologie de couche S (SLH), dans chaque cas du *Bacillus anthracis ;*
- la protéine de fusion (F), la protéine de nucléocapside (N), la phosphoprotéine (P), la protéine de la matrice (M), la glycoprotéine (G), la grande protéine (L ; ARN polymérase), la protéine non structurelle 1 (NS1), la protéine non structurelle 2 (NS2), la petite protéine hydrophobe (SH), le facteur d'élongation M2-1, et la protéine de régulation de la transcription M2-2, dans chaque cas du virus syncytial respiratoire (RSV) ;
- la glycoprotéine L (UL1), l'uracil-ADN glycosylase UL2, la protéine UL3, la protéine UL4, la protéine de réplication de l'ADN UL5, la protéine portail UL6, la protéine de maturation du virion UL7, l'ADN hélicase UL8, la protéine de liaison de l'origine de réplication UL9, la glycoprotéine M (UL10), la protéine UL11, l'exonucléase alcaline UL12, la sérine-thréonine protéine kinase UL13, la protéine tégumentaire UL14, la terminase (UL15), la protéine tégumentaire UL16, la protéine UL17, la protéine de la capside VP23 (UL18), la protéine majeure de la capside VP5 (UL19), la protéine membranaire UL20, la protéine tégumentaire UL21, la glycoprotéine H (UL22), la thymidine kinase UL23, la protéine UL24, la protéine UL25, la protéine de la capside P40 (UL26, VP24, VP22A), la glycoprotéine B (UL27), la protéine ICP18.5 (UL28), la protéine majeure de liaison à l'ADN ICP8 (UL29), l'ADN polymérase UL30, la protéine de la matrice nucléaire UL31, la glycoprotéine de l'enveloppe UL32, la protéine UL33, la protéine membranaire nucléaire interne UL34, la protéine de la capside VP26 (UL35), la grande protéine tégumentaire UL36, la protéine d'assemblage de la capside UL37, la protéine VP19C (UL38), la ribonucléotide réductase (grande sous-unité) UL39, la ribonucléotide réductase (petite sous-unité) UL40, la protéine tégumentaire/protéine d'arrêt de l'hôte du virion VHS (UL41), le facteur de processivité de l'ADN polymérase UL42, la protéine membranaire UL43, la glycoprotéine C (UL44), la protéine membranaire UL45, les protéines tégumentaires VP11/12 (UL46), la protéine tégumentaire VP13/14 (UL47), la protéine de maturation du virion VP16 (UL48, Alpha-TIF), la protéine d'enveloppe UL49, la dUTP diphosphatase UL50, la protéine tégumentaire UL51, la protéine du complexe ADN hélicase/primase UL52, la glycoprotéine K (UL53), la protéine de régulation de la transcription IE63 (ICP27, UL54), la protéine UL55, la protéine UL56, la protéine de réplication virale ICP22 (IE68, US1), la protéine US2, la sérine/thréonine-protéine kinase US3, la glycoprotéine G (US4), la glycoprotéine J (US5), la glycoprotéine D (US6), la glycoprotéine I (US7), la glycoprotéine E (US8), la protéine tégumentaire US9, la protéine de la capside/tégumentaire US10, la protéine Vmw21 (US11), la protéine ICP47 (IE12, US12), l'activateur majeur de la transcription ICP4 (IE175, RS1), l'E3 ubiquitine ligase ICPO (IE110), la protéine liée à la latence 1 (LRP1), la protéine liée à la latence 2 (LRP2), le facteur de neurovirulence RL1 (ICP34.5), et le produit de transcription associé à la latence (LAT), dans chaque cas du virus de l'herpès simplex (HSV) ; et
- la protéine ESAT-6, la protéine ESX-1, la protéine CFP10, la protéine TB10.4, la protéine MPT63, la protéine MPT64, la protéine MPT83, la protéine MTB12, la protéine MTB8, la protéine AG85A, la protéine AG85B, les protéines type Rpf, la protéine KATG, la protéine PPE18, la protéine MTB32, la protéine MTB39, la crystalline, la protéine HSP65, la protéine PST-S, et la protéine HBHA, l'antigène de filtrat de 10 kDa EsxB, la sérine protéase PepA, la protéine de liaison à la fibronectine D FbpD, la protéine sécrétée MPT51, la lipoprotéine de liaison au phosphate périplasmique PSTS1 (PBP-1), la lipoprotéine de liaison au phosphate périplasmique PSTS3 (PBP-3, Phos-1), la protéine de la famille PPE PPE14, la protéine de la famille PPE PPE68, la protéine MTB72F, la DnaK chaperonne moléculaire, la lipoprotéine de surface cellulaire MPT83, la lipoprotéine P23, la protéine perméase du système de transport du phosphate PstA, l'antigène de 14 kDa, la protéine de liaison à la fibronectine C FbpC1, l'alanine déshydrogénase TB43, et la glutamine synthétase 1, dans chaque cas du *Mycobacterium tuberculosis.*

**6.** Composition pharmaceutique selon l'une quelconque des revendications 1 à 3, ledit antigène protéique ou peptidique étant associé à l'allergie ou la maladie allergique et étant dérivé d'une source sélectionnée dans la liste constituée : du pollen de graminées, du pollen d'arbre, du pollen de fleur, du pollen d'herbe, des acariens de la poussière, des moisissures, des animaux, d'un aliment, et du venin d'insecte.

**7.** Composition pharmaceutique selon l'une quelconque des revendications 1 à 3, ledit antigène protéique ou peptidique étant associé à la maladie auto-immune et étant sélectionné dans la liste constituée :

• de la protéine basique de la myéline (MBP), de la protéine protéolipide (PLP), et de la glycoprotéine des oligodendrocytes de la myéline (MOG), dans chaque cas associée à la sclérose en plaques (MS) ;
• de CD44, la préproinsuline, la proinsuline, l'insuline, l'acide glutamique décarboxylase (GAD65), l'antigène de l'insulinome type tyrosine phosphatase 2 (IA2), le transporteur du zinc (ZnT8), et la protéine du choc thermique 60 (HSP60), dans chaque cas associé ▪ e au diabète de type 1 ;
• de la protéine de liaison au rétinoïde de l'interphotorécepteur (IRBP) associée à l'uvéite auto-immune ;
• du récepteur de l'acétylcholine AchR, et du récepteur du facteur de croissance type insuline 1 (IGF-1 R), dans chaque cas associé à la Myasthenia gravis ;
• de la protéine M des streptocoques bêta-hémolytiques (pseudo-auto-antigènes) associée à la fièvre rhumatoïde ;
• du facteur inhibiteur de la migration des macrophages associé à l'arthrite ;
• du complexe Ro/La RNP, de l'alpha- et bêta-fodrine, l'auto-antigène des cellules des îlots, la poly(ADP)ribose polymérase (PARP), NuMA, NOR-90, l'auto-antigène Ro60, et l'antigène p27, dans chaque cas associé au syndrome de Sjögren ;
• de l'auto-antigène Ro60, des lipoprotéines de faible densité, des antigènes Sm du complexe des petites ribonucléoprotéines nucléaires U-1 (B/B', D1, D2, D3, E, F, G), et des ribonucléoprotéines RNP, dans chaque cas associé·e·s au lupus érythémateux ;
• d'oxLDL, bêta(2)GPI, HSP60/65, et oxLDL/bêta(2)GPI, dans chaque cas associé à l'athérosclérose ;
• du récepteur bêta(1)-adrénergique cardiaque associé à la cardiomyopathie idiopathique dilatée (DCM) ;
• de l'histidyl-ARNt synthétase (HisRS) associée à la myosite ;
• de la topoisomérase I associée au scléroderme ;
• de l'IL-17 ; et
• des protéines du choc thermique.

**8.** Composition pharmaceutique selon l'une quelconque des revendications 1 à 3, ledit antigène protéique ou peptidique étant associé à un cancer ou une maladie tumorale et étant sélectionné dans la liste constituée de : p53, CA125, EGFR, Her2/neu, hTERT, PAP, MAGE-A1, MAGE-A3, mésothéline, MUC-1, NY-ESO-1, GP100, MART-1, tyrosinase, PSA, PSCA, PSMA VEGF, VEGFR1, VEGFR2, Ras, CEA et WT1.

**9.** Composition pharmaceutique selon l'une quelconque des revendications 1 à 8, ledit complexe servant à l'utilisation comme adjuvant.

**10.** Composition pharmaceutique selon l'une quelconque des revendications 1 à 9, ledit cargo moléculaire d'acide nucléique étant un ARN immunostimulateur (ARNis).

**11.** Composition pharmaceutique selon l'une quelconque des revendications 1 à 10, les peptides ou protéines cationiques ou polycationiques comprenant des peptides de polyarginine et/ou polylysine et au moins un résidu cystéine.

**12.** Composition pharmaceutique selon l'une quelconque des revendications 1 à 10, lesdits peptides cationiques étant sélectionnés parmi les peptides selon la formule (I)

$$(Arg)_l ; (Lys)_m ; (His)_n ; (Orn)_o ; (Xaa)_x,$$

l + m + n + o + x = 3 à100, et
l, m, n ou o = indépendamment l'un de l'autre est n'importe quel nombre sélectionné parmi 0, 1, 2, 3, 4, 5, 6, 7, 8, 9, 10, 11, 12, 13, 14, 15, 16, 17, 18, 19, 20, 21 à 30, 31 à 40, 41 à 50, 51 à 60, 61 à 70, 71 à 80, 81 à 90 et 91 à 100, à condition que la teneur totale d'Arg, Lys, His et Orn représente au moins 10 % de tous les acides aminés du peptide cationique ; et que Xaa soit n'importe quel acide aminé sélectionné parmi les acides aminés d'origine naturelle (= existant naturellement) ou non d'origine naturelle excepté Arg, Lys, His ou Orn ; et
x = n'importe quel nombre sélectionné parmi 0, 1, 2, 3, 4, 5, 6, 7, 8, 9, 10, 11, 12, 13, 14, 15, 16, 17, 18, 19, 20, 21 à 30, 31 à 40, 41 à 50, 51 à 60, 61 à 70, 71 à 80, 81 à 90, à condition, que la teneur totale de Xaa n'excède pas 90 % de tous les acides aminés du peptide cationique,
$(Arg)_l$ ; $(Lys)_m$ ; $(His)_n$ ; $(Orn)_o$ ; et x sont tels que définis ci-dessus ; Xaa est n'importe quel acide aminé sélectionné parmi les acides aminés d'origine naturelle (= existant naturellement) ou non d'origine naturelle excepté Arg, Lys, His, Orn ; à condition que la teneur totale de l'Arg (arginine), Lys (lysine), His (histidine) et Orn (ornithine) représente au moins 10 % de tous les acides aminés de l'oligopeptide.

**13.** Kit ou kit de parties comprenant :

(a) un complexe tel que défini selon l'une quelconque des revendications 1 à 3 et 9 à 12 ; et
(b) au moins un antigène protéique ou peptidique ou son fragment, variant et/ou dérivé fonctionnel tel que défini selon l'une quelconque des revendications 1 et 4 à 8.

**14.** Composition pharmaceutique selon l'une quelconque des revendications 1 à 12, ou kit ou kit de parties selon la revendication 13, pour l'utilisation comme vaccin.

**15.** Complexe tel que défini selon l'une quelconque des revendications 1 à 3 et 9 à 12, pour l'utilisation en thérapie en combinaison avec au moins un antigène protéique ou peptidique ou son fragment, variant et/ou dérivé fonctionnel tel que défini selon l'une quelconque des revendications 1 et 4 à 8.

**16.** Antigène protéique ou peptidique ou son fragment, variant et/ou dérivé fonctionnel tel que défini selon l'une quelconque des revendications 1 et 4 à 8 pour l'utilisation en thérapie en combinaison avec un complexe tel que défini selon l'une quelconque des revendications 1 à 3 et 9 à 12.

**17.** Complexe selon la revendication 15 pour l'utilisation dans la thérapie de ; ou antigène protéique ou peptidique ou son fragment, variant et/ou dérivé fonctionnel selon la revendication 16 pour l'utilisation dans la thérapie de :

(i) une maladie infectieuse ;
(ii) une allergie ou une maladie allergique ;
(iii) une maladie auto-immune ; ou
(iv) un cancer ou une maladie tumorale.

**18.** Boîte pharmaceutique, comprenant :

(a) un complexe tel que défini selon l'une quelconque des revendications 1 à 3 et 9 à 12 ; et
(b) les instructions décrivant l'utilisation dudit complexe en thérapie en combinaison avec au moins un antigène protéique ou peptidique ou son fragment, variant et/ou dérivé fonctionnel tel que défini selon l'une quelconque des revendications 1 et 4 à 8,

la boîte pharmaceutique comprenant en outre au moins un antigène protéique ou peptidique ou son fragment, variant et/ou dérivé fonctionnel tel que défini selon l'une quelconque des revendications 1 et 4 à 8.

**19.** Boîte pharmaceutique selon la revendication 18, lesdites instructions décrivant en outre l'utilisation dans la thérapie de :

(i) une maladie infectieuse ;
(ii) une allergie ou une maladie allergique ;
(iii) une maladie auto-immune ; ou
(iv) un cancer ou une maladie tumorale.

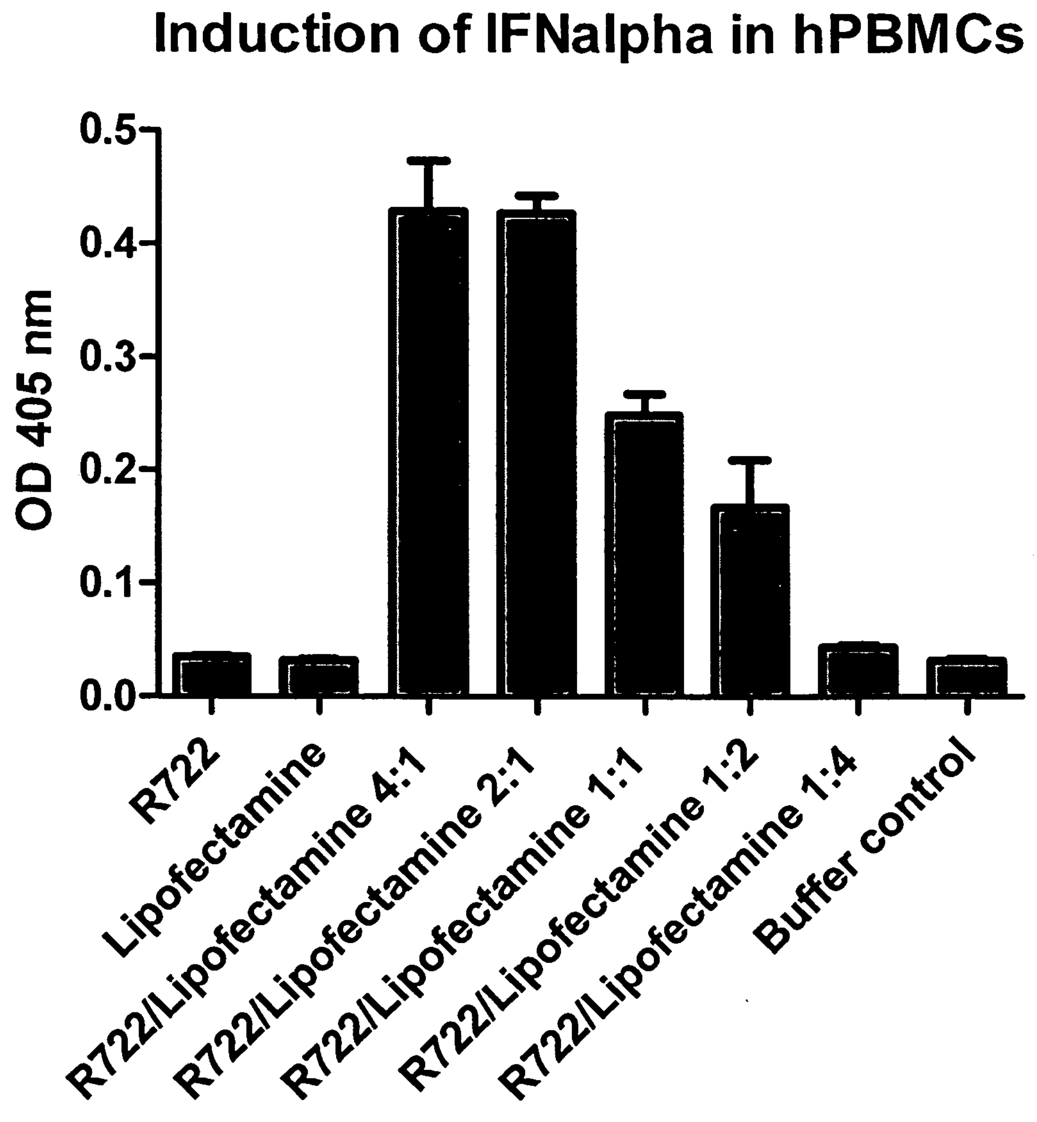
Induction of IFNalpha in hPBMCs
OD 405 nm
0.5
0.4
0.3
0.2
0.1
0.0
R722
Lipofectamine
R722/Lipofectamine 4:1
R722/Lipofectamine 2:1
R722/Lipofectamine 1:1
R722/Lipofectamine 1:2
R722/Lipofectamine 1:4
Buffer control

Fig. 1

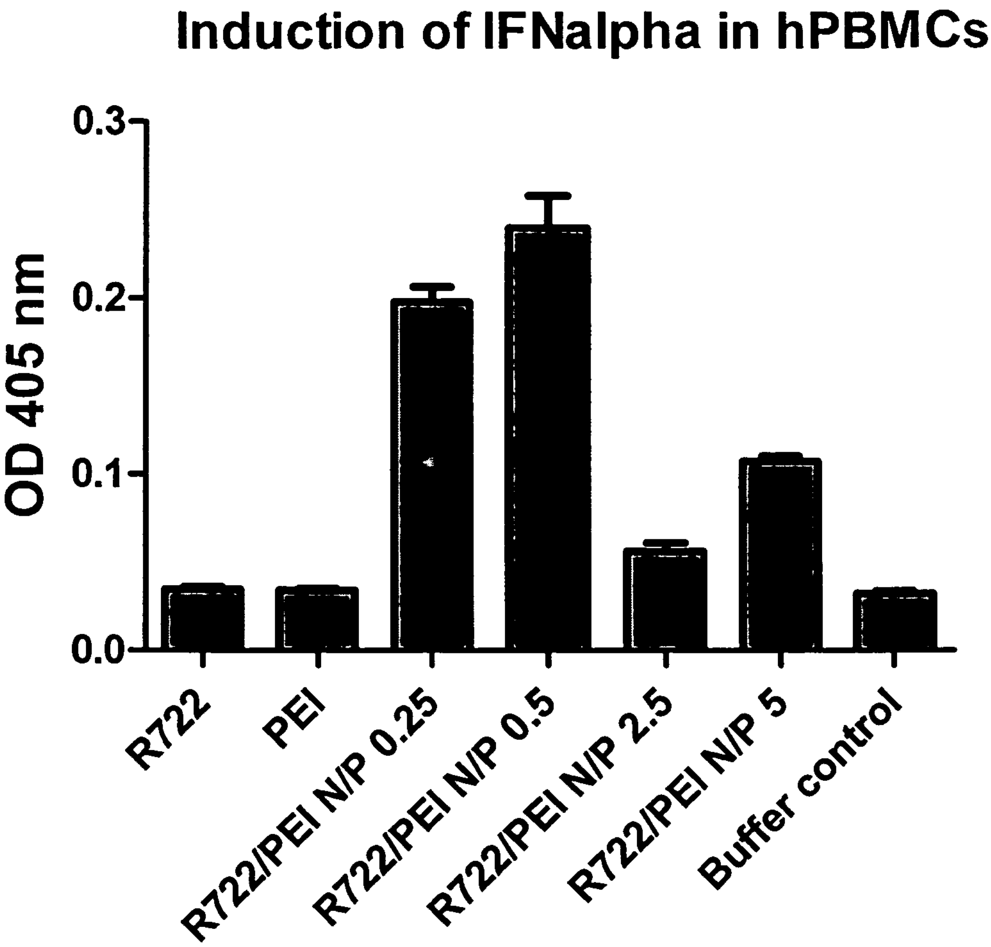
Induction of IFNalpha in hPBMCs
OD 405 nm
0.3
0.2
0.1
0.0
R722
PEI
R722/PEI N/P 0.25
R722/PEI N/P 0.5
R722/PEI N/P 2.5
R722/PEI N/P 5
Buffer control

Fig. 2

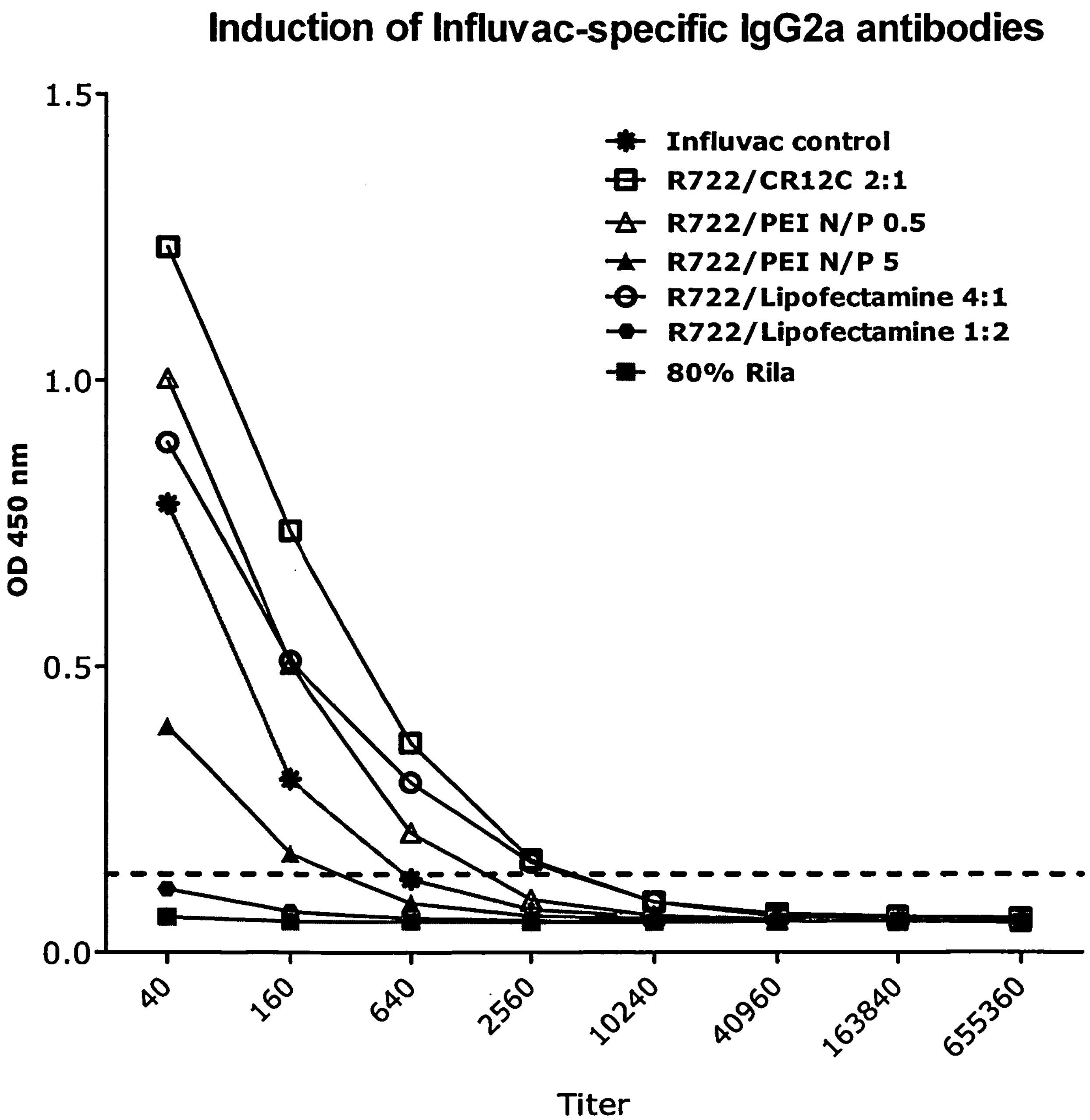
Induction of Influvac-specific IgG2a antibodies
1.5
1.0
0.5
0.0
OD 450 nm
Influvac control
R722/CR12C 2:1
R722/PEI N/P 0.5
R722/PEI N/P 5
R722/Lipofectamine 4:1
R722/Lipofectamine 1:2
80% Rila
40
160
640
2560
10240
40960
163840
655360
Titer

Fig. 3

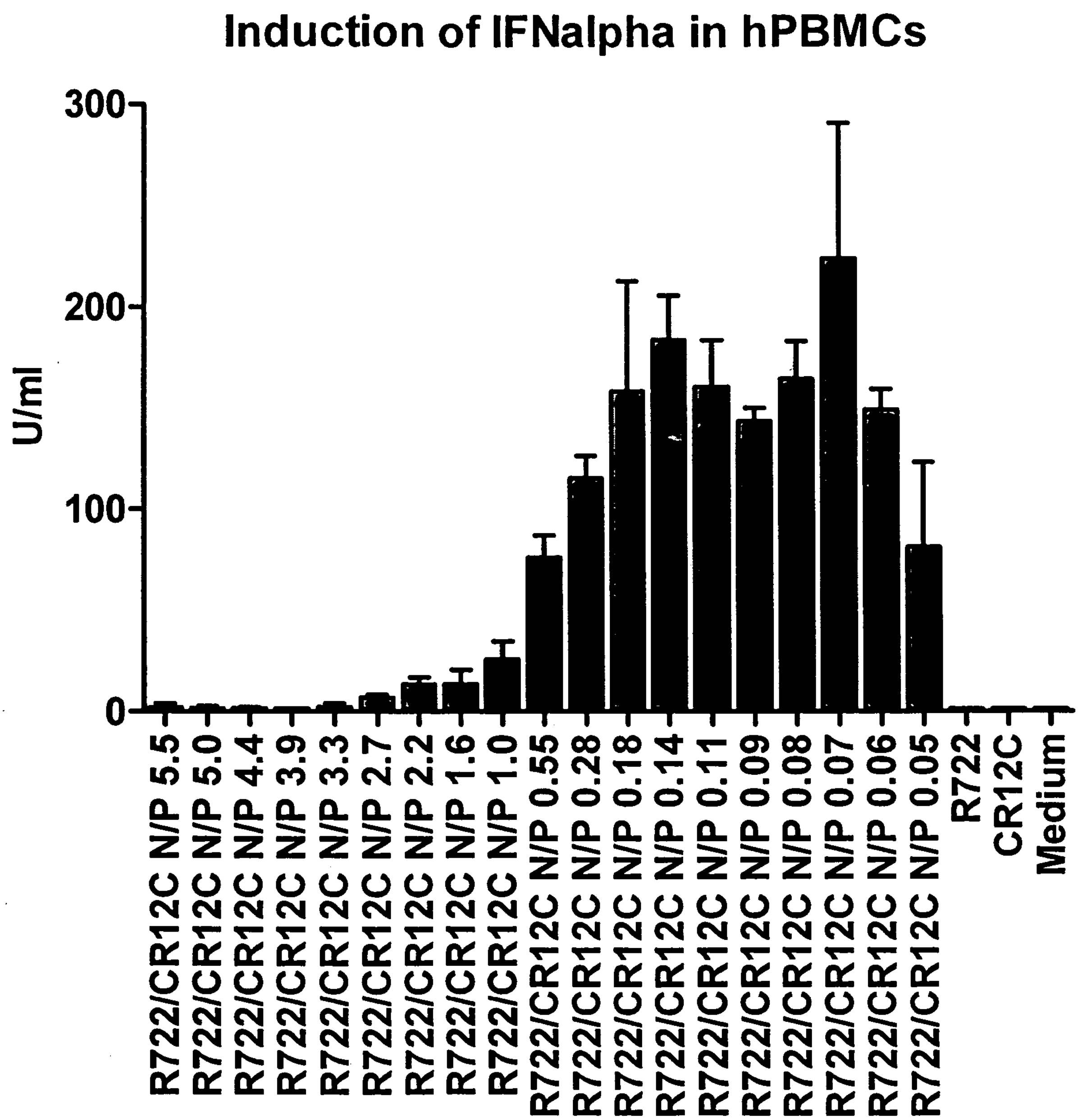

Induction of IFNalpha in hPBMCs
300
200
100
0
U/ml
R722/CR12C N/P 5.5
R722/CR12C N/P 5.0
R722/CR12C N/P 4.4
R722/CR12C N/P 3.9
R722/CR12C N/P 3.3
R722/CR12C N/P 2.7
R722/CR12C N/P 2.2
R722/CR12C N/P 1.6
R722/CR12C N/P 1.0
R722/CR12C N/P 0.55
R722/CR12C N/P 0.28
R722/CR12C N/P 0.18
R722/CR12C N/P 0.14
R722/CR12C N/P 0.11
R722/CR12C N/P 0.09
R722/CR12C N/P 0.08
R722/CR12C N/P 0.07
R722/CR12C N/P 0.06
R722/CR12C N/P 0.05
R722
CR12C
Medium


Fig. 4

# Uptake study of negatively charged complexes in hPBMC

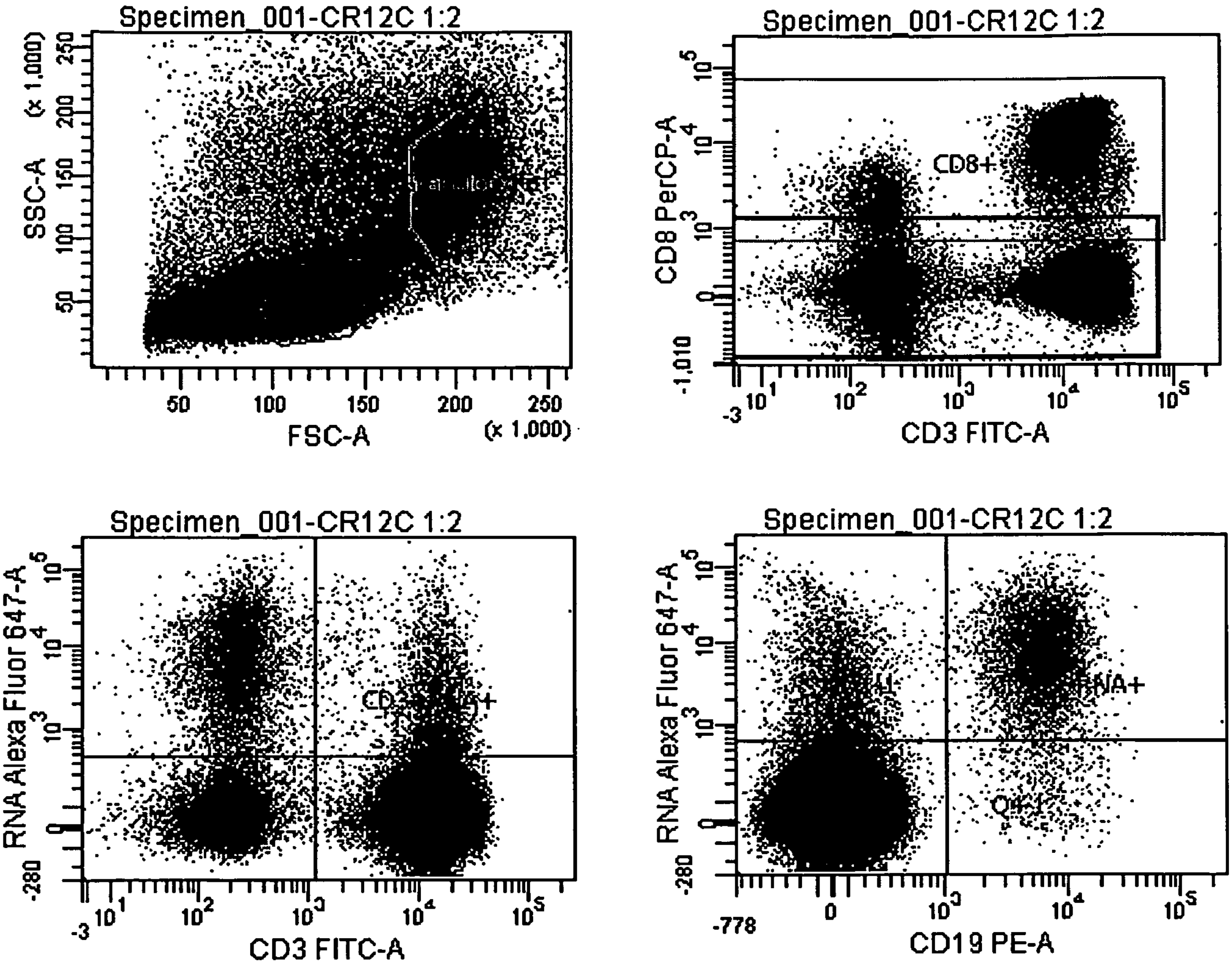


Fig. 5

# Uptake study of positively charged complexes in hPBMC

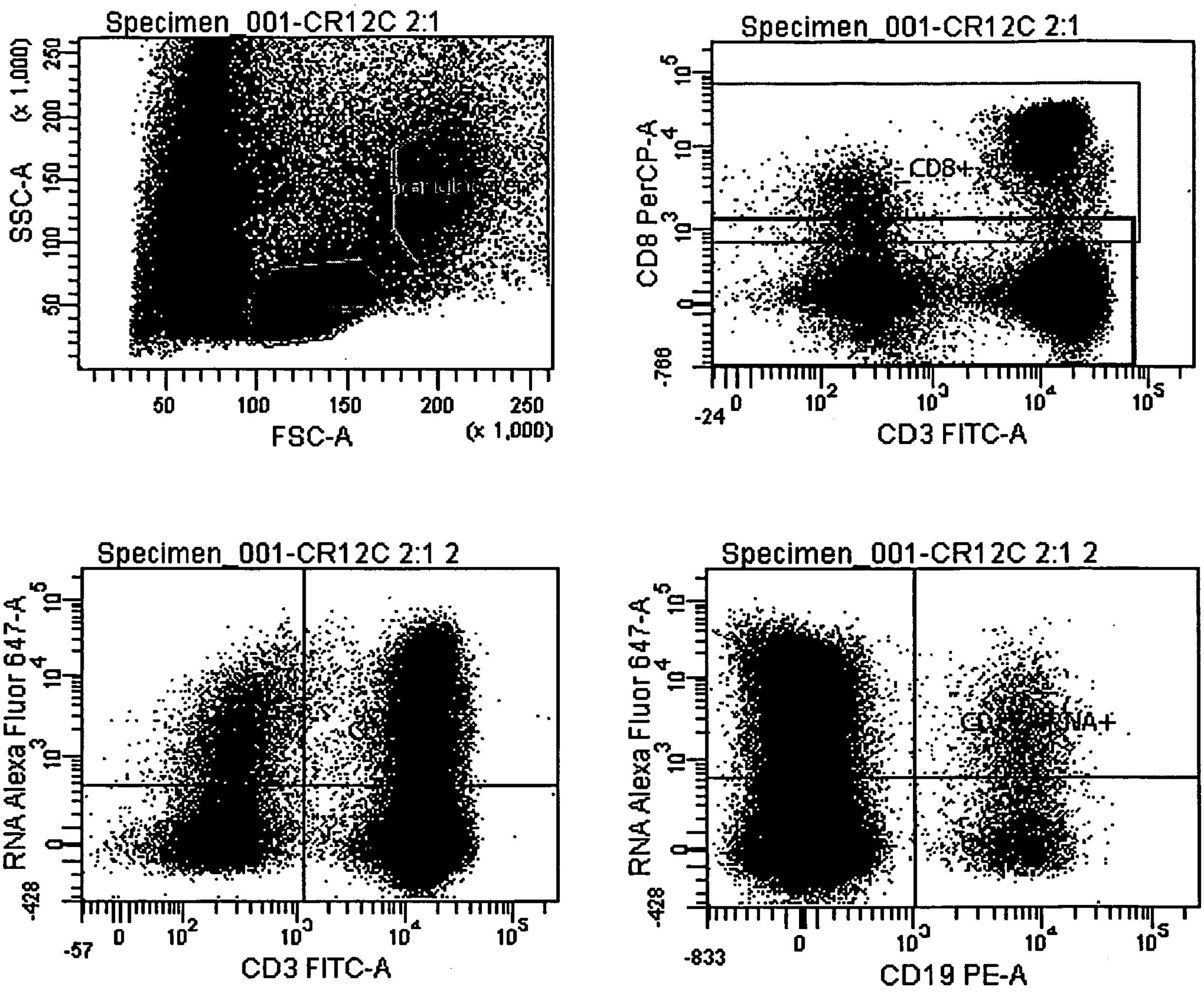


Fig. 6

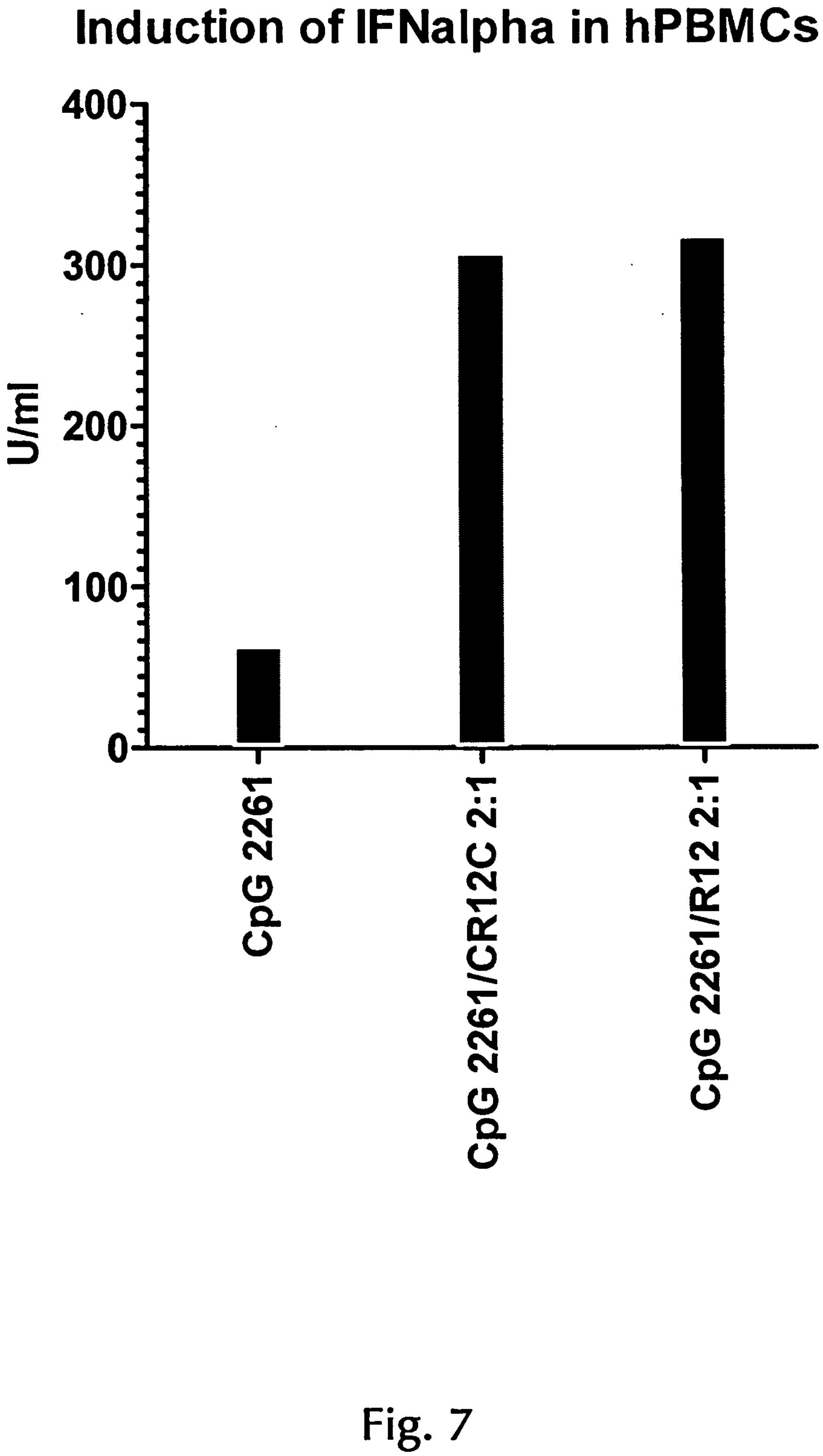
Induction of IFNalpha in hPBMCs
400
300
200
100
0
U/ml
CpG 2261
CpG 2261/CR12C 2:1
CpG 2261/R12 2:1

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009030481 A
- WO 2010037539 A
- WO 0154720 A
- WO 2012013326 A
- WO 2010003193 A
- WO 03086280 A
- EP 2012000418 W

### Non-patent literature cited in the description


- **GUY, B.** *Nat Rev Microbiol,* 2007, vol. 5 (7), 505-17
- **O'HAGAN, D. T. ; E. DE GREGORIO.** *Drug Discov Today,* 2009, vol. 14 (11-12), 541-51
- **HUBER et al.** *Clincal and Vaccine Immunology,* 2006, vol. 13 (9), 981-990
- **MEYLAN, E. ; J. TSCHOPP et al.** *Nature,* 2006, vol. 442 (7098), 39-44
- **FOERG, C. ; MERKLE, H.P.** *J Pharm Sci,* 2008, vol. 97, 144-62
- **SCHEEL, B et al.** *Eur J Immunol,* 2004, vol. 34, 537-47
- **SCHEEL, B et al.** *Eur J Immunol,* 2005, vol. 35, 1557-1566
- **HEIL, F et al.** *Science,* 2004, vol. 303, 1526-9
- **DIEBOLD, S.S. et al.** *Science,* 2004, vol. 303, 1529-31
- **HORNUNG, V. et al.** *Nat Med,* 2005, vol. 11, 263-70
- **FOTIN-MLECZEK et al.** *J Immunother,* 2011, vol. 34, 1-15
- **AKASHI.** *Curr. Opin. Genet. Dev.,* 2001, vol. 11 (6), 660-666
- **HOLCIK et al.** *Proc. Natl. Acad. Sci. USA,* 1997, vol. 94, 2410-2414
- **LAI et al.** *Development,* 1995, vol. 121, 2349-2360
- Purification of PCR Products. **MEZEI ; STORTS.** PCR Technology: Current Innovation. CRC Press, 2001
- Absorption, Circular Dichroism and ORD of Polypeptides. **URRY et al.** Modern Physical Methods in Biochemistry. Elsevier, 1985
- **KARLIN et al.** *PNAS USA,* 1993, vol. 90, 5873-5877
- **ALTSCHUL et al.** *Nucleic Acids Res.,* 1997, vol. 25, 3389-3402
- **ZAMORE.** *Nat. Struct. Biol.,* 2001, vol. 9, 746-750
- **SHARP.** *Genes Dev.,* 2001, vol. 5, 485-490
- **HANNON.** *Nature,* 2002, vol. 41, 244-251
- **STARK et al.** *Annu. Rev. Biochem.,* 1998, vol. 67, 227-264
- **HE ; KATZE.** *Viral Immunol.,* 2002, vol. 15, 95-119
- **ELBASHIR et al.** *Nature,* 2001, vol. 411, 494-498
- **HEMMI H et al.** 408. *Nature,* 2000, 740-5
- **BAUER S et al.** *Proc NatlAcadSci USA,* 2001, vol. 98, 9237-42
- **MEYLAN, E. ; TSCHOPP, J.** Toll-like receptors and RNA helicases: two parallel ways to trigger antiviral responses. *Mol. Cell,* 2006, vol. 22, 561-569